# EUROPÄISCHE PATENTANMELDUNG

(11) **EP 2 857 388 A1**
(43) Veröffentlichungstag der Anmeldung: **08.04.2015**
(21) Anmeldenummer: 13004730.1
(22) Anmeldetag: 01.10.2013
(51) Int. Cl.: C07D 271/06, A61P 29/00, A61K 31/4245

(54) **Sulfon-haltige Azole**

(71) Anmelder: Grünenthal GmbH, 52078 Aachen (DE)
(72) Erfinder: Reich, Melanie, 52072 Aachen (DE); Schunk, Stefan, 52080 Aachen (DE); Jostock, Ruth, 52223 Stolberg (DE)

(57) **Zusammenfassung**

Die vorliegende Erfindung betrifft sulfon-haltige Azole, Verfahren zu deren Herstellung, Arzneimittel enthaltend diese Verbindungen und die Verwendung von sulfon-haltige Azolen zur Behandlung von Schmerz sowie entzündlichen Erkrankungen.

## Beschreibung

Die vorliegende Erfindung betrifft sulfon-haltige Azole, Verfahren zu deren Herstellung, Arzneimittel enthaltend diese Verbindungen und die Verwendung von sulfon-haltige Azolen zur von Schmerz und endzündlichen Erkrankungen.

Im Gegensatz zur konstitutiven Expression des Bradykinin 2 Rezeptors (B2R) wird der Bradykinin 1 Rezeptor (B1 R) in den meisten Geweben nicht oder nur schwach exprimiert. Allerdings ist die Expression des B1 R auf verschiedenen Zellen induzierbar. Beispielsweise erfolgt im Verlauf von Entzündungsreaktionen eine rasche und ausgeprägte Induktion des B1 R auf neuronalen Zellen aber auch verschiedenen peripheren Zellen wie Fibroblasten, Endothelzellen, Granulozyten, Makrophagen und Lymphozyten. Somit kommt es im Verlauf von Entzündungsreaktionen zu einem Switch von einer B2R zu einer B1R Dominanz auf den beteiligten Zellen. Wesentlich an dieser B1 R Heraufregulation beteiligt sind die Zytokine Interleukin-1 (IL-1) und Tumor Nekrose Faktor alpha (TNFα) (Passos et al. J. Immunol. 2004, 172, 1839-1847). Nach Aktivierung mit spezifischen Liganden können B1 R-exprimierende Zellen anschließend selbst entzündungsfördernde Zytokine wie IL-6 und IL-8 sezernieren (Hayashi et al., Eur. Respir. J. 2000, 16, 452-458). Dies führt zur Einwanderung weiterer Entzündungszellen, z.B. neutrophiler Granulozyten (Pesquero et al., PNAS 2000, 97, 8140-8145). Über diese Mechanismen kann das B1R-System zur Chronifizierung von Erkrankungen beitragen. Dies belegt eine Vielzahl tierexperimenteller Untersuchungen (Übersichten in Leeb-Lundberg et al., Pharmacol Rev. 2005, 57, 27-77 und Pesquero et al., Biol. Chem. 2006, 387, 119-126). Auch am Menschen zeigt sich eine verstärkte Expression des B1R, z.B. auf Enterozyten und Makrophagen im betroffenen Gewebe von Patienten mit entzündlichen Darmerkrankungen (Stadnicki et al., Am. J. Physiol. Gastrointest. Liver Physiol. 2005, 289, G361-366) bzw. auf T-Lymphozyten von Patienten mit Multipler Sklerose (Pratet al., Neurology. 1999; 53,2087-2092) oder eine Aktivierung des Bradykinin-B2R-B1R Systems im Verlauf von Infektionen mit Staphyloccocus aureus (Bengtson et al., Blood 2006, 108, 2055-2063). Infektionen mit Staphyloccocus aureus sind verantwortlich für Krankheitsbilder wie oberflächliche Infektionen der Haut bis hin zu septischem Schock.

Aufgrund der dargestellten pathophysiologischen Zusammenhänge ergibt sich für die Anwendung von B1R-Antagonisten ein großes therapeutisches Potential bei akuten und insbesondere chronisch-entzündlichen Erkrankungen. Hierzu gehören Erkrankungen der Atemwege (Asthma bronchiale, Allergien, COPD (chronic-obstruktive pulmonary disease), zystische Fibrose usw.), entzündliche Darmerkrankungen (Ulcerative Colitis, CD/Crohn's disease usw.), neurologische Erkrankungen (Multiple Sklerose, Neurodegeneration usw.), Entzündungen der Haut (atopische Dermatitis, Psoriasis, bakterielle Infektionen usw.) und Schleimhäute (M. Behcet, Pelvitis, Prostatitis usw.), rheumatische Erkrankungen (rheumatoide Arthritis, Osteoarthritis usw.), septischen Schock und Reperfusionssyndrom (nach Herzinfarkt oder Schlaganfall).

Darüber hinaus ist das Bradykinin(Rezeptor)-System auch an der Regulation der Angiogenese beteiligt (Potential als Angiogenese-Inhibitor bei Krebs sowie Makula-Degeneration am Auge) und B1 R-knockout Mäuse sind geschützt vor der Induktion von Übergewicht durch eine besonders fettreiche Ernährung (Pesquero et al., Biol. Chem. 2006, 387, 119-126). B1R-Antagonisten eignen sich daher auch zur Behandlung von Fettleibigkeit.

B1R-Antagonisten sind insbesondere geeignet zur Behandlung von Schmerz, insbesondere Entzündungsschmerz und neuropathischem Schmerz (Calixto et al., Br. J. Pharmacol 2004, 1-16), hier insbesondere von diabetischer Neuropathie (Gabra et al., Biol. Chem. 2006, 387, 127-143). Weiterhin eignen sie sich zur Behandlung von Migräne.

Bei der Entwicklung von B1R-Modulatoren besteht jedoch das Problem, dass der humane und der Ratten-B1-Rezeptor sich so stark unterscheiden, dass viele Verbindungen, die gute B1R-Modulatoren am humanen Rezeptor sind, nur eine schlechte oder keine Affinität zum Ratten-Rezeptor aufweisen. Es besteht weiterhin der Bedarf an neuen B1R-Modulatoren, wobei B1R-Modulatoren, die sowohl an den Ratten-Rezeptor als auch an den humanen Rezeptor binden, besondere Vorteile bieten.

Eine Aufgabe der vorliegenden Erfindung bestand daher darin, neue Verbindungen zur Verfügung zu stellen, die sich insbesondere als pharmakologische Wirkstoffe in Arzneimitteln eignen, vorzugsweise in Arzneimitteln zur Behandlung von Störungen oder Krankheiten, die zumindest teilweise durch B1 R-Rezeptoren vermittelt werden.

Diese Aufgabe wird durch die erfindungsgemäßen sulfon-haltigen Azolen Verbindungen gelöst. Aus WO 2010/020556 A1 sind bestimmte Sulfonamide, die jeweils eine Azol-Einheit enthalten bekannt. Aus WO 2010/02556 A1 ist jedoch kein Hinweis auf die erfindungsgemäßen Sulfone der vorliegenden Erfindung zu entnehmen.

Der erste Gegenstand der vorliegenden Erfindung sind daher Verbindungen allgemeiner Formel (I), 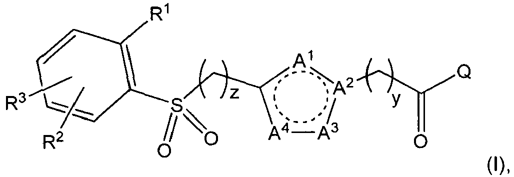 worin
y für 0, 1 oder 2 steht;
z für 1 oder 2 steht;
R¹ für F, Cl, Br, CN, CF₃, CHF₂, CH₂F, C₁₋₄-Alkyl, C₃₋₆-Cycloalkyl, OH, OCH₃, OCF₃, OCHF₂, OCH₂F, O-C₁₋₄-Alkyl, NH₂, N(H)C₁₋₄-Alkyl oder N(C₁₋₄-Alkyl)₂ steht;
R² und R³ jeweils unabhängig voneinander für H, F, Cl, Br, CN, CF₃, CHF₂, CH₂F, C₁₋₄-Alkyl, C₃₋₆-Cycloalkyl, OH, OCH₃, OCF₃, OCHF₂, OCH₂F, O-C₁₋₄-Alkyl, NH₂, N(H)C₁₋₄-Alkyl oder N(C₁₋₄-Alkyl)₂ stehen; oder zwei benachbarte Substituenten R¹ und R² oder R² und R³ oder R¹ und R³ gemeinsam mit den 2 sie verbindenden C-Atomen bilden einen C₅₋₆-Cycloalkyl oder einen 5-, 6- oder 7-gliedrigen Heterocyclyl, der ein oder zwei O-Atome enthält, wobei besagter Cycloalkyl oder Heterocycloalkyl gesättigt oder ungesättigt, jedoch nicht aromatisch sein kann, und unsubstituiert oder substituiert mit einem oder mehreren Substituenten, jeweils unabhängig ausgewählt aus F, Cl, CN, CF₃; CHF₂; CH₂F; C₁₋₄-Alkyl; O-C₁₋₄Alkyl; OCF₃; OCHF₂ oder OCH₂F, sein kann;
A¹, A³ und A⁴ jeweils unabhängig voneinander für O, N oder CR⁴ stehen;
worin jedes R⁴ unabhängig für H, F, Cl, Br, CN, CF₃, CHF₂, CH₂F, C₁₋₄-Alkyl, C₃₋₆-Cycloalkyl, OH, OCH₃, OCF₃, OCHF₂, OCH₂F, O-C₁₋₄-Alkyl, NH₂, N(H)C₁₋₄-Alkyl oder N(C₁₋₄-Alkyl)₂ steht;
A² für C oder N steht;
unter der Bedingung, dass A¹, A², A³ und A⁴ und das C-Atom, welches A¹ und A⁴ verbindet, einen 5-gliedrigen Heteroaryl bilden;
und
Q steht für Formel (i), (ii) oder (iii), 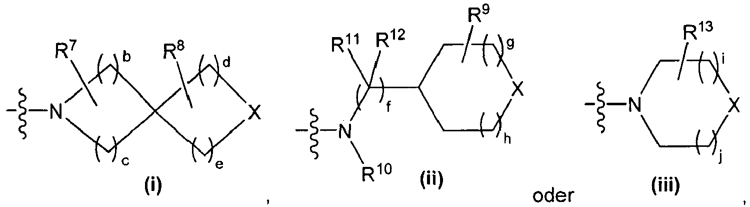 worin
b, c, d und e jeweils unabhängig voneinander für 0, 1, 2, 3 oder 4 stehen,
unter der Bedingung, dass die Summe (b + c) und die Summe (d + e) jeweils wenigstens 2 ist; f für 0, 1, 2, 3 oder 4 steht;
g, h, i und j jeweils unabhängig voneinander für 0, 1 oder 2 stehen;
R⁷, R⁸, R⁹ und R¹³ jeweils unabhängig voneinander für 0, 1, 2, 3, 4 Substituenten stehen, jeweils unabhängig voneinander ausgewählt aus =O, F, Cl, CN, CF₃, CHF₂, CH₂F, C₁₋₄-Alkyl, O-C₁₋₄-Alkyl, OCF₃, OCHF₂ oder OCH₂F;
oder zwei Substituenten R⁹ und R¹³ bilden eine C₁₋₃-Alkylen Gruppe;
R¹⁰ für H, C₁₋₄-Alkyl oder C₃₋₆-Cycloalkyl steht;
jedes R¹¹ und jedes R¹² jeweils unabhängig voneinander für H, F, Cl, CN, C₁₋₄-Alkyl, C₃₋₆-Cycloalkyl, OH, O-CH₃, O-CF₃, O-C₁₋₄-Alkyl, NH₂, N(H)C₁₋₄-Alkyl oder N(C₁₋₄-Alkyl)₂ stehen;
oder ein R¹¹ und ein R¹² gemeinsam mit den sie verbindenden C-Atomen einen C₃₋₆-Cycloalkyl oder 4-7-gliedrigen Heterocycloalkyl bilden;
X für O; N-(C₁₋₄-Alkylen)ₘ-(Z)ₙ-(C₁₋₄-Alkylen)ₒ-B¹ oder C(R¹⁸)-(C₁₋₄-Alkylen)ₚ-(Z)_{q}-(C₁₋₄-Alkylen)ᵣ-B² steht; worin
m, n und o jeweils unabhängig voneinander für 0 oder 1 stehen, unter der Bedingung, dass, falls n für 1 steht und Z für O steht, m für 1 steht;
p, q und r jeweils unabhängig voneinander für 0 oder 1 stehen;
R¹⁸ für H, C₁₋₄-Alkyl, C₃₋₆-Cycloalkyl, OH, O-C₁₋₄-Alkyl, NH₂, N(H)C₁₋₄-Alkyl, N(C₁₋₄-Alkyl)₂, Aryl, Heteroaryl oder 4-7-gliedrigen Heterocycloalkyl steht; und
Z bedeutet O oder C=O;
B¹ bedeutet H, Alkyl, Cycloalkyl, Heteroaryl, 4-12-gliedrigen Heterocycloalkyl, NR¹⁹R²⁰ oder Aryl,
B² bedeutet H, Heteroaryl, 4-12-gliedrigen Heterocycloalkyl oder NR¹⁹R²⁰;
worin R¹⁹ und R²⁰ jeweils unabhängig voneinander für H, C₁₋₄-Alkyl oder C₃₋₆-Cycloalkyl stehen,
worin die vorgenannten Substituenten C₁₋₄-Alkyl, C₁₋₄-Alkylen, C₃₋₆-Cycloalkyl, Heterocycloalkyl, Aryl und Heteroaryl jeweils unsubstituiert oder ein- oder mehrfach substituiert mit gleichen oder verschiedenen Substituenten sind, und die vorgenannten C₁₋₄-Alkyl und C₁₋₄-Alkylen jeweils linear oder verzweigt sein können;
in Form einen individuellen Stereoisomers oder einer Mischung aus Stereoisomeren; in Form eines Tautomers; der freien Verbindung, eines N-Oxids und/oder in Form eines Solvats und/oder eines physiologisch verträglichen Salzes.

Der Ausdruck "C₁₋₄-Alkyl" umfasst im Sinne dieser Erfindung acyclische gesättigte Kohlenwasserstoffreste mit 1, 2, 3 oder 4 C-Atomen, die verzweigt- oder geradkettig (unverzweigt) sowie unsubstituiert oder ein- oder mehrfach, beispielsweise 2-, 3-, 4- oder 5-fach, mit gleichen oder verschiedenen Resten substituiert sein können. Vorzugsweise können die Alkylreste ausgewählt sein aus der Gruppe bestehend aus Methyl, Ethyl, n-Propyl, iso-Propyl, n-Butyl, sec-Butyl, iso-Butyl und tert-Butyl.

Im Sinne dieser Erfindung bedeutet der Ausdruck "C₃₋₆-Cycloalkyl" cyclische gesättigte Kohlenwasserstoffe mit mit 3, 4, 5 oder 6 Kohlenstoffatomen, die unsubstituiert oder an einem oder mehreren Ringgliedern einfach oder mehrfach, beispielsweise mit 2, 3, 4 oder 5, gleichen oder verschiedenen Resten substituiert sein können. Vorzugsweise kann C₃₋₆-Cycloalkyl ausgewählt sein aus der Gruppe bestehend aus Cyclopropyl, Cyclobutyl, Cyclopentyl und Cyclohexyl.

Der Begriff "4-7-gliedrigen Heterocyclolalkyl" bzw. "4-12-gliedrigen Heterocyclolalkyl" umfasst gesättigte oder ungesättigte,aber nicht aromatische, Cycloalkyle mit 4 bis 7 bzw. 4 bis 12 Ringgliedern, in denen ein, zwei oder drei Kohlenstoffatome durch ein Heteroatom jeweils unabhängig voneinander ausgewählt aus der Gruppe S, N oder O ersetzt sind, wobei die Ringglieder unsubstituiert oder ein- oder mehrfach substituiert sein können. Die Bindung des Heterocycloalkyls an die übergeordnete allgemeine Struktur kann über jedes beliebige und mögliche Ringglied des Heterocycloalkyl-Restes erfolgen. Die Heterocycloalkyl-Reste können auch mit weiteren gesättigten, (partiell) ungesättigten oder aromatischen oder heteroaromatischen Ringsystemen kondensiert sein, die wiederum unsubstituiert oder einfach oder mehrfach, beispielsweise 2-, 3-, 4- oder 5-fach, substituiert sein können. Bevorzugt sind Heterocycloalkyl-Reste aus der Gruppe Azetidinyl, Oxetanyl, Tetrahydrofuranyl, Azepanyl, Dioxanyl, Dioxolanyl, Morpholinyl, Tetrahydropyranyl, Pyrrolidinyl, Piperazinyl, Piperidinyl, Pyrazolidinyl, Pyrazolinonyl oder Thiomorpholinyl.

Der Ausdruck "Aryl" bedeutet im Sinne dieser Erfindung aromatische Kohlenwasserstoffe, insbesondere Phenyle und Naphthyle. Die Aryl-Reste können auch mit weiteren gesättigten, (partiell) ungesättigten oder aromatischen Ringsystemen kondensiert sein. Jeder Aryl-Rest kann unsubstituiert oder einfach oder mehrfach, beispielsweise 2-, 3-, 4- oder 5-fach, substituiert vorliegen, wobei die Aryl-Substituenten gleich oder verschieden und in jeder beliebigen und möglichen Position des Aryls sein können. Vorteilhafterweise ist Aryl ausgewählt aus der Gruppe bestehend aus Phenyl, 1-Naphthyl und 2-Naphthyl, welche jeweils unsubstituiert oder ein- oder mehrfach, beispielsweise mit 2, 3, 4 oder 5 Resten, substituiert sein können.

Der Ausdruck "Heteroaryl" steht im Sinne der vorliegenden Erfindung für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sein können und das Heteroaryl unsubstituiert oder ein- oder mehrfach, beispielsweise 2-, 3-, 4- oder 5-fach, mit gleichen oder verschiedenen Resten substituiert sein kann. Die Substituenten können in jeder beliebigen und möglichen Position des Heteroaryls gebunden sein. Der Heterocyclus kann auch Teil eines bi- oder polycyclischen, insbesondere eines mono-, bi- oder tricyclischen Systems sein, das dann insgesamt mehr als 7-gliedrig sein kann, vorzugsweise bis zu 14-gliedrig. Bevorzugte Heteroatome sind unabhängig voneinander ausgewählt aus der Gruppe bestehend aus N, O und S. Vorzugsweise kann der Heteroarylrest ausgewählt werden aus der Gruppe bestehend aus Pyrrolyl, Indolyl, Furyl (Furanyl), Benzofuranyl, Thienyl (Thiophenyl), Benzimidazolyl, Benzothienyl, Benzothiadiazolyl, Benzothiazolyl, Benzotriazolyl, Benzodioxolanyl, Benzodioxanyl, Benzooxazolyl, Benzooxadiazolyl, Imidazothiazolyl, Dibenzofuranyl, Dibenzothienyl, Pyrazolo[1,5-a]pyrimidinyl, Phtalazinyl, Pyrazolyl, Imidazolyl, Thiazolyl, Oxazolyl, Isooxazolyl, Oxadiazolyl, Oxathiazolyl, Triazinyl, Tetrazolyl, Isooxazolyl, Pyridinyl (Pyridyl), Pyridazinyl, Pyrimidinyl, Pyrazinyl, Pyranyl, Indazolyl, Purinyl, Indolizinyl, Chinolinyl, Isochinolinyl, Chinazolinyl, Chinoxalinyl, Carbazolyl, Phenazinyl und Phenothiazinyl, insbesondere aus der Gruppe bestehend aus Thienyl (Thiophenyl), Pyridinyl (Pyridyl), Pyrimidinyl, Thiazolyl, Thiadiazolyl, Triazolyl, Imidazolyl, Oxazolyl, Pyridazinyl, Pyrazinyl, Isooxazolyl, Oxadiazolyl, Chinazolinyl, Chinolinyl und Isochinolinyl. Besonders bevorzugt kann der Heteroarylrest ausgewählt werden aus der Gruppe bestehend aus Isooxazolyl, Oxazolyl, Pyridazinyl, Pyrazinyl, Thienyl, Imidazoyl, Thiazolyl, Triazolyl, Pyridinyl und Pyrimidinyl.

Der Ausdruck "C₁₋₃-Alkylen Gruppe" bzw. "C₁₋₄-Alkylen Gruppe" umfasst im Sinne der vorliegenden Erfindung acyclische gesättigte Kohlenwasserstoffreste mit 1, 2 oder 3 bzw. 1, 2, 3 oder 4 C-Atomen, die verzweigt- oder geradkettig (unverzweigt) sowie unsubstituiert oder ein- oder mehrfach, beispielsweise 2-, 3-, 4- oder 5-fach, mit gleichen oder verschiedenen Resten substituiert sein können und die einen entsprechenden Rest mit der übergeordneten allgemeinen Struktur verknüpfen. Vorzugsweise können die Alkylen-Gruppen ausgewählt sein aus der Gruppe bestehend aus CH₂, CH₂CH₂, CH(CH₃), CH₂CH₂CH₂, CH(CH₃)CH₂, CH₂CH(CH₃), CH(CH₂CH₃), und C(CH₃)₂. Besonders bevorzugt können die Alkylen-Gruppen ausgewählt sein aus der Gruppe bestehend aus CH₂, CH₂CH₂ und CH₂CH₂CH₂.

Im Zusammenhang mit "Alkyl", "Alkylen", "Cycloalkyl" und "Heterocycloalkyl" versteht man unter dem Begriff substituiert" im Sinne dieser Erfindung die Substitution eines Wasserstoffrestes durch F, Cl, Br, I, CF₃, OCF₃, CN, NH₂, NH-C₁₋₄-Alkyl, NH-C₁₋₃-Alkylen-OH, C₁₋₄-Alkyl, N(C₁₋₄-Alkyl)₂, N(C₁₋₃-Alkylen-OH)₂, NO₂ SH, S-C₁₋₄-Alkyl, C₁₋₃-Alkyl, S-Benzyl, O-C₁₋₄-Alkyl, OH, O-C₁₋₃-Alkylen-OH, =O, O-Benzyl, C(=O)C₁₄-Alkyl, CO₂H, CO₂-C₁₋₄-Alkyl, Phenyl, Phenoxy, Benzyl, Naphthyl, Thienyl und Pyridinyl, wobei unter mehrfach substituierten Resten solche Reste zu verstehen sind, die entweder an verschiedenen oder an gleichen Atomen mehrfach, beispielsweise zweifach oder dreifach, substituiert sind, beispielsweise dreifach am gleichen C-Atom wie im Falle von CF₃ oder CH₂CF₃ oder an verschiedenen Stellen wie im Falle von CH(Cl)-CH=CH-CHCl₂. Die Mehrfachsubstitution kann mit gleichen oder verschiedenen Substituenten erfolgen, wie beispielsweise im Falle von CH(OH)-CH=CH-CHCl₂. Bevorzugt ist hierbei die Substitution eines oder mehrerer Wasserstoffreste durch F, CN, CF₃, NH₂, N(CH₃)₂, OH, Phenyl, O-CF₃ oder O-C₁₋₄-Alkyl.

In Bezug auf "Aryl" und "Heteroaryl" versteht man im Sinne dieser Erfindung unter "substituiert" die ein- oder mehrfache, beispielsweise 2-, 3-, 4- oder 5-fache, Substitution eines oder mehrerer Wasserstoffatome des entsprechenden Ringsystems durch F, Cl, Br, I, CN, NH₂, N(CH₃)₂, C(=O)NH₂, C(=O)N(CH₃)₂, 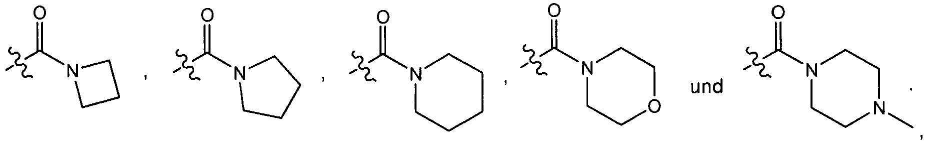 N(H)C₁₋₄-Alkyl, N(H)C₁₋₃-Alkylen-OH, N(C₁₋₄-Alkyl)₂, N(C₁₋₃-Alkylen-OH)₂, N(H)Hetaryl¹, N(Hetaryl¹)₂, N(C₁₄-Alkyl)Hetaryl¹, Pyrrolinyl, Piperazinyl, N-Methyl-piperazinyl, Morpholinyl, Azetidinyl, Piperidinyl, Thiazolinyl, Azepanyl, Diazepanyl, (C₁₋₃-Alkylen)-Azetidinyl, (C₁₋₃-Alkylen)-Pyrrolinyl, (C₁₋₃-Alkylen)-Piperidinyl, (C₁₋₃-Alkylen)-Morpholinyl, (C₁₋₃-Alkylen)-Piperazinyl, (C₁₋₃-Alkylen)-Thiazolinyl, (C₁₋₃-Alkylen)-Azepanyl, (C₁₋₃-Alkylen)-Diazepanyl, NO₂, SH, S-C₁₋₄-Alkyl, OH, O-C₁₋₄-Alkyl, O-C₁₋₃-Alkylen-OH, C(=O)C₁₋₄-Alkyl, NHSO₂C₁₋₄-Alkyl, NHCOC₁₋₄-Alkyl, CO₂H, CH₂SO₂-Phenyl, CO₂-C₁₋₄-Alkyl, OCF₃, CF₃, OCHF₂, CHF₂, OCH₂, CFH₂, OCH₂O, OCH₂CH₂O, OC(CH₃)₂CH₂, C₁₋₄-Alkyl, C₃₋₆-Cycloalkyl, O-C₃₋₆-Cycloalkyl, Pyrrolidinyl, Imidazolyl, Benzyloxy, Phenoxy, Phenyl, Naphthyl, Pyridinyl, Pyrimidinyl, C₁₋₃-Alkylen-Hetaryl¹, Benzyl, Thienyl, Furyl oder für über eine C₁₋₃-Alkylen-Gruppe gebundenes OCF₃, OH, O-C₁₋₄-Alkyl, SH, S-C₁₋₄-Alkyl, C₃₋₆-Cycloalkyl, O-C₃₋₆-Cycloalkyl, N(C₁₋₄-Alkyl)₂, C(=O)-N(C₁₋₄-Alkyl)₂, Phenyl, Pyridyl oder Pyrimidyl, wobei Hetaryl¹ für Phenyl, Thiazolyl, Thienyl oder Pyridinyl steht, an einem oder verschiedenen Atomen, wobei die vorstehend genannten Substituenten - sofern nicht anders angegeben - ggf. ihrerseits mit den genannten Substituenten substituiert sein können. Die Mehrfachsubstitution von Aryl und Heteroaryl kann mit gleichen oder verschiedenen Substituenten erfolgen. Bevorzugte Substituenten für Aryl und Heteroaryl können ausgewählt werden aus der Gruppe bestehend aus-O-C₁₋₄-Alkyl, unsubstituiertem C₁₋₄-Alkyl, F, Cl, CN, CF₃, OCF₃, OH, NH₂, N(CH₃)₂, Pyrrolidinyl, Phenyl, Naphthyl, Thiazolyl, Thienyl und Pyridinyl, insbesondere aus der Gruppe bestehend aus tert-Butyl, F, Cl, CN, CF₃, CH₃; OCH₃, OCF₃, Pyrrolidinyl, NH₂ und N(CH₃)₂.

Im Rahmen der vorliegenden Erfindung bezeichnet das in Formeln verwendete Symbol 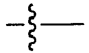 eine Verknüpfung eines entsprechenden Restes an die jeweilige übergeordnete allgemeine Struktur.

Unter dem Begriff "physiologisch verträgliches Salz" versteht man im Sinne dieser Erfindung Salze der erfindungsgemäßen Verbindungen mit anorganischen oder organischen Säuren oder anorganischen oder organischen Basen, die physiologisch -insbesondere bei Anwendung am Menschen und/ oder Säugetierverträglich sind.

In einer Ausführungsform des ersten Erfindungsgegenstands ist die Verbindung der Formel (I) dadurch gekennzeichnet, dass y für 0 oder 1 steht und z für 1 steht, so dass sich die Formeln (I-a) oder (I-b) ergeben:

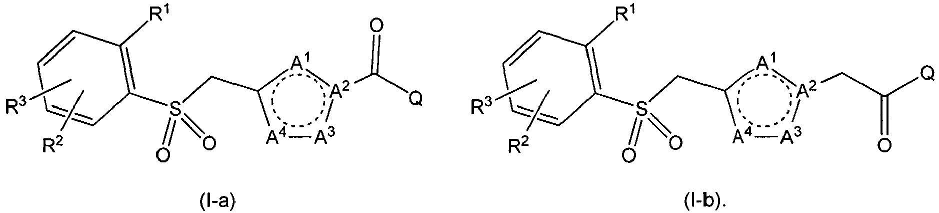

Bevorzugt steht y für 0 und z für 1, so dass bevorzugte Verbindungen die Formel (I-a) aufweisen.

Eine weitere Ausführungsform des ersten Erfindungsgegenstands ist eine Verbindung der Formel (I), die dadurch gekennzeichnet, dass
R¹ für F, Cl, CN, C₁₋₄-Alkyl oder O-C₁₋₄-Alkyl steht, bevorzugt für F, Cl, CN, CH₃ oder OCH₃, und insbesondere bevorzugt für Cl oder CH₃.

Eine weitere Ausführungsform des ersten Erfindungsgegenstands ist eine Verbindung der Formel (I), die dadurch gekennzeichnet, dass
R² und R³ für F, Cl, CN, C₁₋₄-Alkyl oder O-C₁₋₄-Alkyl stehen, bevorzugt für F, Cl, CN, CH₃ oder OCH₃, und insbesondere bevorzugt für Cl, CH₃ oder OCH₃.

In einer weiteren Ausführungsform des ersten Erfindungsgegenstands steht die Teilstruktur 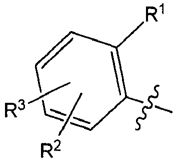 der Formel (I) für einen Rest, der ausgewählt wird aus 4-Methoxy-2,6-dimethyl-phenyl, 4-Methoxy-2,5-dimethyl-phenyl, 4-Methoxy-2,3-dimethyl-phenyl, 2-Chlor-6-methyl-phenyl, 2,3-Dichlorphenyl, 2,4-Dichlorphenyl, 2,5-Dichlorphenyl, 2,6-Dichlorphenyl, 4-Chlor-2,5-dimethyl-phenyl, 4-Chlor-2,6-dimethylphenyl und 4-Chlor-2,3-dimethyl-phenyl.
Bevorzugt steht die Teilstruktur 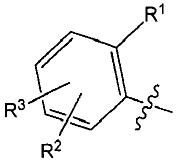 der Formel (I) für einen 4-Methoxy-2,6-dimethyl-phenyl oder 2-Chlor-6-methyl-phenyl.

In einer weiteren Ausführungsform des ersten Erfindungsgegenstands ist die Verbindung der Formel (I) dadurch gekennzeichnet, dass A² für C steht, und eines von A¹, A³ und A⁴ für O steht und die beiden verbleibenden von A¹, A³ und A⁴ für N stehen, so dass sich eine Verbindung der Formel (I-1) ergibt:

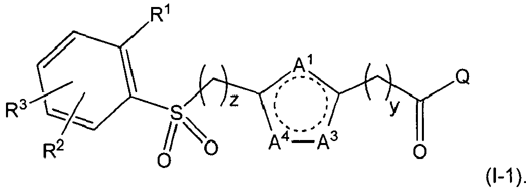

Bevorzugt steht A² für C, A¹ für O und A³ und A⁴ jeweils für N, so dass sich eine Verbindung der Formel (I-1a) ergibt; oder
A² steht für C, A³ für O und A¹ und A⁴ jeweils für N, so dass sich eine Verbindung der Formel (I-1b) ergibt; oder
A² für C, A⁴ für O und A¹ und A³ jeweils für N, so dass sich eine Verbindung der Formel (I-1c) ergibt: 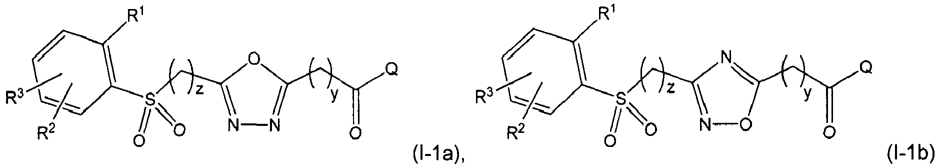 oder

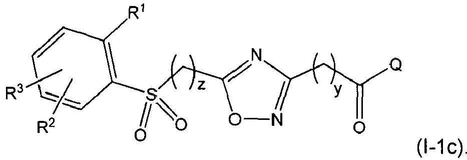

In einer weiteren Ausführungsform des ersten Erfindungsgegenstands ist die Verbindung der Formel (I) dadurch gekennzeichnet, dass A² für N steht, und A¹, A³ und A⁴ unabhängig voneinander für N oder CR⁴ stehen,
wobei jedes R⁴ jeweils unabhängig voneinander für H, F, Cl, CN, CF₃, CHF₂, CH₂F, C₁₋₄-Alkyl oder C₃₋₆-Cycloalkyl steht,
so dass sich eine Verbindung der Formel (I-2) ergibt:

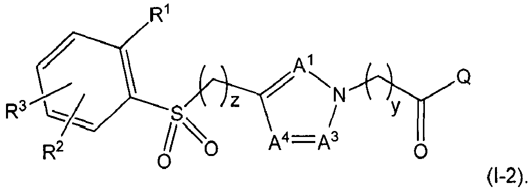

Bevorzugt stehen A² und A³ für N und A¹ und A⁴ für CR⁴, so dass sich eine Verbindung der Formel (I-2a) ergibt; oder
stehen A¹ und A² für N und A³ und A⁴ jeweils für CR⁴, so dass sich eine Verbindung der Formel (I-2b) ergibt; oder
stehen A², A³ und A⁴ jeweils für N und steht A¹ für CR⁴, so dass sich eine Verbindung der Formel (I-2c) ergibt: 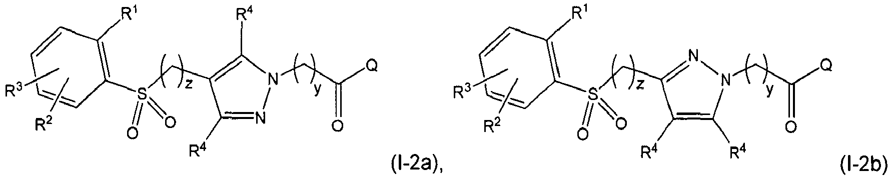 oder

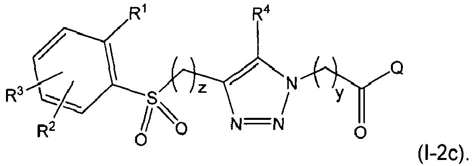

Bevorzugt ist die Verbindung der Formel (I) ausgewählt aus (I-1) oder (I-2), bevorzugt aus (I-1a), (I-1b), (I-1c), (I-2a), (I-2b) oder (I-2c), worin jeweils y für 0 und z für 1 steht.

Weiterhin bevorzugt ist die Verbindung der Formel (I) ausgewählt aus (I-1) oder (I-2), bevorzugt aus (I-1a), (I-1b), (I-1c), (I-2a), (I-2b) oder (I-2c), worin jeweils y für 1 und z für 1 steht. Eine weitere Ausführungsform des ersten Erfindungsgegenstandes ist dadurch gekennzeichnet, dass in der Verbindung der Formel (I), bevorzugt der Formel (I-1) oder (I-2), insbesondere bevorzugt der Formel (I-1a), (I-1b), (I-1c), (I-2a), (I-2b) oder (I-2c), die Teilstruktur Q für Formel (i), (ii) oder (iii), 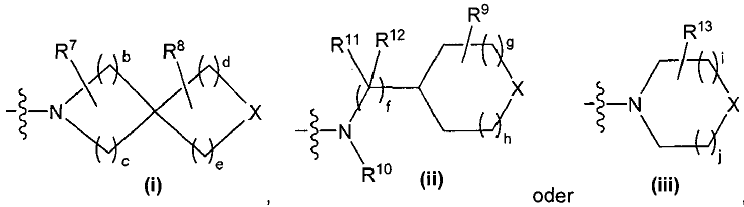 steht.

Bevorzugt steht Q für Formel (i), (ii) oder (iii),
worin
b, c, d und e jeweils unabhängig voneinander für 0, 1, 2, 3 oder 4 stehen,
unter der Bedingung, dass die Summe (b + c) und die Summe (d + e) jeweils wenigstens 2; f für 0, 1 oder 2 steht;
g, h, i und j jeweils unabhängig voneinander für 0, 1 oder 2 stehen;
R⁷, R⁸, R⁹ und R¹³ jeweils unabhängig voneinander für 0, 1, 2, 3, 4 Substituenten stehen, jeweils unabhängig voneinander ausgewählt aus =O, F, Cl, CH₃ oder OCH₃;
oder zwei Substituenten R¹³ bilden eine C₁₋₃-Alkylen Gruppe;
R¹⁰ für H oder Cyclopropyl oder CH₃ steht;
R¹¹ und R¹² jeweils unabhängig für H, CH₃, CH₂CH₃, OH oder OCH₃ stehen,
oder ein R¹¹ und ein R¹² gemeinsam mit sie verbindenden C-Atomen einen Cyclopropyl, Cyclobutyl, Cyclopentyl oder Cyclohexyl bilden;
X für O oder N-(C₁₋₄-Alkylen)ₘ-(Z)ₙ-(C₁₋₄-Alkylen)ₒ-B¹ oder C(R¹⁸)-(C₁₋₄-Alkylen)ₚ-(Z)_{q}-(C₁₋₄-Alkylen)ᵣ-B² steht;
worin
Z für O oder C=O steht;
m, n und o jeweils unabhängig für 0 oder 1 stehen, unter der Bedingung, dass, falls n für 1 steht und Z für O steht, m für 1 steht;
p, q und r jeweils unabhängig für 0 oder 1 stehen;
und
R¹⁸ für H, C₁₋₄-Alkyl, C₃₋₆-Cycloalkyl, OH, O-C₁₋₄-Alkyl, NH₂, N(H)C₁₋₄-Alkyl, N(C₁₋₄-Alkyl)₂, Aryl, Heteroaryl oder 4-7-gliedrigen Heterocycloalkyl steht und
B¹ für H, Alkyl, Cycloalkyl, Heteroaryl, 4-12-gliedrigen Heterocycloalkyl, NR¹⁹R²⁰ oder Aryl steht, oder
B² für H, Heteroaryl, 4-12-gliedrigen Heterocycloalkyl oder NR¹⁹R²⁰ steht;
worin
R¹⁹ und R²⁰ jeweils unabhängig für H, C₁₋₄-Alkyl oder C₃₋₆-Cycloalkyl stehen,
worin
jedes Aryl und jedes Heteroaryl unsubstituiert oder substituiert mit 1, 2, 3 oder 4 Substituenten ist, unabhängig voneinander ausgewählt aus F, Cl, CN, CF₃, CH₃, CH₂CH₃, CH(CH₃)₂, OH, OCH₃, OCF₃, Cyclopropyl, NH₂, N(H)(CH₃), N(CH₃)₂, 1-Morpholinyl, 1-Piperazinyl, 4-Methylpiperazin-1-yl, 1-Piperidinyl und 1-Pyrrolidinyl;
jedes C₁₋₄-Alkyl und jedes C₁₋₄-Alkylen linear oder verzweigt ist und unsubstituiert oder substituiert mit 1, 2 oder 3 Substituenten ist, unabhängig voneinander ausgewählt aus F, Cl, CF₃, CH₃, CH₂CH₃, CH(CH₃)₂, OH oder OCH₃;
und
jedes C₃₋₆-Cycloalkyl und jedes Heterocycloalkyl gesättigt oder ungesättigt, jedoch nicht aromatisch ist, und unsubstituiert oder substituiert mit 1, 2, 3 oder 4 Substituenten ist, unabhängig voneinander ausgewählt aus F, Cl, CN, CF₃, CH₃, CH₂CH₃, CH(CH₃)₂, OH, OCH₃, OCF₃, Cyclopropyl, NH₂, N(H)(CH₃), N(CH₃)₂, 1-Morpholinyl, 1-Piperazinyl, 4-Methyl-piperazin-1-yl, 1-Piperidinyl und 1-Pyrrolidinyl.

In einer weiteren Ausführungsform des ersten Erfindungsgegenstandes ist die Verbindung Formel (I) dadurch gekennzeichnet, dass
R⁷ und R⁸ jeweils unabhängig voneinander oder R⁹ oder R¹³ für 0, 1 oder 2 Substituenten stehen, unabhängig voneinander ausgewählt aus =O, F, Cl, CH₃ oder OCH₃,
bevorzugt stehen R⁷ und R⁸ jeweils für 0 Substituenten oder steht R⁹ für 0 Substituenten oder steht R¹³ für 0 Substituenten oder stehen zwei R¹³ jeweils für CH₃,
X für N-(C₁₋₄-Alkylen)ₘ-B¹ steht,
worin m für 0 oder 1 steht und B¹ für Heteroaryl, 4-12-gliedrigen Heterocycloalkyl oder Aryl steht, oder
X für C(R¹⁸)-(O)_{q}-(C₁₋₄-Alkylen)ᵣ-B² steht;
worin R¹⁸ für H steht, q für 0 oder 1 steht, r für 0 oder 1 steht und
B² für 4-12-gliedrigen Heterocycloalkyl oder Heteroaryl steht oder
oder B² für N(CH₃)₂, N(CH₃)(CH₂CH₃), N(CH₃)(CH(CH₃)₂), N(CH₃)(C(CH₃)₃), N(CH₃)(cyclopropyl), N(CH₂CH₃)₂ oder N(CH₂CH₃)(cyclopropyl) steht;
worin besagter Heteroaryl ausgewählt ist aus 2-Pyridyl, 3-Pyridyl, 4-pyridyl, 2-Pyrimidinyl, 4-Pyrimidinyl, 5-Pyrimidinyl, 3-Ppyridazinyl, 4-Pyridazinyl und 2-Pyrazinyl;
worin besagter 4-12-gliedriger Heterocycloalkyl ausgewählt ist aus Tetrahydrofuranyl, Tetrahydropyranyl, Morpholinyl, Piperazinyl, Piperidinyl und Pyrrolidinyl;
worin besagter Heteroaryl, besagter 4-12-gliedriger Heterocycloalkyl oder besagter Aryl unsubstituiert oder substituiert mit 1, 2, 3 oder 4 Substituenten ist, unabhängig voneinander ausgewählt aus F, Cl, CN, CF₃, CH₃, CH₂CH₃, CH(CH₃)₂, OH, OCH₃, OCF₃, Cyclopropyl, NH₂, N(H)(CH₃), N(CH₃)₂, 1-Morpholinyl, 1-Piperazinyl, 4-Methyl-piperazin-1-yl, 1-Piperidinyl und 1-Pyrrolidinyl.

In einer bevorzugten Ausführungsform des ersten Erfindungsgegenstandes ist die Verbindung gemäß Formel (I), bevorzugt gemäß Formel (I-1) oder (I-2), insbesondere bevorzugt gemäß Formel (I-1a), (I-1b), (I-1c), (I-2a), (I-2b) oder (I-2c) dadurch gekennzeichnet, dass Q ausgewählt ist aus einer der Formeln (i-1) bis (i-46): 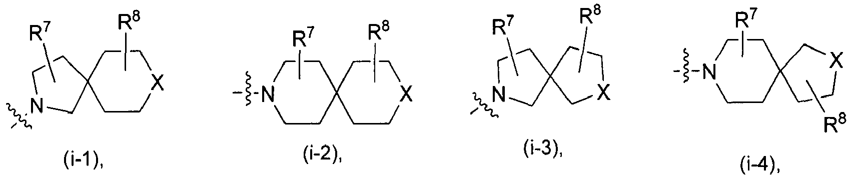 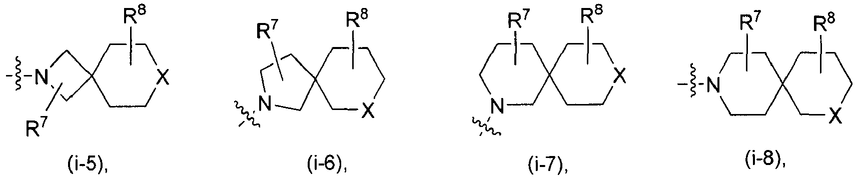 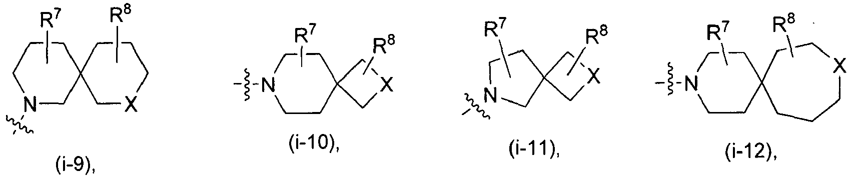 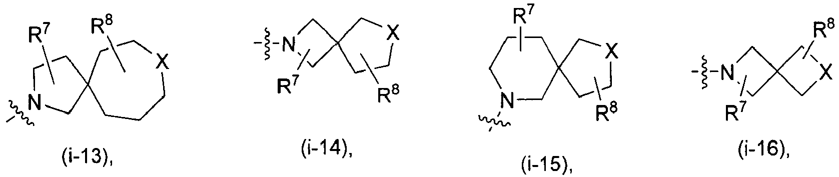 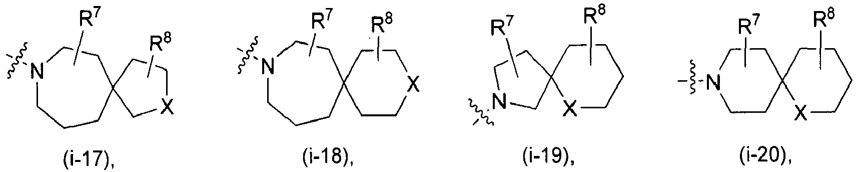 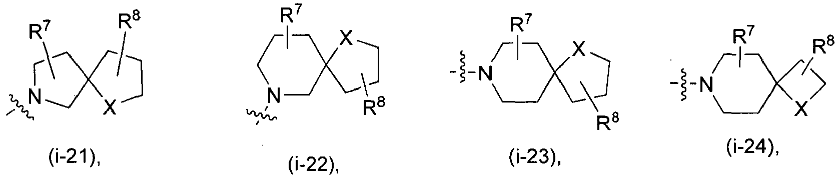 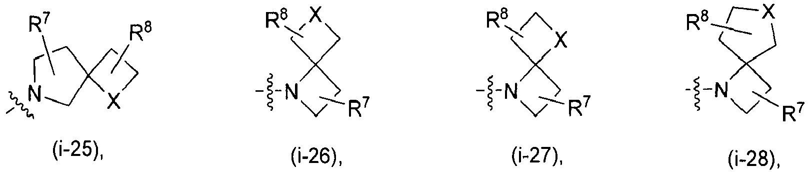 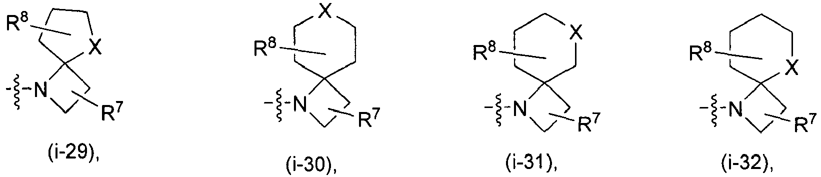 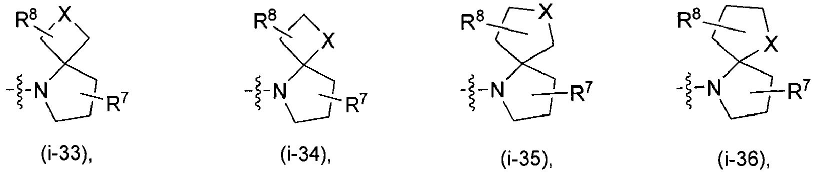 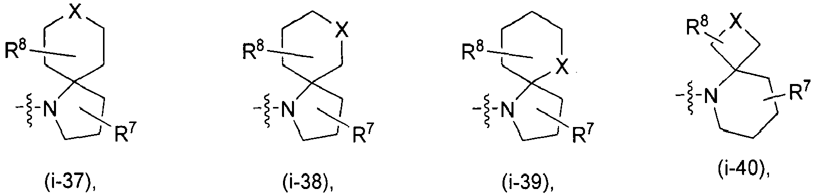 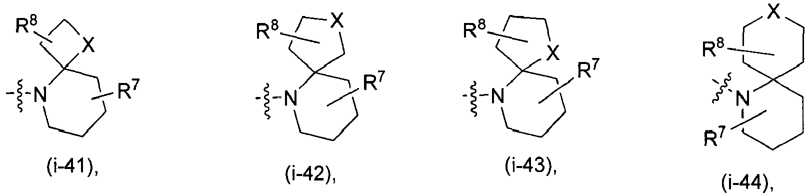 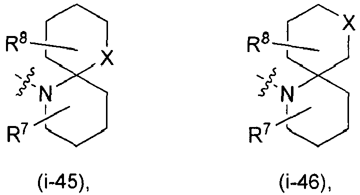 bevorzugt ist Q ausgewählt aus einer der Formeln (i-1) bis (i-46), worin R⁷ und R⁸ jeweils für 0 Substituenten stehen,
weiter bevorzugt ist Q ausgewählt aus einer der Formeln (i-1) bis (i-5), insbesondere bevorzugt aus einer der Formeln (i-1) bis (i-5), worin R⁷ und R⁸ jeweils für 0 Substituenten stehen.

In einer weiteren bevorzugten Ausführungsform des ersten Erfindungsgegenstandes ist die Verbindung gemäß Formel (I), bevorzugt gemäß Formel (I-1) oder (I-2), insbesondere bevorzugt gemäß Formel (I-1a), (I-1b), (I-1c), (I-2a), (I-2b) oder (I-2c) dadurch gekennzeichnet, dass Q ausgewählt ist aus einer der Formeln (ii-1) bis (ii-36): 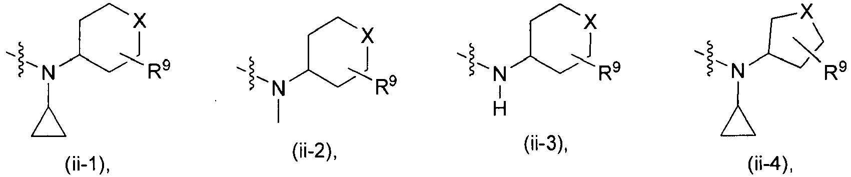 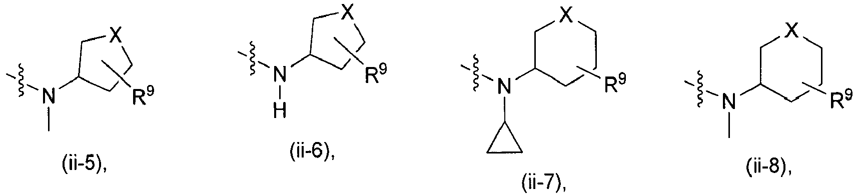 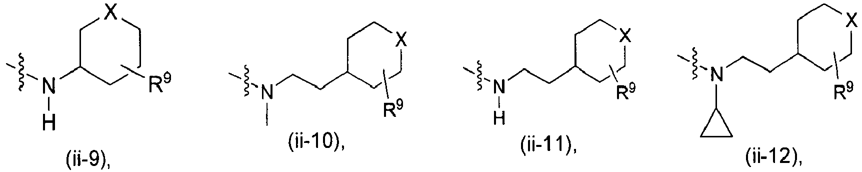 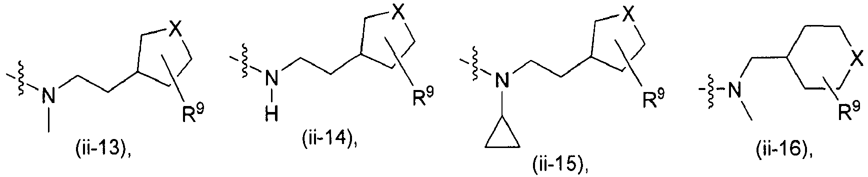 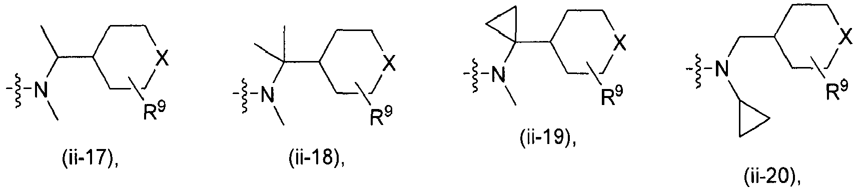 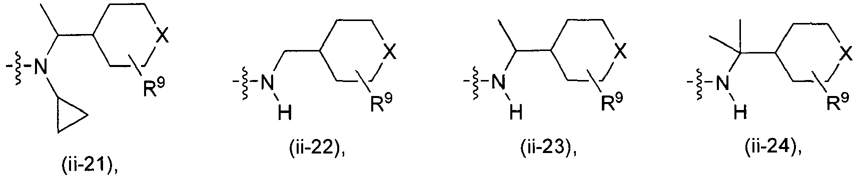 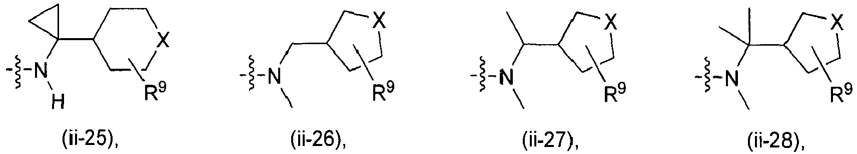 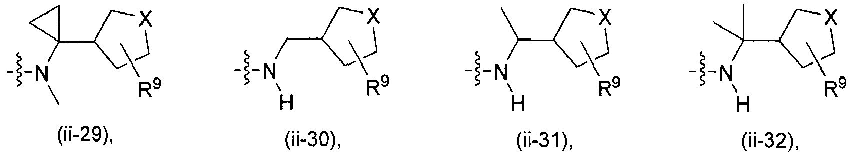 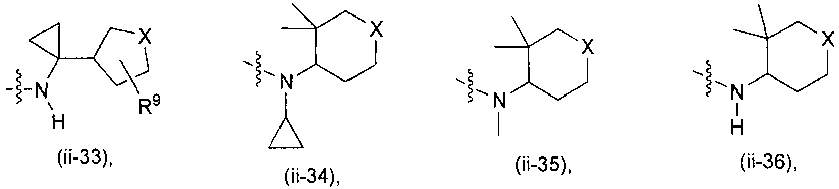 bevorzugt ist Q ausgewählt aus einer der Formeln (ii-1) bis (ii-36), worin R⁹ jeweils für 0 Substituenten steht,
bevorzugt ist Q ausgewählt aus einer der Formeln (ii-1) bis (ii-12), weiter bevorzugt ist Q ausgewählt aus einer der Formeln (ii-1) bis (ii-9), bevorzugt worin R⁹ jeweils für 0 Substituenten steht, und aus einer der Formeln (ii-34) bis (ii-36).

In einer weiteren bevorzugten Ausführungsform des ersten Erfindungsgegenstandes ist die Verbindung gemäß Formel (I), bevorzugt gemäß Formel (I-1) oder (I-2), insbesondere bevorzugt gemäß Formel (I-1a), (I-1b), (I-1c), (I-2a), (I-2b) oder (I-2c) dadurch gekennzeichnet, dass Q ausgewählt ist aus einer der Formeln (iii-1) bis (iii-9) 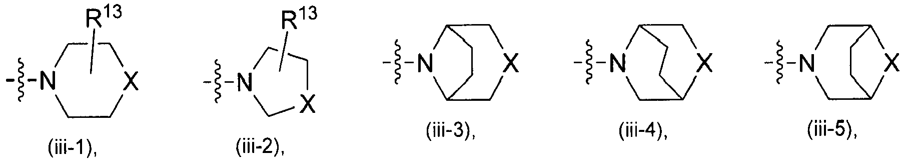 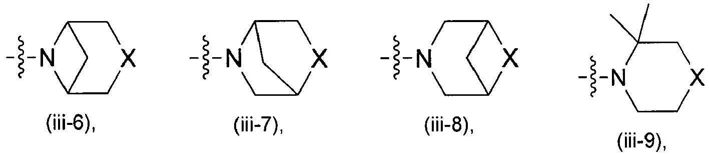 bevorzugt ist Q ausgewählt aus einer der Formeln (iii-1) bis (iii-9), bevorzugt worin R¹³ jeweils für 0 Substituenten steht,
weiter bevorzugt ist Q ausgewählt aus einer der Formeln (iii-1) bis (iii-3) und (iii-9), bevorzugt worin R¹³ jeweils für 0 Substituenten steht.

In einer Ausführungsform des ersten Erfindungsgegenstandes ist die Verbindung gemäß Formel (I), bevorzugt gemäß Formel (I-1) oder (I-2), insbesondere bevorzugt gemäß Formel (I-1a), (I-1b), (I-1c), (I-2a), (I-2b) oder (I-2c) dadurch gekennzeichnet, dass
X für N-(C₁₋₄-Alkylen)ₘ-(Z)ₙ-(C₁₋₄-Alkylen)ₒ-B¹ steht,
worin m, n und o jeweils unabhängig voneinander für 0 oder 1 stehen, unter der Bedingung, dass, falls n für 1 steht und Z für O steht, m für 1 steht und
Z bedeutet O oder C=O;
B¹ bedeutet H, C₁₋₄-Alkyl, Cycloalkyl, Heteroaryl, 4-12-gliedrigen Heterocycloalkyl, NR¹⁹R²⁰ oder Aryl,
worin R¹⁹ und R²⁰ jeweils unabhängig voneinander für H, C₁₋₄-Alkyl oder C₃₋₆-Cycloalkyl stehen.

Bevorzugt steht B¹ für Heteroaryl, 4-12-gliedrigen Heterocycloalkyl oder Aryl steht,
worin besagter Heteroaryl ausgewählt ist aus 2-Pyridyl, 3-Pyridyl, 4-pyridyl, 2-Pyrimidinyl, 4-Pyrimidinyl, 5-Pyrimidinyl, 3-Ppyridazinyl, 4-Pyridazinyl und 2-Pyrazinyl;
worin besagter 4-12-gliedriger Heterocycloalkyl ausgewählt ist aus Tetrahydrofuranyl, Tetrahydropyranyl, Morpholinyl, Piperazinyl, Piperidinyl und Pyrrolidinyl;
worin besagter Heteroaryl, besagter 4-12-gliedriger Heterocycloalkyl oder besagter Aryl unsubstituiert oder substituiert mit 1, 2, 3 oder 4 Substituenten ist, unabhängig voneinander ausgewählt aus F, Cl, CN, CF₃, CH₃, CH₂CH₃, CH(CH₃)₂, OH, OCH₃, OCF₃, Cyclopropyl, NH₂, N(H)(CH₃), N(CH₃)₂, 1-Morpholinyl, 1-Piperazinyl, 4-Methyl-piperazin-1-yl, 1-Piperidinyl und 1-Pyrrolidinyl.

In einer weiteren Ausführungsform des ersten Erfindungsgegenstandes ist die Verbindung gemäß Formel (I), bevorzugt gemäß Formel (I-1) oder (I-2), insbesondere bevorzugt gemäß Formel (I-1a), (I-1b), (I-1c), (I-2a), (I-2b) oder (I-2c) dadurch gekennzeichnet, dass
X für N-(C₁₋₄-Alkylen)ₘ-B¹ steht,
worin m für 0 oder 1 steht und B¹ für Heteroaryl, 4-12-gliedrigen Heterocycloalkyl oder Aryl steht,
worin besagter Heteroaryl ausgewählt ist aus 2-Pyridyl, 3-Pyridyl, 4-pyridyl, 2-Pyrimidinyl, 4-Pyrimidinyl, 5-Pyrimidinyl, 3-Ppyridazinyl, 4-Pyridazinyl und 2-Pyrazinyl;
worin besagter 4-12-gliedriger Heterocycloalkyl ausgewählt ist aus Tetrahydrofuranyl, Tetrahydropyranyl, Morpholinyl, Piperazinyl, Piperidinyl und Pyrrolidinyl;
worin besagter Heteroaryl, besagter 4-12-gliedriger Heterocycloalkyl oder besagter Aryl unsubstituiert oder substituiert mit 1, 2, 3 oder 4 Substituenten ist, unabhängig voneinander ausgewählt aus F, Cl, CN, CF₃, CH₃, CH₂CH₃, CH(CH₃)₂, OH, OCH₃, OCF₃, Cyclopropyl, NH₂, N(H)(CH₃), N(CH₃)₂, 4-Morpholinyl, 1-Piperazinyl, 4-Methyl-piperazin-1-yl, 1-Piperidinyl und 1-Pyrrolidinyl.

In einer bevorzugten Ausführungsform des ersten Erfindungsgegenstandes ist die Verbindung gemäß Formel (I), bevorzugt gemäß Formel (I-1) oder (I-2), insbesondere bevorzugt gemäß Formel (I-1a), (I-1b), (I-1c), (I-2a), (I-2b) oder (I-2c) dadurch gekennzeichnet,
dass X ausgewählt ist aus N-(C₁₋₄-Alkylen)ₘ-(Z)ₙ-(C₁₋₄-Alkylen)ₒ-B¹,
worin
m = 0, n = 0 und o = 0 ist
und B¹ steht für einen Rest, ausgewählt aus H, CH₃, 4-Tetrahydropyranyl, 4-Methyl-cyclohexyl, 4-Fluor-cyclohexyl, Cyclohexyl, 4-Pyridinyl, 2-Dimethylamino-4-pyridinyl, 2-Fluor-4-pyridinyl, 2-Methoxy-4-pyridinyl, 3-Pyridinyl, 6-Methoxy-3-pyridinyl, 6-Pyrrolidin-1-yl-3-pyridinyl, 2-Pyridinyl, 4-Trifluormethyl-2-pyridinyl, 4-Fluor-2-pyridinyl, 3,5-Dimethyl-2-pyridinyl, 4-Methoxy-3,5-dimethyl-2-pyridinyl, 4-Pyrimidinyl, 2,6-Dimethyl-4-pyrimidinyl, 2-Isopropyl-6-methyl-4-pyrimidinyl, 2-Dimethylamino-4-pyrimidinyl, 2-Pyrrolidin-1-yl-4-pyrimidinyl und 6-Methyl-4-pyrimidinyl,
oder
m = 1, C₁₋₄-Alkylen = CH₂, n = 0 und o = 0 ist
und B¹ steht für einen Rest, ausgewählt aus H, CH₃, 4-Tetrahydropyranyl, 4-Methyl-cyclohexyl, 4-Fluor-cyclohexyl, Cyclohexyl, 4-Pyridinyl, 2-Dimethylamino-4-pyridinyl, 2-Fluor-4-pyridinyl, 2-Methoxy-4-pyridinyl, 3-Pyridinyl, 6-Methoxy-3-pyridinyl, 6-Pyrrolidin-1-yl-3-pyridinyl, 2-Pyridinyl, 4-Trifluormethyl-2-pyridinyl, 4-Fluor-2-pyridinyl, 3,5-Dimethyl-2-pyridinyl, 4-Methoxy-3,5-dimethyl-2-pyridinyl, 4-Pyrimidinyl, 2,6-Dimethyl-4-pyrimidinyl, 2-Isopropyl-6-methyl-4-pyrimidinyl, 2-Dimethylamino-4-pyrimidinyl, 2-Pyrrolidin-1-yl-4-pyrimidinyl, 6-Methyl-4-pyrimidinyl, 5-Isooxazolyl
und 3-Methyl-5-isooxazolyl,
oder
m = 0, n = 1, Z = C=0 und o = 0 ist
und und B¹ steht für einen Rest, ausgewählt aus 4-Pyridinyl, 2-Dimethylamino-4-pyridinyl, 2-Fluor-4-pyridinyl, 2-Methoxy-4-pyridinyl, 3-Pyridinyl, 6-Methoxy-3-pyridinyl, 6-Pyrrolidin-1-yl-3-pyridinyl, 2-Pyridinyl, 4-Trifluormethyl-2-pyridinyl, 4-Fluor-2-pyridinyl, 3,5-Dimethyl-2-pyridinyl, 4-Methoxy-3,5-dimethyl-2-pyridinyl, 4-Pyrimidinyl, 2,6-Dimethyl-4-pyrimidinyl, 2-Isopropyl-6-methyl-4-pyrimidinyl, 2-Dimethylamino-4-pyrimidinyl, 2-Pyrrolidin-1-yl-4-pyrimidinyl und 6-Methyl-4-pyrimidinyl,
oder
m = 1, C₁₋₄-Alkylen = CH₂, n = 1, Z = C=O und o = 0 ist
und B¹ steht für einen Rest, ausgewählt aus 1-Pyrrolidinyl, 4-Methyl-1-piperazinyl, 1-Piperazinyl, 1-Piperidinyl und 4-Morpholinyl.

In einer weiteren Ausführungsform des ersten Erfindungsgegenstandes ist die Verbindung gemäß Formel (I), bevorzugt gemäß Formel (I-1) oder (I-2), insbesondere bevorzugt gemäß Formel (I-1a), (I-1b), (I-1c), (I-2a), (I-2b) oder (I-2c) dadurch gekennzeichnet,
dass X für C(R¹⁸)-(C₁₋₄-Alkylen)ₚ-(Z)_{q}-(C₁₋₄-Alkylen)ᵣ-B² steht,
worin
Z für O oder C=O steht;
p, q und r jeweils unabhängig für 0 oder 1 stehen;
R¹⁸ für H, C₁₋₄-Alkyl, C₃₋₆-Cycloalkyl, OH, O-C₁₋₄-Alkyl, NH₂, N(H)C₁₋₄-Alkyl, N(C₁₋₄-Alkyl)₂, Aryl, Heteroaryl oder 4-7-gliedrigen Heterocycloalkyl steht;
und
B² für H, Heteroaryl, 4-12-gliedrigen Heterocycloalkyl oder NR¹⁹R²⁰ steht;
worin
R¹⁹ und R²⁰ jeweils unabhängig für H, C₁₋₄-Alkyl oder C₃₋₆-Cycloalkyl stehen,
worin
jedes Aryl und jedes Heteroaryl unsubstituiert oder substituiert mit 1, 2, 3 oder 4 Substituenten ist, unabhängig voneinander ausgewählt aus F, Cl, CN, CF₃, CH₃, CH₂CH₃, CH(CH₃)₂, OH, OCH₃, OCF₃, Cyclopropyl, NH₂, N(H)(CH₃), N(CH₃)₂, 4-Morpholinyl, 1-Piperazinyl, 4-Methylpiperazin-1-yl, 1-Piperidinyl und 1-Pyrrolidinyl;
jedes C₁₋₄-Alkyl und jedes C₁₋₄-Alkylen linear oder verzweigt ist und unsubstituiert oder substituiert mit 1, 2 oder 3 Substituenten ist, unabhängig voneinander ausgewählt aus F, Cl, CF₃, CH₃, CH₂CH₃, CH(CH₃)₂, OH oder OCH₃;
und
jedes C₃₋₆-Cycloalkyl und jedes Heterocycloalkyl gesättigt oder ungesättigt, jedoch nicht aromatisch ist, und unsubstituiert oder substituiert mit 1, 2, 3 oder 4 Substituenten ist, unabhängig voneinander ausgewählt aus F, Cl, CN, CF₃, CH₃, CH₂CH₃, CH(CH₃)₂, OH, OCH₃, OCF₃, Cyclopropyl, NH₂, N(H)(CH₃), N(CH₃)₂, 4-Morpholinyl, 1-Piperazinyl, 4-Methyl-piperazin-1-yl, 1-Piperidinyl und 1-Pyrrolidinyl.

In einer bevorzugten Ausführungsform des ersten Erfindungsgegenstandes ist die Verbindung gemäß Formel (I), bevorzugt gemäß Formel (I-1) oder (I-2), insbesondere bevorzugt gemäß Formel (I-1a), (I-1b), (I-1c), (I-2a), (I-2b) oder (I-2c) dadurch gekennzeichnet,
dass X ausgewählt ist aus C(R¹⁸)-(O)q-(C₁₋₄-Alkylen)ᵣ-B²,
worin R¹⁸ für H steht, q für 0 oder 1 steht, r für 0 oder 1 steht und B² für 4-12-gliedrigen Heterocycloalkyl oder Heteroaryl oder für N(CH₃)₂, N(CH₃)(CH₂CH₃), N(CH₃)(CH(CH₃)₂), N(CH₃)(C(CH₃)₃), N(CH₃)(cyclopropyl), N(CH₂CH₃)₂ oder N(CH₂CH₃)(cyclopropyl) steht,
worin besagter Heteroaryl ausgewählt ist aus 2-Pyridyl, 3-Pyridyl, 4-pyridyl, 2-Pyrimidinyl, 4-Pyrimidinyl, 5-Pyrimidinyl, 3-Ppyridazinyl, 4-Pyridazinyl und 2-Pyrazinyl;
worin besagter 4-12-gliedriger Heterocycloalkyl ausgewählt ist aus Tetrahydrofuranyl, Tetrahydropyranyl, Morpholinyl, Piperazinyl, Piperidinyl und Pyrrolidinyl;
worin besagter Heteroaryl oder besagter 4-12-gliedriger Heterocycloalkyl unsubstituiert oder substituiert mit 1, 2, 3 oder 4 Substituenten ist, unabhängig voneinander ausgewählt aus F, Cl, CN, CF₃, CH₃, CH₂CH₃, CH(CH₃)₂, OH, OCH₃, OCF₃, Cyclopropyl, NH₂, N(H)(CH₃), N(CH₃)₂, 4-Morpholinyl, 1-Piperazinyl, 4-Methyl-piperazin-1-yl, 1-Piperidinyl und 1-Pyrrolidinyl.

In einer bevorzugten Ausführungsform des ersten Erfindungsgegenstandes ist die Verbindung gemäß Formel (I), bevorzugt gemäß Formel (I-1) oder (I-2), insbesondere bevorzugt gemäß Formel (I-1a), (I-1b), (I-1c), (I-2a), (I-2b) oder (I-2c) dadurch gekennzeichnet,
dass X ausgewählt ist aus C(R¹⁸)-(C₁₋₄-Alkylen)ₚ-(Z)_{q}-(C₁₋₄-Alkylen)ᵣ-B²,
worin R¹⁸ für H steht, p = 0, q = 0 und r = 0 ist
und B² steht für einen Rest, ausgewählt aus 4-Pyridinyl, 2-Dimethylamino-4-pyridinyl, 2-Fluor-4-pyridinyl, 2-Methoxy-4-pyridinyl, 1-Pyrrolidinyl, 4-Methyl-1-piperazinyl, 1-Piperazinyl, 1-Piperidinyl,
4-Methoxy-1-piperidinyl oder 4-Morpholinyl, Dimethylamino und Cyclopropylamino;
oder
worin R¹⁸ für OH steht, p = 0, q = 0 und r = 0 ist
und B² steht für H;
oder
worin R¹⁸ für H steht, p = 0, q = 1, Z = O und r = 0 ist
und B² steht für einen Rest, ausgewählt aus 4-Pyridinyl, 2-Dimethylamino-4-pyridinyl, 2-Fluor-4-pyridinyl, 2-Methoxy-4-pyridinyl, 3-Pyridinyl, 6-Methoxy-3-pyridinyl, 6-Pyrrolidin-1-yl-3-pyridinyl, 2-Pyridinyl, 4-Trifluormethyl-2-pyridinyl, 4-Fluor-2-pyridinyl, 3,5-Dimethyl-2-pyridinyl, 4-Methoxy-3,5-dimethyl-2-pyridinyl,4-Pyrimidinyl, 2,6-Dimethyl-4-pyrimidinyl, 2-Isopropyl-6-methyl-4-pyrimidinyl, 2-Dimethylamino-4-pyrimidinyl, 2-Pyrrolidin-1-yl-4-pyrimidinyl und 6-Methyl-4-pyrimidinyl;
oder
worin R¹⁸ für H steht, p = 1, C₁₋₄-Alkylen = CH₂, q = 0 und r = 0 ist
und B² steht für einen Rest, ausgewählt aus 4-Pyridinyl, 2-Dimethylamino-4-pyridinyl, 2-Fluor-4-pyridinyl, 2-Methoxy-4-pyridinyl, 3-Pyridinyl, 6-Methoxy-3-pyridinyl, 6-Pyrrolidin-1-yl-3-pyridinyl, 2-Pyridinyl, 4-Trifluormethyl-2-pyridinyl, 4-Fluor-2-pyridinyl, 3,5-Dimethyl-2-pyridinyl, 4-Methoxy-3,5-dimethyl-2-pyridinyl,4-Pyrimidinyl, 2,6-Dimethyl-4-pyrimidinyl, 2-Isopropyl-6-methyl-4-pyrimidinyl, 2-Dimethylamino-4-pyrimidinyl, 2-Pyrrolidin-1-yl-4-pyrimidinyl, 6-Methyl-4-pyrimidinyl, 1-Pyrrolidinyl, 4-Methyl-1-piperazinyl, 1-Piperazinyl, 1-Piperidinyl, 4-Methoxy-1-piperidinyl und 4-Morpholinyl.

In einer weiteren Ausführungsform des ersten Erfindungsgegenstandes ist die Verbindung gemäß Formel (I), bevorzugt gemäß Formel (I-1) oder (I-2), insbesondere bevorzugt gemäß Formel (I-1 a), (I-1 b), (I-1 c), (I-2a), (I-2b) oder (I-2c) dadurch gekennzeichnet,
dass
R¹ für F, Cl, CN, CH₃ oder OCH₃ steht; und
R² und R³ unabhängig voneinander für H, F, Cl, CN, CH₃ oder OCH₃ stehen,
z für 1 steht und y für 0 oder 1, bevorzugt für 0, steht;
A¹, A³ und A⁴ jeweils unabhängig voneinander für O, N oder CR⁴ stehen,
worin jedes R⁴ unabhängig für H oder CH₃ steht;
A² für C oder N steht;
Q ist ausgewählt aus einer der Formeln (i-1), (i-2), (i-3), (i-4), 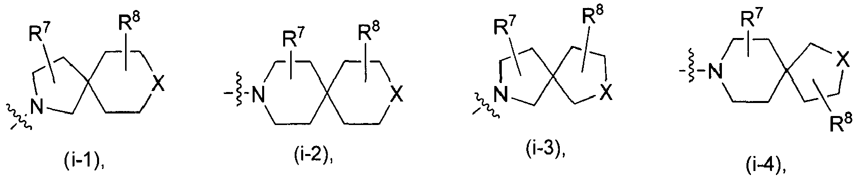 worin
R⁷ und R⁸ unabhängig voneinander für 0, 1 oder 2 Substituenten steht, jeweils unabhängig voneinander ausgewählt aus =O, F, CH₃ oder OCH₃;
oder Q ist ausgewählt aus einer der Formeln (ii-1) bis (ii-12) und (ii-34) bis (ii-36): 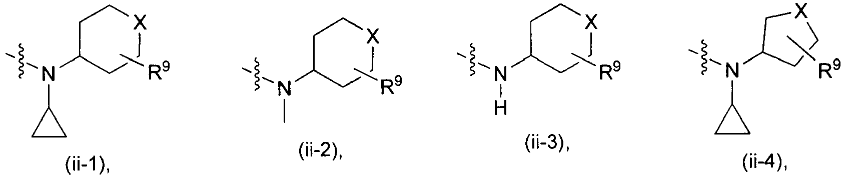 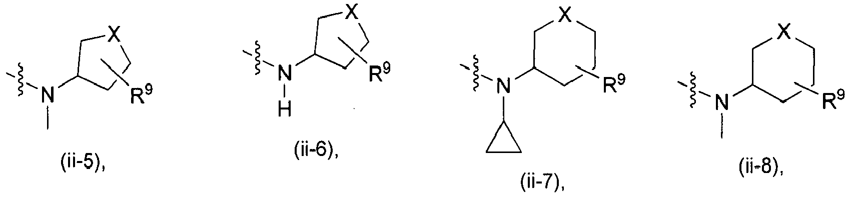 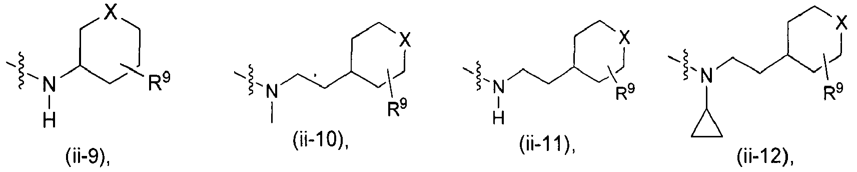 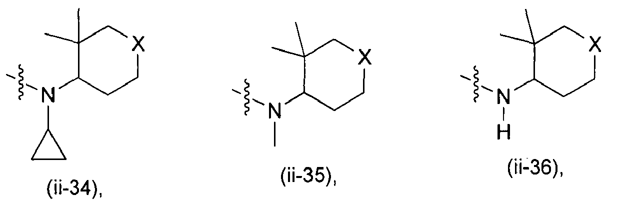 worin
R⁹ für 0, 1 oder 2 Substituenten steht, jeweils unabhängig voneinander ausgewählt aus =O, F, CH₃ oder OCH₃;
oder Q ist ausgewählt aus einer der Formeln (iii-1), (iii-2), (iii-3) und (iii-9): 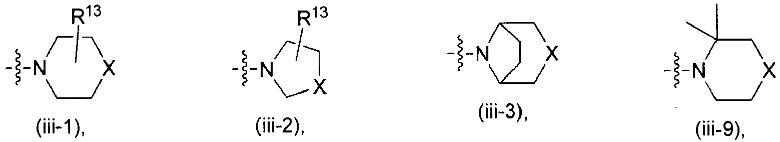 worin R¹³ für 0, 1 oder 2 Substituenten steht, jeweils unabhängig voneinander ausgewählt aus =O, F, CH₃ oder OCH₃, steht;
und worin
X für N-(C₁₋₄-Alkylen)ₘ-B¹ steht,
worin m für 0 oder 1 steht;
und B¹ ausgewählt ist aus 2-Pyridyl, 3-Pyridyl, 4-Pyridyl, 2-Pyrimidinyl oder 4-Pyrimidinyl, jeweils unsubstituiert oder substituiert mit 1, 2 oder 3 Substituenten, jeweils unabhängig voneinander ausgewählt aus F, Cl, CN, CF₃, C₁₋₄-Alkyl, OH, O-C₁₋₄-Alkyl, OCF₃, Cyclopropyl, NH₂, N(H)-C₁₋₄-Alkyl, N(C₁₋₄-Alkyl)₂, 4-Morpholinyl, 1-Piperazinyl, 4-Methyl-piperazin-1-yl, 1-Piperidinyl und 1-Pyrrolidinyl;
oder
X für C(R¹⁸)-(O)_{q}-(C₁₋₄-Alkylen)ᵣ-B² steht,
worin R¹⁸ für H steht, q für 0 oder 1 steht, r für 0 oder 1 steht und B² ausgewählt ist aus 2-Pyridyl, 3-Pyridyl, 4-Pyridyl, 2-Pyrimidinyl oder 4-Pyrimidinyl, 4-Morpholinyl, 1-Piperazinyl, 1-Piperidinyl und Pyrrolidinyl,
jeweils unsubstituiert oder substituiert mit 1, 2 oder 3 Substituenten, jeweils unabhängig voneinander ausgewählt aus F, Cl, CN, CF₃, CH₃, CH₂CH₃, CH(CH₃)₂, OH, OCH₃, OCF₃,
Cyclopropyl, NH₂, N(H)(CH₃), N(CH₃)₂, 4-Morpholinyl, 1-Piperazinyl, 4-Methyl-piperazin-1-yl, 1-Piperidinyl und 1-Pyrrolidinyl,
oder B² für N(CH₃)₂, N(CH₃)(CH₂CH₃), N(CH₃)(CH(CH₃)₂), N(CH₃)(C(CH₃)₃), N(CH₃)(cyclopropyl), N(CH₂CH₃)₂ oder N(CH₂CH₃)(cyclopropyl) steht.

In einer weiteren bevorzugten Ausführungsform der vorliegenden Erfindung können die erfindungsgemäßen Verbindungen ausgewählt sein aus der Gruppe bestehend aus
CC-01 3-[[(4-Methoxy-2,6-dimethyl-phenyl)sulfonyl]-methyl]-N-[(1S,3R)-3-(4-methyl-piperazin-1-yl)-cyclohexyl]-[1,2,4]oxadiazol-5-carbonsäure amid
CC-02 3-[[(4-Methoxy-2,6-dimethyl-phenyl)sulfonyl]-methyl]-N-[1-[5-(trifluormethyl)-pyridin-2-yl]-piperidin-4-yl]-[1,2,4]oxadiazol-5-carbonsäure amid
CC-03 2-[3-[[(4-Methoxy-2,6-dimethyl-phenyl)sulfonyl]-methyl]-[1,2,4]oxadiazol-5-yl]-N-[1-[5-(trifluor-methyl)-pyridin-2-yl]-piperidin-4-yl]-acetamid
CC-04 N-(4-Dimethylamino-cyclohexyl)-3-[[(4-methoxy-2,6-dimethyl-phenyl)sulfonyl]-methyl]-[1,2,4]oxa-diazol-5-carbonsäure amid
CC-05 N-(4-Dimethylamino-cyclohexyl)-2-[3-[[(4-methoxy-2,6-dimethyl-phenyl)sulfonyl]-methyl]-[1,2,4]oxadiazol-5-yl]-acetamid
CC-06 2-[3-[[(4-Methoxy-2,6-dimethyl-phenyl)sulfonyl]-methyl]-[1,2,4]oxadiazol-5-yl]-N-methyl-N-[2-(1-pyridin-4-yl-piperidin-4-yl)-ethyl]-acetamid
CC-09 3-[[(2-Chlor-6-methyl-phenyl)sulfonyl]-methyl]-N-[1-[5-(trifluormethyl)-pyridin-2-yl]-piperidin-4-yl]-[1,2,4]oxadiazol-5-carbonsäure amid
CC-10 3-[[(4-Methoxy-2,6-dimethyl-phenyl)sulfonyl]-methyl]-N-methyl-N-[2-(1-pyridin-4-yl-piperidin-4-yl)-ethyl]-[1,2,4]oxadiazol-5-carbonsäure amid
CC-11 3-[[(2-Chlor-6-methyl-phenyl)sulfonyl]-methyl]-N-(4-dimethylamino-cyclohexyl)-[1,2,4]oxadiazol-5-carbonsäure amid
CC-13 3-[[(4-Chlor-2,5-dimethyl-phenyl)sulfonyl]-methyl]-N-(4-dimethylamino-cyclohexyl)-[1,2,4]oxa-diazol-5-carbonsäure amid
CC-14 3-[[(2-Chlor-6-methyl-phenyl)sulfonyl]-methyl]-N-methyl-N-[2-(1-pyridin-4-yl-piperidin-4-yl)-ethyl]-[1,2,4]oxadiazol-5-carbonsäure amid
CC-16 3-[[(4-Chlor-2,5-dimethyl-phenyl)sulfonyl]-methyl]-N-methyl-N-[2-(1-pyridin-4-yl-piperidin-4-yl)-ethyl]-[1,2,4]oxadiazol-5-carbonsäure amid
CC-17 N-(3,3-Dimethyl-piperidin-4-yl)-3-[[(4-methoxy-2,6-dimethyl-phenyl)sulfonyl]-methyl]-[1,2,4]oxa-diazol-5-carbonsäure amid
CC-18 N-(3,3-Dimethyl-piperidin-4-yl)-2-[3-[[(4-methoxy-2,6-dimethyl-phenyl)sulfonyl]-methyl]-[1,2,4]oxadiazol-5-yl]-acetamid
CC-20 3-[[(2-Chlor-6-methyl-phenyl)sulfonyl]-methyl]-N-(2,2-dimethyl-4-pyrrolidin-1 -yl-cyclohexyl)-[1,2,4]oxadiazol-5-carbonsäure amid
CC-21 N-(2,2-Dimethyl-4-pyrrolidin-1-yl-cyclohexyl)-3-[[(4-methoxy-2,6-dimethyl-phenyl)sulfonyl]-methyl]-[1,2,4]oxadiazol-5-carbonsäure amid
CC-22 3-[[(4-Chlor-2,5-dimethyl-phenyl)sulfonyl]-methyl]-N-(2,2-dimethyl-4-pyrrolidin-1-yl-cyclohexyl)-[1,2,4]oxadiazol-5-carbonsäure amid
CC-23 N-(2,2-Dimethyl-4-pyrrolidin-1-yl-cyclohexyl)-2-[3-[[(4-methoxy-2,6-dimethyl-phenyl)sulfonyl]-methyl]-[1,2,4]oxadiazol-5-yl]-acetamid
CC-24 3-[[(4-Chlor-2,5-dimethyl-phenyl)sulfonyl]-methyl]-N-(3,3-dimethyl-piperidin-4-yl)-[1,2,4]oxadiazol-5-carbonsäure amid
CC-26 N-(2,2-Dimethyl-4-pyrrolidin-1-yl-cyclohexyl)-2-[5-[[(4-methoxy-2,6-dimethyl-phenyl)sulfonyl]-methyl]-[1,2,4]oxadiazol-3-yl]-acetamid
CC-27 2-[5-[[(4-Methoxy-2,6-dimethyl-phenyl)sulfonyl]-methyl]-[1,2,4]oxadiazol-3-yl]-N-[1-(2-methoxy-ethyl)-3,3-dimethyl-piperidin-4-yl]-acetamid
CC-28 N-(4-Dimethylamino-cyclohexyl)-2-[5-[[(4-methoxy-2,6-dimethyl-phenyl)sulfonyl]-methyl]-[1,2,4]oxadiazol-3-yl]-acetamid
CC-29 N-(2,2-Dimethyl-4-pyrrolidin-1-yl-cyclohexyl)-2-[5-[[(4-methoxy-2,6-dimethyl-phenyl)sulfonyl]-methyl]-[1,3,4]oxadiazol-2-yl]-acetamid
CC-30 N-(1-Acetyl-3,3-dimethyl-piperidin-4-yl)-2-[5-[[(4-methoxy-2,6-dimethyl-phenyl)sulfonyl]-methyl]-[1,2,4]oxadiazol-3-yl]-acetamid
CC-31 2-[5-[[(4-Methoxy-2,6-dimethyl-phenyl)sulfonyl]-methyl]-[1,3,4]oxadiazol-2-yl]-N-[1-(2-methoxy-ethyl)-3,3-dimethyl-piperidin-4-yl]-acetamid
CC-32 2-[5-[[(4-Methoxy-2,6-dimethyl-phenyl)sulfonyl]-methyl]-[1,2,4]oxadiazol-3-yl]-N-(4-pyrrolidin-1-yl-cyclohexyl)-acetamid
CC-33 N-[2,2-Dimethyl-4-(4-methyl-piperazin-1-yl)-cyclohexyl]-2-[5-[[(4-methoxy-2,6-dimethyl-phenyl)sulfonyl]-methyl]-[1,2,4]oxadiazol-3-yl]-acetamid
CC-34 N-(4-Dimethylamino-cyclohexyl)-2-[5-[[(4-methoxy-2,6-dimethyl-phenyl)sulfonyl]-methyl]-[1,3,4]oxadiazol-2-yl]-acetamid
CC-35 N-[4-(Cyclopropylamino)-2,2-dimethyl-cyclohexyl]-2-[5-[[(4-methoxy-2,6-dimethyl-phenyl)sulfonyl]-methyl]-[1,2,4]oxadiazol-3-yl]-acetamid
CC-36 N-(2,2-Dimethyl-4-pyridin-4-yl-cyclohexyl)-2-[5-[[(4-methoxy-2,6-dimethyl-phenyl)sulfonyl]-methyl]-[1,3,4]oxadiazol-2-yl]-acetamid
CC-37 N-(2,2-Dimethyl-4-pyridin-4-yl-cyclohexyl)-2-[5-[[(4-methoxy-2,6-dimethyl-phenyl)sulfonyl]-methyl]-[1,2,4]oxadiazol-3-yl]-acetamid
CC-38 N-(1-Acetyl-3,3-dimethyl-piperidin-4-yl)-2-[3-[[(3,4-dichlorphenyl)sulfonyl]-methyl]-[1,2,4]oxa-diazol-5-yl]-acetamid
CC-40 2-[5-[[(4-Methoxy-2,6-dimethyl-phenyl)sulfonyl]-methylj-[1,3,4]oxadiazol-2-yl]-N-(4-pyrrolidin-1-yl-cyclohexyl)-acetamid
CC-41 N-[1-(2-Methoxy-ethyl)-3,3-dimethyl-piperidin-4-yl]-2-[3-(p-tolylsulfonyl-methyl)-[1,2,4]oxadiazol-5-yl]-acetamid
CC-42 2-[5-[[(4-Methoxy-2,6-dimethyl-phenyl)sulfonyl]-methyl]-[1,2,4]oxadiazol-3-yl]-N-piperidin-4-yl-acetamid
CC-43 2-[3-[[(4-Chlor-2,5-dimethyl-phenyl)sulfonyl]-methyl]-[1,2,4]oxadiazol-5-yl]-N-(2,2-dimethyl-4-pyrrolidin-1-yl-cyclohexyl)-acetamid
CC-44 N-[2,2-Dimethyl-4-(4-methyl-piperazin-1-yl)-cyclohexyl]-2-[3-(p-tolylsulfonyl-methyl)-[1,2,4]oxa-diazol-5-yl]-acetamid
CC-45 2-[3-[[(4-Chlor-2,5-dimethyl-phenyl)sulfonyl]-methyl]-[1,2,4]oxadiazol-5-yl]-N-[2,2-dimethyl-4-(4-methyl-piperazin-1-yl)-cyclohexyl]-acetamid
CC-46 N-(1-Acetyl-3,3-dimethyl-piperidin-4-yl)-2-[3-[[(4-chlor-2,5-dimethyl-phenyl)sulfonyl]-methyl]-[1,2,4]oxadiazol-5-yl]-acetamid
CC-47 N-(1-Acetyl-3,3-dimethyl-piperidin-4-yl)-2-[3-[[(2,3-dichlor-phenyl)sulfonyl]-methyl]-[1,2,4]oxa-diazol-5-yl]-acetamid
CC-48 2-[3-[[(2,3-Dichlor-phenyl)sulfonyl]-methyl]-[1,2,4]oxadiazol-5-yl]-N-[2,2-dimethyl-4-(4-methyl-piperazin-1-yl)-cyclohexyl]-acetamid
CC-50 2-[3-[[(3,4-Dichlorphenyl)sulfonyl]-methyl]-[1,2,4]oxadiazol-5-yl]-N-(4-dimethylamino-cyclohexyl)-acetamid
CC-52 N-(3,3-Dimethyl-piperidin-4-yl)-2-[5-[[(4-methoxy-2,6-dimethyl-phenyl)sulfonyl]-methyl]-[1,2,4]oxa-diazol-3-yl]-acetamid
CC-53 2-[3-[[(3,4-Dichlorphenyl)sulfonyl]-methyl]-[1,2,4]oxadiazol-5-yl]-N-(2,2-dimethyl-4-pyrrolidin-1-yl-cyclohexyl)-acetamid
CC-54 2-[3-[[(2,3-Dichlor-phenyl)sulfonyl]-methyl]-[1,2,4]oxadiazol-5-yl]-N-piperidin-4-yl-acetamid
CC-57 2-[3-[[(4-Chlor-2,5-dimethyl-phenyl)sulfonyl]-methyl]-[1,2,4]oxadiazol-5-yl]-N-[4-(cyclopropyl-amino)-2,2-dimethyl-cyclohexyl]-acetamid
CC-59 2-[3-[[(2,3-Dichlor-phenyl)sulfonyl]-methyl]-[1,2,4]oxadiazol-5-yl]-N-(4-dimethylamino-cyclohexyl)-acetamid
CC-62 2-[3-[[(4-Chlor-2,5-dimethyl-phenyl)sulfonyl]-methyl]-[1,2,4]oxadiazol-5-yl]-N-(3,3-dimethyl-piperidin-4-yl)-acetamid
CC-69 N-(1-Acetyl-3,3-dimethyl-piperidin-4-yl)-2-[5-[[(4-methoxy-2,6-dimethyl-phenyl)sulfonyl]-methyl]-[1,3,4]oxadiazol-2-yl]-acetamid
CC-70 2-[3-[[(4-Chlor-2,5-dimethyl-phenyl)sulfonyl]-methyl]-[1,2,4]oxadiazol-5-yl]-N-(4-pyrrolidin-1-yl-cyclohexyl)-acetamid
CC-71 2-[3-[[(2,3-Dichlor-phenyl)sulfonyl]-methyl]-[1,2,4]oxadiazol-5-yl]-N-(4-pyrrolidin-1-yl-cyclohexyl)-acetamid
CC-72 2-[3-[[(2,3-Dichlor-phenyl)sulfonyl]-methyl]-[1,2,4]oxadiazol-5-yl]-N-[1-(2-methoxy-ethyl)-3,3-di-methyl-piperidin-4-yl]-acetamid
CC-77 2-[3-[[(4-Chlor-2,5-dimethyl-phenyl)sulfonyl]-methyl]-[1,2,4]oxadiazol-5-yl]-N-[1-(2-methoxy-ethyl)-3,3-dimethyl-piperidin-4-yl]-acetamid
CC-80 2-[3-[[(4-Chlor-2,5-dimethyl-phenyl)sulfonyl]-methyl]-[1,2,4]oxadiazol-5-yl]-N-(2,2-dimethyl-4-pyridin-4-yl-cyclohexyl)-acetamid
CC-81 2-[3-[[(4-Chlor-2,5-dimethyl-phenyl)sulfonyl]-methyl]-[1,2,4]oxadiazol-5-yl]-N-(4-dimethylamino-cyclohexyl)-acetamid
CC-86 N-[2,2-Dimethyl-4-(4-methyl-piperazin-1-yl)-cyclohexyl]-2-[5-[[(4-methoxy-2,6-dimethylphenyl)sulfonyl)-methyl]-[1,3,4]oxadiazol-2-yl]-acetamid
CC-88 N-(3,3-Dimethyl-piperidin-4-yl)-2-[5-[[(4-methoxy-2,6-dimethyl-phenyl)sulfonyl]-methyl]-[1,3,4]oxadiazol-2-yl]-N-methyl-acetamid
CC-90 2-[3-[[(2-Chlor-6-methyl-phenyl)sulfonyl]-methyl]-[1,2,4]oxadiazol-5-yl]-N-piperidin-4-yl-acetamid
CC-91 2-[3-[[(2-Chlor-6-methyl-phenyl)sulfonyl]-methyl]-[1,2,4]oxadiazol-5-yl]-N-(2,2-dimethyl-4-pyrrolidin-1-yl-cyclohexyl)-acetamid
CC-93 2-[3-[[(2-Chlor-6-methyl-phenyl)sulfonyl]-methyl]-[1,2,4]oxadiazol-5-yl]-N-(4-pyrrolidin-1-yl-cyclohexyl)-acetamid
CC-94 2-[3-[[(2-Chlor-6-methyl-phenyl)sulfonyl]-methyl]-[1,2,4]oxadiazol-5-yl]-N-[1-(2-methoxy-ethyl)-3,3-dimethyl-piperidin-4-yl]-acetamid
CC-95 2-[3-[[(2-Chlor-6-methyl-phenyl)sulfonyl]-methyl]-[1,2,4]oxadiazol-5-yl]-N-[4-(cyclopropylamino)-2,2-dimethyl-cyclohexyl]-acetamid
CC-96 2-[3-[[(2-Chlor-6-methyl-phenyl)sulfonyl]-methyl]-[1,2,4]oxadiazol-5-yl]-N-(1,3,3-trimethyl-piperidin-4-yl)-acetamid
CC-97 2-[5-[[(4-Methoxy-2,6-dimethyl-phenyl)sulfonyl]-methyl]-[1,3,4]oxadiazol-2-yl]-N-piperidin-4-yl-acetamid
CC-98 2-[3-[[(2-Chlor-6-methyl-phenyl)sulfonyl]-methyl]-[1,2,4]oxadiazol-5-yl]-N-[2,2-dimethyl-4-(4-methyl-piperazin-1-yl)-cyclohexyl]-acetamid
CC-1002-[3-[[(4-Chlor-2,5-dimethyl-phenyl)sulfonyl]-methyl]-[1,2,4]oxadiazol-5-yl]-N-(3,3-dimethyl-piperidin-4-yl)-N-methyl-acetamid
CC-1 01 N-(3,3-Dimethyl-piperidin-4-yl)-2-[5-[[(4-methoxy-2,6-dimethyl-phenyl)sulfonyl]-methyl]-[1,2,4]oxadiazol-3-yl]-N-methyl-acetamid
CC-1022-[5-[[(4-Methoxy-2,6-dimethyl-phenyl)sulfonyl]-methyl]-[1,2,4]oxadiazol-3-yl]-N-(1,3,3-trimethyl-piperidin-4-yl)-acetamid
CC-1052-[3-[[(4-Chlor-2,5-dimethyl-phenyl)sulfonyl]-methyl]-[1,2,4]oxadiazol-5-yl]-N-(1,3,3-trimethyl-piperidin-4-yl)-acetamid
CC-1062-[3-[[(2,3-Dichlor-phenyl)sulfonyl]-methyl]-[1,2,4]oxadiazol-5-yl]-N-(2,2-dimethyl-4-pyrrolidin-1-yl-cyclohexyl)-acetamid
CC-1082-[3-[[(2-Chlor-6-methyl-phenyl)sulfonyl]-methyl]-[1,2,4]oxadiazol-5-yl]-N-cyclopropyl-N-(4-dimethylamino-cyclohexyl)-acetamid
CC-1092-[3-[[(2,3-Dichlor-phenyl)sulfonyl]-methyl]-[1,2,4]oxadiazol-5-yl]-N-(2,2-dimethyl-4-pyridin-4-yl-cyclohexyl)-acetamid
CC-1102-[3-[[(2-Chlor-6-methyl-phenyl)sulfonyl]-methyl]-[1,2,4]oxadiazol-5-yl]-N-(3,3-dimethyl-piperidin-4-yl)-N-methyl-acetamid
CC-1112-[5-[[(4-Methoxy-2,6-dimethyl-phenyl)sulfonyl]-methyl]-[1,3,4]oxadiazol-2-yl]-N-(1,3,3-trimethyl-piperidin-4-yl)-acetamid
CC-112 N-Cyclopropyl-2-[3-[[(2,3-dichlor-phenyl)sulfonyl]-methyl]-[1,2,4]oxadiazol-5-yl]-N-(4-dimethyl-amino-cyclohexyl)-acetamid
CC-1132-[3-[[(4-Chlor-2,5-dimethyl-phenyl)sulfonyl]-methyl]-[1,2,4]oxadiazol-5-yl]-N-cyclopropyl-N-(4-dimethylamino-cyclohexyl)-acetamid
CC-1142-[3-[[(2,3-Dichlor-phenyl)sulfonyl]-methyl]-[1,2,4]oxadiazol-5-yl]-N-(1,3,3-trimethyl-piperidin-4-yl)-acetamid
CC-116 N-Cyclopropyl-N-(4-dimethylamino-cyclohexyl)-2-[5-[[(4-methoxy-2,6-dimethyl-phenyl)sulfonyl]-methyl]-[1,2,4]oxadiazol-3-yl]-acetamid
CC-118 N-Cyclopropyl-N-(4-dimethylamino-cyclohexyl)-2-[4-[[(4-methoxy-2,6-dimethyl-phenyl)sulfonyl]-methyl]-3,5-dimethyl-1H-pyrazol-1-yl]-acetamid
CC-1202-[3-[[(2-Chlor-6-methyl-phenyl)sulfonyl]-methyl]-[1,2,4]oxadiazol-5-yl]-N-(3,3-dimethyl-piperidin-4-yl)-acetamid
CC-123N-[4-(Cyclopropylamino)-2,2-dimethyl-cyclohexyl]-2-[3-[[(2,3-dichlor-phenyl)sulfonyl]-methyl]-[1,2,4]oxadiazol-5-yl]-acetamid
CC-124 N-Cyclopropyl-N-(4-dimethylamino-cyclohexyl)-2-[5-[[(4-methoxy-2,6-dimethyl-phenyl)sulfonyl]-methyl]-[1,3,4]oxadiazol-2-yl]-acetamid
CC-127 N-(2,2-Dimethyl-4-pyridin-4-yl-cyclohexyl)-2-[3-[[(4-methoxy-2,6-dimethyl-phenyl)sulfonyl)-methyl]-5-methyl-1 H-pyrazol-1-yl]-acetamid
CC-1282-[3-[[(2-Chlor-6-methyl-phenyl)sulfonyl]-methyl]-[1,2,4]oxadiazol-5-yl]-N-(4-dimethylamino-cyclohexyl)-acetamid
CC-129[3-[[(4-Methoxy-2,6-dimethyl-phenyl)sulfonyl]-methyl]-[1,2,4]oxadiazol-5-yl]-[4-(4-methyl-piperazin-1-yl)-piperidin-1-yl]-methanon
CC-1302-[3-[[(4-Methoxy-2,6-dimethyl-phenyl)sulfonyl]-methyl]-[1,2,4]oxadiazol-5-yl]-1-(2-methyl-2,8-diazaspiro[4.4]nonan-8-yl)-ethanon
CC-1312-[3-[[(4-Methoxy-2,6-dimethyl-phenyl)sulfonyl]-methyl]-[1,2,4]oxadiazol-5-yl]-1-[4-(4-methyl-piperazin-1-yl)-piperidin-1-yl]-ethanon
CC-1332-[3-[[(4-Methoxy-2,6-dimethyl-phenyl)sulfonyl]-methyl]-[1,2,4]oxadiazol-5-yl]-1-(4-pyridin-4-yl-piperazin-1-yl)-ethanon
CC-1342-[3-[[(4-Methoxy-2,6-dimethyl-phenyl)sulfonyl]-methyl]-[1,2,4]oxadiazol-5-yl]-1-(3-pyridin-4-yl-3,8-diazaspiro[4.5]decan-8-yl)-ethanon
CC-1352-[3-[[(4-Methoxy-2,6-dimethyl-phenyl)sulfonyl]-methyl]-[1,2,4]oxadiazol-5-yl]-1-[(1S, 5R)-3-pyridin-4-yloxy-8-azabicyclo[3.2.1]octan-8-yl]-ethanon
CC-136[3-[[(4-Methoxy-2,6-dimethyl-phenyl)sulfonyl]-methyl]-[1,2,4]oxadiazol-5-yl]-(4-pyridin-4-yl-piperazin-1-yl)-methanon
CC-137 [3-[[(2-Chlor-6-methyl-phenyl)sulfonyl]-methyl]-[1,2,4]oxadiazol-5-yl]-(4-pyridin-4-yl-piperazin-1-yl)-methanon
CC-138 [3-[[(2-Chlor-6-methyl-phenyl)sulfonyl]-methyl]-[1,2,4]oxadiazol-5-yl]-[4-(4-methyl-piperazin-1-yl)-piperidin-1-yl]-methanon
CC-139 [3-[[(4-Methoxy-2,6-dimethyl-phenyl)sulfonyl]-methyl]-[1,2,4]oxadiazol-5-yl]-(3-pyridin-4-yl-3,8-diazaspiro[4.5]decan-8-yl)-methanon
CC-141 [3-[[(4-Chlor-2,5-dimethyl-phenyl)sulfonyl]-methyl]-[1,2,4]oxadiazol-5-yl]-[4-(4-methyl-piperazin-1-yl)-piperidin-1-yl]-methanon
CC-142 [3-[[(4-Chlor-2,5-dimethyl-phenyl)sulfonyl]-methyl]-[1,2,4]oxadiazol-5-yl]-(2-methyl-2,8-diaza-spiro[4.4]nonan-8-yl)-methanon
CC-143 [3-[[(2-Chlor-6-methyl-phenyl)sulfonyl]-methyl]-[1,2,4]oxadiazol-5-yl]-(2-methyl-2,8-diazaspiro-[4.4]nonan-8-yl)-methanon
CC-144 [3-[[(4-Chlor-2,5-dimethyl-phenyl)sulfonyl]-methyl]-[1,2,4]oxadiazol-5-yl]-(4-pyridin-4-yl-piperazin-1-yl)-methanon
CC-145 [3-[[(2-Chlor-6-methyl-phenyl)sulfonyl]-methyl]-[1,2,4]oxadiazol-5-yl]-(3-pyridin-4-yl-3,8-diazaspiro[4.5]decan-8-yl)-methanon
CC-146 [3-[[(4-Chlor-2,5-dimethyl-phenyl)sulfonyl]-methyl]-[1,2,4]oxadiazol-5-yl]-(9-pyridin-4-yl-3,9-diazaspiro[5.5]undecan-3-yl)-methanon
CC-147 [3-[[(4-Methoxy-2,6-dimethyl-phenyl)sulfonyl]-methyl]-[1,2,4]oxadiazol-5-yl]-(2-methyl-2,8-diazaspiro[4.4]nonan-8-yl)-methanon
CC-148 [3-[[(2-Chlor-6-methyl-phenyl)sulfonyl]-methyl]-[1,2,4]oxadiazol-5-yl]-(9-pyridin-4-yl-3,9-diazaspiro[5.5]undecan-3-yl)-methanon
CC-149 [3-[[(4-Methoxy-2,6-dimethyl-phenyl)sulfonyl]-methyl]-[1,2,4]oxadiazol-5-yl]-[(1S,5R)-3-pyridin-4-yloxy-8-azabicyclo[3.2.1]octan-8-yl]-methanon
CC-150 [3-[[(2-Chlor-6-methyl-phenyl)sulfonyl]-methyl]-[1,2,4]oxadiazol-5-yl]-[(1S,5R)-3-pyridin-4-yloxy-8-azabicyclo[3.2.1]octan-8-yl]-methanon
CC-151 [3-[[(4-Chlor-2,5-dimethyl-phenyl)sulfonyl]-methyl]-[1,2,4]oxadiazol-5-yl]-[(1S,5R)-3-pyridin-4-yloxy-8-azabicyclo[3.2.1]octan-8-yl]-methanon
CC-152 [3-[[(4-Chlor-2,5-dimethyl-phenyl)sulfonyl]-methyl]-[1,2,4]oxadiazol-5-yl]-(3-pyridin-4-yl-3,8-diazaspiro[4.5]decan-8-yl)-methanon
CC-1531-[2,2-Dimethyl-4-(pyridin-4-yl-methyl)-piperidin-1-yl]-2-[3-[[(4-methoxy-2,6-dimethyl-phenyl)-sulfonyl]-methyl]-[1,2,4]oxadiazol-5-yl]-ethanon
CC-154 N-(4-Dimethylamino-cyclohexyl)-2-[3-[[(4-methoxy-2,6-dimethyl-phenyl)sulfonyl]-methyl]-5-methyl-1 H-pyrazol-1-yl]-acetamid
CC-155 N-(2,2-Dimethyl-4-pyrrolidin-1-yl-cyclohexyl)-2-[3-[[(4-methoxy-2,6-dimethyl-phenyl)sulfonyl]-methyl]-5-methyl-1 H-pyrazol-1-yl]-acetamid
CC-1562-[3-[[(4-Methoxy-2,6-dimethyl-phenyl)sulfonyl]-methyl]-5-methyl-1H-pyrazol-1-yl]-N-(4-pyrrolidin-1-yl-cyclohexyl)-acetamid
CC-1572-[3-[[(4-Methoxy-2,6-dimethyl-phenyl)sulfonyl]-methyl]-5-methyl-1H-pyrazol-1-yl]-N-[1-(2-methoxy-ethyl)-3,3-dimethyl-piperidin-4-yl]-acetamid
CC-158N-(2,2-Dimethyl-4-pyrrolidin-1-yl-cyclohexyl)-2-[4-[[(4-methoxy-2,6-dimethyl-phenyl)sulfonyl]-methyl]-1H-[1,2,3]triazol-1-yl]-acetamid
CC-159N-(1-Acetyl-3,3-dimethyl-piperidin-4-yl)-2-[3-[[(4-methoxy-2,6-dimethyl-phenyl)sulfonyl]-methyl]-5-methyl-1 H-pyrazol-1-yl]-acetamid
CC-160 2-[4-[[(4-Methoxy-2,6-dimethyl-phenyl)sulfonyl]-methyl]-1H-[1,2,3]triazol-1-yl]-N-[1-(2-methoxy-ethyl)-3,3-dimethyl-piperidin-4-yl]-acetamid
CC-161 N-(1-Acetyl-3,3-dimethyl-piperidin-4-yl)-2-[4-[[(4-methoxy-2,6-dimethyl-phenyl)sulfonyl]-methyl]-1H-[1,2,3]triazol-1-yl]-acetamid
CC-162N-[2,2-Dimethyl-4-(4-methyl-piperazin-1-yl)-cyclohexyl]-2-[4-[[(4-methoxy-2,6-dimethylphenyl)sulfonyl]-methyl]-1H-[1,2,3]triazol-1-yl]-acetamid
CC-163 N-[2,2-Dimethyl-4-(4-methyl-piperazin-1-yl)-cyclohexyl]-2-[3-[[(4-methoxy-2,6-dimethyl-phenyl)sulfonyl]-methyl]-5-methyl-1H-pyrazol-1-yl]-acetamid
CC-1642-[4-[[(4-Methoxy-2,6-dimethyl-phenyl)sulfonyl]-methyl]-3,5-dimethyl-1H-pyrazol-1-yl]-N-(4-pyrrolidin-1-yl-cyclohexyl)-acetamid
CC-1652-[4-[[(4-Methoxy-2,6-dimethyl-phenyl)sulfonyl]-methyl]-3,5-dimethyl-1H-pyrazol-1-yl]-N-[1-(2-methoxy-ethyl)-3,3-dimethyl-piperidin-4-yl]-acetamid
CC-166 N-[2, 2-D imethyl-4-(4-methyl-piperazi n-1-yl)-cycloh exyl]-2-[4-[[(4-methoxy-2, 6-d imethyl-phenyl)sulfonyl]-methyl]-3,5-dimethyl-1 H-pyrazol-1-yl]-acetamid
CC-1672-[4-[[(4-Methoxy-2,6-dimethyl-phenyl)sulfonyl]-methyl]-1H-[1,2,3]triazol-1-yl]-N-(4-pyrrolidin-1-yl-cyclohexyl)-acetamid
CC-168 N-(4-Dimethylamino-cyclohexyl)-2-[4-[[(4-methoxy-2,6-dimethyl-phenyl)sulfonyl]-methyl]-3,5-dimethyl-1 H-pyrazol-1-yl]-acetamid
CC-169 N-(4-Dimethylamino-cyclohexyl)-2-[4-[[(4-methoxy-2,6-dimethyl-phenyl)sulfonyl]-methyl]-1H-[1,2,3]triazol-1-yl]-acetamid
CC-170 N-(2,2-Dimethyl-4-pyrrolidin-1-yl-cyclohexyl)-2-[4-[[(4-methoxy-2,6-dimethyl-phenyl)sulfonyl]-methyl]-3,5-dimethyl-1H-pyrazol-1-yl]-acetamid
CC-171 N-(3,3-Dimethyl-piperidin-4-yl)-2-[3-[[(4-methoxy-2,6-dimethyl-phenyl)sulfonyl]-methyl]-5-methyl-1 H-pyrazol-1-yl]-acetamid
CC-172 N-(2,2-Dimethyl-4-pyridin-4-yl-cyclohexyl)-2-[4-[[(4-methoxy-2,6-dimethyl-phenyl)sulfonyl]-methyl]-3,5-dimethyl-1 H-pyrazol-1-yl]-acetamid
CC-1732-[4-[[(4-Methoxy-2,6-dimethyl-phenyl)sulfonyl]-methyl]-3,5-dimethyl-1H-pyrazol-1-yl]-N-piperidin-4-yl-acetamid
CC-174N-[4-(Cyclopropylamino)-2,2-dimethyl-cyclohexyl]-2-[4-([(4-methoxy-2,6-dimethyl-phenyl)sulfonyl]-methyl]-3,5-dimethyl-1 H-pyrazol-1-yl]-acetamid
CC-175 N-[4-(Cyclopropylamino)-2,2-dimethyl-cyclohexyl]-2-[4-[[(4-methoxy-2,6-dimethyl-phenyl)sulfonyl]-methyl]-1 H-[1,2,3]triazol-1-yl]-acetamid
CC-176 N-(3,3-Dimethyl-piperidin-4-yl)-2-[4-[[(4-methoxy-2,6-dimethyl-phenyl)sulfonyl]-methyl]-1 H-[1,2,3]triazol-1-yl]-acetamid
CC-177 N-[4-(Cyclopropylamino)-2,2-dimethyl-cyclohexyl]-2-[3-[[(4-methoxy-2,6-dimethyl-phenyl)sulfonyl]-methyl]-5-methyl-1 H-pyrazol-1-yl]-acetamid
CC-178 N-(2,2-Dimethyl-4-pyridin-4-yl-cyclohexyl)-2-[4-[[(4-methoxy-2,6-dimethyl-phenyl)sulfonyl]-methyl]-1 H-[1,2,3]triazol-1-yl]-acetamid
CC-179N-(1-Acetyl-3,3-dimethyl-piperidin-4-yl)-2-[4-[[(4-methoxy-2,6-dimethyl-phenyl)sulfonyl]-methyl]-3,5-dimethyl-1 H-pyrazol-1-yl]-acetamid
CC-180 N-(3,3-Dimethyl-piperidin-4-yl)-2-[4-[[(4-methoxy-2,6-dimethyl-phenyl)sulfonyl]-methyl]-3,5-dimethyl-1 H-pyrazol-1-yl]-acetamid
CC-181 N-(3,3-Dimethyl-piperidin-4-yl)-2-[4-[[(4-methoxy-2,6-dimethyl-phenyl)sulfonyl]-methyl]-1 H-[1,2,3]-triazol-1-yl]-N-methyl-acetamid
CC-1822-[4-[[(4-Methoxy-2,6-dimethyl-phenyl)sulfonyl]-methyl]-1H-[1,2,3]triazol-1-yl]-N-piperidin-4-yl-acetamid
CC-1832-[4-[[(4-Methoxy-2,6-dimethyl-phenyl)sulfonyl]-methyl]-1H-[1,2,3]triazol-1-yl]-N-(1,3,3-trimethyl-piperidin-4-yl)-acetamid
CC-1842-[4-[[(4-Methoxy-2,6-dimethyl-phenyl)sulfonyl]-methyl]-3,5-dimethyl-1H-pyrazol-1-yl]-N-(1,3,3-trimethyl-piperidin-4-yl)-acetamid
CC-185 N-(3,3-Dimethyl-piperidin-4-yl)-2-[4-[[(4-methoxy-2,6-dimethyl-phenyl)sulfonyl]-methyl]-3,5-dimethyl-1 H-pyrazol-1-yl]-N-methyl-acetamid
CC-186N-Cyclopropyl-N-(4-dimethylamino-cyclohexyl)-2-[4-[[(4-methoxy-2,6-dimethyl-phenyl)sulfonyl]-methyl]-1H-[1,2,3]triazol-1-yl]-acetamid
CC-1882-[3-[[(4-Methoxy-2,6-dimethyl-phenyl)sulfonyl]-methyl]-5-methyl-1H-pyrazol-1-yl]-N-(1,3,3-trimethyl-piperidin-4-yl)-acetamid
SC-01 3-[[(4-Methoxy-2,6-dimethyl-phenyl)sulfonyl]-methyl]-N-(1-pyridin-4-yl-piperidin-4-yl)-[1,2,4]-oxadiazol-5-carbonsäure amid
SC-02 [3-[[(4-Methoxy-2,6-dimethyl-phenyl)sulfonyl]-methyl]-[1,2,4]oxadiazol-5-yl]-(7-pyridin-4-yl-2,7-diazaspiro[3.5]nonan-2-yl)-methanon
SC-03 [3-[[(4-Methoxy-2,6-dimethyl-phenyl)sulfonyl]-methyl]-[1,2,4]oxadiazol-5-yl]-(3-pyridin-4-yloxy-pyrrolidin-1-yl)-methanon
SC-04 [3-[[(4-Methoxy-2,6-dimethyl-phenyl)sulfonyl]-methyl]-[1,2,4]oxadiazol-5-yl]-(8-pyridin-4-yl-3,8-diazaspiro[4.5]decan-3-yl)-methanon
SC-05 [3-[[(4-Methoxy-2,6-dimethyl-phenyl)sulfonyl]-methyl]-[1,2,4]oxadiazol-5-yl]-(8-pyridin-4-yl-3,8-diazaspiro[4.4]nonan-3-yl)-methanon
SC-06 3-[[(4-Methoxy-2,6-dimethyl-phenyl)sulfonyl]-methyl]-N-[1-[5-(trifluormethyl)-pyridin-2-yl]-pyrrolidin-3-yl]-[1,2,4]oxadiazol-5-carbonsäure amid
SC-07 [3-[[(4-Methoxy-2,6-dimethyl-phenyl)sulfonyl]-methyl]-[1,2,4]oxadiazol-5-yl]-[3-[(4-methyl-piperazin-1-yl)-methyl]-pyrrolidin-1-yl]-methanon
SC-08 [3-[[(4-Methoxy-2,6-dimethyl-phenyl)sulfonyl]-methyl]-[1,2,4]oxadiazol-5-yl]-[4-(pyridin-4-yl-methyl)-piperidin-1-yl]-methanon
SC-09 [3-[[(4-Methoxy-2,6-dimethyl-phenyl)sulfonyl]-methyl]-[1,2,4]oxadiazol-5-yl]-(9-pyridin-4-yloxy-3-azaspiro[5.5]undecan-3-yl)-methanon
SC-10 [3-[[(4-Methoxy-2,6-dimethyl-phenyl)sulfonyl]-methyl]-[1,2,4]oxadiazol-5-yl]-(9-pyridin-4-yl-3,9-diazaspiro[5.5]undecan-3-yl)-methanon
SC-11 2-[3-[[(4-Methoxy-2,6-dimethyl-phenyl)sulfonyl]-methyl]-[1,2,4]oxadiazol-5-yl]-1-(9-pyridin-4-yl-3,9-diazaspiro[5.5]undecan-3-yl)-ethanon
SC-12 N-(3,3-Dimethyl-1-pyridin-4-yl-piperidin-4-yl)-3-[[(4-methoxy-2,6-dimethyl-phenyl)sulfonyl]-methyl]-[1,2,4]oxadiazol-5-carbonsäure amid
SC-13 N-[2,2-Dimethyl-4-(4-methyl-piperazin-1-yl)-cyclohexyl]-2-[3-[[(4-methoxy-2,6-dimethyl-phenyl)-sulfonyl]-methyl]-[1,2,4]oxadiazol-5-yl]-acetamid
SC-14 N-(3,3-Dimethyl-1-pyridin-4-yl-piperidin-4-yl)-2-[3-[[(4-methoxy-2,6-dimethyl-phenyl)sulfonyl]-methyl]-[1,2,4]oxadiazol-5-yl]-acetamid
SC-16 N-[2,2-Dimethyl-4-(4-methyl-piperazin-1-yl)-cyclohexyl]-3-[[(4-methoxy-2,6-dimethyl-phenyl)-sulfonyl]-methyl]-[1,2,4]oxadiazol-5-carbonsäure amid
SC-18 3-[[(4-Methoxy-2,6-dimethyl-phenyl)sulfonyl]-methyl]-N-(4-pyridin-4-yl-cyclohexyl)-[1,2,4]oxa-diazol-5-carbonsäure amid
SC-19 3-[[(4-Methoxy-2,6-dimethyl-phenyl)sulfonyl]-methyl]-N-[4-(pyridin-4-yl-methyl)-cyclohexyl]-[1,2,4]oxadiazol-5-carbonsäure amid
SC-20 2-[3-[[(4-Methoxy-2,6-dimethyl-phenyl)sulfonyl]-methyl]-[1,2,4]oxadiazol-5-yl]-N-methyl-N-[1-(4-methyl-cyclohexyl)-piperidin-4-yl]-acetamid
SC-21 2-[3-[[(4-Methoxy-2,6-dimethyl-phenyl)sulfonyl]-methyl]-[1,2,4]oxadiazol-5-yl]-N-methyl-N-(1-tetrahydro-pyran-4-yl-piperidin-4-yl)-acetamid
SC-22 N-Cyclopropyl-2-[3-[[(4-methoxy-2,6-dimethyl-phenyl)sulfonyl]-methyl]-[1,2,4]oxadiazol-5-yl]-N-(1-pyridin-4-yl-piperidin-4-yl)-acetamid
SC-27 2-[3-[[(4-Methoxy-2,6-dimethyl-phenyl)sulfonyl]-methyl]-[1,2,4]oxadiazol-5-yl]-N-methyl-N-[1-(pyridin-4-carbonyl)-piperidin-4-yl]-acetamid
SC-30 3-[[(4-Methoxy-2,6-dimethyl-phenyl)sulfonyl]-methyl]-N-[(4-pyridin-4-yl-cyclohexyl)-methyl]-[1,2,4]oxadiazol-5-carbonsäure amid
SC-31 N-(2,2-Dimethyl-4-pyridin-4-yl-cyclohexyl)-3-[[(4-methoxy-2,6-dimethyl-phenyl)sulfonyl]-methyl]-[1,2,4]oxadiazol-5-carbonsäure amid
SC-32 N-(2,2-Dimethyl-4-pyridin-4-yl-cyclohexyl)-2-(3-[[(4-methoxy-2,6-dimethyl-phenyl)sulfonyl]-methyl]-[1,2,4]oxadiazol-5-yl]-acetamid
SC-33 2-[3-[[(4-Methoxy-2,6-dimethyl-phenyl)sulfonyl]-methyl]-[1,2,4]oxadiazol-5-yl]-N-methyl-N-(4,4,9-trimethyl-9-azaspiro[5.5]undecan-3-yl)-acetamid
SC-35 N-(4-Hydroxy-2,2-dimethyl-cyclohexyl)-2-[3-[[(4-methoxy-2,6-dimethyl-phenyl)sulfonyl]-methyl]-[1,2,4]oxadiazol-5-yl]-acetamid
SC-36 N-(3,3-Dimethyl-tetrahydro-pyran-4-yl)-2-[3-[[(4-methoxy-2,6-dimethyl-phenyl)sulfonyl]-methyl]-[1,2,4]oxadiazol-5-yl]-acetamid
SC-37 2-[3-[[(4-Methoxy-2,6-dimethyl-phenyl)sulfonyl]-methyl]-[1,2,4]oxadiazol-5-yl]-N-(4-pyridin-4-yl-cyclohexyl)-acetamid
SC-39 N-(3,3-Dimethyl-1-tetrahydro-pyran-4-yl-piperidin-4-yl)-2-[3-[[(4-methoxy-2,6-dimethyl-phenyl)-sulfonyl]-methyl]-[1,2,4]oxadiazol-5-yl]-acetamid
SC-40 2-[3-[[(4-Methoxy-2,6-dimethyl-phenyl)sulfonyl]-methyl]-[1,2,4]oxadiazol-5-yl]-N-[4-(2-methoxy-pyridin-4-yl)-2,2-dimethyl-cyclohexyl]-acetamid
SC-41 2-[3-[[(4-Methoxy-2,6-dimethyl-phenyl)sulfonyl]-methyl]-[1,2,4]oxadiazol-5-yl]-1-(4-pyridin-4-yl-piperidin-1-yl)-ethanon
SC-42 N-[1-(2,6-Dimethyl-pyrimidin-4-yl)-piperidin-4-yl]-2-[3-[[(4-methoxy-2,6-dimethyl-phenyl)sulfonyl]-methyl]-[1,2,4]oxadiazol-5-yl]-N-methyl-acetamid
SC-43 3-[[(4-Methoxy-2,6-dimethyl-phenyl)sulfonyl]-methyl]-N-methyl-N-[(1S,3R)-3-(4-methyl-piperazin-1-yl)-cyclohexyl]-[1,2,4]oxadiazol-5-carbonsäure amid
SC-44 2-[3-[[(4-Methoxy-2,6-dimethyl-phenyl)sulfonyl]-methyl]-[1,2,4]oxadiazol-5-yl]-N-methyl-N-[(1S,3R)-3-(4-methyl-piperazin-1-yl)-cyclohexyl]-acetamid
SC-46 2-[3-[[(4-Methoxy-2,6-dimethyl-phenyl)sulfonyl]-methyl]-[1,2,4]oxadiazol-5-yl]-N-methyl-N-[1-(3-piperidin-1-yl-propanoyl)-piperidin-4-yl]-acetamid
SC-48 N-[1-(4-Fluor-cyclohexyl)-3,3-dimethyl-piperidin-4-yl]-2-[3-[[(4-methoxy-2,6-dimethyl-phenyl)-sulfonyl]-methyl]-[1,2,4]oxadiazol-5-yl]-acetamid
SC-49 3-[[(2-Chlor-6-methyl-phenyl)sulfonyl]-methyl]-N-methyl-N-[(1S,3R)-3-(4-methyl-piperazin-1-yl)-cyclohexyl]-[1,2,4]oxadiazol-5-carbonsäure amid
SC-50 2-[3-[[(2-Chlor-6-methyl-phenyl)sulfonyl]-methyl]-[1,2,4]oxadiazol-5-yl]-N-methyl-N-[(1S,3R)-3-(4-methyl-piperazin-1-yl)-cyclohexyl]-acetamid
SC-51 2-[3-[[(4-Methoxy-2,6-dimethyl-phenyl)sulfonyl]-methyl]-[1,2,4]oxadiazol-5-yl]-1-[4-[(6-methoxy-pyridin-3-yl)-methyl]-piperazin-1-yl]-ethanon
SC-52 2-[3-[[(4-Methoxy-2,6-dimethyl-phenyl)sulfonyl]-methyl]-[1,2,4]oxadiazol-5-yl]-1-[4-[(2-pyrrolidin-1-yl-pyridin-4-yl)-methyl]-piperazin-1-yl]-ethanon
SC-55 2-[3-[[(4-Methoxy-2,6-dimethyl-phenyl)sulfonyl]-methyl]-[1,2,4]oxadiazol-5-yl]-N-[1-[(6-methoxy-pyridin-3-yl)-methyl]-piperidin-4-yl]-N-methyl-acetamid
SC-57 2-[3-[[(4-Methoxy-2,6-dimethyl-phenyl)sulfonyl]-methyl]-[1,2,4]oxadiazol-5-yl]-N-methyl-N-[1-[(2-pyrrolidin-1-yl-pyridin-4-yl)-methyl]-piperidin-4-yl]-acetamid
SC-58 2-[3-[[(4-Methoxy-2,6-dimethyl-phenyl)sulfonyl]-methyl]-[1,2,4]oxadiazol-5-yl]-N-methyl-N-[4-(4-methyl-piperazin-1-yl)-cyclohexyl]-acetamid
SC-59 [3-[[(4-Methoxy-2,6-dimethyl-phenyl)sulfonyl]-methyl]-[1,2,4]oxadiazol-5-yl]-[4-[(6-methoxy-pyridin-3-yl)-methyl]-piperazin-1-yl]-methanon
SC-60 [3-[[(4-Methoxy-2,6-dimethyl-phenyl)sulfonyl]-methyl]-[1,2,4]oxadiazol-5-yl]-[4-[(2-pyrrolidin-1-yl-pyridin-4-yl)-methyl]-piperazin-1-yl]-methanon
SC-61 [3-[[(4-Methoxy-2,6-dimethyl-phenyl)sulfonyl]-methyl]-[1,2,4]oxadiazol-5-yl]-[4-[(6-pyrrolidin-1-yl-pyridin-3-yl)-methyl]-piperazin-1-yl]-methanon
SC-62 2-[3-[[(4-Methoxy-2,6-dimethyl-phenyl)sulfonyl]-methyl]-[1,2,4]oxadiazol-5-yl]-1-[4-[(6-pyrrolidin-1-yl-pyridin-3-yl)-methyl]-piperazin-1-yl]-ethanon
SC-63 2-[3-[[(4-Methoxy-2,6-dimethyl-phenyl)sulfonyl]-methyl]-[1,2,4]oxadiazol-5-yl]-N-methyl-N-(1-chinazolin-4-yl-piperidin-4-yl)-acetamid
SC-64 2-[3-[[(4-Methoxy-2,6-dimethyl-phenyl)sulfonyl]-methyl]-[1,2,4]oxadiazol-5-yl]-N-[4-(4-methoxy-piperidin-1-yl)-cyclohexyl]-N-methyl-acetamid
SC-65 2-[3-[[(4-Methoxy-2,6-dimethyl-phenyl)sulfonyl]-methyl]-[1,2,4]oxadiazol-5-yl]-N-methyl-N-[1-[(6-pyrrolidin-1-yl-pyridin-3-yl)-methyl]-piperidin-4-yl]-acetamid
SC-66 N-[1-(2-Dimethylami no-pyrimidin-4-yl)-piperidin-4-yl]-2-[3-[[(4-methoxy-2,6-dimethyl-phenyl)-sulfonyl]-methyl]-[1,2,4]oxadiazol-5-yl]-N-methyl-acetamid
SC-67 N-[1-(2-Dimethylamino-pyridin-4-yl)-piperidin-4-yl]-2-[3-[[(4-methoxy-2,6-dimethyl-phenyl)-sulfonyl]-methyl]-[1,2,4]oxadiazol-5-yl]-N-methyl-acetamid
SC-68 N-[1-(2-Dimethylamino-pyrimidin-4-yl)-piperidin-4-yl]-3-[[(4-methoxy-2,6-dimethyl-phenyl)sulfonyl]-methyl]-N-methyl-[1,2,4]oxadiazol-5-carbonsäure amid
SC-69 N-[1-(2-Dimethylamino-pyridin-4-yl)-piperidin-4-yl]-3-[[(4-methoxy-2,6-dimethyl-phenyl)sulfonyl]-methyl]-N-methyl-[1,2,4]oxadiazol-5-carbonsäure amid
SC-70 N-[1-(3-Fluor-pyridin-4-yl)-piperidin-4-yl]-2-[3-[[(4-methoxy-2,6-dimethyl-phenyl)sulfonyl]-methyl]-[1,2,4]oxadiazol-5-yl]-N-methyl-acetamid
SC-72 2-[3-[[(4-Methoxy-2,6-dimethyl-phenyl)sulfonyl]-methyl]-[1,2,4]oxadiazol-5-yl]-N-methyl-N-[1-[(3-methyl-isoxazol-5-yl)-methyl]-piperidin-4-yl]-acetamid
SC-73 2-[3-[[(4-Methoxy-2,6-dimethyl-phenyl)sulfonyl]-methyl]-[1,2,4]oxadiazol-5-yl]-N-methyl-N-[1-[2-(4-methyl-piperazin-1-yl)-2-oxo-ethyl]-piperidin-4-yl]-acetamid
SC-77 2-[3-[[(4-Methoxy-2,6-dimethyl-phenyl)sulfonyl]-methyl]-[1,2,4]oxadiazol-5-yl]-N-[1-[(4-methoxy-3,5-dimethyl-pyridin-2-yl)-methyl]-piperidin-4-yl]-N-methyl-acetamid
SC-79 N-[1-(2-Isopropyl-6-methyl-pyrimidin-4-yl)-piperidin-4-yl]-2-[3-[[(4-methoxy-2,6-dimethyl-phenyl)sulfonyl]-methyl]-[1,2,4]oxadiazol-5-yl]-N-methyl-acetamid
SC-80 2-[3-[[(4-Methoxy-2,6-dimethyl-phenyl)sulfonyl]-methyl]-[1,2,4]oxadiazol-5-yl]-N-methyl-N-[1-(2-oxo-2-pyrrolidin-1-yl-ethyl)-piperidin-4-yl]-acetamid
SC-81 2-[3-[[(4-Methoxy-2,6-dimethyl-phenyl)sulfonyl]-methyl]-[1,2,4]oxadiazol-5-yl]-N-methyl-N-[1-(6-methyl-pyrimidin-4-yl)-piperidin-4-yl]-acetamid
SC-82 N-[1-(3-Fluor-pyridin-4-yl)-piperidin-4-yl]-3-[[(4-methoxy-2,6-dimethyl-phenyl)sulfonyl]-methyl]-N-methyl-[1,2,4]oxadiazol-5-carbonsäure amid
SC-83 2-[4-[[(4-Methoxy-2,6-dimethyl-phenyl)sulfonyl]-methyl]-3,5-dimethyl-1H-pyrazol-1-yl]-N-methyl-N-[(1S,3R)-3-(4-methyl-piperazin-1-yl)-cyclohexyl]-acetamid
SC-84 2-[4-[[(4-Methoxy-2,6-dimethyl-phenyl)sulfonyl]-methyl]-1H-[1,2,3]triazol-1-yl]-N-methyl-N-[(1S,3R)-3-(4-methyl-piperazin-1-yl)-cyclohexyl]-acetamid
SC-85 2-[3-[[(4-Methoxy-2,6-dimethyl-phenyl)sulfonyl]-methyl]-[1,2,4]oxadiazol-5-yl]-N-methyl-N-(2-oxo-1-pyridin-4-yl-piperidin-4-yl)-acetamid
SC-86 [5-[[(4-Methoxy-2,6-dimethyl-phenyl)sulfonyl]-methyl]-[1,3,4]oxadiazol-2-yl]-[3-[(4-methylpiperazin-1-yl)-methyl]-pyrrolidin-1-yl]-methanon
SC-87 N-(3,3-Dimethyl-piperidin-4-yl)-5-[[(4-methoxy-2,6-dimethyl-phenyl)sulfonyl]-methyl]-[1,3,4]-oxadiazol-2-carbonsäure amid
SC-88 [5-[[(4-Methoxy-2,6-dimethyl-phenyl)sulfonyl]-methyl]-[1,2,4]oxadiazol-3-yl]-[3-[(4-methyl-piperazin-1-yl)-methyl]-pyrrolidin-1-yl]-methanon
SC-89 5-[[(4-Methoxy-2,6-dimethyl-phenyl)sulfonyl]-methyl]-N-methyl-N-[(1S,3R)-3-(4-methyl-piperazin-1-yl)-cyclohexyl]-[1,2,4]oxadiazol-3-carbonsäure amid
SC-90 N-(3,3-Dimethyl-piperidin-4-yl)-5-[[(4-methoxy-2,6-dimethyl-phenyl)sulfonyl]-methyl]-[1,2,4]oxa-diazol-3-carbonsäure amid
SC-91 5-[[(4-Methoxy-2,6-dimethyl-phenyl)sulfonyl]-methyl]-N-methyl-N-[(1S,3R)-3-(4-methyl-piperazin-1-yl)-cyclohexyl]-[1,3,4]oxadiazol-2-carbonsäure amid
SC-92 2-[4-[[(4-Methoxy-2,6-dimethyl-phenyl)sulfonyl]-methyl]-3,5-dimethyl-1H-pyrazol-1-yl]-1-[3-[(4-methyl-piperazin-1-yl)-methyl]-pyrrolidin-1-yl]-ethanon
SC-93 2-[4-[[(4-Methoxy-2,6-dimethyl-phenyl)sulfonyl]-methyl]-1H-[1,2,3]triazol-1-yl]-1-[3-[(4-methyl-piperazin-1-yl)-methyl]-pyrrolidin-1-yl]-ethanon
jeweils
in Form der freien Verbindung, eines Tautomers oder eines N-Oxids;
in Form eines Racemates, eines individuellen Stereoisomers oder einer Mischung aus Stereoisomeren und/oder in Form eines Solvats und/oder eines physiologisch verträglichen Salzes.

Die oben verwendete Nummerierung der einzelnen Ausführungsformen der erfindungsgemäßen Verbindungen wird in den nachfolgenden Erläuterungen der vorliegenden Erfindung, insbesondere in der Beschreibung der Beispiele, beibehalten.

Gemäß einem Aspekt der vorliegenden Erfindung weisen die erfindungsgemäßen Verbindungen vorzugweise eine antagonistische Wirkung am humanen B1 R-Rezeptor oder dem B1 R-Rezeptor der Ratte auf. In einer bevorzugten Ausführungsform der Erfindung weisen die erfindungsgemäßen Verbindungen eine antagonistische Wirkung sowohl am humanen B1 R- Rezeptor (hB1 R) als auch am B1R-Rezeptor der Ratte (rB1 R) auf.

In einer bevorzugten Ausführungsform der vorliegenden Erfindung weisen die erfindungsgemäßen Verbindungen im FLIPR-Assay bei einer Konzentration von 10 µM am humanen B1 R-Rezeptor und/ oder am B1 R-Rezeptor der Ratte eine Inhibition von mindestens 15%, 25%, 50%, 70%, 80% oder 90 % auf. Ganz besonders bevorzugt sind Verbindungen, die eine Inhibition am humanen B1R-Rezeptor und am B1 R-Rezeptor der Ratte von mindestens 70%, insbesondere von mindestens 80% und insbesondere bevorzugt von mindestens 90% bei einer Konzentration von 10 µM aufweisen.

Die agonistische bzw. antagonistische Wirkung von Substanzen kann am Bradykinin Rezeptor 1 (B1R) der Spezies Mensch und Ratte mit ektopisch exprimierenden Zelllinien (CHO K1 Zellen) und mit Hilfe eines Ca²⁺-sensitiven Farbstoffs (Fluo-4) im Fluorescent Imaging Plate Reader (FLIPR) quantifiziert werden. Die Angabe in % Aktivierung wird bezogen auf das Ca²⁺-Signal nach Zugabe von Lys-Des-Arg⁹-Bradykinin (0,5 nM), bzw. Des-Arg⁹-Bradykinin (100 nM). Antagonisten führen zu einer Unterdrückung des Ca²⁺-Einstroms nach der Zugabe des Agonisten. Angegeben werden % Inhibition im Vergleich zu der maximal erreichbaren Inhibition.

Vorzugsweise wirken die erfindungsgemäßen Verbindungen beispielsweise auf den im Zusammenhang mit verschiedenen Erkrankungen relevanten B1R, so dass sie sich als pharmazeutischer Wirkstoff in Arzneimitteln eignen.

Ein weiterer Gegenstand der Erfindung sind daher Arzneimittel enthaltend wenigstens eine erfindungsgemäße Verbindung, sowie gegebenenfalls geeignete Zusatz- und/oder Hilfsstoffe und/ oder gegebenenfalls weitere Wirkstoffe.

Die erfindungsgemäßen Arzneimittel enthalten neben mindestens einer erfindungsgemäßen Verbindung gegebenenfalls geeignete Zusatz- und/ oder Hilfsstoffe, so auch Trägermaterialien, Füllstoffe, Lösungsmittel, Verdünnungsmittel, Farbstoffe und/ oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/ Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, nasal, buccal, rektal oder topisch, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen in einem Depot, in gelöster Form oder in einem Pflaster, gegebenenfalls unter Zusatz von die Haupenetration fördernden Mitteln, sind geeignete perkutane Applikationszubereitungen. Oral oder perkutan anwendbare Zubereitungsformen können die erfindungsgemäßen Verbindungen verzögert freisetzen. Die erfindungsgemäßen Verbindungen können auch in parenteralen Langzeitdepotformen wie z. B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, insbesondere 0,01 bis 5 mg/kg wenigstens einer erfindungsgemäßen Verbindung appliziert.

In einer Form des Arzneimittels liegt eine enthaltene erfindungsgemäße Verbindung ggf. als reines Diastereomer und/ oder Enantiomer, als Razemat oder als nicht-äquimolare oder äquimolare Mischung der Diastereomere und/ oder Enantiomere vor. Dem Fachmann sind geeignete Methoden zur Trennung von Stereoisomeren bekannt.

B1 R ist insbesondere am Schmerzgeschehen beteiligt. Entsprechend können die erfindungsgemäßen substituierten Verbindungen insbesondere zur Herstellung eines Arzneimittels zur Behandlung von Schmerz, insbesondere von akutem, viszeralem, neuropathischem oder chronischem Schmerz oder Entzündungsschmerz, verwendet werden.

Ein weiterer Gegenstand der Erfindung ist daher die Verwendung wenigstens einer erfindungsgemäßen Verbindung zur Herstellung eines Arzneimittels zur Behandlung von Schmerz, insbesondere von akutem, viszeralem, neuropathischem oder chronischem Schmerz. Eine bestimmte Ausführungsform der vorliegenden Erfindung ist die Verwendung zumindest einer der erfindungsgemäßen Verbindungen zur Herstellung eines Arzneimittels zur Behandlung von Entzündungsschmerz.

Gegenstand der Erfindung ist auch die Verwendung wenigstens einer erfindungsgemäßen Verbindung zur Behandlung von Schmerz, insbesondere von akutem, viszeralem, neuropathischem oder chronischem Schmerz oder Entzündungsschmerz.

Ein weiterer Gegenstand der Erfindung ist die Verwendung wenigstens einer erfindungsgemäßen Verbindung zur Herstellung eines Arzneimittels zur Behandlung von Diabetes, Erkrankungen der Atemwege, zum Beispiel Asthma bronchiale, Allergien, COPD/chronic-obstruktive pulmonary disease oder zystische Fibrose; entzündlichen Darmerkrankungen, beispielsweise Ulcerative Colitis oder CD/Crohn's disease; neurologischen Erkrankungen, beispielsweise Multiple Sklerose, neurodegenerativen Erkrankungen, fibrotischen Erkrankungen, Entzündungen der Haut, beispielsweise ätopische Dermatitis, Psoriasis oder bakterielle Infektionen; rheumatischen Erkrankungen, beispielsweise rheumatoide Arthritis oder Osteoarthritis; septischem Schock; Reperfusionssyndrom, zum Beispiel nach Herzinfarkt oder Schlaganfall, Fettleibigkeit; und als Angiognese-Inhibitor.

Gegenstand der Erfindung ist auch die Verwendung wenigstens einer erfindungsgemäßen Verbindung zur Behandlung einer der vorstehend genannten Indikationen.

Dabei kann es in einer der vorstehenden Verwendungen bevorzugt sein, wenn eine verwendete Verbindung als reines Diastereomer und/ oder Enantiomer, als Razemat oder als nicht-äquimolare oder äquimolare Mischung der Diastereomere und/ oder Enantiomere vorliegt.

Ein weiterer Gegenstand der Erfindung ist schließlciheine erfindungsgemäße Verbindung zur Behandlung von Schmerz, insbesondere von akutem Schmerz, viszeralem Schmerz, neuropathischen Schmerz, chronischen Schmerz oder Entzündungsschmerz; Migräne; Diabetes; Atemwegserkrankungen; entzündlichen Darmerkrankungen; neurologischen Erkrankungen; neurodegenerativen Erkrankungen, fibrotischen Erkrankungen, Entzündungen der Haut; rheumatischen Erkrankungen; septischen Schock; Reperfusionssyndrom; Fettleibigkeit und/oder Angiogenese.

Ein weiterer Gegenstand der Erfindung ist ein Verfahren zur Behandlung, insbesondere in einer der vorgenannten Indikationen, eines nichthumanen Säugetieres oder Menschen, das oder der eine Behandlung der entsprechenden Indikation benötigt, durch Verabreichung einer therapeutisch wirksamen Dosis einer erfindungsgemäßen Verbindung oder eines erfindungsgemäßen Arzneimittels.

Ein weiterer Gegenstand der Erfindung ist ein Verfahren zur Behandlung von Schmerzen, insbesondere einer der vorgenannten Schmerzformen, eines nichthumanen Säugetieres oder Menschen, das oder der eine Behandlung insbesondere von Schmerzen, insbesondere von akuten, viszeralen, neuropathischen oder chronischem Schmerzen oder Entzündungsschmerzen benötigt, durch Verabreichung einer therapeutisch wirksamen Dosis einer erfindungsgemäßen Verbindung, oder eines erfindungsgemäßen Arzneimittels.

Ein weiterer Gegenstand der Erfindung ist ein Verfahren zur Herstellung der erfindungsgemäßen Verbindungen wie in der Beschreibung sowie den Beispielen ausgeführt.

### Beispiele:

### Abkürzungsverzeichnis

äq = Aquivalent(e); BINAP = (2,2-Bis(diphenylphosphino)-1,1'-binaphthyl); Boc = *tert*.-Butyloxycarbonyl; Cbz = Benzyloxycarbonyl; d = Tag(e); DAST = Diethylaminosulfur trifluorid; DC = Dünnschichtchromatographie; DCM = Dichlormethan; DIBAL-H = Diisobutylaluminiumhydrid; DIPEA = Diisopropylethylamin; DME = 1,2-Dimethoxyethan; DMF = N,N-Dimethylformamid; DMS = Dimethyl-sulfid; DMSO = Dimethylsulfoxid; EDCI = N-(3-Dimethylaminopropyl)-N'-ethylcarbodiimid Hydrochlorid; ges. = gesättigt(e); h = Stunde(n); HATU = O-(7-Azabenzotriazol-1-yl)N,N,N',N'-tetramethyluronium hexafluorphosphat; HOBt = 1-Hydroxybenzotriazol; konz. = konzentriert(e); LAH = Lithiumaluminiumhydrid; MeOH = Methanol; min = Minute(n); M = Molar; org. = organisch(e); PCC = Pyridiniumchlorochromat; PTSA = p-Toluolsulfonsäure; R.t. = Retentionszeit; RT = Raumtemperatur; TEA = Triethylamin; TFA = Trifluoressigsäure; THF = Tetrahydrofuran; TOSMIC = p-Toluolsulfonylmethylisocyanid; Wt-% = Gewichtsprozent(e); Xantphos = 4,5-Bis(diphenylphosphino)-9,9-dimethylxanthen

Die eingesetzten Chemikalien und Lösungsmittel wurden kommerziell bei den herkömmlichen Anbietern (Acros, Aldrich, Fluka, Lancaster, Maybridge, TCI, Fluorochem, Tyger, ABCR, Fulcrum, FrontierScientific, Milestone etc.) bezogen. Die Reaktionen wurden z.T. unter Inertgas (Stickstoff) durchgeführt. Die Ausbeuten der hergestellten Verbindungen sind nicht optimiert. Die Mischungsverhältnisse von Lösungsmitteln sind stets im Verhältnis Volumen / Volumen angegeben.

Eingesetzte Mengenäquivalente der Reagenzien, sowie Lösungsmittelmengen, Reaktionstemperaturen und -zeiten können bei unterschiedlichen Reaktionen, die nach derselben Methode durchgeführt wurden, leicht variieren. Aufarbeitungs- und Aufreinigungsmethoden wurden den charakteristischen Eigenschaften der Verbindungen entsprechend angepasst.

### A. Synthese der Carbonsäure bzw. -ester-Bausteine (ACI)

### Synthese des Bausteins ACI-1:

### Ethyl 3-(((4-methoxy-2,6-dimethylphenyl)sulfonyl)methyl)-1,2,4-oxadiazole-5-carboxylate (ACI-1)

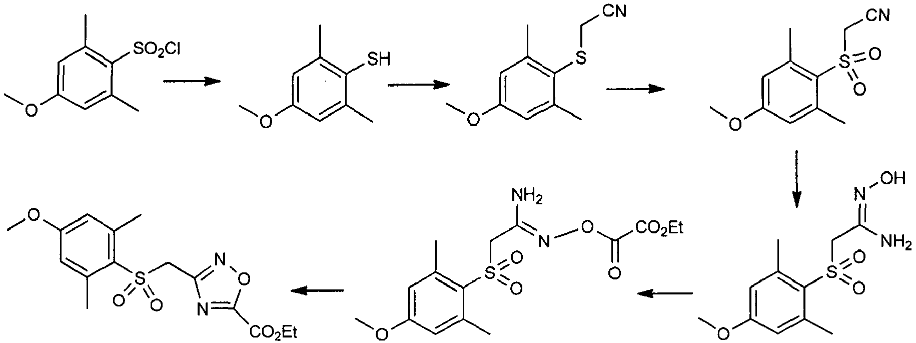

**Stufe 1:** Eine Lösung aus 4-Methoxy-2,6-dimethylbenzol-1-sulfonyl chlorid (91,88 mmol, 1,0 äq) in DCM (100 ml) wurde bei 0°C tropfenweise zu einer Lösung auf Triphenylphosphin (367,5 mmol, 4,0 äq) in DCM (100 ml) und DMF (6 ml) gegeben und 2 h bei 25°C gerührt. 1 M HCl (150 ml) wurde zugegeben und das Gemisch 1 h bei 25°C gerührt. Die org. Phase wurde abgetrennt und unter vermindertem Druck konzentriert. Aus dem Rückstand wurde mit Hexan ein Feststoff ausgefällt und abfiltriert. Das Filtrat wurde unter vermindertem Druck konzentriert, der Rückstand in Diethylether (500 ml) gelöst und mit 2 M NaOH (2 x 250 ml) gewaschen. Die vereinigten wässrigen Phasen wurden mit konz. HCl auf pH 2 eingestellt und mit Diethylether (1000 ml) extrahiert. Die org. Phase wurde mit Wasser (2 x 250 ml) und ges. NaCl-Lösung (2 x 250 ml) gewaschen, über Natriumsulfat getrocknet und unter vermindertem Druck konzentriert. Ausbeute: 51 %.

**Stufe 2:** Zu einer Suspension aus NaH (51,06 mmol, 1,1 äq, 60 % in Mineralöl) in THF (150 ml) wurde bei 0°C tropfenweise eine Lösung aus 4-Methoxy-2,6-dimethylbenzolthiol (46,42 mmol, 1,0 äq) in THF (25 ml) gegeben und 30 min bei dieser Temperatur gerührt. Bei 0°C wurde eine Lösung aus Bromacetonitril (55,7 mmol, 1,2 äq) in THF (25 ml) zugegeben und 1 h bei 25°C gerührt. Anschließend wurde Wasser (100 ml) zugegeben und mit Ethylacetat (600 ml) extrahiert. Die org. Phase wurde mit Wasser (2 x 150 ml) und ges. NaCl-Lösung (2 x 150 ml) gewaschen, über Natriumsulfat getrocknet und unter vermindertem Druck konzentriert. Der Rückstand wurde säulenchromatographisch (4 % Ethylacetat in Hexan) gereinigt. Ausbeute: 71 %.

**Stufe 3:** 2-((4-Methoxy-2,6-dimethylphenyl)thio)acetonitril (33,33 mmol, 1,0 äq) wurde zu einer Mischung aus Essigsäure:Wasser:Ethanol (2:2:3, 35 ml) gegeben und 10 min gerührt. Die Reaktionsmischung wurde auf 0°C gekühlt und Oxon (66,66 mmol. 2,0 äq) wurde portionsweise zugegeben und die Mischung dann 16 h bei RT gerührt. Das Gemisch wurde mit DCM (500 ml) verdünnt, mit Wasser (2 x 100 ml) und ges. NaCl-Lösung (2 x 100 ml) gewaschen, über Natriumsulfat getrocknet und unter vermindertem Druck konzentriert. Der Rückstand wurde ohne weitere Aufreinigung in den nächsten Schritt eingesetzt. Ausbeute: 80 %.

**Stufe 4:** Hydroxylamine HCl (18,9 mmol, 1,5 äq) und TEA (2,0 äq) wurden in 1,4-Dioxan (30 ml) gelöst und 15 min bei 25°C gerührt. Anschließend wurde eine Lösung aus 2-((4-Methoxy-2,6-dimethylphenyl)-sulfonyl)acetonitril (12,6 mmol, 1,0 äq) in 1,4-Dioxan (15 ml) zugegeben und 14 h auf Siedetemperatur erhitzt. Der entstandene Feststoff wurde abfiltriert und das Filtrat konzentriert. Der Rückstand wurde ohne weitere Aufreinigung in den nächsten Schritt eingesetzt. Ausbeute: 79 %.

**Stufe 5:** Zu einer Lösung aus (Z)-N'-Hydroxy-2-((4-methoxy-2,6-dimethylphenyl)sulfonyl)acetimidamid (3,68 mmol, 1,0 äq) und TEA (2,0 äq) in DCM (15 ml) wurde bei 0°C Chloroxoessigsäure-ethylester (7,68 mmol, 2,0 äq) tropfenweise zugegeben. Anschließend wurde auf 25°C erwärmt und 20 min gerührt. Die Reaktionslösung wurde mit DCM (100 ml) verdünnt, mit Wasser (25 ml) und ges. NaCl-Lösung (25 ml) gewaschen, über Natriumsulfat getrocknet und unter vermindertem Druck konzentriert. Das Rohprodukt wurde ohne weitere Aufreinigung in die nächste Stufe eingesetzt. Ausbeute: 72 %.

**Stufe 6:** (Z)-Ethyl 2-(((1-amino-2-((4-methoxy-2,6-dimethylphenyl)sulfonyl)ethylidene)amino)oxy)-2-oxoacetat (1,0 g, 2,688 mmol) wurde in Toluol (10 ml) gelöst und 24 h auf Siedetemperatur erhitzt. Das Lösungsmittel wurde unter vermindertem Druck eingeengt, der Rückstand in Ethylacetat (200 ml) aufgenommen, mit Wasser und ges. NaCl-Lösung (je 50 ml) gewaschen, über Natriumsulfat getrocknet und unter vermindertem Druck konzentriert. Nach säulenchromatographischer Aufreinigung (Kieselgel, 20% Ethylacetat in Hexan) erhielt man einen weißen Feststoff. Ausbeute: 52 %.

### Synthese des Bausteins ACI-2:

### 2-(3-((4-Methoxy-2,6-dimethylphenylsulfonyl)methyl)-1,2,4-oxadiazol-5-yl)essigsäure (ACI-2)

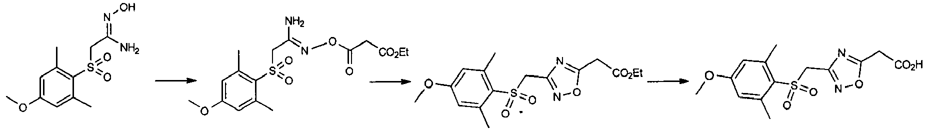

**Stufe 1:** TEA (2.1 ml, 14.7 mmol, 2.0 äq.) wurde zu einer Lösung aus (Z)-N'-Hydroxy-2-((4-methoxy-2,6-dimethylphenyl)sulfonyl)acetimidamid [Stufe 4, ACI-1] (2.0 g, 7.3 mmol, 1.0 äq) in trockenem DCM (30 ml) bei 0°C zugegeben. Dann wurde Malonsäure-monoethylesterchlorid (2.2 g, 14.7 mmol, 2.0 äq.) zum Reaktionsgemisch getropft. Das Reaktionsgemisch wurde auf RT erwärmt und 2 h gerührt. Das Reaktionsgemisch wurde anschließend mit DCM (50 ml) verdünnt, mit Wasser (50 ml) und ges. NaCl-Lösung (25 ml) gewaschen und über Natriumsulfat getrocknet. Das Lösungsmittel wurde unter vermindertem Druck konzentriert und das Rohprodukt ohne weitere Aufreinigung in die nächste Stufe eingesetzt. Ausbeute: 74%.

**Stufe 2:** (Z)-Ethyl 3-(((1-amino-2-((4-methoxy-2,6-dimethylphenyl)sulfonyl)ethyliden)amino)oxy)-3-oxo-propanoat (2.0 g, 5.18 mmol, 1.0 äq.) wurde in trockenem Toluol (20 ml) gelöst und 16 h refluxiert. Nach vollständiger Umsetzung wurde das Lösungsmittel unter vermindertem Druck konzentriert. Der Rückstand wurde säulenchromatographisch (Kieselgel, 50% DCM/Hexan) aufgereinigt. Ausbeute: 42%.

**Stufe 3:** Eine 1 N Lösung LiOH·H₂O (3 ml) wurde bei 0°C zu Ethyl 2-(3-(((4-methoxy-2,6-dimethylphenyl)-sulfonyl)methyl)-1,2,4-oxadiazol-5-yl)acetat (500 mg, 1.35 mmol, 1.0 äq.), gelöst in MeOH (5 ml), gegeben und das Reaktionsgemisch 3 h bei RT gerührt. Das MeOH wurde unter vermindertem Druck konzentriert, die zurückbleibende wässrige Phase mit Wasser verdünnt (20 ml) und mit Ethylacetat (2 x 20 ml) gewaschen. Die wässrige Phase wurde mit 2 N HCl sauer gestellt (pH2) und mit Ethylacetat gewaschen (2 x 20 ml). Die vereinigten organischen Phasen wurden über Natriumsulfat getrocknet und unter vermindertem Druck aufkonzentriert. Ausbeute: 65%.

### Synthese des Bausteins ACI-3:

### Ethyl 3-(((2-chlor-6-methylphenyl)sulfonyl)methyl)-1,2,4-oxadiazol-5-carboxylat (ACI-3)

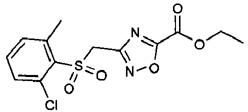

Die Synthese erfolgte in Analogie zu ACI-1, wobei in Stufe 4 TEA/DCM durch K₂CO₃/THF ersetzt wurde.

### Synthese des Bausteins ACI-4:

### 2-(4-(((4-Methoxy-2,6-dimethylphenyl)sulfonyl)methyl)-3,5-dimethyl-1H-pyrazol-1-yl)essigsäure (ACI-4)

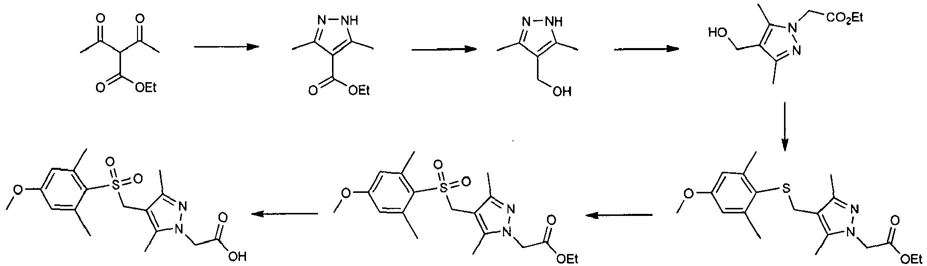

Stufe 1: Zu einer Lösung aus Ethyl 2-acetyl-3-oxobutanoat (10,0 g, 58,13 mmol, 1,0 äq) in Ethanol (100 ml) wurde bei 0°C Hydrazin hydrat (5,8 g, 116,27 mmol, 2,0 äq) gegeben und das Gemisch 20 h bei RT gerührt. Anschließend wurde das Reaktionsgemisch konzentriert, in Wasser (100 ml) aufgenommen und mit Ethylacetat (2 x 150 ml) extrahiert. Die vereinigten organischen Phasen wurden mit ges. NaCl-Lösung (100 ml) extrahiert, und über Natriumsulfat getrocknet. Das Lösungsmittel wurde unter vermindertem Druck konzentriert und Hexan (2 x 50 ml) wurde hinzugegeben. Ausbeute: 56 %.

**Stufe 2:** Eine Lösung aus Ethyl 3,5-dimethyl-1H-pyrazol-4-carboxylat (5,5 g, 32,73 mmol, 1,0 äq) in THF (25 ml) wurde zu einer Suspension aus LAH (2,48 g, 65,47 mmol, 2,0 äq) in THF (50 ml) bei 0°C zugetropft und das Reaktonsgemisch anschließend 16 h bei RT gerührt. Zu dem Gemisch wurde gesättigte Na₂SO₄ Lösung gegeben, dann wurde über Celite filtriert und mit THF (100 ml) gewaschen. Das Filtrat wurde unter vermindertem Druck konzentriert und Diethylether hinzugegeben, um das gewünschte Produkt als weissen Feststoff zu erhalten. Ausbeute: 60%.

**Stufe 3:** Zu einem Gemisch aus (3,5-Dimethyl-1H-pyrazol-4-yl)methanol (2,5 g 19,84 mmol,1,0 äq) und K₂CO₃ (4,9 g, 29,76 mmol, 1,5 äq) in Aceton (20 ml) wurde eine Lösung aus Ethylbromacetat (2,7 g, 19,84 mmol, 1,0 äq) in Aceton (15 ml) zugetropft und das Gemisch anschließend 16 h refluxiert. Dann wurde das Reaktionsgemisch auf RT gekühlt und konzentriert. Der Rückstand wurde in Ethylacetat (100 ml) aufgenommen, mit Wasser (50 ml) und ges. NaCl Lösung (50 ml) gewaschen und über Na₂SO₄ getrocknet. Dann wurde unter vermindertem Druck konzentriert und das Roprodukt säulenchromatographisch gereinigt (Kieselgel; 2% MeOH/DCM). Ausbeute: 31 %.

**Stufe 4:** Zu einer Lösung aus Ethyl 2-(4-(hydroxymethyl)-3,5-dimethyl-1H-pyrazol-1-yl)acetat (1,3 g, 6,132 mmol, 1,0 äq) in THF (50 ml) wurde eine Lösung aus 4-Methoxy-2,6-dimethylbenzothiol [Stufe 1, **ACI-1**] (1,03 g, 6,132 mmol, 1,0 äq) in THF (10 ml) gegeben, gefolgt von PTSA (233 mg, 1,22 mmol, 0,2 äq) und das Reaktonsgemisch anschließend 16 h refluxiert. Dann wurde das Gemisch auf RT abgekühlt, mit Ethylacetat (100 ml) verdünnt und mit Wasser (50 ml) und ges. NaCl-Lösung (50 ml) extrahiert. Es wurde über Natriumsulfat getrocknet, das Lösungsmittel unter vermindertem Druck konzentriert und Hexan (2 x 50 ml) wurde hinzugegeben. Ausbeute: 67%.

**Stufe 5:** Ethyl 2-(4-(((4-methoxy-2,6-dimethylphenyl)thio)methyl)-3,5-dimethyl-1H-pyrazol-1-yl)acetat (1,5 g, 4,14 mmol, 1,0 äq) wurde zu einer Mischung aus Essigsäure:Wasser:Ethanol (2:2:3, 21 ml) gegeben und 10 min gerührt. Die Reaktionsmischung wurde auf 0°C gekühlt, Oxon (5,0 g, 8,28 mmol, 2,0 äq) wurde portionsweise zugegeben und die Mischung dann 2 h bei RT gerührt. Das Gemisch wurde mit Ethylacetat (100 ml) verdünnt, mit Wasser (2 x 50 ml) und ges. NaCl-Lösung (50 ml) gewaschen, über Natriumsulfat getrocknet und unter vermindertem Druck konzentriert. Der Rückstand wurde ohne weitere Aufreinigung in den nächsten Schritt eingesetzt. Ausbeute 91 %.

**Stufe 6:** Ethyl 2-(4-(((4-methoxy-2,6-dimethylphenyl)suifonyl)methyl)-3,5-dimethyl-1H-pyrazol-1-yl)acetat (1,5 g, 3,80 mmol, 1,0 äq) wurde in einer Mischung aus THF und MeOH (1:1, 30 ml) gelöst. Eine Lösung aus LiOH·H₂O (0,497 g, 11,42 mmol, 3,0 äq) in Wasser (15 ml) wurde bei RT zugegeben und die Mischung 1 h gerührt. Nach DC-Kontrolle wurde die Reaktionslösung konzentriert, der Rückstand in Wasser (50 ml) aufgenommen und mit ges. NaHSO₄ Lösung sauer gestellt. Dann wurde mit Ethylacetat (2 x 50 ml) extrahiert. Die vereinigten org. Phasen wurden mit Wasser (50 ml) und ges. NaCl Lösung (50 ml) gewaschen, über Natriumsulfat getrocknet und unter vermindertem Druck bis zur Trockene konzentriert. Ausbeute: 94%.

### Synthese des Bausteins ACI-5:

### Ethyl 2-(4-(((4-methoxy-2,6-dimethylphenyl)sulfonyl)methyl)-1H-1,2,3-triazol-1-yl)acetat (ACI-5)

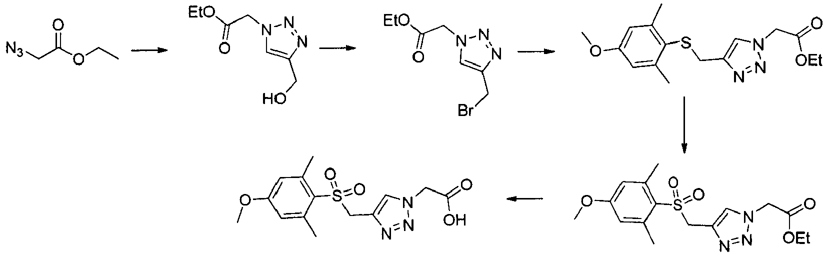

Stufe 1: Zu einer Suspension aus Cul auf Amberlyst A-21 (0,441 g, 2,325 mmol, 0,10 äq) in DCM (20 ml) wurde Ethyl 2-azidoacetat (3,0 g, 23,25 mmol, 1,0 äq) gegeben und das Gemisch auf 5°C gekühlt. Eine Lösung Propargylalkohol (1,23 g, 22,08 mmol, 0,95 äq) in DCM (20 ml) wurde hinzugetropfet und das Reaktionsgemisch 12 h bei RT gerührt. Das Gemisch wurde anschließend filtriert und das Filtrat und unter vermindertem Druck konzentriert. Das Rohprodukt wurde säulenchromatographisch (Kieselgel, 2% MeOH / DCM) gereinigt. Ausbeute: 58%.

**Stufe 2:** Zu einer Lösung aus Ethyl 2-(4-(hydroxymethyl)-1H-1,2,3-triazol-1-yl)acetat (2,5 g, 13,51 mmol, 1,0 äq) in DCM (100 ml) wurde bei 0°C PBr₃ (1,46 g, 5,4 mmol, 0,4 äq) gegeben und das Reaktionsgemisch 2 h bei RT gerührt. Zu dem Gemisch wurde Wasser (50 ml) und DCM (100 ml) hinzugegeben und die Phasen getrennt. Die wässrige Phase wurde mit DCM (100 ml) extrahiert und die vereinigten organischen Phasen mit ges. NaCl-Lösung (80 ml) gewaschen. Die organische Phase wurde über Natriumsulfat getrocknet und unter vermindertem Druck konzentriert. Ausbeute: 57%.

**Stufe 3:** Zu einer Suspension aus NaH (337 mg, 8,426 mmol, 1,1 äq) in THF (20 ml) wurde bei 0°C eine Lösung aus 4-Methoxy-2,6-dimethylbenzothiol (1,415 g, 8,426 mmol, 1,1 äq) in THF (10 ml) gegeben und das Reaktionsgemisch 30 min bei RT gerührt. Eine Lösung aus Ethyl 2-(4-(bromomethyl)-1 H-1,2,3-triazol-1-yl)-acetat (1,9 g, 7,66 mmol) in THF (10 ml) wurde bei 0°C hinzugegeben und das Gemisch 2 h bei RT gerührt. Zu dem Gemisch wurde Wasser (80 ml) hinzugegeben und anschließend mit Ethylacetat (2 x 100 ml) extrahiert. Die vereinigten organischen Phasen wurden mit ges. NaCl-Lösung (80 ml) gewaschen, über Natriumsulfat getrocknet und unter vermindertem Druck konzentriert. Das Rohprodukt wurde säulenchromatographisch (Kieselgel, 50% Ethylacetat / Hexan) gereinigt. Ausbeute: 70%.

**Stufe 4:** Ethyl 2-(4-(((4-methoxy-2,6-dimethylphenyl)thio)methyl)-1H-1,2,3-triazol-1-yl)acetat (1,8 g, 5,37 mmol, 1,0 äq) wurde zu einer Mischung aus Essigsäure:Wasser:Ethanol (2:2:3, 28 ml) gegeben und 10 min gerührt. Die Reaktionsmischung wurde auf 0°C gekühlt, Oxon (6,6 g, 10,74 mmol, 2,0 äq) wurde portionsweise zugegeben und die Mischung dann 16 h bei RT gerührt. Das Gemisch wurde mit DCM (200 ml) verdünnt, mit Wasser (80 ml) und ges. NaCl-Lösung (80 ml) gewaschen, über Natriumsulfat getrocknet und unter vermindertem Druck konzentriert. Der Rückstand wurde ohne weitere Aufreinigung in den nächsten Schritt eingesetzt. Ausbeute 76%.

**Stufe 5:** Ethyl 2-(4-(((4-methoxy-2,6-dimethylphenyl)sulfonyl)methyl)-1H-1,2,3-triazol-1-yl)acetat (400 mg, 1,09 mmol, 1.0 äq) wurde in einer Mischung aus THF und MeOH (1:1, 10 ml) gelöst. Eine Lösung aus LiOH·H₂O (137 mg, 3,26 mmol 3,0 äq) in Wasser (2 ml) wurde bei RT zugegeben und die Mischung 2 h gerührt. Nach DC-Kontrolle wurde die Reaktionslösung konzentriert, der Rückstand in Wasser (20 ml) aufgenommen und mit NaHSO₄ Lösung (pH 2-3) sauer gestellt. Dann wurde mit DCM (2 x 50 ml) extrahiert. Die vereinigten org. Phasen wurden mit Wasser (40 ml) und ges. NaCl Lösung (40 ml) gewaschen, über Natriumsulfat getrocknet und unter vermindertem Druck bis zur Trockene konzentriert. Ausbeute: 84%.

### Synthese des Bausteins ACI-6:

### Ethyl 5-(((4-methoxy-2,6-dimethylphenyl)sulfonyl)methyl)-1,2,4-oxadiazol-3-carboxylat (ACI-6)

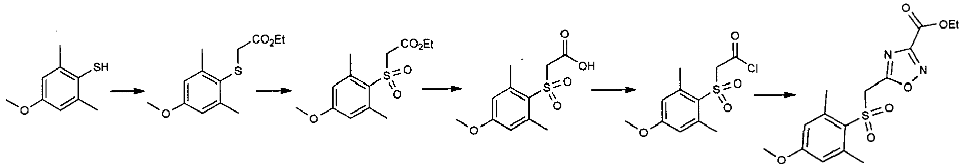

**Stufe 1:** Zu einer Suspension aus NaH (2,86 g, 71,9 mmol, 1,1 äq, 60 % in Mineralöl) in THF (150 ml) wurde eine Lösung aus 4-Methoxy-2,6-dimethylbenzothiol (11,0 g, 65,48 mmol, 1,0 äq) in THF (50 ml) bei 0 °C hinzugetropft und das resultierende Gemisch 30 min bei 0°C gerührt. Eine Lösung aus Ethyl bromacetat (13,12 g, 78,57 mmol, 1,2 äq) in THF (50 ml) wurde anschließend bei 0°C zu dem Reaktionsgemisch gegeben und es wurde 1 h bei RT erührt. Zu dem Gemisch wurde Eis-Wasser (100 ml) hinzugegeben und es wurde mit Ethylacetat (2 x 300 ml) extrahiert. Die organischen Phasen wurden mit Wasser (2 x 150 ml) und ges. NaCl-Lösung (2 x 150 ml) gewaschen, über Natriumsulfat getrocknet und unter vermindertem Druck konzentriert. Das Rohprodukt wurde säulenchromatographisch (Kieselgel, 5% Ethylacetat / Hexan) gereinigt. Ausbeute: 55%.

**Stufe 2:** Ethyl 2-((4-methoxy-2,6-dimethylphenyl)thio)acetat (9,10 g, 35,83 mmol, 1,0 äq) wurde zu einer Mischung aus Essigsäure:Wasser:Ethanol (2:2:3, 70 ml) gegeben und 10 min gerührt. Die Reaktionsmischung wurde auf 0°C gekühlt, Oxon (44,0 g, 71,66 mmol, 2,0 äq) wurde portionsweise zugegeben und die Mischung dann 16 h bei RT gerührt. Das Gemisch wurde mit DCM (300 ml) verdünnt, mit Wasser (2 x 100 ml) und ges. NaCl-Lösung (2 x 100 ml) gewaschen, über Natriumsulfat getrocknet und unter vermindertem Druck konzentriert. Das Rohprodukt wurde anschließend säulenchromatographisch (Kieselgel, 10% Ethylacetat / Hexan) aufgereinigt. Ausbeute: 78%.

**Stufe 3:** Ethyl 2-((4-methoxy-2,6-dimethylphenyl)sulfonyl)acetat (2,0 g, 6,99 mmol, 1,0 äq) wurde in einer Mischung aus THF und MeOH (1:1, 40 ml) gelöst. Eine Lösung aus LiOH·H₂O (881 mg, 20,97 mmol, 3,0 äq) in Wasser (10 ml) wurde bei RT zugegeben und die Mischung 4 h gerührt. Nach DC-Kontrolle wurde die Reaktionslösung konzentriert, der Rückstand in Wasser (50 ml) aufgenommen und mit 1 N HCl Lösung (pH 4) sauer gestellt. Dann wurde mit DCM (2 x 100 ml) extrahiert. Die vereinigten org. Phasen wurden mit ges. NaCl Lösung (100 ml) gewaschen, über Natriumsulfat getrocknet und unter vermindertem Druck bis zur Trockene konzentriert. Ausbeute: 66%.

**Stufe 4 & 5:** Oxalyl chlorid (0,996 ml, 11,61 mmol, 3,0 äq) wurde bei 0°C zu einer Lösung aus 2-((4-Methoxy-2,6-dimethylphenyl)sulfonyl)essigsäure (1,0 g, 3,87 mmol, 1,0 äq) in DCM (20 ml) hinzugetropft und das resultierende Gemisch 2 h bei RT gerührt. Das Reakionsgemisch wurde unter vermindertem Druck konzentriert (unter Inertgas). Das so erhaltene Säurechlorid wurde in THF (20 ml) gelöst und Ethyl 2-amino-2-(hydroxyimino)acetat (562 mg, 4,26 mmol, 1,1 äq) wurde hinzugegeben. Das Reaktonsgemisch wurde anschließend 2 h bei RT gerührt. Dann wurde die Reaktionslösung konzentriert und der Rückstand in 1,4-Dioxan (20 ml) aufgenommen. Molsieb (1,6 g) wurde hinzugegeben und das Gemisch 48 h refluxiert. Anschließend wurde filtriert, das Filtrat unter vermindertem Druck konzentriert und der Rückstand säulenchromatographisch (Kieselgel, 25% Ethylacetat / Hexan) gereinigt. Ausbeute: 36%.

### Synthese des Bausteins ACI-7:

### Ethyl 5-(((4-methoxy-2,6-dimethylphenyl)sulfonyl)methyl)-1,3,4-oxadiazol-2-carboxylat (ACI-7)

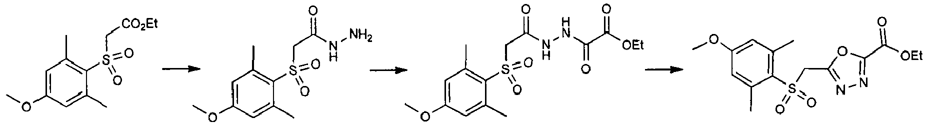

**Stufe 1:** Ein Gemisch aus Hydrazine hydrat (1,04 g, 20,80 mmol, 1,5 äq) und Ethyl 2-((4-methoxy-2,6-dimethylphenyl)sulfonyl)acetat (Stufe 3, ACI-6) (4,0 g, 13,98 mmol, 1,0 äq) in EtOH (40 ml) wurde 14 h refluxiert. Nach DC-Kontrolle wurde die Reaktionslösung konzentriert und das Rohprodukt ohne weitere Aufreinigung in den nächsten Schritt eingesetzt. Ausbeute: 67%.

**Stufe 2:** Ethyl oxalyl chlorid (3,8 g, 27,84 mmol, 2,0 äq) wurde bei -10°C zu einem Gemisch aus 2-((4-Methoxy-2,6-dimethylphenyl)sulfonyl)acetohydrazid (3,80 g, 13,97 mmol, 1,0 äq) und K₂CO₃ (7,7 g, 55,88 mmol, 4,0 äq) in THF (30 ml) gegeben und und das resultierende Gemisch 30 min bei -10°C gerührt. Nach DC-Kontrolle wurde mit Ethylacetat (100 ml) verdünnt, mit Wasser (60 ml) und ges. NaCl-Lösung (60 ml) gewaschen, über Natriumsulfat getrocknet und unter vermindertem Druck konzentriert. Der Rückstand wurde ohne weitere Aufreinigung in den nächsten Schritt eingesetzt. Ausbeute: 54%.

**Stufe 3:** Zu einer Lösung aus Ethyl 2-(2-(2-((4-methoxy-2,6-dimethylphenyl)sulfonyl)acetyl)hydrazinyl)-2-oxoacetat (1,0 g, 2,69 mmol, 1,0 äq) und TEA (0,75 ml, 5,38 mmol, 2,0 äq) in DCM (20 ml) wurde bei 0°C eine Lösung aus p-Toluolsulfonyl chlorid (614 mg, 3,23 mmol, 1,2 äq) in DCM (5 ml) hinzugegeben und das resultierende Gemisch 3 h bei RT gerührt. Nach DC-Kontrolle wurde mit DCM (50 ml) verdünnt, mit NH₄Cl Lösung (30 ml), Wasser (30 ml) und ges. NaCl-Lösung (30 ml) gewaschen, über Natriumsulfat getrocknet und unter vermindertem Druck konzentriert. Das Rohprodukt wurde säulenchromatographisch (Kieselgel, 40% Ethylacetat / Hexan) gereinigt. Ausbeute: 84%.

### Synthese des Bausteins ACI-8:

### 2-(3-(((2-Chlor-6-methylphenyl)sulfonyl)methyl)-1,2,4-oxadiazol-5-yl)essigsäure (ACI-8)

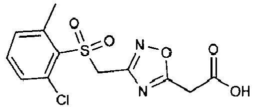

Die Synthese erfolgte in Analogie zu **ACI-2.**

### B. Synthese der Aminbausteine

### Synthese des Aminbausteins AMN-2:

### 7-(Pyridin-4-yl)-2,7-diazaspiro[3.5]nonan dihydrochlorid (AMN-2)

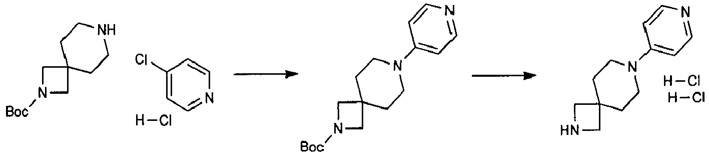

Stufe 1: tert-Butyl 2,7-diazaspiro[3.5]nonan-2-carboxylat (0,6 g, 2,651 mmol, 1,0 äq), 4-Chlorpyridin HCl (1,193 g, 7,953 mmol, 3 äq) und DIPEA (2,254 ml, 13,255 mmol, 5,0 äq) wurden in 2-Propanol (10 ml) 15 h refluxiert. Gesättigte Natriumhydrogencarbonatlösung (20 ml) und Ethylacetat (50 ml) wurden zugegeben, Phasen getrennt und die wässrige Phase mit Ethylacetat (3 x 50 ml) extrahiert. Die vereinigten organischen Phasen wurden mit ges. NaCl-Lösung (50 ml) gewaschen, über Magnesiumsulfat getrocknet und unter vermindertem Druck konzentriert. Das Rohprodukt wurde säulenchromatographisch (Kieselgel) mit Ethylacetat / Ethanol + wässrige Ammoniaklösung (10:1:0,05) gereinigt. Ausbeute: 60%.

**Stufe 2:** tert-Butyl 7-(pyridin-4-yl)-2,7-diazaspiro[3.5]nonane-2-carboxylate (0,47 g, 1,549 mmol, 1,0 äq) wurde in Ethanol (11 ml) gelöst und unter Eiskühlung mit Acetylchlorid (0,544 ml, 7,745 mmol, 5,0 äq) versetzt. Nach 16 h rühren bei RT wurde die Reaktionslösung unter vermindertem Druck konzentriert, wobei ein heller Niederschlag ausfiel. Die Reaktionslösung wurde mit Diethylether (20 ml) verdünnt, der Niederschlag abfiltriert, mit Diethylether gewaschen und im Vakuum getrocknet. Ausbeute: 93%.

### Synthese des Aminbausteins AMN-4:

### 8-(Pyridin-4-yl)-2,8-diazaspiro[4.5]decan dihydrochlorid (AMN-4)

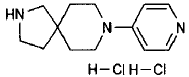

Die Synthese wurde analog der Synthese des Amins **AMN-2** durchgeführt.

### Synthese des Aminbausteins AMN-5:

### 8-Pyridin-4-YI-3,8-diazaspiro[4.4]nonan dihydrochlorid (AMN-5)

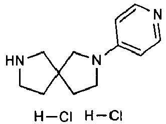

Die Synthese wurde analog der Synthese des Amins **AMN-2** durchgeführt.

### Synthese des Aminbausteins AMN-9:

### 9-Pyridin-4-yloxy-3-azaspiro[5.5]undecan dihydrochlorid (AMN-09)

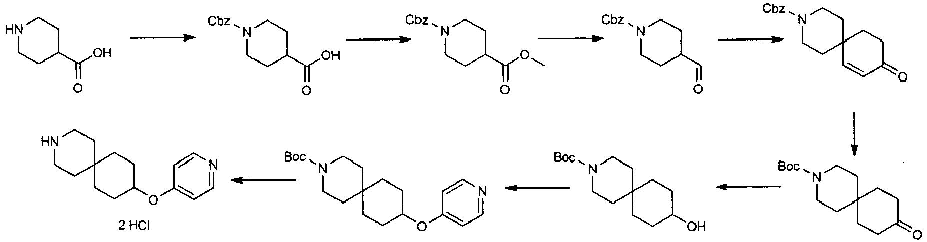

**Stufe 1:** Zu Piperidin-4-carbonsäure (25 g) in THF (75 ml) wurde Wasser (75 ml) gegeben, gefolgt von Natriumbicarbonat (30,8 g). Das Gemisch wurde auf to 0°C gekühlt und Cbz chlorid (38,9 ml) hinzugetropft. Anschließend wurde das Reaktionsgemisch 5 h bei RT gerührt (DC-Kontrolle). Nach vollständiger Umsetzung wurde das organische Lösungsmittel abdestilliert und der Rückstand in Wasser (200 ml) aufgenommen, mit Ethylacetat (2 x 150 ml) gewaschen. Die wässrige Phase wurde mit verdünnter wässriger HCl Lösung sauer gestellt und mit Ethylacetat extrahiert. Die organische Phase wurde getrocknet (Na₂SO₄) und unter Vakuum aufkonzentriert. Ausbeute: 96%.

**Stufe 2:** 1-(Benzyloxycarbonyl)piperidin-4-carbonsäure (48,5 g) in Methanol (485 ml) wurde auf 0°C gekühlt und Thionylchlorid (13,34 ml) hinzugetropft. Das Gemisch wurde anschließend 20 min refluxiert (DC-Kontrolle). Nach vollständiger Umsetzung wurde das Methanol abdestilliert, der Rückstand in Wasser (15 ml) aufgenommen und mit Ethylacetat (2 x 150 ml). Die vereinigten organischen Phasen wurden mit Wasser und ges. NaCl-Lösung extrahiert, getrocknet (Na₂SO₄) und unter Vakuum aufkonzentriert. Ausbeute: 67%.

**Stufe 3:** Eine Lösung 1-Benzyl 4-methyl piperidin-1,4-dicarboxylat (10 g) in Toluol (100 ml) unter Stickstoff wurde auf -78°C gekühlt. Anschließend wurde DIBAL-H (60,9 ml) bei -78°C hinzugetropft und das Gemisch 1 h bei dieser Temperatur gerührt (DC-Kontrolle). Aufgrund unvollständiger Umsetzung wurde weitere 0.2 äq DIBAL-H hinzugegeben und weitere 30 min gerührt. Zu dem Reaktionsgemisch wurden langsam bei -78°C Methanol (40 ml), gefolgt von ges. Natriumchloridlösung (40 ml) hinzugegeben. Das Gemisch wurde über Celite filtriert und das Lösungsmittel unter vermindertem Druck entfernt. Der Rückstand wurde mit Ethylacetat (3 x 75 ml) extrahiert, getrocknet (Na₂SO₄) und unter vermindertem Druck aufkonzentriert. Das so erhaltene Rohprodukt wurde säulenchromatographisch aufgereinigt (Kieselgel, 20% Ethylacetat / Hexan). Ausbeute: 49%.

**Stufe 4:** Methylvinylketon (1,64 ml), Ethanol (5 ml) and Wasser (5 ml) wurden zu Benzyl 4-formyl-piperidin-1-carboxylat (5 g) gegeben. Anschließend wurde das Gemisch zu einer kochenden Lösung Kaliumhydroxid (0,22 g) in Ethanol (10 ml) gegeben und das resultierende Reaktionsgemisch 1 h refluxiert. Nach vollständiger Umsetzung wurde das Gemisch auf Wasser (25 ml) gegeben und mit Ethylacetat (2 x 50 ml) extrahiert. Die vereinigten organischen Phasen wurden getrocknet (Na₂SO₄) und unter vermindertem Druck aufkonzentriert. Das so erhaltene Rohprodukt wurde säulenchromatographisch aufgereinigt (Kieselgel, 25% Ethylacetat / Hexan). Ausbeute: (46%).

**Stufe 5:** Boc-Anhydrid (9,4 ml) und Kaliumcarbonat (7,56 g) wurden zu Benzyl 9-oxo-3-azaspiro[5.5]-undec-7-en-3-carboxylat (8,2 g) in EtOH / Wasser (9:1) (200 ml) gegeben. Anschließend wurde Pd/C (1 g) hinzugegeben und 4 h bei 80 psi hydrogenolysiert. Nach vollständiger Umsetzung wurde das Gemisch über Celite filtriert und mit Ethanol und Ethylacetat nachgespült. Das Filtrat wurde getrocknet (Na₂SO₄) und unter Vakuum aufkonzentriert. Der Rückstand wurde in Ethylacetat und Wasser aufgenommen und die wässrige Phase mit Ethylacetat extrahiert. Die vereinigten organischen Phasen wurden getrocknet (Na₂SO₄) und unter vermindertem Druck aufkonzentriert. Das so erhaltene Rohprodukt wurde säulenchromatographisch aufgereinigt (Kieselgel, 20% Ethylacetat / Hexan). Ausbeute: 40%.

**Stufe 6:** *tert*-Butyl 9-oxo-3-azaspiro[5.5]undecan-3-carboxylat (1,5 g) wurde in THF (7,5 ml) gelöst und auf-5°C gekühlt. Anschließend wurde NaBH₄ (0,212 g) hinzugegeben und das Gemisch 1 h bei RT gerührt. Nach vollständiger Umsetzung wurde zu dem Gemisch Essigsäure gegeben und das Methanol anschließend abdestilliert. Der Rückstand wurde in Wasser (50 ml) aufgenommen und mit Ethylacetat (2 x 50 ml) extrahiert. Die vereinigten organischen Phasen wurden getrocknet (Na₂SO₄) und unter vermindertem Druck aufkonzentriert. Das so erhaltene Rohprodukt wurde säulenchromatographisch aufgereinigt (Kieselgel, 30% Ethylacetat / Hexan). Ausbeute: 80%.

**Stufe 7:** Zu Natriumhydrid (0,89 g) in DMSO (20 ml) wurde 4-Chlorpyridin Hydrochlorid (1,3 g) gegeben und das Gemisch 10 min gerührt. Anschließend wurde *tert*-Butyl 9-hydroxy-3-azaspiro[5.5]undecan-3-carboxylat (2,0 g) in DMSO (20 ml) langsam hinzugegeben und das Gemisch über Nacht gerührt (DC-Kontrolle: Umsetzung ca. 30-35 %). Eine katalytische Menge Natriumiodid wurde hinzugegeben und das Reaktionsgemisch 8 h bei 80°C gerührt. Zu dem Reaktionsgemisch wurde Methanol und NaHCO₃ Lösung gegeben und 20 min gerührt. Dann wurde mit Ethylacetat extrahiert und wiederum mit NaHCO₃ Lösung und kaltem Wasser gewaschen. Die organische Phase wurde getrocknet (Na₂SO₄) und unter vermindertem Druck aufkonzentriert. Das so erhaltene Rohprodukt wurde säulenchromatographisch aufgereinigt (Kieselgel, 70% Ethylacetat / Hexan). Ausbeute: 40%.

**Stufe 8:** *tert*-Butyl 9-(pyridin-4-yloxy)-3-azaspiro[5.5]undecan-3-carboxylat (1 g, 2,886 mmol) wurde in Methanol (2 ml) gelöst, mit Chlorwasserstoff in Methanol (1,25 mol/l, 11,5 ml) versetzt und 30 min refluxiert. Das Lösungsmittel wurde unter vermindertem Druck entfernt und der Rückstand in einer geringen Menge Ethanol gelöst. Anschließend wurde Aceton (ca. 25 ml) hinzugegeben, das Gemisch 30 min bei 0°C gerührt und schließlich der entstandene Feststoff abgesogen. Ausbeute: >99%.

### Synthese des Aminbausteins AMN-10:

### 9-Pyridin-4-yl-3,9-diazaspiro[5.5]undecan dihydrochlorid (AMN-10)

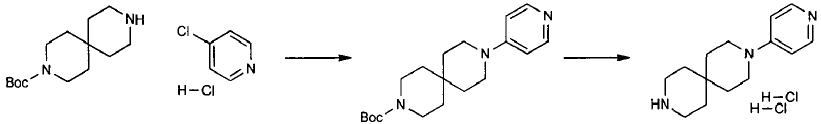

**Stufe 1:** *tert*-Butyl 3,9-diazaspiro[5.5]undecan-3-carboxylat (10 g, 39,308 mmol, 1,0 Äq.), 4-Chlorpyridin HCl (17,69 g, 117,924 mmol, 3 äq) und DIPEA (26,732 ml, 157,232 mmol, 4 äq) wurden in 2-Propanol (75 ml) 15 h refluxiert. Gesättigte Natriumhydrogencarbonatlösung (50 ml) und Ethylacetat (100 ml) wurden zugegeben, die Phasen getrennt und die wässrige Phase mit Ethylacetat (5 x 100 ml) extrahiert. Die vereinigten organischen Phasen wurden mit ges. NaCl-Lösung (100 ml) gewaschen, über Magnesiumsulfat getrocknet und unter vermindertem Druck eingeengt. Das Rohprodukt wurde säulenchromatographisch (Kieselgel) mit Ethylacetat / DCM / Methanol + wässrige Ammoniaklösung (1:1:0,25) gereinigt. Ausbeute: 54%.

**Stufe 2:** *tert*-Butyl 9-(pyridin-4-yl)-3,9-diazaspiro[5.5]undecan-3-carboxylat (2,8 g, 8,447 mmol, 1,0 äq) wurde in Methanol (17 ml) gelöst, mit Chlorwasserstoff in Methanol (1,25 mol/l, 34 ml) versetzt und 2 h refluxiert. Das Lösungsmittel wurde unter vermindertem Druck entfernt, der Rückstand in Ethanol (10 ml) aufgenommen und gekühlt. Aceton (100 ml) wurde zugegeben und es wurde 30 min im Eisbad gerührt. Der Niederschlag wurde abgesogen, mit Diethylether gewaschen und unter Vakuum getrocknet. Ausbeute: 95 %.

### Synthese des Aminbausteins AMN-11:

### 3,3-Dimethyl-1-(pyridin-4-yl)piperidin-4-amin (AMN-11)

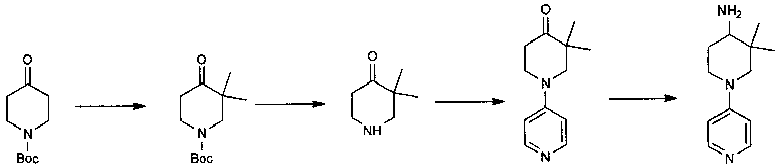

**Stufe 1:** Eine Lösung aus tert-Butyl 4-oxopiperidin-1-carboxylat (20 g, 100.5 mmol, 1 äq) in THF wurde bei 0°C zu einer Suspension aus ^{t}BuOK (28 g, 251,25 mmol, 2,5 äq) gegeben. Das Reaktionsgemisch wurde 30 min bei RT gerührt, danach wurde lodmethan (15,5 ml, 251 mmol, 2,5 äq) zugetropft. Das Gemisch wurde 20 h bei RT gerührt und anschließend mit Ethylacetat (500 ml) verdünnt. Die organische Phase wurde mit ges. NaCl-Lösung (2 x 200 ml) gewaschen und über Natriumsulfat getrocknet. Das Lösungsmittel wurde unter vermindertem Druck eingeengt und das Rohprodukt säulenchromatographisch (Kieselgel, 5% EtOAc/Hexan) aufgereinigt. Ausbeute: 18%.

**Stufe 2:** tert-Butyl 3,3-dimethyl-4-oxopiperidin-1-carboxylat (2,5 g, 11 mmol, 1 äq) wurde 1,5 h bei RT in einer Mischung aus TFA (25 ml) in DCM (100 ml) gerührt. Das Lösungsmittel und die überschüssige TFA wurden unter vermindertem Druck entfernt, das Rohprodukt in DCM (2x 50 ml) aufgenommen und wieder eingeengt. Das so erhaltene Produkt wurde ohne weitere Aufreinigung in die nächste Stufe eingesetzt.

**Stufe 3:** DIPEA (9,4 ml, 55 mmol, 5 äq) wurde bei 0°C zu einer Lösung aus 3,3-Dimethylpiperidin-4-on (1,2 g, 11 mmol, 1 äq) in n-BuOH (70 ml) gegeben und 20 h bei RT gerührt. 4-Brompyridin (4,27 g, 22 mmol, 2 äq) wurde hinzugefügt und das Reaktionsgemisch anschließend 20 h refluxiert. Die Mischung wurde unter vermindertem Druck aufkonzentriert und das Rohprodukt säulenchromatographisch (Kieselgel, 10% MeOH/DCM) gereinigt. Ausbeute: 67%.

**Stufe 4:** Eine Lösung aus 3,3-Dimethyl-1-(pyridin-4-yl)piperidin-4-on (1,5 g, 7,35 mmol, 1 äq) und Ammoniumacetat (5,6 g, 73,5 mmol, 10 äq) in MeOH (80 ml) wurde 2 h bei RT gerührt. NaCNBH₄ (680 mg, 11 mmol, 1,5 äq) wurde portionsweise zum Reaktionsgemisch gegeben und es wurde 60 h bei RT gerührt. Das Gemisch wurde unter vermindertem Druck aufkonzentriert und das Rohprodukt säulenchromatographisch (Aluminumoxid, 1 % MeOH/DCM) gereinigt. Ausbeute: 66%.

### Synthese des Aminbausteins AMN-12:

### 2,2-Dimethyl-4-(4-methylpiperazin-1-yl)cyclohexanamin (AMN-12)

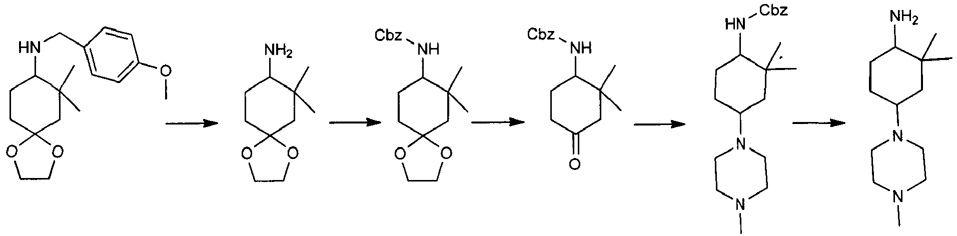

**Stufe** 1: Zu einer Lösung aus N-(4-Methoxybenzyl)-7,7-dimethyl-1,4-dioxaspiro[4.5]decan-8-amin [Stufe 2, **AMN-26**] (2,3 g, 7,54 mmol, 1,0 äq) in MeOH (30 ml) wurde Pd(OH)₂ (690 mg) gegeben und das Reaktionsgemisch 2 h bei RT hydriert (Ballondruck). Nach vollständiger Umsetzung (DC-Kontrolle) wurde das Reaktionsgemisch durch Kieselgur filtriert und das Filtrat am Rotationsverdampfer aufkonzentriert. Das Rohprodukt wurde säulenchromatographisch (neutrales Aluminiumoxid, 1% MeOH/DCM) aufgereinigt. Ausbeute: 72%.

**Stufe 2:** Zu einer Lösung aus 7,7-Dimethyl-1,4-dioxaspiro[4.5]decan-8-amin (1,3 g, 7,027 mmol, 1,0 äq) und TEA (3,89 ml, 28,10 mmol, 4,0 äq) in DCM (25 ml) wurde bei 0°C eine 50%ige Cbz-Cl-Lösung (2,56 ml, 8,432 mmol, 1,2 äq) in Toluol getropft und 2 h bei RT gerührt. Nach vollständiger Umsetzung (DC-Kontrolle) wurde das Reaktionsgemisch mit DCM (100 ml) verdünnt und mit Wasser (75 ml), ges.NH₄Cl-Lösung (75 ml) und ges. NaCl-Lösung (75 ml) gewaschen. Die organische Phase wurde über Natrium-sulfat getrocknet und das Lösungsmittel am Rotationsverdampfer einrotiert. Das Rohprodukt wurde säulenchromatographisch (Kieselgel, 15% Ethylacetat/Hexan) aufgereinigt. Ausbeute: 34%.

**Stufe 3:** Benzyl (7,7-dimethyl-1,4-dioxaspiro[4.5]decan-8-yl)carbamat (1,4 g, 4,3887 mmol, 1,0 äq) wurde in MeOH (14 ml) vorgelegt, mit 2N HCl (14 ml) versetzt und 18 h bei RT gerührt. Das Lösungsmittel wurde unter vermindertem Druck abgedampft und der Rückstand mit Wasser (10 ml) verdünnt. Es wurde mit ges. NaHCO₃-Lösung (20 ml) basisch gestellt und mit Ethylacetat (2 x 50 ml) extrahiert. Die vereinigten organischen Phasen wurden mit Wasser (50 ml) und ges. NaCl-Lösung (50 ml) gewaschen. Anschließend wurde über Natriumsulfat getrocknet und das Lösungsmittel am Rotationsverdampfer einrotiert. Das Rohprodukt wurde ohne weitere Aufreinigung in die nächste Stufe eingesetzt. Ausbeute: 91%.

**Stufe 4:** N-Methylpiperazin (0,620 ml, 5,6 mmol, 2,0 äq) wurde bei 0°C zu einer Lösung aus Benzyl (2,2-dimethyl-4-oxocyclohexyl)carbamat (770 mg, 2,8 mmol, 1,0 äq) in DCM (15 ml) gegeben und 2 h bei RT gerührt. Na(OAc)₃BH (1,78 g, 8,4 mmol, 3,0 äq) wurde portionsweise bei 0°C zum Reaktionsgemisch gegeben und es wurde 18 h bei RT gerührt. Das Reaktionsgemisch wurde anschließend mit DCM (60 ml) verdünnt und mit Wasser (50 ml) und ges. NaCl-Lösung (50 ml) gewaschen. Die organische Phase wurde über Natriumsulfat getrocknet und das Lösungsmittel am Rotationsverdampfer einrotiert. Das Rohprodukt wurde säulenchromatographisch (neutrales Aluminiumoxid, 1 % MeOH/DCM) aufgereinigt. Ausbeute: 44%.

**Stufe 5:** Zu einer Lösung aus Benzyl (2,2-dimethyl-4-(4-methylpiperazin-1-yl)cyclohexyl)carbamat (500 mg, 1,393 mmol, 1,0 äq) in MeOH (6 ml) wurde Pd(OH)₂ (150 mg) gegeben und das Reaktionsgemisch 2 h bei RT hydriert (Ballondruck). Nach vollständiger Umsetzung (DC-Kontrolle) wurde das Reaktionsgemisch durch Kieselgur filtriert und das Filtrat am Rotationsverdampfer aufkonzentriert. Das Rohprodukt wurde ohne weitere Aufreinigung in die nächste Stufe eingesetzt. Ausbeute: 95%.

### Synthese des Aminbausteins AMN-14:

### 4-(Pyridin-4-yl)cyclohexanamin (AMN-14)

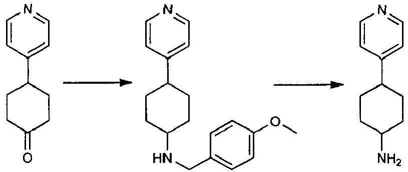

Stufe 1: 4-Methoxybenzylamin (1,48 ml, 11,3 mmol, 1,2 äq) wurde bei 0°C zu einer Lösung aus 4-(Pyridin-4-yl)cyclohexanon [Stufe 4, **AMN-25]** (1,65 g, 9,42 mmol, 1,0 äq) in DCM (100 ml) gegeben und 1 h bei RT gerührt. Na(OAc)₃BH (6,0 g, 28,26 mmol, 3,0 äq) wurde portionsweise bei 0°C zugegeben und 16 h bei RT gerührt. Die Reaktionsmischung wurde mit DCM (50 ml) verdünnt, mit Wasser (50 ml) und ges. NaCl-Lösung (50 ml) gewaschen und über Natriumsulfat getrocknet. Das Lösungsmittel wurde unter vermindertem Druck abgedampft und das Rohprodukt säulenchromatographisch (Kieselgel, 5% MeOH/DCM) aufgereinigt. Ausbeute: 79%.

**Stufe 2:** N-(4-Methoxybenzyl)-4-(pyridin-4-yl)cyclohexanamin (500 mg, 1,69 mmol, 1,0 äq) wurde in MeOH (20 ml) gelöst und 15 min mit Argon entgast. Pd(OH)₂ (150 mg) wurde zugegeben und unter Ballondruck (H₂) 5 h bei RT hydriert. Nach vollständiger Umsetzung (DC-Kontrolle) wurde die Reaktionsmischung über Celite abfiltriert und das Filtrat unter vermindertem Druck konzentriert. Der Rückstand wurde ohne weitere Aufreinigung in die nächste Stufe eingesetzt. Ausbeute: 94%.

### Synthese des Aminbausteins AMN-15:

### 4-(Pyridin-4-ylmethyl)cyclohexanamin (AMN-15)

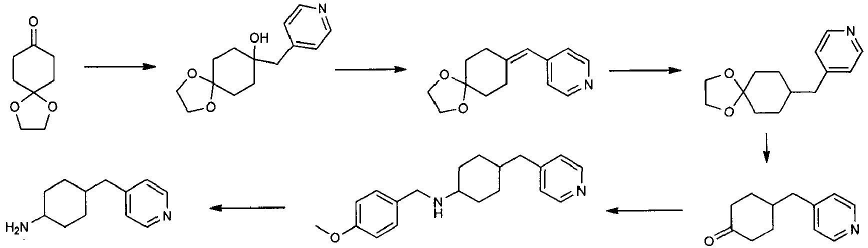

**Stufe 1:** Zu einer Lösung aus Diisopropylamin (7,66 ml, 54,48 mmol, 1,7 äq) in THF (50 ml) wurde bei - 78°C tropfenweise n-BuLi (32,04 ml, 48,07 mmol, 1,5 äq, 1,5 M Lösung in Hexan) gegeben, auf RT aufgetaut und 10 min bei RT gerührt. Eine Lösung aus 4-Methylpyridin (3,136 ml, 32,05 mmol, 1,0 äq) in THF (25 ml) wurde bei -78°C tropfenweise zum Reaktionsgemisch gegeben und es wurde 1 h bei dieser Temperatur gerührt. Anschließend wurde 1,4-Dioxaspiro[4.5]decan-8-on (5,0 g, 32,05 mmol, 1,0 äq) in THF (25 ml) zugegeben und 12 h bei RT gerührt. Nach DC-Kontrolle wurde das Reaktionsgemisch mit ges. Ammoniumchloridlösung (100 ml) hydrolisiert und mit Ethylacetat (2 x 200 ml) extrahiert. Die vereinigten organischen Phasen wurden mit Wasser (150 ml) und ges. NaCl-Lösung (150 ml) gewaschen und über Natriumsulfat getrocknet. Das Lösungsmittel wurde unter vermindertem Druck konzentriert und das Rohprodukt säulenchromatographisch (Kieselgel, 2% MeOH in DCM) aufgereinigt. Ausbeute: 50%.

**Stufe 2:** SOCl₂ (10 ml) wurde bei -10°C zu 8-(Pyridin-4-ylmethyl)-1,4-dioxaspiro[4.5]decan-8-ol (4,0 g, 16,06 mmol, 1,0 äq), gelöst in Pyridin (50 ml), getropft und bei dieser Temperatur 15 min gerührt. Nach vollständiger Umsetzung (DC-Kontrolle) wurde das Reaktionsgemisch auf Eis (100 g) gegossen, mit ges. Natriumhydrogencarbonat-Lösung basisch gestellt und mit DCM (2 x 150 ml) extrahiert. Die vereinigten organischen Phasen wurden mit Wasser (100 ml) und ges. NaCl Lösung (100 ml) gewaschen und über Natriumsulfat getrocknet. Das Lösungsmittel wurde unter vermindertem Druck konzentriert und das Rohprodukt säulenchromatographisch (Kieselgel, 3% MeOH/DCM) aufgereinigt. Ausbeute: 67%.

**Stufe 3:** 4-(1,4-Dioxaspiro[4.5]decan-8-ylidenmethyl)pyridin (1,7 g, 7,29 mmol, 1.0 äq) wurde in MeOH (35 ml) gelöst und 15 min mit Argon entgast. Pd(OH)₂ (1,7 g) wurde zugegeben und es wurde 14 h unter Ballondruck (H₂) bei RT hydriert. Nach DC-Kontrolle wurde die Reaktionsmischung über Celite abfiltriert und das Filtrat unter vermindertem Druck konzentriert. Der Rückstand wurde ohne weitere Aufreinigung in die nächste Stufe eingesetzt. Ausbeute: 76%.

**Stufe 4:** 4-(1,4-Dioxaspiro[4.5]decan-8-ylmethyl)pyridin (1,3 g g, 5,57 mmol, 1.0 äq) wurde in THF (40 ml) gelöst, mit 2N HCl (20 ml) versetzt und 3 h bei RT gerührt. Nach DC-Kontrolle wurde das Lösungsmittel unter vermindertem Druck konzentriert, der Rückstand mit Wasser (50 ml) verdünnt, mit Natriumhydrogencarbonat basisch eingestellt und mit Ethylacetat (100 ml) extrahiert. Die organische Phase wurde mit Wasser (50 ml) gewaschen und über Natriumsulfat getrocknet. Das Lösungsmittel wurde unter vermindertem Druck konzentriert und das Rohprodukt ohne weitere Reinigung in die nächste Stufe eingesetzt. Ausbeute: 61 %.

**Stufe 5:** 4-Methoxybenzylamin (528 µl, 4,06 mmol, 1,2 äq) wurde bei 0°C zu einer Lösung aus 4-(Pyridin-4-ylmethyl)cyclohexanon (641 µl, 3,39 mmol, 1,0 äq) in DCM (15 ml) gegeben und 1 h bei RT gerührt. Anschließend wurde bei 0°C Na(OAc)₃BH (1,078 g, 5,0 mmol, 1,5 äq) portionsweise zugegeben und 16 h bei RT gerührt. Die Reaktionsmischung wurde unter vermindertem Druck konzentriert, der Rückstand säulenchromatographisch (neutrales Aluminiumoxid, 0,5% MeOH in DCM) gereinigt. Ausbeute: 33%.

**Stufe 6:** N-(4-Methoxybenzyl)-4-(pyridin-4-ylmethyl)cyclohexanamin (500 mg, 1,68 mmol, 1,0 äq) wurde in MeOH (15 ml) gelöst und 15 min mit Argon entgast. Pd(OH)₂ (250 mg) wurde zugegeben und es wurde 5 h unter Ballondruck (H₂) bei RT hydriert. Nach DC-Kontrolle wurde die Reaktionsmischung über Celite abfiltriert und das Filtrat unter vermindertem Druck konzentriert. Der Rückstand wurde ohne weitere Aufreinigung in die nächste Stufe eingesetzt. Ausbeute: 78%.

### Synthese des Aminbausteins AMN-25.

### (4-(Pyridin-4-yl)cyclohexyl)methanamin (AMN-25)

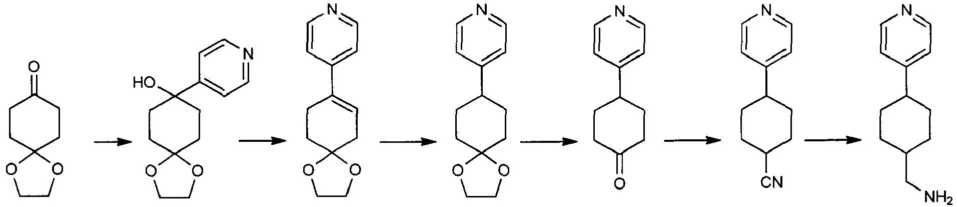

**Stufe 1:** Eine Lösung aus 4-Brompyridin (6,3 g, 40,0 mmol, 1,25 äq) in trockenem THF (50 ml) wurde bei -78°C zu einer Lösung aus n-BuLi (32 ml, 64,0 mmol, 2,0 äq, 2M Lösung in Hexan) in THF (60 ml) getropft und das Reaktionsgemisch bei dieser Temperatur 1,5 h gerührt. Eine Lösung aus 1,4-Dioxaspiro-[4.5]decan-8-on (5,0 g, 32,0 mmol, 1,0 äq.) in THF (50 ml) wurde bei -78°C zum Reaktionsgemisch gegeben und 2 h bei dieser Temperatur gerührt. Nach vollständiger Umsetzung (DC-Kontrolle) wurde das Reaktionsgemisch gekühlt und mit Wasser (100 ml) versetzt. Es wurde mit Ethylacetat (2 x 200 ml) extrahiert. Die vereinigten organischen Phasen wurden mit Wasser (100 ml) und ges. NaCl-Lösung (100 ml) gewaschen und über Natriumsulfat getrocknet. Das Lösungsmittel wurde unter vermindertem Druck abgedampft und das Rohprodukt säulenchromatographisch (Kieselgel, 2% MeOH/DCM) aufgereinigt. Ausbeute: 64%.

**Stufe 2:** SOCl₂ (10,5 ml) wurde bei -10°C zu einer Lösung aus 8-(Pyridin-4-yl)-1,4-dioxaspiro[4.5]decan-8-ol (6,0 g, 25,5 mmol, 1,0 äq) in Pyridin (50 ml) gegeben und das Reaktionsgemisch 15 min bei dieser Temperatur gerührt. Nach vollständiger Umsetzung (DC-Kontrolle) wurde das Reaktionsgemisch auf Eis (100 g) gegossen, mit ges. Natriuhydrogencarbonat-Lösung (100 ml) neutralisiert und mit DCM (2 x 150 ml) extrahiert. Die vereinigten organischen Phasen wurden mit Wasser (100 ml) und ges. NaCl-Lösung (100 ml) gewaschen und über Natriumsulfat getrocknet. Das Lösungsmittel wurde unter vermindertem Druck abgedampft und das Rohprodukt säulenchromatographisch (Kieselgel, 1% MeOH/DCM) aufgereinigt. Ausbeute: 63%.

**Stufe 3:** 4-(1,4-Dioxaspiro[4.5]dec-7-en-8-yl)pyridin (3,5 g, 16,1 mmol, 1,0 äq) wurde in MeOH (80 ml) gelöst und 15 min mit Argon entgast. Pd(OH)₂ (1,75 g, 50 Gew.%) wurde zugegeben und es wurde unter Ballondruck (H₂) 3 h bei RT hydriert. Nach vollständiger Umsetzung (LCMS-Kontrolle) wurde die Reaktionsmischung über Celite abfiltriert und das Filtrat unter vermindertem Druck aufkonzentriert. Der Rückstand wurde ohne weitere Aufreinigung in die nächste Stufe eingesetzt. Ausbeute: 84%.

**Stufe 4:** 4-(1,4-Dioxaspiro[4.5]decan-8-yl)pyridin (3,0 g, 13,6 mmol, 1,0 äq) wurde in THF (136 ml) gelöst, mit 2N HCl (13,6 ml) versetzt und 3 h bei RT gerührt. Nach vollständiger Umsetzung (DC-Kontrolle) wurde das Lösungsmittel unter vermindertem Druck abgedampft. Der Rückstand wurde mit Wasser (50 ml) verdünnt, mit Natriumhydrogencarbonat basisch gestellt und mit Ethylacetat (2 x 100 ml) extrahiert. Die vereinigten organischen Phasen wurden mit Wasser (80 ml) gewaschen und über Natriumsulfat getrocknet. Das Lösungsmittel wurde unter vermindertem Druck abgedampft und das Rohprodukt wurde ohne weitere Aufreinigung in die nächste Stufe eingesetzt. Ausbeute: 69%.

**Stufe 5:** t-BuOK (1,535 g, 13,714 mmol, 2,4 äq) wurde bei -5°C portionsweise zu einer Mischung aus 4-(Pyridin-4-yl)cyclohexanon (1,0 g, 5,714 mmol, 1,0 äq) und TOSMIC (1,44 g, 7,428 mmol, 1,3 äq) in DME (20 ml) und EtOH (0.3 ml) gegeben. Das Reaktionsgemisch wurde auf RT erwärmt und 14 h gerührt. Die Mischung wurde mit Eiswasser (100 ml) verdünnt und mit DCM (2 x 100 ml) extrahiert. Die vereinigten organischen Phasen wurden mit Wasser (100 ml) und ges. NaCl-Lösung (100 ml) gewaschen und über Natriumsulfat getrocknet. Das Lösungsmittel wurde unter vermindertem Druck eingeengt und das Rohprodukt säulenchromatographisch (Kieselgel, 1 % MeOH/DCM) aufgereinigt. Ausbeute: 56%.

**Stufe 6:** 4-(Pyridin-4-yl)cyclohexancarbonitril (200 mg, 1,075 mmol, 1,0 äq) wurde in THF (20 ml) gelöst und bei 0°C mit BH₃·DMS (347 µl, 4,30 mmol, 4,0 äq) und BF₃·Et₂O (130 µl, 1,075 mmol, 1,0 äq) versetzt. Das Reaktionsgemisch wurde auf RT erwärmt und danach 14 h refluxiert. Die Mischung wurde bei 0°C mit MeOH (50 ml) gequenched und unter vermindertem Druck aufkonzentriert. Das Rohprodukt wurde säulenchromatographisch (neutrales Aluminumoxid, 10% MeOH/DCM) aufgereinigt. Ausbeute: 73%.

### Synthese des Aminbausteins AMN-26:

### 2,2-Dimethyl-4-(pyridin-4-yl)cyclohexanamin (AMN-26)

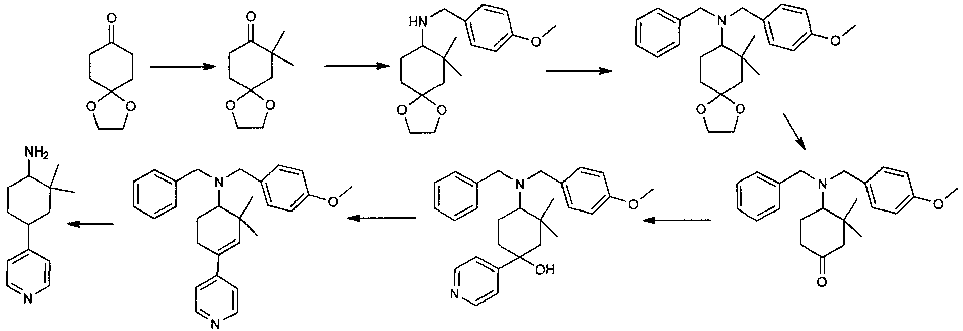

**Stufe 1:** Zu einer Lösung aus 1,4-Dioxaspiro[4.5]decan-8-on (25,0 g, 160,07 mmol, 1,0 äq) in THF (500 ml) wurde bei 0°C tropfenweise eine Lösung aus t-BuOK (39,0 g, 352,157 mmol, 2,2 äq) in THF (250 ml) gegeben. Nach vollständiger Zugabe wurde ebenfalls langsam Methyliodid (60 ml, 960,143 mmol, 6,0 äq) bei gleicher Temperatur zugetropft und die Reaktion 16 h bei RT gerührt. Die Reaktionsmischung wurde über Celite abfiltriert, das Filtrat unter vermindertem Druck konzentriert und der Rückstand säulenchromatographisch (Kieselgel, 3% Ethylacetat in Hexan) gereinigt. Ausbeute: 21 %.

**Stufe 2:** Eine Mischung aus 4-Methoxybenzylamin (2,8 g, 15,217 mmol, 1,0 äq) und 7,7-Dimethyl-1,4-dioxaspiro[4.5]decan-8-on (2,8 g, 15,217 mmol, 1,0 äq) in DCM (100 ml) wurde für 3 h bei RT gerührt. Anschließend wurde Na(OAc)₃BH (9,67 g, 45,65 mmol, 3,0 äq) portionsweise bei 0°C zugegeben und 20 h bei RT gerührt. Die Reaktionslösung wurde mit DCM (100 ml) verdünnt, mit Wasser und ges. NaCl-Lösung (je 100 ml) gewaschen, über Natriumsulfat getrocknet und unter vermindertem Druck konzentriert. Nach säulenchromatographischer Reinigung (10% Ethylacetat in Hexan) erhielt man das gewünschte produkt. Ausbeute: 46%.

**Stufe 3:** N-(4-Methoxybenzyl)-7,7-dimethyl-1,4-dioxaspiro[4.5]decan-8-amin (2,52 g, 8,26 mmol, 1,0 äq) wurde in DMF (15 ml) gelöst, NaH (991 mg, 24,78 mmol, 3,0 äq, 60% in Mineralöl) wurde bei 0°C zugegeben, gefolgt von Benzylbromid. Das Reaktionsgemisch wurde 16 h bei RT gerührt, mit Eiswasser (100 ml) verdünnt und mit Ethylacetat (2 x 150 ml) extrahiert. Die vereinigten org. Phasen wurden mit Wasser und ges. NaCl-Lösung (je 150 ml) gewaschen, über Natriumsulfat getrocknet und unter vermindertem Druck konzentriert. Nach säulenchromatographischer Reinigung (Kieselgel, 5% Ethylacetat in Hexan) erhielt man das gewünschte produkt. Ausbeute: 92%.

**Stufe 4:** N-Benzyl-N-(4-methoxybenzyl)-7,7-dimethyl-1,4-dioxaspiro[4.5]decan-8-amin (3,0 g, 7,597 mmol, 1,0 äq) wurde in MeOH/Aceton (1:1, 30 ml) gelöst, bei 0°C mit 2N HCl (30 ml) versetzt und 18 h bei RT gerührt. Nach DC-Kontrolle wurde das Lösungsmittel unter vermindertem Druck konzentriert, der Rückstand mit Wasser (100 ml) verdünnt, mit Natriumhydrogencarbonat Lösung (50 ml) basisch eingestellt und mit Ethylacetat (2 x 100 ml) extrahiert. Die organische Phase wurde mit Wasser und ges. NaCl-Lösung (je 100 ml) gewaschen und über Natriumsulfat getrocknet. Das Lösungsmittel wurde unter vermindertem Druck konzentriert und das Rohprodukt ohne weitere Reinigung in die nächste Stufe eingesetzt. Ausbeute: 95%.

**Stufe 5:** Eine Lösung aus 4-Brompyridin (2,52 g, 16, mmol, 2,2 äq) in THF (10 ml) wurde bei -78°C zu einer Lösung aus n-BuLi (8,73 ml, 21,82 mmol, 3,0 äq, 2,5 M Lösung in Hexan) in THF (10 ml) getropft und 45 min gerührt. Anschließend wurde bei -78°C eine Lösung aus 4-(Benzyl(4-methoxybenzyl)amino)-3,3-dimethylcyclohexanon (2,55 g, 2,27 mmol, 1,0 äq) in THF (10 ml) zugegeben, 45 min bei dieser Temperatur gerührt, auf RT erwärmt und weitere 16 h gerührt. Die Reaktionsmischung wurde mit ges. Ammoniumchloridlösung (100 ml) bei 0°C hydrolisiert und mit Ethylacetat (2 x 150 ml) extrahiert. Die vereinigten org. Phasen wurden mit Wasser und ges. NaCl-Lösung (je 150 ml) gewaschen, über Natriumsulfat getrocknet und unter vermindertem Druck konzentriert. Nach säulenchromatographischer Reinigung (Kieselgel, 60% Ethylacetat in Hexan) erhielt man das gewünschte produkt. Ausbeute: 36%.

**Stufe 6:** SOCl₂ (10 ml) wurde bei -10°C zu 4-(Benzyl(4-methoxybenzyl)amino)-3,3-dimethyl-1-(pyridin-4-yl)cyclohexanol (1,15 g, 2,67 mmol, 1,0 äq), gelöst in Pyridin (12,5 ml), getropft und bei dieser Temperatur 10 min gerührt. Nach vollständiger Umsetzung (DC-Kontrolle) wurde das Reaktionsgemisch auf Eis (100 g) gegossen, mit ges. Natriumhydrogencarbonat-Lösung auf pH9-10 eingestellt und mit DCM (2 x 150 ml) extrahiert. Die vereinigten organischen Phasen wurden mit Wasser (150 ml) und ges. NaCl Lösung (150 ml) gewaschen und über Natriumsulfat getrocknet. Das Lösungsmittel wurde unter vermindertem Druck konzentriert und das Rohprodukt säulenchromatographisch (Kieselgel, 25% Ethylacetat in Hexan) aufgereinigt. Ausbeute: 66 %.

**Stufe 7:** N-Benzyl-N-(4-methoxybenzyl)-2,2-dimethyl-4-(pyridin-4-yl)cyclohex-3-enamin (500 mg, 1,213 mmol, 1.0 äq) wurde in MeOH (10 ml) gelöst und 15 min mit Stickstoff entgast. Pd(OH)₂ (500 mg) wurde zugegeben und das Gemisch unter Ballondruck (H₂) 8 h bei RT hydriert. Nach DC-Kontrolle wurde die Reaktionsmischung über Celite abfiltriert und das Filtrat unter vermindertem Druck konzentriert. Der Rückstand wurde säulenchromatographisch gereinigt (neutrales Aluminiumoxid, 5% MeOH in DCM). Ausbeute: 80%.

### Synthese des Aminbausteins AMN-27:

### N,3,8,8-Tetramethyl-3-azaspiro[5.5]undecan-9-amin (AMN-27)

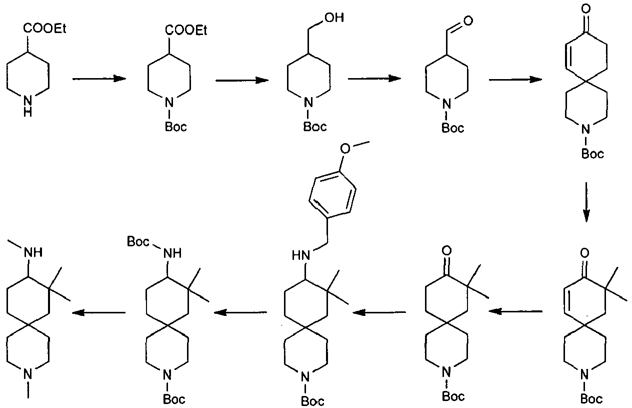

**Stufe 1:** Di-t-butyldicarbonat (20,80 g, 95,39 mmol, 1,5 äq) wurde bei 0°C zu einer Lösung aus Ethyl piperidin-4-carboxylat (10,0 g, 63,7 mmol, 1 äq) und TEA (17,68 ml, 127,0 mmol, 2,0 äq) in DCM (200 ml) gegeben und 16 h bei RT gerührt. Die Reaktionslösung wurde mit DCM (100 ml) verdünnt und mit Wasser und ges. NaCl-Lösung (je 100 ml) gewaschen, über Natriumsulfat getrocknet und unter vermindertem Druck konzentriert. Das Rohprodukt wurde ohne weitere Aufreinigung in die nächste Stufe eingesetzt. Ausbeute: 94%.

**Stufe 2:** Eine Lösung aus 1-t-Butyl 4-ethyl piperidin-1,4-dicarboxylat (15,42 g, 60,0 mmol, 1,0 äq) in THF (80 ml) wurde bei 0°C zu einer Suspension aus LAH (3,33 g, 90,0 mmol, 1,5 äq) in THF (120 ml) getropft und anschließend 1 h bei RT gerührt. Die Reaktionsmischung wurde mit THF:Wasser (9:1, 36 ml) und 10%iger NaOH-Lösung (7,2 ml) unter Eiskühlung hydrolisiert, über Celite abfiltriert und das Filtrat unter vermindertem Druck konzentriert. Das Rohprodukt wurde ohne weitere Aufreinigung in die nächste Stufe eingesetzt. Ausbeute: 77%.

**Stufe 3:** Oxalsäurechlorid (10,54 g, 111,62 mmol, 2,0 äq) wurde in DCM (100 ml) gelöst und auf -78°C gekühlt. Bei dieser Temperatur wurde eine Mischung aus DMSO (15,83 ml, 223,26 mmol, 4,0 äq) und DCM (100 ml) zugetropft. Anschließend wurde eine Lösung aus tert-Butyl 4-(hydroxymethyl)piperidin-1-carboxylat (12,0 g, 55,81 mmol, 1,0 äq) in DCM (100 ml) zugetropft und 30 min gerührt. TEA (38,71 ml, 279,05 mmol, 5,0 äq) wurde bei -78°C zugegegeben und die Reaktionsmischung langsam auf RT aufgetaut und weitere 30 min gerührt. Die Reaktionsmischung wurde mit ges. Ammoniumchloridlösung (200 ml) hydrolisiert und mit DCM (100 ml) verdünnt. Nach Trennung der Phasen wurde die org. Phase mit ges. NaCl-Lösung (150 ml) gewaschen, über Natriumsulfat getrocknet und unter vermindertem Druck konzentriert. Das Rohprodukt wurde ohne weitere Aufreinigung in die nächste Stufe eingesetzt. Ausbeute: 95%.

**Stufe 4:** Bei 0°C wurde 3 M KOH in Ethanol (7,5 ml) zu einer Lösung aus t-Butyl 4-formylpiperidin-1-carboxylat (11,29 g, 53,0 mmol, 1,0 äq) und Methylvinylketon (4,893 g, 69,9 mmol, 1,3 äq) in THF (100 ml) gegeben und 16 h bei RT gerührt. Die Reaktionslösung wurde unter vermindertem Druck konzentriert, der Rückstand in Wasser (100 ml) und KHSO₄-Lösung bei Eiskühlung aufgenommen und mit DCM (2 x 150 ml) extrahiert. Die vereinigten org. Phasen wurden mit ges. NaCl-Lösung (100 ml) gewaschen, über Natriumsulfat getrocknet und unter vermindertem Druck konzentriert. Das Rohprodukt wurde säulenchromatographisch (Kieselgel, 10-12% Ethylacetat in Hexan) gereinigt. Ausbeute: 25%.

**Stufe 5:** KO^{t}Bu (7,61 g, 67,92 mmol, 3,0 äq) wurde bei 0°C zu einer Lösung aus tert-Butyl 9-oxo-3-azaspiro[5.5]undec-7-en-3-carboxylat (22,64 mmol, 1,0 äq) in THF (60 ml) gegeben und 30 min bei dieser Temperatur gerührt. Anschließend wurde Methyliodid (5,66 ml, 90,56 mmol, 4,0 äq) tropfenweise zugegeben und weitere 2 h bei RT gerührt. Die Reaktionsmischung wurde mit Wasser (100 ml) verdünnt, mit Ethylacetat (2 x 150 ml) extrahiert. Die org. Phase wurden mit ges. NaCl-Lösung (100 ml) gewaschen, über Natriumsulfat getrocknet und unter vermindertem Druck konzentriert. Nach säulenchromatographischer Reinigung (Kieselgel, 10-12% Ethylacetat in Hexan) erhielt man das gewünschte Produkt. Ausbeute: 42%.

**Stufe 6:** tert-Butyl 10,10-dimethyl-9-oxo-3-azaspiro[5.5]undec-7-en-3-carboxylat (7,0 g, 23,89 mmol, 1,0 äq) wurde in MeOH (70 ml) gelöst, mit Argon 15 min entgast und mit Pd-C (1,4 g) versetzt. Die Reaktionsmischung wurde unter Ballondruck (H₂) 4 h bei RT hydriert, anschließend über Celite filtriert und das Filtrat unter vermindertem Druck konzentriert. Ausbeute: 90%.

**Stufe 7:** AcOH (0,2 ml) wurde zu einer Lösung aus tert-Butyl 8,8-dimethyl-9-oxo-3-azaspiro[5.5]undecan-3-carboxylat (30, g, 10,17 mmol, 1,0 äq) und 4-Methoxybenzylamin (1,32 ml, 10,17 mmol, 1,0 äq) in DCM (30 ml) gegeben und 1 h bei RT gerührt. Anschließend wurde Na(OAc)₃BH (4,31 g, 20,34 mmol, 2,0 äq) portionsweise zugegeben und 16 h bei RT gerührt. Die Reaktionsmischung wurde mit DCM (100 ml) verdünnt, mit ges. Natriumhydrogencarbonatlösung (2 x 60 ml) und ges. NaCl-Lösung (60 ml) gewaschen, über Natriumsulfat getrocknet und unter vermindertem Druck konzentriert. Nach säulenchromatographischer Reinigung (Kieselgel, 4% MeOH in DCM) erhielt man einen farblosen Feststoff. Ausbeute: 66%.

**Stufe 8:** Eine Mischung aus tert-Butyl 9-((4-methoxybenzyl)amino)-8,8-dimethyl-3-azaspiro[5.5]undecan-3-carboxylat (2,8g 6,73 mmol, 1,0 äq), Boc₂O (1,76 g, 8,08 mmol, 1,2 äq) und Pd(OH)₂ (500 mg) in Ethyl-acetat (30 ml) wurde unter Ballondruck (H₂) 2 h bei RT hydriert. Die Reaktionsmischung wurde über Celite abfiltriert, das Filtrat unter vermindertem Druck konzentriert und das Rohprodukt säulenchromatographisch (Kieselgel, 20% Ethylacetat in Hexan) gereinig. Ausbeute: 78%.

**Stufe 9:** Zu einer Suspension aus LAH (460 mg, 12,12 mmol, 6,0 äq) in THF (10 ml) wurde eine Lösung aus t-Butyl 9-((tert-butoxycarbonyl)amino)-8,8-dimethyl-3-azaspiro[5.5]undecan-3-carboxylat (800 mg, 2,02 mmol, 1,0 äq) in THF (6 ml) gegeben und 2 h auf Siedetemperatur erhitzt. Die Reaktionsmischung wurde mit THF:H₂O (9:1, 4,2 ml) und 10%ige NaOH-Lösung (1 ml) bei 0°C hydrolisiert, mit THF (50 ml) verdünnt und 1 h bei RT gerührt. Anschließend wurde über Celite abfiltriert und das Filtrat unter vermindertem Druck konzentriert. Das entstandene Amin wurde ohne weitere Reinigung in der nächsten Stufe eingesetzt. Ausbeute: 94%.

### Synthese des Aminbausteins AMN-28:

### 4-Amino-3,3-dimethylcyclohexanol (AMN-28)

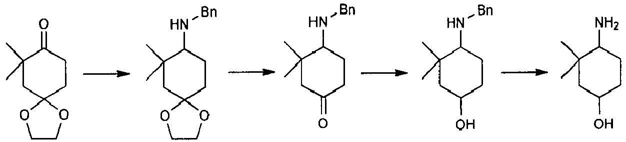

**Stufe 1:** Eine Mischung aus 7,7-Dimethyl-1,4-dioxaspiro[4.5]decan-8-on [Stufe 1, AMN-26] (1,5 g, 8,15 mmol, 1,0 äq) und Benzylamin (900 µl, 8,15 mmol, 1,0 äq) in DCM (75 ml) wurde 3 h bei RT gerührt. Na(OAc)₃BH (5,18 g, 24,45 mmol, 3,0 äq) wurde portionsweise bei 0°C zum Reaktionsgemisch gegeben und 20 h bei RT gerührt. Das Reaktionsgemisch wurde mit DCM (100 ml) verdünnt, mit Wasser (100 ml) und ges. NaCl-Lösung (100 ml) gewaschen und über Natriumsulfat getrocknet. Das Lösungsmittel wurde unter vermindertem Druck abgedampft und das Rohprodukt säulenchromatographisch (neutrales Aluminiumoxid, 5% MeOH/DCM) aufgereinigt. Ausbeute: 36%.

Stufe 2: Konz. HCl (2 ml) wurde zu einer Lösung aus N-Benzyl-7,7-dimethyl-1,4-dioxaspiro[4.5]decan-8-amin (600 mg, 2,18 mmol, 1,0 äq) in Aceton (2 ml) gegeben und 1 h bei RT gerührt. Das Lösungsmittel wurde unter vermindertem Druck eingeengt. Der Rückstand wurde in Wasser (10 ml) gelöst, mit 2N NaOH-Lösung basisch gestellt und mit DCM (2 x 100 ml) extrahiert. Die vereinigten organischen Phasen wurden mit Wasser (50 ml) und ges. NaCl-Lösung (50 ml) gewaschen und über Natriumsulfat getrocknet. Das Lösemittel wurde unter vermindertem Druck eingeengt und das Rohprodukt in die nächste Stufe eingesetzt. Ausbeute: 500 mg

**Stufe 3:** NaBH₄ (98 mg, 2,5 mmol, 1,2 äq) wurde bei 0°C zu einer rührenden Lösung aus 4-(Benzyl-amino)-3,3-dimethylcyclohexanon (400 mg, 2,16 mmol, 10 äq) in MeOH (6 ml) gegeben und das Reaktionsgemisch 1 h bei RT gerührt. Nach vollständiger Umsetzung (DC-Kontrolle) wurde das Reaktionsgemisch unter vermindertem Druck aufkonzentriert und mit DCM (50 ml) verdünnt. Die organische Phase wurde mit Wasser (20 ml) und ges. NaCl-Lösung (20 ml) gewaschen und über Natriumsulfat getrocknet. Das Lösungsmittel wurde unter vermindertem Druck abgedampft und das Rohprodukt säulenchromatographisch aufgereinigt. Ausbeute: 79%.

**Stufe 4:** Zu einer Lösung aus 4-(Benzylamino)-3,3-dimethylcyclohexanol (400 mg, 1,71 mmol, 1,0 äq) in MeOH (5 ml) wurde Pd-C(100 mg) gegeben und 15 min mit N₂ entgast. Das Reaktionsgemisch wurde 1 h bei RT unter Ballondruck hydriert. Nach vollständiger Umsetzung (DC-Kontrolle) wurde das Reaktionsgemisch durch Celite filtriert und das Filtrat unter vermindertem Druck aufkonzentriert. Das Rohprodukt wurde ohne weitere Aufreinigung in die nächste Stufe eingesetzt. Ausbeute:240 mg.

### Synthese des Aminbausteins AMN-29:

### 3,3-Dimethyltetrahydro-2H-pyran-4-amin (AMN-29)

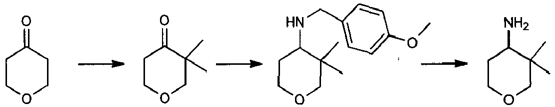

**Stufe 1:** Eine Lösung aus Dihydro-2H-pyran-4(3H)-on (10 g, 99,88 mmol, 1,0 äq) in THF (60 ml) wurde bei 0°C tropfenweise zu einer Lösung aus Bu^{t}OK (24,65 g, 219,73 mmol, 2,2 äq) in THF (200 ml) gegeben. Nach vollständiger Zugabe wurde Methyliodid (37,4 ml, 599,28 mmol, 6,0 äq) bei dieser Temperatur zugetropft und das Reaktionsgemisch 1 h bei RT gerührt. Das Reaktionsgemisch wurde mit Eiswasser (200 ml) verdünnt und mit Diethylether (2 x 300 ml) extrahiert. Die vereinigten org. Phasen wurden mit Wasser (300 ml) und ges. NaCl-Lösung (300 ml) gewaschen, über Natriumsulfat getrocknet und vorsichtig (Verbindung ist leicht flüchtig) unter vermindertem Druck eingeengt. Das Rohprodukt wurde säulenchromatographisch (Kieselgel, 1,5 % Ethylacetat / Hexan) aufgereinigt. Ausbeute: 10%.

**Stufe 2:** Eine Mischung aus 3,3-Dimethyldihydro-2H-pyran-4(3H)-on (1,0 g, 7,81 mmol, 1,0 äq) und 4-Methoxybenzylamin (2,02 ml, 15,62 mmol, 2,0 äq) in DCM (20 ml) wurde 2 h bei RT gerührt. Na(OAc)₃BH (4,96 g, 24,43 mmol, 3,0 äq) wurde portionsweise bei 0°C zum Reaktionsgemisch gegeben und 16 h bei RT gerührt. Das Reaktionsgemisch wurde mit DCM (150 ml) verdünnt, mit Wasser (80 ml) und ges. NaCl-Lösung (80 ml) gewaschen und über Natriumsulfat getrocknet. Das Lösungsmittel wurde unter vermindertem Druck abgedampft. Das Rohprodukt wurde in DCM (100 ml) gelöst und mit 2N HCl (50 ml) gewaschen. Die wässrige Phase wurde mit NaHCO₃ basisch gestellt und mit DCM (2 x 75 ml) extrahiert. Die organische Phase wurde mit Wasser (50 ml) und ges. NaCl-Lösung (50 ml) gewaschen und über Natriumsulfat getrocknet. Das Lösungsmittel wurde unter vermindertem Druck abgedampft. Ausbeute: 10%.

**Stufe 3:** Eine Lösung aus N-(4-Methoxybenzyl)-3,3-dimethyltetrahydro-2H-pyran-4-amin (200 mg, 0,80 mmol, 1,0 äq) in MeOH (4 ml) wurde 15 min mit N₂ entgast und danach mit 20% Pd(OH)₂ (200 mg) versetzt. Das Reaktionsgemisch wurde 2 h bei RT unter Ballondruck hydriert. Nach vollständiger Umsetzung (DC-Kontrolle) wurde das Reaktionsgemisch durch Celite filtriert, mit MeOH (2 x 5 ml) gewaschen und das Filtrat unter vermindertem Druck aufkonzentriert. Das Rohprodukt wurde ohne weitere Aufreinigung in die nächste Stufe eingesetzt. Ausbeute: 97%.

### Synthese des Aminbausteins AMN-30:

### 3,3-Dimethyl-1-(tetrahydro-2H-pyran-4-yl)piperidin-4-amin (AMN-30)

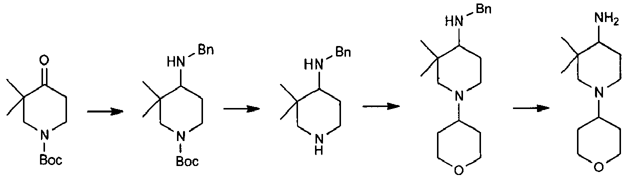

**Stufe 1:** Eine Mischung aus tert-Butyl 3,3-dimethyl-4-oxopiperidin-1-carboxylat [Stufe 1, **AMN-11]** (3,0 g, 13,27 mmol, 1,0 äq) und Benzylamin (1,4 ml, 13,27 mmol, 1,0 äq) in MeOH (30 ml) wurde 1 h bei RT gerührt. NaCNBH₃ (2,09 g, 33,17 mmol, 2,5 äq) wurde portionsweise bei 0°C zum Reaktionsgemisch gegeben und es wurde 16 h bei RT gerührt. Das Reaktionsgemisch wurde aufkonzentriert, mit DCM (200 ml) verdünnt und mit Wasser (100 ml) und ges. NaCl-Lösung (100 ml) gewaschen und über Natriumsulfat getrocknet. Das Lösungsmittel wurde unter vermindertem Druck abgedampft und das Rohprodukt säulenchromatographisch (neutrales Aluminumoxid, 15% Ethylacetat/Hexan) aufgereinigt. Ausbeute: 35%.

**Stufe 2:** tert-Butyl 4-(benzylamino)-3,3-dimethylpiperidin-1-carboxylat (1,09 g, 3,44 mmol, 1 äq) wurde in DCM (15 ml) gelöst und bei 0°C mit TFA (3 ml) versetzt. Das Reaktionsgemisch wurde 2 h bei RT gerührt. Nach vollständiger Umsetzung wurde das Lösungsmittel unter vermindertem Druck abgedampft, der Rückstand in Toluol aufgenommen und wieder eingeengt. Das Rohprodukt wurde ohne weitere Aufreinigung in die nächste Stufe eingesetzt.

**Stufe 3:** Tetrahydropyran-4-on (389 µl, 3,44 mmol, 1,0 äq.) wurde bei 0°C zu einer Lösung aus N-Benzyl-3,3-dimethylpiperidin-4-amin (3,44 mmol, 1,0 äq) in MeOH (10 ml) gegeben und 1 h bei RT gerührt. NaCNBH₃ (541 mg, 8,6 mmol, 2,5 äq) wurde bei 0°C zum Reaktionsgemisch gegeben und 8 h bei RT gerührt. Das Reaktionsgemisch wurde aufkonzentriert, mit DCM (100 ml) verdünnt. Anschließend wurde mit Wasser (50 ml) und ges. NaCl-Lösung (50 ml) gewaschen und über Natriumsulfat getrocknet. Das Lösungsmittel wurde unter vermindertem Druck abgedampft und das Rohprodukt säulenchromatographisch (Kieselgel, 10% MeOH/DCM) aufgereinigt. Ausbeute: 39%.

Stufe 4: Eine Lösung aus N-Benzyl-3,3-dimethyl-1-(tetrahydro-2H-pyran-4-yl)piperidin-4-amin (400 mg, 1,324 mmol, 1,0 äq) in MeOH (5 ml) wurde 15 min mit N₂ entgast und danach mit Pd-C(160 mg) versetzt. Das Reaktionsgemisch wurde 5 h bei RT unter Ballondruck hydriert. Nach vollständiger Umsetzung (DC-Kontrolle) wurde das Reaktionsgemisch durch Celite filtriert und das Filtrat unter vermindertem Druck aufkonzentriert. Das Rohprodukt wurde ohne weitere Aufreinigung in die nächste Stufe eingesetzt. Ausbeute: 71%.

### Synthese des Aminbausteins AMN-31:

### 4-(2-Methoxypyridin-4-yl)-2,2-dimethylcyclohexanamin (AMN-31)

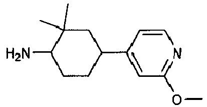

Die Synthese wurde analog der Synthese des Amins AMN-26 durchgeführt.

### Synthese des Aminbausteins AMN-35:

### 1-(4-(Methylamino)piperidin-1-yl)-3-(piperidin-1-yl)propan-1-on hydrochlorid (AMN-35)

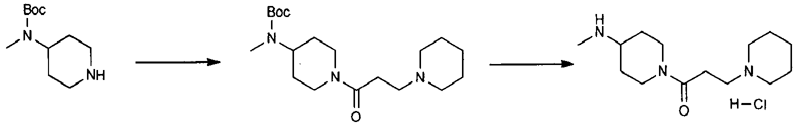

Stufe 1: 3-(Piperidin-1-yl)propansäure (0,5 g, 3,183 mmol, 1 äq) wurde in DCM (40 ml) und DIPEA (1,35 ml, 7,958 mmol, 2,5 äq) gelöst, auf 0°C gekühlt, mit EDCI (0,693 g, 3,82 mmol, 1,2 äq) und HOBT (0,064 g, 0,637 mmol, 0,2 äq) versetzt und 15 min gerührt. t-Butyl methyl(piperidin-4-yl)carbamat (0,678 g, 3,183 mmol, 1,0 äq) wurde zugegeben und 16 h bei RT gerührt. Die Reaktionsmischung wurde mit Ethylacetat (100 ml) verdünnt, mit 10%iger Ammoniumchloridlösung, ges. Natriumhydrogencarbonatlösung und ges. NaCl-Lösung gewaschen, über Natriumsulfat getrocknet und unter vermindertem Druck reduziert. Das Rohprodukt wurde säulenchromatographisch (Kieselgel, Ethylacetat:Ethanol 5:1 + Ammoniak) gereinigt. Ausbeute: 78%.

Stufe 2: tert-Butyl methyl(1-(3-(piperidin-1-yl)propanoyl)piperidin-4-yl)carbamae (0,87 g, 2,461 mmol, 1,0 äq) wurde in Ethanol gelöst, unter Eiskühlung mit Acetylchlorid (0,87 ml, 12,305 mmol, 5 äq) versetzt und 16 h bei RT gerührt. Die Reaktionslösung wurde halb konzentriert, der entstandene Niederschlag abfiltriert, mit Diethylether gewaschen und unter vermindertem Druck getrocknet. Ausbeute: >99%.

### Synthese des Aminbausteins AMN-36:

### 1-((6-Methoxypyridin-3-yl)methyl)piperazin dihydrochlorid (AMN-36)

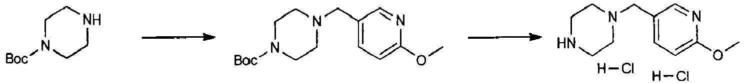

**Stufe 1:** 6-Methoxynicotinaldehyd (0,419 g, 3,062 mmol, 1,14 äq) und tert-butyl piperazine-1-carboxylat (0,5 g, 2,686 mmol, 1,0 äq) wurden in DCM (17 ml) gelöst. Essigsäure (0,359 ml, 6,285 mmol, 2,34 äq) und Natriumtriacetoxyborhydrid (0,783 g, 3,76 mmol 1,4 äq) wurden zugegeben und die Mischung 48 h bei RT gerührt. Das Reaktionsgemisch wurde mit ges. Natriumhydrogencarbonatlösung hydrolisiert, die Phasen getrennt und die wässrige Phase mit DCM (2 x 50 ml) extrahiert. Die vereinigten org. Phasen wurden mit ges. NaCl-Lösung gewaschen, über Natriumsulfat getrocknet und konzentriert. Das Rohprodukt wurde säulenchromatographisch (Kieselgel, Ethylacetat:Cyclohexan 5:1) gereinigt. Ausbeute: 96%.

**Stufe 2:** tert-Butyl 4-((6-methoxypyridin-3-yl)methyl)piperazin-1-carboxylat (0,78 g, 2,538 mmol, 1,0 äq) wurde in Ethanol (10 ml) gelöst, unter Eiskühlung mit Acetylchlorid (0,9 ml, 12,69 mmol, 5 äq) versetzt und 16 h bei RT gerührt. Nach DC-Kontrolle wurde 2 äq Acetylchlorid nachdosiert und 2 h auf 50°C erwärmt Die Reaktionslösung wurde halb konzentriert, der entstandene Niederschlag abfiltriert, mit Diethylether gewaschen und unter vermindertem Druck getrocknet. Ausbeute: >99%.

### Synthese des Aminbausteins AMN-37:

### 1-((2-(Pyrrolidin-1-yl)pyridin-4-yl)methyl)piperazin dihydrochlorid (AMN-37)

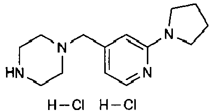

Die Synthese wurde analog der Synthese des Amins **AMN-36** durchgeführt.

### Synthese des Aminbausteins AMN-40:

### 1-((6-Methoxypyridin-3-yl)methyl)-N-methylpiperidin-4-amin hydrochlorid (AMN-40)

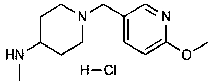

Die Synthese wurde analog der Synthese des Amins **AMN-36** durchgeführt.

### Synthese des Aminbausteins AMN-41:

### N-Methyl-1-((2-(pyrrolidin-1-yl)pyridin-4-yl)methyl)piperidin-4-amin hydrochlorid (AMN-41)

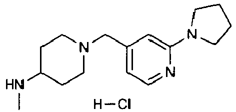

Die Synthese wurde analog der Synthese des Amins **AMN-36** durchgeführt.

### Synthese des Aminbausteins AMN-42:

### N-Methyl-4-(4-methylpiperazin-1-yl)cyclohexanamin hydrochlorid (AMN-42)

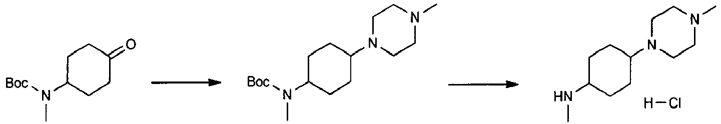

**Stufe 1:** 1-Methylpiperazin (0,25 g, 2,257 mmol, 1,14 äq) und tert-Butyl methyl(4-oxocyclohexyl)carbamat (0,45 g, 1,98 mmol, 1,0 äq) wurden in DCM (19 ml) gelöst, Essigsäure (0,266 ml, 4,633 mmol, 2,34 äq) und Natriumtriacetoxyborhydrid (0,58 g, 2,772 mmol 1,4 äq) wurden zugegeben und es wurde 16 h bei RT gerührt. Das Reaktionsgemisch wurde mit ges. Natriumhydrogencarbonatlösung hydrolisiert, die Phasen getrennt und die wässrige Phase mit DCM (2 x 50 ml) extrahiert. Die vereinigten org. Phasen wurden mit ges. NaCl-Lösung gewaschen, über Natriumsulfat getrocknet und konzentriert. Das Rohprodukt wurde säulenchromatographisch (Kieselgel, Ethylacetat:Ethanol 5:1 + Ammoniak) gereinigt. Ausbeute: 76%.

**Stufe 2:** tert-Butyl methyl(4-(4-methylpiperazin-1-yl)cyclohexyl)carbamat (0,46 g, 1,477 mmol, 1,0 äq) wurde in Ethanol (5 ml) gelöst, unter Eiskühlung mit Acetylchlorid (0,52 ml, 7,385 mmol, 5,0 äq) versetzt und 16 h bei RT gerührt. Die Reaktionslösung wurde konzentriert, der Rückstand 2x in DCM aufgenommen, konzentriert und im Hochvakuum getrocknet. Ausbeute: >99%.

### Synthese des Aminbausteins AMN-43:

### 1-((6-(Pyrrolidin-1-yl)pyridin-3-yl)methyl)piperazin hydrochlorid (AMN-43)

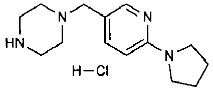

Die Synthese wurde analog der Synthese des Amins **AMN-36** durchgeführt.

### Synthese des Aminbausteins AMN-45:

### 4-(4-Methoxypiperidin-1-yl)-N-methylcyclohexanamin hydrochlorid (AMN-45)

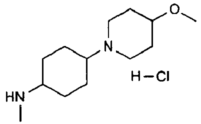

Die Synthese wurde analog der Synthese des Amins **AMN-42** durchgeführt.

### Synthese des Aminbausteins AMN-46:

### N-Methyl-1-((6-(pyrrolidin-1-yl)pyridin-3-yl)methyl)piperidin-4-amin hydrochlorid (AMN-46)

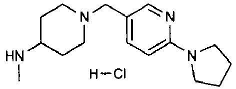

Die Synthese wurde analog der Synthese des Amins **AMN-36** durchgeführt.

### Synthese des Aminbausteins AMN-53:

### (1S,3R)-N-Methyl-3-(4-methylpiperazin-1-yl)cyclohexanamin (AMN-53)

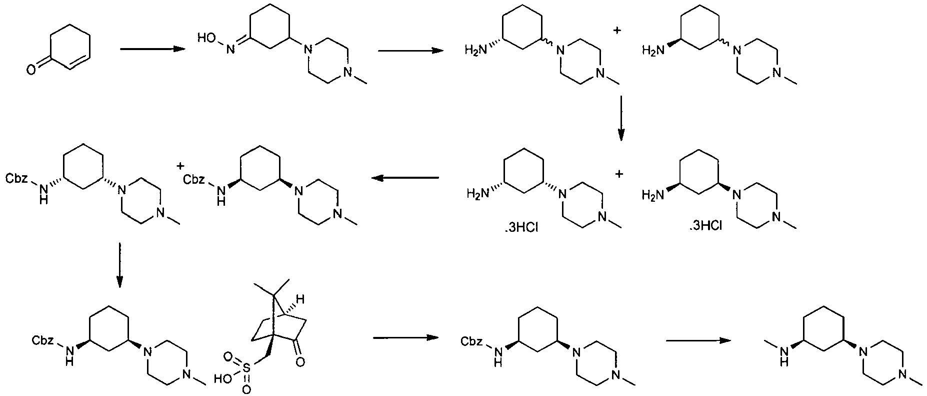

**Stufe 1:** Zu einer Lösung aus 2-Cyclohexen-1-on (100,0 g, 1041,6 mmol 1,0 äq) in Ethanol (300 ml) wurde N-Methylpiperazin (104,16 g, 1041,6 mmol, 1,0 äq) hinzugetropft und das Gemisch 5 h bei RT gerührt. Dann wurde die Mischung mit Ethanol (600 ml) verdünnt, auf 0°C gekühlt und K₂CO₃ (172,48 g, 1249,9 mmol, 1,2 äq) gefolgt von NH₂OH·HCl (86,7 g, 1249,9 mmol, 1,2 äq) portionsweise hinzugegeben. Es wurde weitere 30 min bei 0°C, gefolgt von 16 h bei RT gerührt. Anschließend wurde das Reaktionsgemisch durch Celite filtriert, mit Ethanol gewaschen (500 ml) und das Filtrat unter vermindertem Druck aufkonzentriert. Das Rohprodukt wurde in THF (100 ml) und n-Hexan (500 ml) aufgenommen und für 3 h gerührt, bevor das gewünschte Produkt abfiltriert wurde. Das Rohprodukt wurde ohne weitere Aufreinigung in die nächste Stufe eingesetzt. Ausbeute: 55%.

**Stufe 2:** Zu einer Suspension aus LAH (13,15 g, 355,45 mmol, 2,5 äq) in THF (600 ml) wurde bei 0°C (E)-3-(4-Methylpiperazin-1-yl)cyclohexanon oxim (30,0 g, 142,18 mmol, 1,0 äq) portionsweise hinzugegeben. Anschließend wurde auf RT erwärmt und 14 h refluxiert. Dann wurde auf 0°C gekühlt und 15 % NaOH Lösung (15 ml) zugegeben. Es wurde filtriert, mit 10% MeOH/ DCM (500 ml) gewaschen und unter vermindertem Druck konzentriert. Ausbeute: 67%.

**Stufe 3:** Zu einer Lösung aus 3-(4-Methylpiperazin-1-yl)cyclohexanamin (59,0 g, 284,26 mmol) in Methanol (70 ml) wurde bei 50°C ethanolische HCl (7N, 150 ml) gegeben und das Gemisch 3 h bei derselben Temperatur gerührt. Die Reaktionsmischung wurde auf RT geühlt und filtriert. Der Feststoff wurde aus MeOH umkristallisiert. Ausbeute: 32%.

**Stufe 4:** Zu einem Gemisch aus (cis)-3-(4-Methylpiperazin-1-yl)cyclohexanamin Trihydrochlorid (5,0 g, 16,39 mmol, 1,0 äq) und K₂CO₃(9,05g, 65,56 mmol, 4,0 äq) in Wasser (15 ml) wurde bei 0°C eine Lösung aus Cbz-Cl (2,85 g, 16,39 mmol, 1,0 äq) in Toluol (25 ml) gegeben und das Reaktionsgemisch 16 h bei RTgerührt. Anschließend wurde die Reaktionsmischung mit Wasser (15 ml) verdünnt und die organische Phase über Natriumsulfat getrocknet und unter vermindertem Druck konzentriert. Das Rohprodukt wurde säulenchromatographisch (Alox neutral, 1 % MeOH/DCM) gereinigt. Ausbeute: 37%.

**Stufe 5:** Ein Gemisch aus Benzyl ((cis)-3-(4-methylpiperazin-1-yl)cyclohexyl)carbamat (2,0 g, 6,04 mmol, 1,0 äq) in Toluol (2,5 ml) und Isopropyl acetat (12 ml) wurde 15 min auf 65°C erwärmt und auf RT gekühlt. (1S)-(+) Camphorsulfonsäure (700 mg, 3,02 mmol, 0,5 äq) und Wasser (0,1 ml) wurden zu dem Gemisch gegeben und es wurde 3 h bei 75°C gerührt. Die Mischung wurde auf RT gekühlt und 3 h gerührt. Anschließend wurde das Gemisch über Nacht bei RT stehen gelassen. Dann wurde filtriert, mit Isopropyl acetat (20 ml) gewaschen und aus EtOH und Isopropyl acetat umkristallisiert. Ausbeute: 14%.

**Stufe 6:** Eine Lösung aus NaOH (0,234 g, 5,852 mmol, 1,1 äq) in Wasser (15 ml) wurde zu einer Suspension aus Benzyl ((1S,3R)-3-(4-methylpiperazin-1-yl)cyclohexyl)carbamat ((1S,4R)-7,7-dimethyl-2-oxo-bicyclo[2.2.1]heptan-1-yl)methanesulfonat (3,0 g, 5,32 mmol, 1,0 äq) in Toluol (15 ml) hinzugegeben und das Gemisch 30 min bei RT gerührt. Anschließend wurde die Reaktionsmischung mit Toluol (15 ml) verdünnt und die organische Phase mit ges. NaCl Lösung gewaschen. Dann wurde über Natriumsulfat getrocknet und unter vermindertem Druck konzentriert. Ausbeute: 96%.

**Stufe 7:** Eine Lösung aus Benzyl ((1S,3R)-3-(4-methylpiperazin-1-yl)cyclohexyl)carbamat (2,0 g, 6,042 mmol, 1,0 äq) in THF (10 ml) wurde bei 0°C zu einer Suspension aus LAH (0,345g, 9,06 mmol, 1,5 äq) in THF (30 ml) hinzugetropft. Anschließend wurde das Gemisch auf RT erwärmt und 30 min refluxiert. Dann wurde auf 0°C gekühlt und 10% NaOH Lösung (0,35 ml) hinzugegeben. Es wurde filtriert, mit THF (2 x 50 ml) gewaschen und und das Filtrat unter vermindertem Druck konzentriert. Ausbeute: 94%.

### Synthese des Aminbausteins AMN-54:

### tert-Butyl 4-amino-3,3-dimethylpiperidin-1-carboxylat (AMN-54)

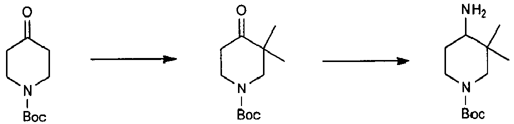

**Stufe 1:** Zu einer Lösung aus t-Butyl 4-oxopiperidin-1-carboxylat (20,0 g, 100,4 mmol, 1,0 äq) in THF (200 ml) wurde Kalium t-butylat (22,48 g, 200,4 mmol, 2,0 äq) bei 0 °C hinzugetropft und das Gemisch 15 min gerührt. Anschließend wurde Mel (15,5 ml, 251,0 mmol, 2,5 äq) langsam hinzueggeben und das Reaktionsgemisch 20 h bei RT gerührt. Nach DC-Kontrolle wurde die Reaktionsmischung mit Eiswasser (500 ml) und Ethylacetat (500 ml) verdünnt. Die organische Phase wurde über Natriumsulfat getrocknet und unter vermindertem Druck konzentriert. Das Rohprodukt wurde säulenchromatographisch (Kieselgel, 3% Ethylacetat in Hexan) gereinigt. Ausbeute: 15%.

**Stufe 2:** Zu einem Gemisch aus t-Butyl 3,3-dimethyl-4-oxopiperidin-1-carboxylat (1,2 g, 5,28 mmol, 1,0 äq) und Ammonium acetat (4,07 g, 52,8 mmol, 10,0 äq) in Methanol (30 ml) wurde AcOH (0,3 ml) gegeben und das Gemisch 3 h bei RT gerührt. NaCNBH₃ (497 mg, 7,92 mmol, 1,5 äq) wurde bei 0°C portionsweise zu dem Reaktionsgemisch gegeben, dann wurde 14 h bei RT gerührt. Nach DC-Kontrolle wurde die Reaktionsmischung und unter vermindertem Druck konzentriert. Das Rohprodukt wurde säulenchromatographisch (Alox neutral, 1,5% MeOH in DCM) gereinigt. Ausbeute: 48%.

### Synthese des Aminbausteins AMN-55:

### 1-(4-Fluorcyclohexyl)-3,3-dimethylpiperidin-4-amin (AMN-55)

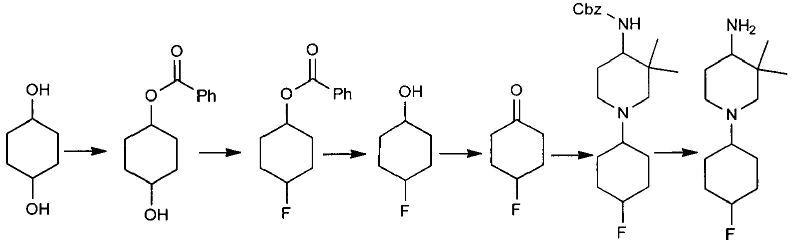

**Stufe 1:** Cyclohexan-1,4-diol (50,0 g, 0,431 mol, 1,0 äq) wurde in Chloroform (750 ml) gelöst und bei 0°C wurden nacheinander Pyridin (104 ml, 1,293 mol, 3,0 äq) und Benzoesäurechlorid (50 ml, 0,344 mol, 0,8 äq) zugegeben und 16 h bei RT gerührt. Nach DC-Kontrolle wurde die Reaktionsmischung mit Chloroform (500 ml) verdünnt, mit Wasser (500 ml), 3M HCl-Lösung (500 ml) und ges. NaCl-Lösung (500 ml) gewaschen, über Natriumsulfat getrocknet und unter vermindertem Druck konzentriert. Das Rohprodukt wurde säulenchromatographisch (Kieselgel, 20% Ethylacetat in Hexan) gereinigt. Ausbeute: 62%.

**Stufe 2:** 4-Hydroxycyclohexyl benzoat (5,0 g, 22,72 mmol, 1,0 äq) wurde in DCM (125 ml) gelöst und bei -78°C wurde DAST (4,5 ml, 34,09 mmol, 1,5 äq) in DCM (125 ml) zugetropft. Es wurde 20 min bei -78°C gerührt, dann auf 0°C erwärmt und 1 h gerührt. Anschließend wurde weitere 2 h bei RT gerührt. Nach DC-Kontrolle wurde die Reaktionsmischung mit DCM (100 ml) verdünnt, mit Wasser (200 ml) und ges. NaCl-Lösung (200 ml) gewaschen, über Natriumsulfat getrocknet und unter vermindertem Druck konzentriert. Das Rohprodukt wurde säulenchromatographisch (Kieselgel, 1,5% Ethylacetat in Hexan) gereinigt. Ausbeute: 21%.

**Stufe 3:** 4-Fluorcyclohexyl benzoat (1,1 g, 4,95 mmol, 1,0 äq) wurde in einer Mischung aus THF und MeOH (1:1, 30 ml) gelöst. Eine 2 M LiOH·H₂O-Lösung (7,5 ml) wurde bei 0°C zugegeben und die Mischung 4 h bei RT gerührt. Nach DC-Kontrolle wurde die Reaktionslösung konzentriert, der Rückstand in Wasser (30 ml) aufgenommen und mit 10%iger MeOH in DCM (100 ml) extrahiert. Die org. Phase wurde über Natriumsulfat getrocknet und unter vermindertem Druck bis zur Trockene konzentriert. Ausbeute: 94%.

**Stufe 4:** Zu einer Mischung aus 4-Fluorcyclohexanol (550 mg, 4,66 mmol, 1,0 äq) und 4Å Molekularsieb (2,0 g) in DCM (37 ml) wurde bei 0°C PCC (1,607g, 7,45 mmol, 1,6 äq) gegeben und 1,5 h bei RT gerührt. Die Reaktionsmischung wurde über Kieselgel filtriert und mehrmals mit DCM gewaschen. Das Filtrat wurde unter vermindertem Druck konzentriert und das Rohprodukt wurde säulenchromatographisch (Kieselgel, 20% Ethylacetat in Hexan) gereinigt. Ausbeute: 70%.

**Stufe 5:** 4-Fluorcyclohexanon (500 mg, 4,31 mmol, 1,0 äq) und Benzyl (3,3-dimethylpiperidin-4-yl)-carbamat (1,13 g, 4,31 mmol, 1,0 äq) wurden in MeOH (43 ml) gelöst und 3 h bei RT gerührt. Die Reaktionsmischung wurde auf 0°C gekühlt, Natriumcyanobrohydrid (866 mg, 12,93 mmol, 3,0 äq) wurde portionsweise zugegeben und die Mischung 14 h bei RT gerührt. Die Reaktionsmischung wurde konzentriert, der Rückstand in Wasser (25 ml) aufgenommen und mit DCM (60 ml) extrahiert. Die org. Phase wurde mit ges. Natriumhydrogencarbonatlösung (25 ml) gewaschen, über Natriumsulfat getrocknet und unter vermindertem Druck konzentriert. Das Rohprodukt wurde säulenchromatographisch (Kieselgel, 15% Ethylacetat in Hexan) gereinigt. Ausbeute: 9%.

**Stufe 6:** Benzyl (1-(4-fluorcyclohexyl)-3,3-dimethylpiperidin-4-yl)carbamat (260 mg, 0,718 mmol, 1,0 äq) wurde in MeOH (10 ml) gelöst und Pd(OH)₂ (130 mg) wurde zugegeben. Die Reaktionslösung wurde 20 min mit N₂ entgast und anschließend 4 h bei RT unter Ballondruck hydriert. Nach DC-Kontrolle wurde die Reaktionsmischung über Celite filtriert und mit Methanol gewaschen. Das Filtrat wurde unter vermindertem Druck konzentriert und das Rohprodukt wurde ohne weitere Reinigung in die nächste Stufe eingesetzt. Ausbeute: 91%.

### Synthese des Aminbausteins AMN-56:

### 4-(Methylamino)-1-(pyridin-4-yl)piperidin-2-on (AMN-56)

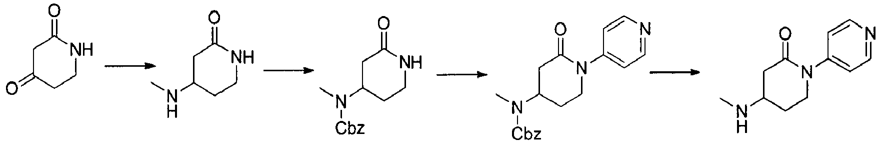

**Stufe 1:** Zu einem Gemisch aus Piperidin-2,4-dion (2,0 g, 17,699 mmol, 1,0 äq) und CH₃NH₂·HCl (2,37 g, 35,398 mmol, 2,0 äq) in MeOH (20 ml) wurde AcOH (0,1 ml) gegeben und das Gemisch 2 h bei RT gerührt. NaBH₃CN (3,29 g, 53,097 mmol, 3,0 äq) wurde bei 0°C protionsweise zu der Mischung hinzugegeben und es wurde 14 h bei RT gerührt. Nach DC- Kontrolle wurde das Reaktionsgemisch filtriert und das Filtrat unter vermindertem Druck konzentriert. Das so erhaltene Roprodukt wurde ohne weitere Aufreinigung in die nächste Stufe eingesetzt. Ausbeute: 81 %.

**Stufe 2:** Zu einem Gemisch aus 4-(Methylamino)piperidin-2-on (1,814 g, 14,173 mmol, 1,0 äq) und K₂CO₃ (3,91 g, 28,346 mmol, 2,0 äq) in THF (50 ml) wurde bei 0°C Cbz-Cl (7,2 ml, 21,259 mmol, 1,5 äq, 50% Lösung in Toluol) gegeben und das Gemisch 14 h bei RT gerührt. Nach DC-Kontrolle wurde die Reaktionsmischung mit DCM (300 ml) verdünnt und mit Wasser (150 ml) und ges. NaCl Lösung (150 ml) gewaschen. Die organische Phase wurde über Natriumsulfat getrocknet und unter vermindertem Druck konzentriert. Das Rohprodukt wurde säulenchromatographisch (Alox neutral, 1% MeOH in DCM) gereinigt. Ausbeute: 27%.

**Stufe 3:** Ein Gemisch aus Benzyl methyl(2-oxopiperidin-4-yl)carbamat (300 mg, 1,145 mmol, 1,0 äq), 4-Brompyridin hydrochlorid (244 mg, 1,259 mmol, 1,1 äq) und Cs₂CO₃ (744 mg, 2,29 mmol, 2,0 äq) in trockenem Toluol wurde 20 min mit N₂ entgast. Dann wurde Pd₂(dba)₃ (104 mg, 0,114 mmol, 0,1 äq) und Xantphos (66 mg, 0,114 mmol, 0,1 äq) hinzugegeben und das Gemisch 14 h bei 90°C in einem geschlosssenen Kolben erhitzt. Das Reaktionsgemisch wurde filtriert und das Filtrat unter vermindertem Druck konzentriert. Das Rohprodukt wurde säulenchromatographisch (Alox neutral, 1 % MeOH in DCM) gereinigt. Ausbeute: 41%.

**Stufe 4:** Eine Lösung aus Benzyl methyl(2-oxo-1-(pyridin-4-yl)piperidin-4-yl)carbamat (300 mg, 0,885 mmol, 1,0 äq) in MeOH wurde 15 min mit N₂ entgast. Anschließend wurde Pd(OH)₂ (240 mg) hinzugegeben und das Reaktionsgemisch 4 h bei RT unter Ballondruck hydriert. Nach DC-Kontrolle wurde das Reaktionsgemisch über Celite filtriert und das Filtrat unter vermindertem Druck konzentriert. Das Rohprodukt wurde säulenchromatographisch (Alox neutral, 3% MeOH in DCM) gereinigt. Ausbeute: 77%.

### Synthese des Aminbausteins AMN-57:

### 1-(3-Fluorpyridin-4-yl)-N-methylpiperidin-4-amin (AMN-57)

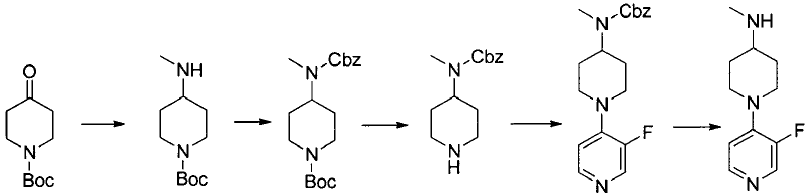

**Stufe 1:** Zu einem Gemisch aus tert-Butyl 4-oxopiperidin-1-carboxylat (20,0 g, 100,50 mmol, 1,0 äq) und CH₃NH₂.HCl (10 g, 150,75 mmol, 1,5 äq) in MeOH (300 ml) wurde AcOH (0,5 ml) gegeben und das Gemisch 2 h bei RT gerührt. NaBH₃CN (12,6 g, 200,00 mmol, 2,0 äq) wurde bei 0°C portionsweise zu der Mischung hinzugegeben und es wurde 14 h bei RT gerührt. Das Gemisch wurde unter vermindertem Druck konzentriert und mit ges. NaHCO₃ Lsöung (150 ml) verdünnt. Es wurde mit DCM (2 x 400 ml) extrahiert und die organische Phase mit ges. NaCl Lösung (200 ml) gewaschen. Anschließend wurde über Natriumsulfat getrocknet und unter vermindertem Druck konzentriert. Das Rohprodukt wurde säulenchromatographisch (Alox neutral, 3% MeOH in DCM) gereinigt. Ausbeute: 57%.

**Stufe 2:** Zu einem Gemisch aus t-Butyl 4-(methylamino)piperidin-1-carboxylat (3,0 g, 14,018 mmol, 1,0 äq) und K₂CO₃ (3,8 g, 28,03 mmol, 2,0 äq) in DCM (46 ml) wurde bei 0°C Cbz-Cl (2,0 ml, 16,82 mmol, 1,2 äq) hinzugegeben und das Gemisch 14 h bei RT gerührt. Die Reaktionsmischung wurde mit DCM (100 ml) verdünnt und mit Wasser (40 ml) und ges. NaCl Lösung (40 ml) gewaschen. Dann wurde die organische Phase wurde über Natriumsulfat getrocknet und unter vermindertem Druck konzentriert. Das Rohprodukt wurde säulenchromatographisch (Kieselgel, 20% Ethylacetat in Hexan) gereinigt. Ausbeute: 62%.

**Stufe 3:** TFA (5 ml) wurde bei 0°C zu einer Lösung aus t-Butyl 4-(((benzyloxy)carbonyl)(methyl)amino)-piperidin-1-carboxylat (2,0 g, 5,747 mmol, 1,0 äq) in DCM (20 ml) gegeben und das Gemisch 1 h bei RT gerührt. Nach DC- Kontrolle wurde das Reaktionsgemisch unter vermindertem Druck konzentriert. Das so erhaltene Roprodukt wurde ohne weitere Aufreinigung in die nächste Stufe eingesetzt.

**Stufe 4:** Kalium tert-butylat (1,27 g, 11,363 mmol, 2,0 äq) wurde zu einem Gemisch aus Benzyl methyl-(piperidin-4-yl)carbamat (1,40 g, 5,681 mmol, 1,0 äq) und 4-Brom-3-fluorpyridin (1,0 g, 5,681 mmol, 1,0 äq) in Toluol (50 ml) gegeben. Dann wurde das Reaktionsgemisch 15 min mit N₂ entgast. BINAP (176 mg, 0,284 mmol, 0,05 äq) und Pd₂(dba)₃ (259 mg, 0,284 mmol, 0,05 äq) wurden zu dem Gemisch gegeben und es wurde 16 h auf 100°C erhitzt. Anschließend wurde die Reaktionsmischung über Celite filtriert, mit 5% MeOH in DCM (2 x 20 ml) gewaschen und das Filtrat unter vermindertem Druck konzentriert. Das Rohprodukt wurde säulenchromatographisch (Kieselgel, 3% MeOH in DCM) gereinigt. Ausbeute: 68%.

**Stufe 5:** Eine Lösung aus Benzyl (1-(3-fluorpyridin-4-yl)piperidin-4-yl)(methyl)carbamat (1,3 g, 3,790 mmol, 1,0 äq) in MeOH (15 ml) wurde 15 min mit N₂ entgast. Anschließend wurde Pd(OH)₂ (400 mg) hinzugegeben und das Reaktionsgemisch 2 h bei RT unter Ballondruck hydriert. Nach DC-Kontrolle wurde das Gemisch über Celite filtriert, mit MeOH (2 x 10 ml) gewaschen und das Filtrat unter vermindertem Druck konzentriert. Das Rohprodukt wurde säulenchromatographisch (Alox neutral, 2% MeOH in DCM) gereinigt. Ausbeute: 63%.

### Synthese des Aminbausteins AMN-58:

### N,N-Dimethyl-4-(4-(methylamino)piperidin-1-yl)pyridin-2-amin (AMN-58)

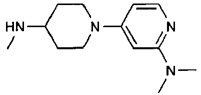

Die Synthese wurde analog der Synthese des Amins **AMN-57** durchgeführt.

### Synthese des Aminbausteins AMN-59:

### N,N-Dimethyl-4-(4-(methylamino)piperidin-1-yl)pyrimidin-2-amin (AMN-59)

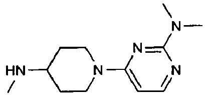

Die Synthese wurde analog der Synthese des Amins **AMN-57** durchgeführt.

### C. Einzelsubstanzsynthesen

### Allgemeine Methode zur Synthese der Beispielverbindungen (SC)

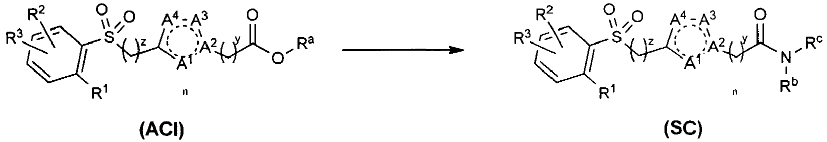

### Allgemeine Arbeitsvorschrift 1 (AAV-1):

Der Ethylester (1,0 äq) wurde in Ethanol und DIPEA (2,0-4,0 äq) gelöst. Das Amin (1,1 äq, als Hydrochlorid oder Base) wurde zugegeben und die Reaktionslösung 16 h auf Siedetemperatur erhitzt. Es fiel ein heller Niederschlag aus. Nach dünnschichtchromatographischer Kontrolle wurde die Reaktionsmischung mit Ethylacetat (ca. 20 ml) verdünnt, eventuell vorher das Ethanol einrotiert. Der entstandene Niederschlag wurde abgesaugt, mit Ethylacetat (10 ml) gewaschen und im Vakuum getrocknet.

### Allgemeine Arbeitsvorschrift 2 (AAV-2):

Der Ethylester (1,0 äq) wurde in Ethanol und DIPEA (2,0-4,0 äq) gelöst. Das Amin (1,1 äq, als Hydrochlorid oder Base) wurde zugegeben und die Reaktionslösung 16 h auf Siedetemperatur erhitzt. Nach dünnschichtchromatographischer Kontrolle wurde die Reaktionsmischung mit Ethylacetat verdünnt, mit ges. Natriumhydrogencarbonatlösung (eventuell zusätzlich mit ges. NaCl-Lösung) gewaschen, über Natriumsulfat oder Magnesiumsulfat getrocknet und unter vermindertem Druck konzentriert. Der Rückstand wurde säulenchromatographisch (Kieselgel, Ethylacetat/Ethanol/Ammoniak (25%ig aq.)) gereinigt.

### Allgemeine Arbeitsvorschrift 3 (AAV-3):

Die Carbonsäure (1,0 äq) wurde in DCM gelöst und bei 0°C mit N-Ethyldiisopropylamin (2,5-5,0 äq) versetzt. EDCI (1,2 äq) und HOBt (0,2 äq) wurden bei 0°C zugegeben und das Reaktionsgemisch 15 min bei RT gerührt. Es wurde wieder auf 0°C gekühlt, das Amin (1,0 äq) zugegeben und zwischen 16 h und 2,5 d bei RT gerührt. Nach vollständiger Umsetzung (DC-Kontrolle) wurde das Reaktionsgemisch mit Ethylacetat verdünnt. Die organische Phase wurde mit 10%iger NH₄Cl-Lösung, ges. NaHCO₃-Lösung und ges. NaCl-Lösung gewaschen und über Natriumsulfat oder Magnesiumsulfat getrocknet und abfiltriert. Das Lösungsmittel wurde eingeengt und der Rückstand säulenchromatographisch (Kieselgel, Ethylacetat/Ethanol/Ammoniak (25%ig aq.) oder Ethylacetat/Cyclohexan) gereinigt.

### Allgemeine Arbeitsvorschrift 4 (AAV-4):

Der Ethylester (1,0 äq) wurde in Ethanol und DIPEA (2,0-4,0 äq) gelöst. Das Amin (1,1 äq, als Hydrochlorid oder Base) wurde zugegeben und die Reaktionslösung 16 h auf Siedetemperatur erhitzt. Nach dünnschichtchromatographischer Kontrolle wurde die Reaktionsmischung mit Kieselgel versetzt und unter vermindertem Druck konzentriert. Der Rückstand wurde säulenchromatographisch (Kieselgel, Ethylacetat/Ethanol oder Cyclohexan/Ammoniak (25%ig aq.)) gereinigt.

### Allgemeine Arbeitsvorschrift 5 (AAV-5):

Der Ethylester (1,0 äq) und das Amin (1,1 äq) wurde in Ethanol und DIPEA (4 äq) gelöst und die Reaktionslösung 6 h auf Siedetemperatur erhitzt. Die Reaktionsmischung wurde unter vermindertem Druck konzentriert, in Ethylacetat gelöst, mit Wasser und mit ges. NaCl-Lösung gewaschen, über Natriumsulfat getrocknet und unter vermindertem Druck konzentriert. Der Rückstand wurde säulenchromatographisch (Alox - neutral, MeOH/DCM) aufgereinigt.

### Allgemeine Arbeitsvorschrift 6 (AAV-6):

Die Carbonsäure (1.0 äq.) wurde in DCM gelöst und DIPEA (2,0-3,0 äq) und HATU (1,0-1,3 äq) bei 0°C hinzugegeben. Anschließend wurde 15 min bei RT gerührt, bevor eine Lösung des Amins (1,0-1,1 äq) in DCM bei 0°C hinzugegeben wurde. Das Reaktionsgemisch wurde 14-24 h bei RT gerührt, anschließend mit DCM verdünnt und mit ges. Ammoniumchloridlösung, ges. Natriumhydrogencarbonatlösung, Wasser und ges. NaCl-Lösung gewaschen, über Natriumsulfat getrocknet und unter vermindertem Druck konzentriert. Der Rückstand wurde säulenchromatographisch (Kieselgel oder neutrales Aluminumoxid, MeOH/DCM) aufgereinigt.

### Allgemeine Arbeitsvorschrift 7 (AAV-7):

Der Ethylester (1,0 äq) und das Amin (1,5 äq) wurde in Ethanol und DIPEA (4 äq) gelöst und die Reaktionslösung 20 h in einem geschlossenen Kolben auf 100°C erhitzt. Die Reaktionsmischung wurde unter vermindertem Druck konzentriert und der Rückstand säulenchromatographisch (Kieselgel, MeOH/DCM) aufgereinigt.

### Allgemeine Arbeitsvorschrift 8 (AAV-8):

Die Carbonsäure (1.0 äq.) wurde in DCM gelöst und DIPEA (2,0-4,0 äq), EDCI (1,5 äq) und HOBt (1,0 äq) bei 0°C hinzugegeben. Anschließend wurde 15 min bei RT gerührt, bevor eine Lösung des Amins (1,2 äq) in DCM bei 0°C hinzugegeben wurde. Das Reaktionsgemisch wurde 14 h bei RT gerührt, anschließend mit DCM verdünnt und mit ges. Ammoniumchloridlösung, ges. Natriumhydrogencarbonat-lösung, Wasser und ges. NaCl-Lösung gewaschen, über Natriumsulfat getrocknet und unter vermindertem Druck konzentriert. Der Rückstand wurde säulenchromatographisch (Kieselgel, MeOH/DCM) oder via prep-DC aufgereinigt.

### Allgemeine Arbeitsvorschrift 9 (AAV-9):

Eine 2 M Lösung (CH₃)₃Al (1,2-3.0 äq) in Toluol wurde bei 0°C zu einer Lösung des Amins (1,1-1.2 äq) in Toluol oder DCM gegeben und anschließend optional 30 min gerührt. Dann wurde eine Lösung des Ethylesters (1,0 äq) in Toluol oder DCM hinzugegeben und das Gemisch 14-16 h bei RT gerührt. Nach vollständiger Umsetzung (DC-Kontrolle) wurde ges. Ammoniumchloridlösung zugegeben und mit DCM extrahiert (2x). Die vereinigten org. Phasen wurden Wasser und ges. NaCl-Lösung gewaschen, über Natriumsulfat getrocknet und unter vermindertem Druck konzentriert. Der Rückstand wurde säulenchromatographisch (Kieselgel oder neutrales Aluminumoxid, MeOH/DCM) gereinigt.

### Allgemeine Arbeitsvorschrift 10 (AAV-10):

Ein Gemisch aus dem Ethylester (1,0 äq), dem Amin (1,0 äq) und DIPEA (2,5 äq) wurde 16 h in EtOH refluxiert. Anschließend wurde unter vermindertem Druck konzentriert und der Rückstand säulenchromatographisch (Kieselgel, MeOH/DCM) und via prep-HPLC gereinigt.

### Allgemeine Arbeitsvorschrift 11 (AAV-11):

Die Carbonsäure (1.0 äq.) wurde in THF gelöst und DIPEA (2,5-4,0 äq) und HATU (1,0-1,2 äq) bei 0°C hinzugegeben. Anschließend wurde 15 min gerührt, bevor eine Lösung des Amins (1,0-1,2 äq) in THF hinzugegeben wurde. Das Reaktionsgemisch wurde 16 h bei RT gerührt, anschließend mit DCM oder Ethylacetat verdünnt und mit ges. Natriumhydrogencarbonatlösung, ges. Ammoniumchloridlösung, Wasser und ges. NaCl-Lösung gewaschen, über Natriumsulfat getrocknet und unter vermindertem Druck konzentriert. Der Rückstand wurde säulenchromatographisch (Kieselgel oder neutrales Aluminumoxid, MeOH/DCM) aufgereinigt.

| **Beispiel Nr.** | **Struktur** | **Name** | **Carbonsäure bzw.-ester (ACI)** | **Amin (AMN)** | **Synthese nach** | **Ausbeute** |
|---|---|---|---|---|---|---|
| **SC-01** | 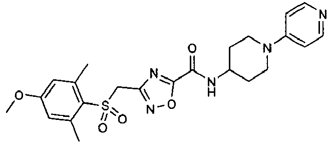 | 3-[[(4-Methoxy-2,6-dimethyl-phenyl)sulfonyl]-methyl]-N-(1-pyridin-4-yl-piperidin-4-yl)-[1,2,4]oxadiazol-5-carbonsäure amid | Ethyl 3-(((4-methoxy-2,6-dimethylphenyl)sulfonyl)methyl)-1,2,4-oxadiazol-5-carboxylat (ACI-1) | 1-(Pyridin-4-yl)piperidin-4-amin dihydrochlorid (AMN-1) | AAV-1 | 70% |
| **SC-02** | 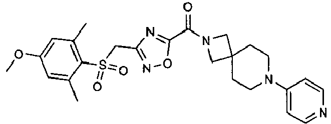 | [3-[[(4-Methoxy-2,6-dimethyl-phenyl)sulfonyl]-methyl]-[1,2,4]oxadiazol-5-yl]-(7-pyridin-4-yl-2,7-diazaspiro[3.5]nonan-2-yl)-methanon | Ethyl 3-(((4-methoxy-2,6-dimethylphenyl)sulfonyl)methyl)-1,2,4-oxadiazol-5-carboxylat (ACI-1) | 7-(Pyridin-4-yl)-2,7-diazaspiro[3.5]nonan dihyrdochlorid(AMN-2) | AAV-1 | 75% |
| **SC-03** | 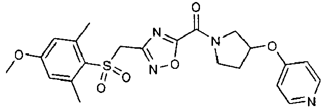 | [3-[[(4-Methoxy-2,6-dimethyl-phenyl)sulfonyl]-methyl]-[1,2,4]oxadiazol-5-yl]-(3-pyridin-4-yloxy-pyrrolidin-1-yl)-methanon | Ethyl 3-(((4-methoxy-2,6-dimethylphenyl)sulfonyl)methyl)-1,2,4-oxadiazol-5-carboxylat (ACI-1) | 4-(Pyrrolidin-3-yloxy)pyridin (AMN-3) | AAV-2 | 72% |
| **SC-04** | 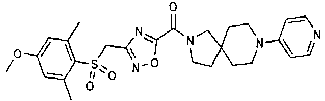 | [3-[[(4-Methoxy-2,6-dimethyl-phenyl)sulfonyl]-methyl]-[1,2,4]oxadiazol-5-yl]-(8-pyridin-4-yl-3,8-diazaspiro[4.5]decan-3-yl)-methanon | Ethyl 3-(((4-methoxy-2,6-dimethylphenyl)sulfonyl)methyl)-1,2,4-oxadiazol-5-carboxylat (ACI-1) | 8-(Pyridin-4-yl)-2,8-diazaspiro[4.5]decan dihydrochlorid(AMN-4) | AAV-1 | 60% |
| **SC-05** | 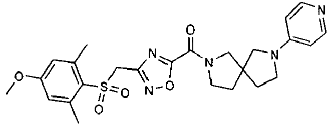 | [3-[[(4-Methoxy-2,6-dimethyl-phenyl)sulfonyl]-methyl]-[1,2,4]oxadiazol-5-yl]-(8-pyridin-4-yl-3,8-diazaspiro[4.4]nonan-3-yl)-methanon | Ethyl 3-(((4-methoxy-2,6-dimethylphenyl)sulfonyl)methyl)-1,2,4-oxadiazol-5-carboxylat (ACI-1) | 2-(Pyridin-4-yl)-2,7-diazaspiro[4.4]nonan dihydrochlorid (AMN-5) | AAV-2 | 58% |
| **SC-06** | 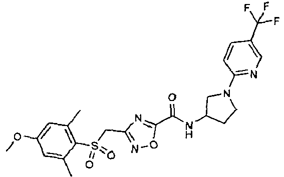 | 3-[[(4-Methoxy-2,6-dimethyl-phenyl)sulfonyl]-methyl]-N-[1-[5-(trifluormethyl)-pyridin-2-yl]-pyrrolidin-3-yl]-[1,2,4]oxadiazol-5-carbonsäure amid | Ethyl 3-(((4-methoxy-2,6-dimethylphenyl)sulfonyl)methyl)-1,2,4-oxadiazol-5-carboxylat (ACI-1) | 1-(5-(Trifluormethyl)pyridin-2-yl)pyrrolidin-3-amin (AMN-6) | AAV-2 | 46% |
| **SC-07** | 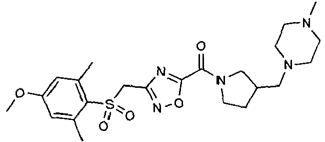 | [3-[[(4-Methoxy-2,6-dimethyl-phenyl)sulfonyl]-methyl]-[1,2,4]oxadiazol-5-yl]-[3-[(4-methyl-piperazin-1-yl)-methyl]-pyrrolidin-1-yl]-methanon | Ethyl 3-(((4-methoxy-2,6-dimethylphenyl)sulfonyl)methyl)-1,2,4-oxadiazol-5-carboxylat (ACI-1) | 1-Methyl-4-(pyrrolidin-3-ylmethyl)piperazin (AMN-7) | AAV-1 | 88% |
| **SC-08** | 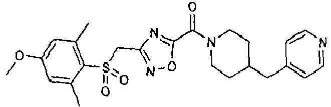 | [3-[[(4-Methoxy-2,6-dimethyl-phenyl)sulfonyl]-methyl]-[1,2,4]oxadiazol-5-yl]-[4-(pyridin-4-yl-methyl)-piperidin-1-yl]-methanon | Ethyl 3-(((4-methoxy-2,6-dimethylphenyl)sulfonyl)methyl)-1,2,4-oxadiazol-5-carboxylat (ACI-1) | 4-(Piperidin-4-ylmethyl)pyridin dihydrochlorid (AMN-8) | AAV-2 | 39% |
| **SC-09** | 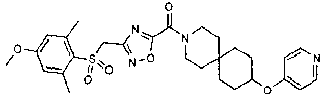 | [3-[[(4-Methoxy-2,6-dimethyl-phenyl)sulfonyl]-methyl]-[1,2,4]oxadiazol-5-yl]-(9-pyridin-4-yloxy-3-azaspiro[5.5]undecan-3-yl)-methanon | Ethyl 3-(((4-methoxy-2,6-dimethylphenyl)sulfonyl)methyl)-1,2,4-oxadiazol-5-carboxylat (ACI-1) | 9-(Pyridin-4-yloxy)-3-azaspiro[5.5]undecan dihydrochlorid (AMN-9) | AAV-2 | 43% |
| **SC-10** | 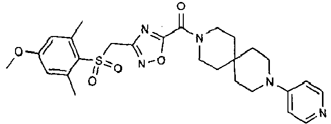 | [3-[[(4-Methoxy-2,6-dimethyl-phenyl)sulfonyl]-methyl]-[1,2,4]oxadiazol-5-yl]-(9-pyridin-4-yl-3,9-diazaspiro[5.5]undecan-3-yl)-methanon | Ethyl 3-(((4-methoxy-2,6-dimethylphenyl)sulfonyl)methyl)-1,2,4-oxadiazol-5-carboxylat (ACI-1) | 3-(Pyridin-4-yl)-3,9-diazaspiro[5.5]undecan dihydrochlorid (AMN-10) | AAV-5 | 20% |
| **SC-11** | 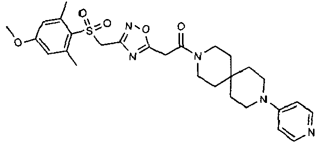 | 2-[3-[[(4-Methoxy-2,6-dimethyl-phenyl)sulfonyl]-methyl]-[1,2,4]oxadiazol-5-yl]-1-(9-pyridin-4-yl-3,9-diazaspiro[5.5]undecan-3-yl)-ethanon | 2-(3-((4-Methoxy-2,6-dimethylphenylsulfonyl)methyl)-1,2,4-oxadiazol-5-yl)essigsäure (ACI-2) | 3-(Pyridin-4-yl)-3,9-diazaspiro[5.5]undecan dihydrochlorid (AMN-10) | AAV-6 | 23% |
| **SC-12** | 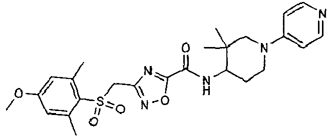 | N-(3,3-Dimethyl-1-pyridin-4-yl-piperidin-4-yl)-3-[[(4-methoxy-2,6-dimethyl-phenyl)sulfonyl]-methyl]-[1,2,4]oxadiazol-5-carbonsäure amid | Ethyl 3-(((4-methoxy-2,6-dimethylphenyl)sulfonyl)methyl)-1,2,4-oxadiazol-5-carboxylat (ACI-1) | 3,3-Dimethyl-1-(pyridin-4-yl)piperidin-4-amin (AMN-11) | AAV-7 | 66% |
| **SC-13** | 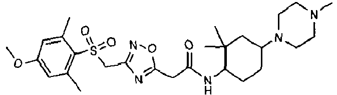 | N-[2,2-Dimethyl-4-(4-methyl-piperazin-1-yl)-cyclohexyl]-2-[3-[[(4-methoxy-2,6-dimethyl-phenyl)sulfonyl]-methyl]-[1,2,4]oxadiazol-5-yl]-acetamid | 2-(3-((4-Methoxy-2,6-dimethylphenylsulfonyl)methyl)-1,2,4-oxadiazol-5-yl)essigsäure (ACI-2) | 2,2-Dimethyl-4-(4-methylpiperazin-1-yl)cyclohexanamin (AMN-12) | AAV-8 | 43% |
| **SC-14** | 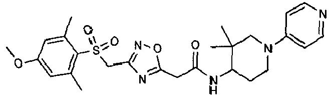 | N-(3,3-Dimethyl-1-pyridin-4-yl-piperidin-4-yl)-2-[3-[[(4-methoxy-2,6-dimethyl-phenyl)sulfonyl]-methyl]-[1,2,4]oxadiazol-5-yl]-acetamid | 2-(3-((4-Methoxy-2,6-dimethylphenylsulfonyl)methyl)-1,2,4-oxadiazol-5-yl)essigsäure (ACI-2) | 3,3-Dimethyl-1-(pyridin-4-yl)piperidin-4-amin (AMN-11) | AAV-8 | 19% |
| **SC-16** | 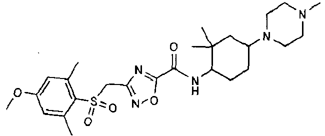 | N-[2,2-Dimethyl-4-(4-methyl-piperazin-1-yl)-cyclohexyl]-3-[[(4-methoxy-2,6-dimethyl-phenyl)sulfonyl]-methyl]-[1,2,4]oxadiazol-5-carbonsäure | Ethyl 3-(((4-methoxy-2,6-dimethylphenyl)sulfonyl)methyl)-1,2,4-oxadiazol-5-carboxylat (ACI-1) | 2,2-Dimethyl-4-(4-methylpiperazin-1-yl)cyclohexanamin (AMN-12) | AAV-9 | 37% |
| **SC-18** | 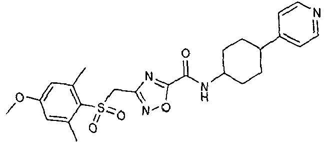 | 3-[[(4-Methoxy-2,6-dimethyl-phenyl)sulfonyl]-methyl]-N-(4-pyridin-4-yl-cyclohexyl)-[1,2,4]oxadiazol-5-carbonsäure amid | Ethyl 3-(((4-methoxy-2,6-dimethylphenyl)sulfonyl)methyl)-1,2,4-oxadiazol-5-carboxylat (ACI-1) | 4-(Pyridin-4-yl)cyclohexanamin (AMN-14) | AAV-10 | 14% |
| **SC-19** | 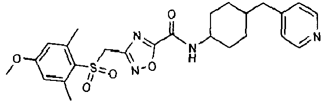 | 3-[[(4-Methoxy-2,6-dimethyl-phenyl)sulfonyl]-methyl]-N-[4-(pyridin-4-yl-methyl)-cyclohexyl]-[1,2,4]oxadiazol-5-carbonsäure amid | Ethyl 3-(((4-methoxy-2,6-dimethylphenyl)sulfonyl)methyl)-1,2,4-oxadiazol-5-carboxylat (ACI-1) | 4-(Pyridin-4-ylmethyl)cyclohexanamin (AMN-15) | AAV-9 | 27% |
| **SC-20** | 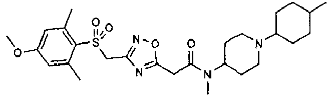 | 2-[3-[[(4-Methoxy-2,6-dimethyl-phenyl)sulfonyl]-methyl]-[1,2,4]oxadiazol-5-yl]-N-methyl-N-[1-(4-methyl-cyclohexyl)-piperidin-4-yl]-acetamid | 2-(3-((4-Methoxy-2,6-dimethylphenylsulfonyl)methyl)-1,2,4-oxadiazol-5-yl)essigsäure (ACI-2) | N-Methyl-1-(4-methylcyclohexyl)piperidin -4-amin (AMN-16) | AAV-3 | 43% |
| **SC-21** | 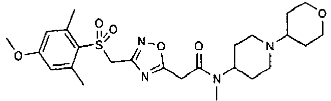 | 2-[3-[[(4-Methoxy-2,6-dimethyl-phenyl)sulfonyl]-methyl]-[1,2,4]oxadiazol-5-yl]-N-methyl-N-(1-tetrahydro-pyran-4-yl-piperidin-4-yl)-acetamid | 2-(3-((4-Methoxy-2,6-dimethylphenylsulfonyl)methyl)-1,2,4-oxadiazol-5-yl)essigsäure (ACI-2) | N-Methyl-1-(tetrahydro-2H-pyran-4-yl)piperidin-4-amin (AMN-17) | AAV-3 | 61% |
| **SC-22** | 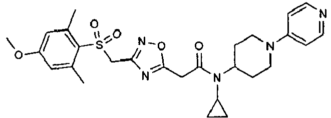 | N-Cyclopropyl-2-[3-[[(4-methoxy-2,6-dimethyl-phenyl)sulfonyl]-methyl]-[1,2,4]oxadiazol-5-yl]-N-(1-pyridin-4-yl-piperidin-4-yl)-acetamid | 2-(3-((4-Methoxy-2,6-dimethylphenylsulfonyl)methyl)-1,2,4-oxadiazol-5-yl)essigsäure (ACI-2) | N-Cyclopropyl-1-(pyridin-4-yl)piperidin-4-amin (AMN-18) | AAV-3 | 63% |
| **SC-27** | 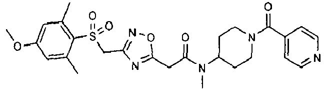 | 2-[3-[[(4-Methoxy-2,6-dimethyl-phenyl)sulfonyl]-methyl]-[1,2,4]oxadiazol-5-yl]-N-methyl-N-[1-(pyridin-4-carbonyl)-piperidin-4-yl]-acetamid | 2-(3-((4-Methoxy-2,6-dimethylphenylsulfonyl)methyl)-1,2,4-oxadiazol-5-yl)essigsäure (ACI-2) | (4-(Methylamino)piperidin-1-yl)(pyridin-4-yl)methanon (AMN-23) | AAV-3 | 60% |
| **SC-30** | 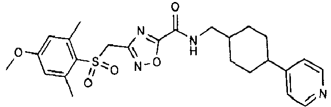 | 3-[[(4-Methoxy-2,6-dimethyl-phenyl)sulfonyl]-methyl]-N-[(4-pyridin-4-yl-cyclohexyl)-methyl]-[1,2,4]oxadiazol-5-carbonsäure amid | Ethyl 3-(((4-methoxy-2,6-dimethylphenyl)sulfonyl)methyl)-1,2,4-oxadiazol-5-carboxylat (ACI-1) | (4-(Pyridin-4-yl)cyclohexyl)methanamin (AMN-25) | AAV-9 | 63% |
| **SC-31** | 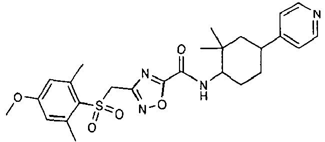 | N-(2,2-Dimethyl-4-pyridin-4-yl-cyclohexyl)-3-[[(4-methoxy-2,6-dimethyl-phenyl)sulfonyl]-methyl]-[1,2,4]oxadiazol-5-carbonsäure amid | Ethyl 3-(((4-methoxy-2,6-dimethylphenyl)sulfonyl)methyl)-1,2,4-oxadiazol-5-carboxylat (ACI-1) | 2,2-Dimethyl-4-(pyridin-4-yl)cyclohexanamin (AMN-26) | AAV-9 | 38% |
| **SC-32** | 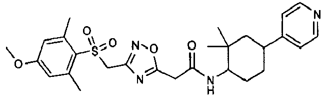 | N-(2,2-Dimethyl-4-pyridin-4-yl-cyclohexyl)-2-[3-[[(4-methoxy-2,6-dimethyl-phenyl)sulfonyl]-methyl]-[1,2,4]oxadiazol-5-yl]-acetamid | 2-(3-((4-Methoxy-2,6-dimethylphenylsulfonyl)methyl)-1,2,4-oxadiazol-5-yl)essigsäure (ACI-2) | 2,2-Dimethyl-4-(pyridin-4-yl)cyclohexanamin (AMN-26) | AAV-8 | 20% |
| **SC-33** | 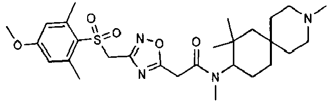 | 2-[3-[[(4-Methoxy-2,6-dimethyl-phenyl)sulfonyl]-methyl]-[1,2,4]oxadiazol-5-yl]-N-methyl-N-(4,4,9-trimethyl-9-azaspiro[5.5]undecan-3-yl)-N-(4-Hydroxy-2, | 2-(3-((4-Methoxy-2,6-dimethylphenylsulfonyl)methyl)-1,2,4-oxadiazol-5-yl)essigsäure (ACI-2) | N,3,8,8-Tetramethyl-3-azaspiro[5.5]undecan-9-amin (AMN-27) | AAV-11 | 17% |
| **SC-35** | 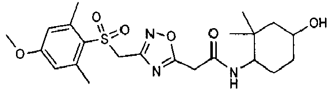 | 2-dimethyl-cyclohexyl)-2-[3-[[(4-methoxy-2,6-dimethyl-phenyl)sulfonyl]-methyl]-[1,2,4]oxadiazol-5-yl]-acetamid | 2-(3-((4-Methoxy-2,6-dimethylphenylsulfonyl)methyl)-1,2,4-oxadiazol-5-yl)essigsäure (ACI-2) | 4-Amino-3,3-dimethylcyclohexanol (AMN-28) | AAV-6 | 29% |
| **SC-36** | 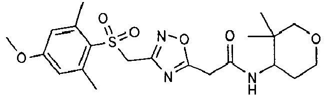 | N-(3,3-Dimethyl-tetrahydro-pyran-4-yl)-2-[3-[[(4-methoxy-2,6-dimethyl-phenyl)sulfonyl]-methyl]-[1,2,4]oxadiazol-5-yl]-acetamid | 2-(3-((4-Methoxy-2,6-dimethylphenylsulfonyl)methyl)-1,2,4-oxadiazol-5-yl)essigsäure (ACI-2) | 3,3-Dimethyltetrahydro-2H-pyran-4-amin (AMN-29) | AAV-8 | 37% |
| **SC-37** | 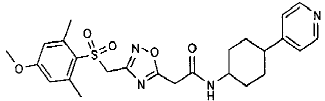 | 2-[3-[[(4-Methoxy-2,6-dimethyl-phenyl)sulfonyl]-methyl]-[1,2,4]oxadiazol-5-yl]-N-(4-pyridin-4-yl-cyclohexyl)-acetamid | 2-(3-((4-Methoxy-2,6-dimethylphenylsulfonyl)methyl)-1,2,4-oxadiazol-5-yl)essigsäure (ACI-2) | 4-(Pyridin-4-yl)cyclohexanamin (AMN-14) | AAV-6 | 38% |
| **SC-39** | 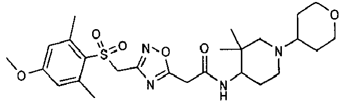 | N-(3,3-Dimethyl-1-tetrahydro-pyran-4-yl-piperidin-4-yl)-2-[3-[[(4-methoxy-2,6-dimethyl-phenyl)sulfonyl]-methyl]-[1,2,4]oxadiazol-5-yl]-acetamid | 2-(3-((4-Methoxy-2,6-dimethylphenylsulfonyl)methyl)-1,2,4-oxadiazol-5-yl)essigsäure (ACI-2) | 3,3-Dimethyl-1-(tetrahydro-2H-pyran-4-yl)piperidin-4-amin (AMN-30) | AAV-6 | 23% |
| **SC-40** | 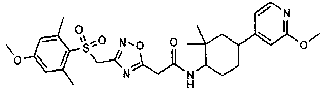 | 2-[3-[[(4-Methoxy-2,6-dimethyl-phenyl)sulfonyl]-methyl]-[1,2,4]oxadiazol-5-yl]-N-[4-(2-methoxy-pyridin-4-yl)-2,2-dimethyl-cyclohexyl]-acetamid | 2-(3-((4-Methoxy-2,6-dimethylphenylsulfonyl)methyl)-1,2,4-oxadiazol-5-yl)essigsäure (ACI-2) | 4-(2-Methoxypyridin-4-yl)-2,2-dimethylcyclohexanamin (AMN-31) | AAV-8 | 61% |
| **SC-41** | 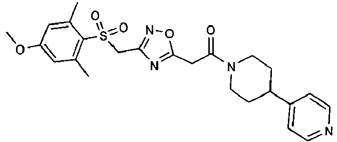 | 2-[3-[[(4-Methoxy-2,6-dimethyl-phenyl)sulfonyl]-methyl]-[1,2,4]oxadiazol-5-yl]-1-(4-pyridin-4-yl-piperidin-1-yl)-ethanon | 2-(3-((4-Methoxy-2,6-dimethylphenylsulfonyl)methyl)-1,2,4-oxadiazol-5-yl)essigsäure (ACI-2) | 4-(Piperidin-4-yl)pyridin (AMN-32) | AAV-6 | 17% |
| **SC-42** | 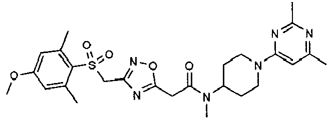 | N-[1-(2,6-Dimethyl-pyrimidin-4-yl)-piperidin-4-yl]-2-[3-[[(4-methoxy-2,6-dimethyl-phenyl)sulfonyl]-methyl]-[1,2,4]oxadiazol-5-yl]-N-methyl-acetamid | 2-(3-((4-Methoxy-2,6-dimethylphenylsulfonyl)methyl)-1,2,4-oxadiazol-5-yl)essigsäure (ACI-2) | 1-(2,6-Dimethylpyrimidin-4-yl)-N-methylpiperidin-4-amin (AMN-33) | AAV-3 | 63% |
| **SC-43** | 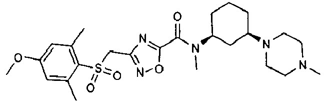 | 3-[[(4-Methoxy-2,6-dimethyl-phenyl)sulfonyl]-methyl]-N-methyl-N-[(1S,3R)-3-(4-methyl-piperazin-1-yl)-cyclohexyl]-[1,2,4]oxadiazol-5-carbonsäure amid | Ethyl 3-(((4-methoxy-2,6-dimethylphenyl)sulfonyl)methyl)-1,2,4-oxadiazol-5-carboxylat (ACI-1) | (1S,3R)-N-Methyl-3-(4-methylpiperazin-1-yl)cyclohexanamin (AMN-53) | AAV-9 | 18% |
| **SC-44** | 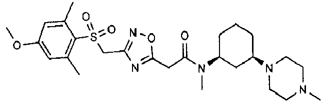 | 2-[3-[[(4-Methoxy-2,6-dimethyl-phenyl)sulfonyl]-methyl]-[1,2,4]oxadiazol-5-yl]-N-methyl-N-[(1S,3R)-3-(4-methyl-piperazin-1-yl)-cyclohexyl]-acetamid | 2-(3-((4-Methoxy-2,6-dimethylphenylsulfonyl)methyl)-1,2,4-oxadiazol-5-yl)essigsäure (ACI-2) | (1 S,3R)-N-Methyl-3-(4-methylpiperazin-1-yl)cyclohexanamin (AMN-53) | AAV-6 | 29% |
| **SC-46** | 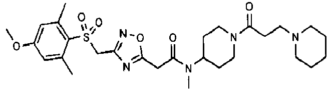 | 2-[3-[[(4-Methoxy-2,6-dimethyl-phenyl)sulfonyl]-methyl]-[1,2,4]oxadiazol-5-yl]-N-methyl-N-[1-(3-piperidin-1-yl-propanoyl)-piperidin-4-yl]-acetamid | 2-(3-((4-Methoxy-2,6-dimethylphenylsulfonyl)methyl)-1,2,4-oxadiazol-5-yl)essigsäure (ACI-2) | 1-(4-(Methylamino)piperidin-1-yl)-3-(piperidin-1-yl)propan-1-on hydrochlorid (AMN-35) | AAV-3 | 66% |
| **SC-48** | 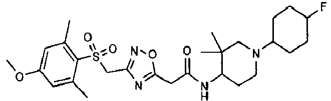 | N-[1-(4-Fluor-cyclohexyl)-3,3-dimethyl-piperidin-4-yl]-2-[3-[[(4-methoxy-2,6-dimethyl-phenyl)sulfonyl]-methyl]-[1,2,4]oxadiazol-5-yl]-acetamid | 2-(3-((4-Methoxy-2,6-dimethylphenylsulfonyl)methyl)-1,2,4-oxadiazol-5-yl)essigsäure (ACI-2) | 1-(4-Fluorcyclohexyl)-3,3-dimethylpiperidin-4-amin (AMN-55) | AAV-6 | 41% |
| **SC-49** | 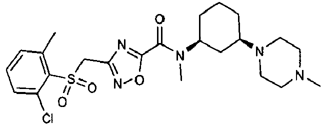 | 3-[[(2-Chlor-6-methyl-phenyl)sulfonyl]-methyl]-N-methyl-N-[(1S,3R)-3-(4-methyl-piperazin-1-yl)-cyclohexyl]-[1,2,4]oxadiazol-5-carbonsäure amid | Ethyl 3-(((2-chlor-6-methylphenyl)sulfonyl)methyl)-1,2,4-oxadiazol-5-carboxylat (ACI-3) | (1S,3R)-N-Methyl-3-(4-methylpiperazin-1-yl)cyclohexanamin (AMN-53) | AAV-9 | 20% |
| **SC-50** | 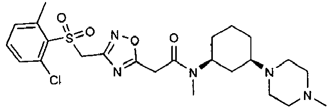 | 2-[3-[[(2-Chlor-6-methyl-phenyl)sulfonyl]-methyl]-[1,2,4]oxadiazol-5-yl]-N-methyl-N-[(1 S,3R)-3-(4-methyl-piperazin-1-yl)-cyclohexyl]-acetamid | 2-(3-(((2-Chlor-6-methylphenyl)sulfonyl)methyl)-1,2,4-oxadiazol-5-yl)essigsäure (ACI-8) | (1S,3R)-N-Methyl-3-(4-methylpiperazin-1-yl)cyclohexanamin (AMN-53) | AAV-11 | 30% |
| **SC-51** | 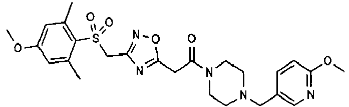 | 2-[3-[[(4-Methoxy-2,6-dimethyl-phenyl)sulfonyl]-methyl]-[1,2,4]oxadiazol-5-yl]-1-[4-[(6-methoxy-pyridin-3-yl)-methyl]-piperazin-1-yl]-ethanon | 2-(3-((4-Methoxy-2,6-dimethylphenylsulfonyl)methyl)-1,2,4-oxadiazol-5-yl)essigsäure (ACI-2) | 1-((6-Methoxypyridin-3-yl)methyl)piperazin dihydrochlorid (AMN-36) | AAV-3 | 57% |
| **SC-52** | 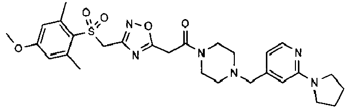 | 2-[3-[[(4-Methoxy-2,6-dimethyl-phenyl)sulfonyl]-methyl]-[1,2,4]oxadiazol-5-yl]-1-[4-[(2-pyrrolidin-1-yl-pyridin-4-yl)-methyl]-piperazin-1-yl]-ethanon | 2-(3-((4-Methoxy-2,6-dimethylphenylsulfonyl)methyl)-1,2,4-oxadiazol-5-yl)essigsäure (ACI-2) | 1-((2-(Pyrrolidin-1-yl)pyridin-4-yl)methyl)piperazin dihydrochlorid (AMN-37) | AAV-3 | 63% |
| **SC-55** | 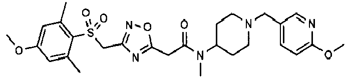 | 2-[3-[[(4-Methoxy-2,6-dimethyl-phenyl)sulfonyl]-methyl]-[1,2,4]oxadiazol-5-yl]-N-[1-[(6-methoxy-pyridin-3-yl)-methyl]-piperidin-4-yl]-N-methyl-acetamid | 2-(3-((4-Methoxy-2,6-dimethylphenylsulfonyl)methyl)-1,2,4-oxadiazol-5-yl)essigsäure (ACI-2) | 1-((6-Methoxypyridin-3-yl)methyl)-N-methylpiperidin-4-amin hydrochlorid (AMN-40) | AAV-3 | 52% |
| **SC-57** | 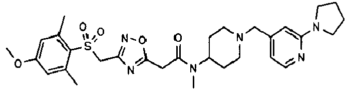 | 2-[3-[[(4-Methoxy-2,6-dimethyl-phenyl)sulfonyl]-methyl]-[1,2,4]oxadiazol-5-yl]-N-methyl-N-[1-[(2-pyrrolidin-1-yl-pyridin-4-yl)-methyl]-piperidin-4-yl]-acetamid | 2-(3-((4-Methoxy-2,6-dimethylphenylsulfonyl)methyl)-1,2,4-oxadiazol-5-yl)essigsäure (ACI-2) | N-Methyl-1-((2-(pyrrolidin-1-yl)pyridin-4-yl)methyl)piperidin-4-amin hydrochlorid (AMN-41) | AAV-3 | 76% |
| **SC-58** | 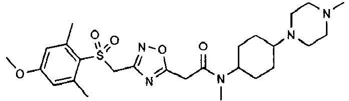 | 2-[3-[[(4-Methoxy-2,6-dimethyl-phenyl)sulfonyl]-methyl]-[1,2,4]oxadiazol-5-yl]-N-methyl-N-[4-(4-methyl-piperazin-1-yl)-cyclohexyl]-acetamid | 2-(3-((4-Methoxy-2,6-dimethylphenylsulfonyl)methyl)-1,2,4-oxadiazol-5-yl)essigsäure (ACI-2) | N-Methyl-4-(4-methylpiperazin-1-yl)cyclohexanamin hydrochlorid (AMN-42) | AAV-3 | 39% |
| **SC-59** | 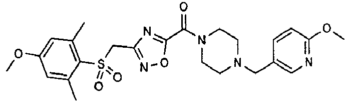 | [3-[[(4-Methoxy-2,6-dimethyl-phenyl)sulfonyl]-methyl]-[1,2,4]oxadiazol-5-yl]-[4-[(6-methoxy-pyridin-3-yl)-methyl]-piperazin-1-yl]-methanon | Ethyl 3-(((4-methoxy-2,6-dimethylphenyl)sulfonyl)methyl)-1,2,4-oxadiazol-5-carboxylat (ACI-1) | 1-((6-Methoxypyridin-3-yl)methyl)piperazin dihydrochlorid (AMN-36) | AAV-4 | 38% |
| **SC-60** | 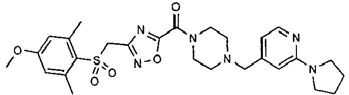 | [3-[[(4-Methoxy-2,6-dimethyl-phenyl)sulfonyl]-methyl]-[1,2,4]oxadiazol-5-yl]-[4-[(2-pyrrolidin-1-yl-pyridin-4-yl)-methyl]-piperazin-1-yl]-methanon | Ethyl 3-(((4-methoxy-2,6-dimethylphenyl)sulfonyl)methyl)-1,2,4-oxadiazol-5-carboxylat (ACI-1) | 1-((2-(Pyrrolidin-1-yl)pyridin-4-yl)methyl)piperazin dihydrochlorid (AMN-37) | AAV-4 | 32% |
| **SC-61** | 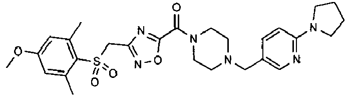 | [3-[[(4-Methoxy-2,6-dimethyl-phenyl)sulfonyl]-methyl]-[1,2,4]oxadiazol-5-yl]-[4-[(6-pyrrolidin-1-yl-pyridin-3-yl)-methyl]-piperazin-1-yl]-methanon | Ethyl 3-(((4-methoxy-2,6-dimethylphenyl)sulfonyl)methyl)-1,2,4-oxadiazol-5-carboxylat (ACI-1) | 1-((6-(Pyrrolidin-1-yl)pyridin-3-yl)methyl)piperazin hydrochlorid (AMN-43) | AAV-4 | 15% |
| **SC-62** | 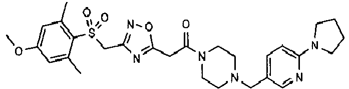 | 2-[3-[[(4-Methoxy-2,6-dimethyl-phenyl)sulfonyl]-methyl]-[1,2,4]oxadiazol-5-yl]-1-[4-[(6-pyrrolidin-1-yl-pyridin-3-yl)-methyl]-piperazin-1-yl]-ethanon | 2-(3-((4-Methoxy-2,6-dimethylphenylsulfonyl)methyl)-1,2,4-oxadiazol-5-yl)essigsäure (ACI-2) | 1-((6-(Pyrrolidin-1-yl)pyridin-3-yl)methyl)piperazin hydrochlorid (AMN-43) | AAV-3 | 43% |
| **SC-63** | 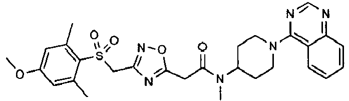 | 2-[3-[[(4-Methoxy-2,6-dimethyl-phenyl)sulfonyl]-methyl]-[1,2,4]oxadiazol-5-yl]-N-methyl-N-(1-chinazolin-4-yl-piperidin-4-yl)-acetamid | 2-(3-((4-Methoxy-2,6-dimethylphenylsulfonyl)methyl)-1,2,4-oxadiazol-5-yl)essigsäure (ACI-2) | N-Methyl-1-(quinazolin-4-yl)piperidin-4-amin (AMN-44) | AAV-3 | 60% |
| **SC-64** | 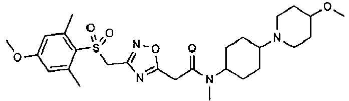 | 2-[3-[[(4-Methoxy-2,6-dimethyl-phenyl)sulfonyl]-methyl]-[1,2,4]oxadiazol-5-yl]-N-[4-(4-methoxy-piperidin-1-yl)-cyclohexyl]-N-methyl-acetamid | 2-(3-((4-Methoxy-2,6-dimethylphenylsulfonyl)methyl)-1,2,4-oxadiazol-5-yl)essigsäure (ACI-2) | 4-(4-Methoxypiperidin-1-yl)-N-methylcyclohexanamin hydrochlorid (AMN-45) | AAV-3 | 22% |
| **SC-65** | 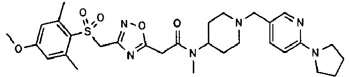 | 2-[3-[[(4-Methoxy-2,6-dimethyl-phenyl)sulfonyl]-methyl]-[1,2,4]oxadiazol-5-yl]-N-methyl-N-[1-[(6-pyrrolidin-1-yl-pyridin-3-yl)-methyl]-piperidin-4-yl]-acetamid | 2-(3-((4-Methoxy-2,6-dimethylphenylsulfonyl)methyl)-1,2,4-oxadiazol-5-yl)essigsäure (ACI-2) | N-Methyl-1-((6-(pyrrolidin-1-yl)pyridin-3-yl)methyl)piperidin-4-amin hydrochlorid (AMN-46) | AAV-3 | 53% |
| **SC-66** | 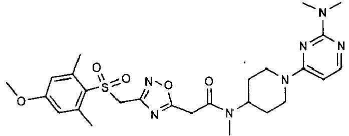 | N-[1-(2-Dimethylamino-pyrimidin-4-yl)-piperidin-4-yl]-2-[3-[[(4-methoxy-2,6-dimethyl-phenyl)sulfonyl]-methyl]-[1,2,4]oxadiazol-5-yl]-N-methyl- | 2-(3-((4-Methoxy-2,6-dimethylphenylsulfonyl)methyl)-1,2,4-oxadiazol-5-yl)essigsäure (ACI-2) | N,N-Dimethyl-4-(4-(methylamino)piperidin-1-yl)pyrimidin-2-amin (AMN-59) | AAV-11 | 51% |
| **SC-67** | 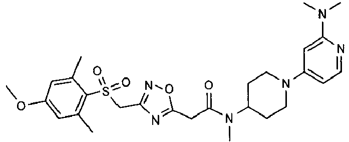 | N-[1-(2-Dimethylamino-pyridin-4-yl)-piperidin-4-yl]-2-[3-[[(4-methoxy-2,6-dimethyl-phenyl)sulfonyl]-methyl]-[1,2,4]oxadiazol-5-yl]-N-methyl- | 2-(3-((4-Methoxy-2,6-dimethylphenylsulfonyl)methyl)-1,2,4-oxadiazol-5-yl)essigsäure (ACI-2) | N,N-Dimethyl-4-(4-(methylamino)piperidin-1-yl)pyridin-2-amin (AMN-58) | AAV-6 | 48% |
| **SC-68** | 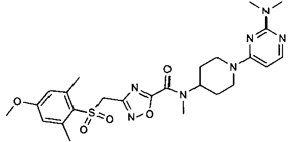 | N-[1-(2-Dimethylamino-pyrimidin-4-yl)-piperidin-4-yl]-3-[[(4-methoxy-2,6-dimethyl-phenyl)sulfonyl]-methyl]-N-methyl-[1,2,4]oxadiazol-5-carbonsäure | Ethyl 3-(((4-methoxy-2,6-dimethylphenyl)sulfonyl)methyl)-1,2,4-oxadiazol-5-carboxylat (ACI-1) | N,N-Dimethyl-4-(4-(methylamino)piperidin-1-yl)pyrimidin-2-amin (AMN-59) | AAV-9 | 40% |
| **SC-69** | 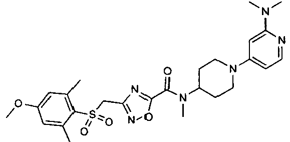 | N-[1-(2-Dimethylamino-pyridin-4-yl)-piperidin-4-yl]-3-[[(4-methoxy-2,6-dimethyl-phenyl)sulfonyl]-methyl]-N-methyl-[1,2,4]oxadiazol-5-carbonsäure amid | Ethyl 3-(((4-methoxy-2,6-dimethylphenyl)sulfonyl)methyl)-1,2,4-oxadiazol-5-carboxylat (ACI-1) | N,N-Dimethyl-4-(4-(methylamino)piperidin-1-yl)pyridin-2-amin (AMN-58) | AAV-9 | 43% |
| **SC-70** | 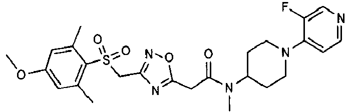 | N-[1-(3-Fluor-pyridin-4-yl)-piperidin-4-yl]-2-[3-[[(4-methoxy-2,6-dimethyl-phenyl)sulfonyl]-methyl]-[1,2,4]oxadiazol-5-yl]-N-methyl-acetamid | 2-(3-((4-Methoxy-2,6-dimethylphenylsulfonyl)methyl)-1,2,4-oxadiazol-5-yl)essigsäure (ACI-2) | 1-(3-Fluorpyridin-4-yl)-N-methylpiperidin-4-amin (AMN-57) | AAV-11 | 44% |
| **SC-72** | 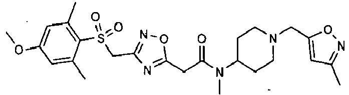 | 2-[3-[[(4-Methoxy-2,6-dimethyl-phenyl)sulfonyl]-methyl]-[1,2,4]oxadiazol-5-yl]-N-methyl-N-[1-[(3-methyl-isoxazol-5-yl)-methyl]-piperidin-4-yl]-acetamid | 2-(3-((4-Methoxy-2,6-dimethylphenylsulfonyl)methyl)-1,2,4-oxadiazol-5-yl)essigsäure (ACI-2) | N-Methyl-1-((3-methylisoxazol-5-yl)methyl)piperidin-4-amin (AMN-47) | AAV-3 | 57% |
| **SC-73** | 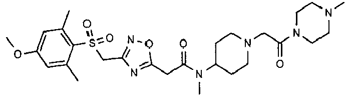 | 2-[3-[[(4-Methoxy-2,6-dimethyl-phenyl)sulfonyl]-methyl]-[1,2,4]oxadiazol-5-yl]-N-methyl-N-[1-(2-(4-methyl-piperazin-1-yl)-2-oxo-ethyl]-piperidin-4-yl]-acetamid | 2-(3-((4-Methoxy-2,6-dimethylphenylsulfonyl)methyl)-1,2,4-oxadiazol-5-yl)essigsäure (ACI-2) | 2-(4-(Methylamino)piperidin-1-yl)-1-(4-methylpiperazin-1-yl)ethanon (AMN-48) | AAV-3 | 56% |
| **SC-77** | 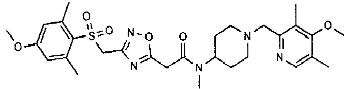 | 2-[3-[[(4-Methoxy-2,6-dimethyl-phenyl)sulfonyl]-methyl]-[1,2,4]oxadiazol-5-yl]-N-[1-[(4-methoxy-3,5-dimethyl-pyridin-2-yl)-methyl]-piperidin-4-yl]-N- | 2-(3-((4-Methoxy-2,6-dimethylphenylsulfonyl)methyl)-1,2,4-oxadiazol-5-yl)essigsäure (ACI-2) | 1-((4-Methoxy-3,5-dimethylpyridin-2-yl)methyl)-N-methylpiperidin-4-amin (AMN-49) | AAV-3 | 68% |
| **SC-79** | 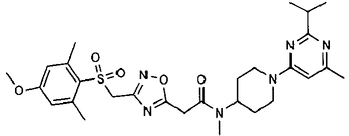 | N-[1-(2-Isopropyl-6-methyl-pyrimidin-4-yl)-piperidin-4-yl]-2-[3-[[(4-methoxy-2,6-dimethyl-phenyl)sulfonyl]-methyl]-[1,2,4]oxadiazol-5-yl]-N-methyl- | 2-(3-((4-Methoxy-2,6-dimethylphenylsulfonyl)methyl)-1,2,4-oxadiazol-5-yl)essigsäure (ACI-2) | 1-(2-Isopropyl-6-methylpyrimidin-4-yl)-N-methylpiperidin-4-amin (AMN-50) | AAV-3 | 67% |
| **SC-80** | 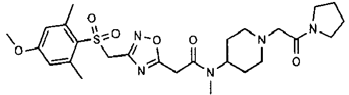 | 2-[3-[[(4-Methoxy-2,6-dimethyl-phenyl)sulfonyl]-methyl]-[1,2,4]oxadiazol-5-yl]-N-methyl-N-[1-(2-oxo-2-pyrrolidin-1-yl-ethyl)-piperidin-4-yl]-acetamid | 2-(3-((4-Methoxy-2,6-dimethylphenylsulfonyl)methyl)-1,2,4-oxadiazol-5-yl)essigsäure (ACI-2) | 2-(4-(Methylamino)piperidin-1-yl)-1-(pyrrolidin-1-yl)ethanon (AMN-51) | AAV-3 | 70% |
| **SC-81** | 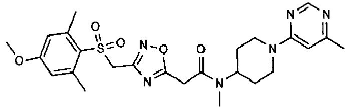 | 2-[3-[[(4-Methoxy-2,6-dimethyl-phenyl)sulfonyl]-methyl]-[1,2,4]oxadiazol-5-yl]-N-methyl-N-[1-(6-methyl-pyrimidin-4-yl)-piperidin-4-yl]-acetamid | 2-(3-((4-Methoxy-2,6-dimethylphenylsulfonyl)methyl)-1,2,4-oxadiazol-5-yl)essigsäure (ACI-2) | N-Methyl-1-(6-methylpyrimidin-4-yl)piperidin-4-amin (AMN-52) | AAV-3 | 61% |
| **SC-82** | 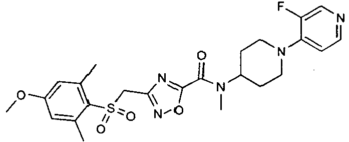 | N-[1-(3-Fluor-pyridin-4-yl)-piperidin-4-yl]-3-[[(4-methoxy-2,6-dimethyl-phenyl)sulfonyl]-methyl]-N-methyl-[1,2,4]oxadiazol-5-carbonsäure amid | Ethyl 3-(((4-methoxy-2,6-dimethylphenyl)sulfonyl)methyl)-1,2,4-oxadiazol-5-carboxylat (ACI-1) | 1-(3-Fluorpyridin-4-yl)-N-methylpiperidin-4-amin (AMN-57) | AAV-9 | 40% |
| **SC-83** | 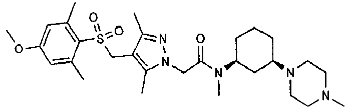 | 2-[4-[[(4-Methoxy-2,6-dimethyl-phenyl)sulfonyl]-methyl]-3,5-dimethyl-1H-pyrazol-1-yl]-N-methyl-N-[(1S,3R)-3-(4-methyl-piperazin-1-yl)-cyclohexyl]- | 2-(4-(((4-Methoxy-2,6-dimethylphenyl)sulfonyl)methyl)-3,5-dimethyl-1 H-pyrazol-1-yl)essigsäure (ACI-4) | (1S,3R)-N-Methyl-3-(4-methylpiperazin-1-yl)cyclohexanamin (AMN-53) | AAV-11 | 39% |
| **SC-84** | 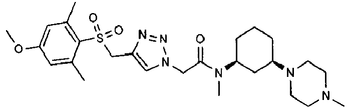 | 2-[4-[[(4-Methoxy-2,6-dimethyl-phenyl)sulfonyl]-methyl]-1H-[1,2,3]triazol-1-yl]-N-methyl-N-[(1S,3R)-3-(4-methyl-piperazin-1-yl)-cyclohexyl]-acetamid | Ethyl 2-(4-(((4-methoxy-2,6-dimethylphenyl)sulfonyl)methyl)-1H-1,2,3-triazol-1-yl)acetat (ACI-5) | (1S,3R)-N-Methyl-3-(4-methylpiperazin-1-yl)cyclohexanamin (AMN-53) | AAV-6 | 29% |
| **SC-85** | 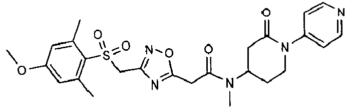 | 2-[3-[[(4-Methoxy-2,6-dimethyl-phenyl)sulfonyl]-methyl]-[1,2,4]oxadiazol-5-yl]-N-methyl-N-(2-oxo-1-pyridin-4-yl-piperidin-4-yl)-acetamid | 2-(3-((4-Methoxy-2,6-dimethylphenylsulfonyl)methyl)-1,2,4-oxadiazol-5-yl)essigsäure (ACI-2) | 4-(Methylamino)-1-(pyridin-4-yl)piperidin-2-on (AMN-56) | AAV-6 | 30% |
| **SC-86** | 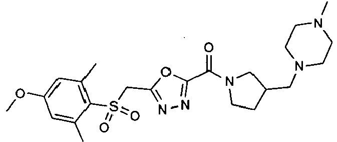 | [5-[[(4-Methoxy-2,6-dimethyl-phenyl)sulfonyl]-methyl]-[1,3,4]oxadiazol-2-yl]-[3-[(4-methyl-piperazin-1-yl)-methyl]-pyrrolidin-1-yl]-methanon | Ethyl 5-(((4-methoxy-2,6-dimethylphenyl)sulfonyl)methyl)-1,3,4-oxadiazol-2-carboxylat (ACI-7) | 1-Methyl-4-(pyrrolidin-3-ylmethyl)piperazin (AMN-7) | AAV-9 | 9% |
| **SC-87** | 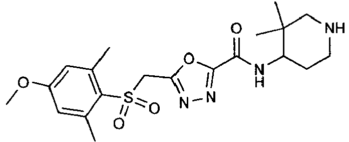 | N-(3,3-Dimethyl-piperidin-4-yl)-5-[[(4-methoxy-2,6-dimethyl-phenyl)sulfonyl]-methyl]-[1,3,4]oxadiazol-2-carbonsäure amid | Ethyl 5-(((4-methoxy-2,6-dimethylphenyl)sulfonyl)methyl)-1,3,4-oxadiazol-2-carboxylat (ACI-7) | tert-Butyl 4-amino-3,3-dimethylpiperidin-1-carboxylat (AMN-54) | siehe unten | |
| **SC-88** | 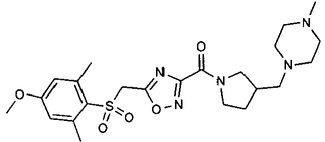 | [5-[[(4-Methoxy-2,6-dimethyl-phenyl)sulfonyl]-methyl]-[1,2,4]oxadiazol-3-yl]-[3-[(4-methyl-piperazin-1-yl)-methyl]-pyrrolidin-1-yl]-methanon | Ethyl 5-(((4-methoxy-2,6-dimethylphenyl)sulfonyl)methyl)-1,2,4-oxadiazol-3-carboxylat (ACI-6) | 1-Methyl-4-(pyrrolidin-3-ylmethyl)piperazin (AMN-7) | AAV-9 | 41% |
| **SC-89** | 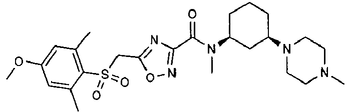 | 5-[[(4-Methoxy-2,6-dimethyl-phenyl)sulfonyl]-methyl]-N-methyl-N-[(1S,3R)-3-(4-methyl-piperazin-1-yl)-cyclohexyl]-[1,2,4]oxadiazol-3-carbonsäure amid | Ethyl 5-(((4-methoxy-2,6-dimethylphenyl)sulfonyl)methyl)-1,2,4-oxadiazol-3-carboxylat (ACI-6) | (1S,3R)-N-Methyl-3-(4-methylpiperazin-1-yl)cyclohexanamin (AMN-53) | AAV-9 | 61% |
| **SC-90** | 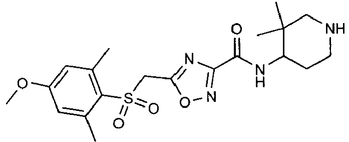 | N-(3,3-Dimethyl-piperidin-4-yl)-5-[[(4-methoxy-2,6-dimethyl-phenyl)sulfonyl]-methyl]-[1,2,4]oxadiazol-3-carbonsäure amid | Ethyl 5-(((4-methoxy-2,6-dimethylphenyl)sulfonyl)methyl)-1,2,4-oxadiazol-3-carboxylat (ACI-6) | tert-Butyl 4-amino-3,3-dimethylpiperidin-1-carboxylat (AMN-54) | siehe unten | |
| **SC-91** | 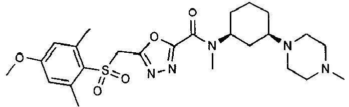 | 5-[[(4-Methoxy-2,6-dimethyl-phenyl)sulfonyl]-methyl]-N-methyl-N-[(1S,3R)-3-(4-methyl-piperazin-1-yl)-cyclohexyl]-[1,3,4]oxadiazol-2-carbonsäure amid | Ethyl 5-(((4-methoxy-2,6-dimethylphenyl)sulfonyl)methyl)-1,3,4-oxadiazol-2-carboxylat (ACI-7) | (1 S,3R)-N-Methyl-3-(4-methylpiperazin-1-yl)cyclohexanamin (AMN-53) | AAV-9 | 5% |
| **SC-92** | 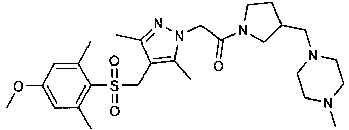 | 2-[4-[[(4-Methoxy-2,6-dimethyl-phenyl)sulfonyl]-methyl]-3,5-dimethyl-1H-pyrazol-1-yl]-1-[3-[(4-methyl-piperazin-1-yl)-methyl]-pyrrolidin-1-yl]-ethanon | 2-(4-(((4-Methoxy-2,6-dimethylphenyl)sulfonyl)methyl)-3,5-dimethyl-1H-pyrazol-1-yl)essigsäure (ACI-4) | 1-Methyl-4-(pyrrolidin-3-ylmethyl)piperazin (AMN-7) | AAV-11 | 59% |
| **SC-93** | 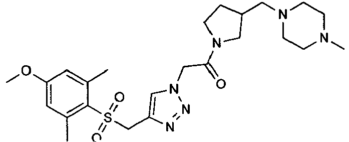 | 2-[4-[[(4-Methoxy-2,6-dimethyl-phenyl)sulfonyl]-methyl]-1H-[1,2,3]triazol-1-yl]-1-[3-[(4-methyl-piperazin-1-yl)-methyl]-pyrrolidin-1-yl]-ethanon | Ethyl 2-(4-(((4-methoxy-2,6-dimethylphenyl)sulfonyl)methyl)-1H-1,2,3-triazol-1-yl)acetat (ACI-5) | 1-Methyl-4-(pyrrolidin-3-ylmethyl)piperazin (AMN-7) | AAV-6 | 17% |

| | | | | | | |
|---|---|---|---|---|---|---|
| **Tabelle: Synthese der Beispielverbindungen (SC)** | | | | | | |

### Synthese der Beispielverbindung SC-87:

### N-(3,3-Dimethyl-piperidin-4-yl)-5-[[(4-methoxy-2,6-dimethyl-phenyl)sulfonyl]-methyl]-[1,3,4]oxadiazol-2-carbonsäure amid (SC-87)

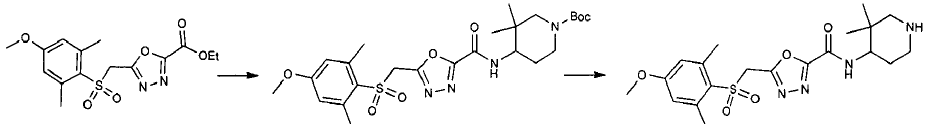

**Stufe 1:** Zu einer Lösung aus Ethyl 5-(((4-methoxy-2,6-dimethylphenyl)sulfonyl)methyl)-1,3,4-oxadiazol-2-carboxylat (**ACI-7**) (500 mg, 1,41 mmol, 1,0 äq) in Ethanol (5 ml) wurde DIPEA (0,6 ml, 3,52 mmol, 2,5 äq) und tert-Butyl 4-amino-3,3-dimethylpiperidin-1-carboxylat (**AMN-54**) (385 mg, 1,69 mmol, 1,2 äq) bei RT himzugegeben und das Gemisch 24 h refluxiert. Das Reaktionsgemisch wurde unter vermindertem Druck konzentriert und das Rohprodukt säulenchromatographisch gereinigt (Kieselgel, 15% Aceton/Hexan). Ausbeute: 23%.

**Stufe 2:** Durch eine Lösung aus tert-Butyl 4-(5-(((4-methoxy-2,6-dimethylphenyl)sulfonyl)methyl)-1,3,4-oxadiazol-2-carboxamido)-3,3-dimethylpiperidin-1-carboxylat (180 mg, 0,335 mmol, 1,0 äq) in Ethylacetat (5 ml) wurde 5 min bei -20°C HCl Gas geleitet. Das Reaktionsgemisch wurde unter vermindertem Druck konzentriert und in Methanol (5 ml) gelöst. Dann wurde Amberlyst A-21 hinzugegeben und 2 h bei RT gerührt. Das Gemisch wurde filtriert und unter vermindertem Druck konzentriert. Ausbeute: 83%.

### Synthese der Beispielverbindung SC-90:

### N-(3,3-Dimethyl-piperidin-4-yl)-5-[[(4-methoxy-2,6-dimethyl-phenyl)sulfonyl]-methyl]-[1,2,4]oxadiazol-3-carbonsäure amid (SC-90)

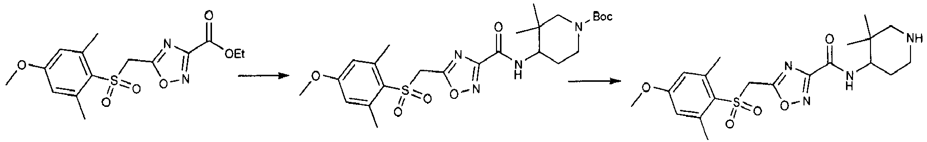

**Stufe 1:** Zu einer Lösung tert-Butyl 4-amino-3,3-dimethylpiperidin-1-carboxylat (**AMN-54**) (1,06 g, 4,65 mmol, 1,1 äq) in DCM (20 ml) wurde eine 2M Lösung aus (CH₃)₃Al in Toluol (6,34 ml, 12,69 mmol, 3,0 äq) bei 0°C hinzugegeben und das Gemisch weitere 30 min gerührt. Eine Lösung aus Ethyl 5-(((4-methoxy-2,6-dimethylphenyl)sulfonyl)methyl)-1,2,4-oxadiazol-3-carboxylat (**ACI-6**) (1,5 g, 4,23 mmol, 1,0 äq) in DCM (10 ml) wurde zum Reaktionsgemisch hinzugegeben und 14 h bei RT gerührt. Das Reaktionsgemisch wurde unter vermindertem Druck konzentriert und das Rohprodukt säulenchromatographisch (Kieselgel, 30% Ethylacetat/Hexan) und anschließend via prep-HPLC gereinigt. Ausbeute: 17%.

**Stufe 2:** Durch eine Lösung aus tert-Butyl 4-(5-(((4-methoxy-2,6-dimethylphenyl)sulfonyl)methyl)-1,2,4-oxadiazol-3-carboxamido)-3,3-dimethylpiperidin-1-carboxylat (300 mg, 0,56 mmol, 1,0 äq) in Ethylacetat (20 ml) wurde 10 min bei -20°C HCl-Gas geleitet und das Gemisch 10 min bei RT gerührt. Das Reaktionsgemisch wurde nach DC-Kontrolle unter vermindertem Druck konzentriert, in DCM (40 ml) gelöst, mit gesättigter NaHCO₃ Lösung gewaschen, über Natriumsulfat getrocknet und unter vermindertem Druck konzentriert. Ausbeute: 88%.

### Analytische Daten - Einzelsubstanzen

### Methode 1:

Material und Methoden für die HPLC-MS Analytik: HPLC: Waters Alliance 2795 mit PDA Waters 2998; MS: Micromass Quattro Micro™ API; Säule: Waters Atlantis^{®} T3, 3 µm, 100 Å, 2,1 x 30 mm; Säulentemperatur: 40°C, Eluent A: gereinigtes Wasser + 0,1% Ameisensäure; Eluent B: Acetonitril (gradient grade) + 0, 1 % Ameisensäure; Gradient: 0% B bis 100% B in 8,8 min, 100% B für 0,4 min, 100% B bis 0% B in 0,01 min, 0% B für 0,8 min; Fluss: 1,0 ml/min; lonisation: ES+, 25 V; Ansatz: 100 µl/min 70% Methanol + 0,2% Ameisensäure; UV: 200 - 400 nm.
oder

### Methode 2:

Material und Methoden für die LC/MS Analytik: **Hardware:** Gekoppeltes Agilent 1290 Infinity UHPLC-TOF System; *LC-Module:* MTP-Handler: Agilent, Modell BenchCel 2R; Thermostatisierter Autoinjektor: Agilent, Modell G4226A; Säulenofen: Agilent, Modell G1316C; DAD: Agilent, Modell G4212A; Binäre Pumpe: Agilent, Modell G4220A; *Time Of Flight Massenspektrometer:* Agilent 6224; Ionenquelle: Dual ESI; **Säule:** Hersteller: Waters; Typ: Acquity UPLC HSS T3 1,8µm (Part No. 186003538); Dimensionen: 2,1 x 50 mm; **Eluenten:** Eluent A: Wasser aus Millipore Reinstwasseranlage: Milli-Q Integral 3 + 0,1 % Ameisensäure; Eluent B: Acetonitril, Merck KGaA: LiChrosolv Hypergrade für LC-MS (1.00029.9010) + 0,1 % Ameisensäure; Ameisensäure: Merck KGaA: Suprapur 98-100% (1.11670.1000); **LC-Methode:** Fluss: 2,5 ml/min; Laufzeit: 1,2 min; Gradient: Start 2% B, 1 min 100% B, 1.09 min 100% B, 1.11 min 2% B, 1.2 min 2% B Stop; Säulentemperatur: 80°C; UV: 190-400 nm; **MS-Methode:** Ionen Polarität: Positiv; Gas Temperatur: 325°C; Gas Fluss: 10 ml/min

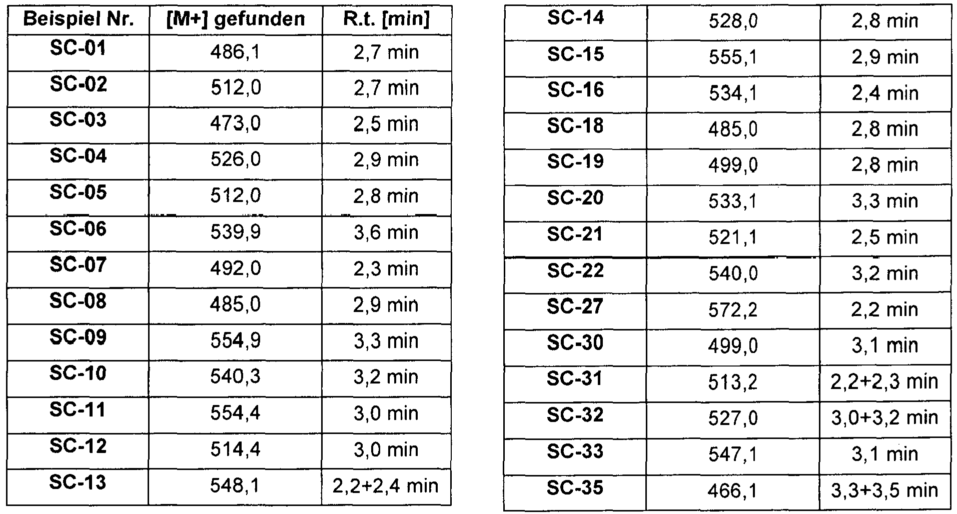

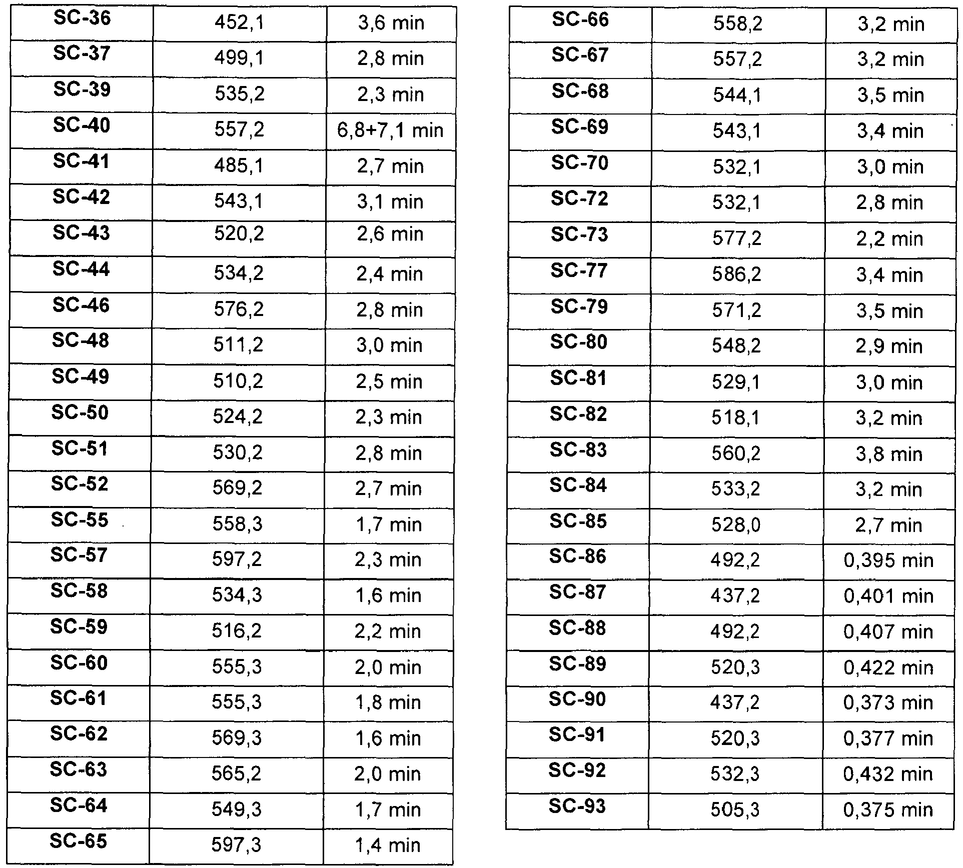

### D. Weitere Verbindungen

Die voranstehend genannten Verbindungen CC-01 bis CC-188 wurden in analoger Weise unter Nutzung der allgemeinen Arbeitsvorschriften hergestellt.

### E. Pharmakologische Methoden

### Funktionelle Untersuchung am Bradykinin Rezeptor 1 (B1R)

Die agonistische bzw. antagonistische Wirkung von Substanzen kann am Bradykinin Rezeptor 1 (B1R) der Spezies Mensch und Ratte mit folgendem Assay bestimmt werden. Gemäß diesem Assay wird der intrazelluläre Ca²⁺-Anstieg nach Gabe eines B1 R Agonisten mit Hilfe eines Ca²⁺-sensitiven Farbstoffs (Typ Fluo-4, Molecular Probes Europe BV, Leiden NL) im Fluorescent Imaging Plate Reader (FLIPR, Molecular Devices, Sunnyvale, USA) und/oder im Novostar (BMG Labtech GmbH, Offenburg, DE) quantifiziert.

### Methode:

Es werden Chinese Hamster Ovary Zellen (CHO K1 Zellen) verwendet, die stabil mit dem humanen B1 R-Gen (hB1R-Zellen) bzw. dem B1 R-Gen der Ratte (rB1R-Zellen) transfiziert sind. Für funktionelle Untersuchungen werden diese Zellen auf schwarze 96-Loch-Platten mit klarem Boden (BD Biosciences, Heidelberg, DE oder Greiner, Frickenhausen, DE) in einer Dichte von 20.000 - 35.000 Zellen/Loch ausplattiert. Über Nacht werden die Zellen bei 37°C und 5 % CO₂ in Kulturmedium (hB1 R-Zellen: Nutrient Mixture Ham's F12, Gibco Invitrogen GmbH, Karlsruhe, Deutschland oder DMEM, Sigma-Aldrich, Taufkirchen, Deutschland; rB1R-Zellen: D-MEM/F12, Gibco Invitrogen GmbH, Karlsruhe, DE) mit 10 Vol-% FBS (Fetal bovine serum, Gibco Invitrogen GmbH, Karlsruhe, DE oder PAN Biotech GmbH, Aidenbach, DE) belassen. Am folgenden Tag werden die Zellen mit 2,13 µM Fluo-4 (Molecular Probes Europe BV, Leiden, NL) in HBSS-Puffer (Hank's buffered saline solution, Gibco Invitrogen GmbH, Karlsruhe, DE) mit 2,5 mM Probenecid (Sigma-Aldrich, Taufkirchen, DE) und 10 mM HEPES (Sigma-Aldrich, Taufkirchen, DE) für 60 min bei 37°C beladen. Anschließend werden die Platten 2 x mit HBSS-Puffer gewaschen und mit HBSS-Puffer versetzt, der zusätzlich 0,1 % BSA (bovines Serumalbumin; Sigma-Aldrich, Taufkirchen, Deutschland), 5,6 mM Glucose und 0,05 % Gelatine (Merck KGaA, Darmstadt, DE) enthält. Nach einer weiteren Inkubation von 20 Minuten bei RT werden die Platten zur Ca²⁺-Messung im FLIPR oder Novostar eingesetzt. Alternativ wird mit Puffer A (15 mM HEPES, 80 mM NaCl, 5 mM KCI, 1,2 mM CaCl₂, 0,7 mM MgSO₄, 2g/L Glucose, 2,5 mM Probenecid) gewaschen und mit Puffer A versetzt mit 2,5 µM Fluo-4 und 0,025 % Pluronic F127 (Sigma-Aldrich, Taufkirchen, DE) beladen. Danach werden die Zellen 2 x mit Puffer A gewaschen und für 30 Minuten mit Puffer A, der zusätzlich 0,05 % BSA und 0,05 % Gelatine enthält bei RT inkubiert und danach zur Ca²⁺-Messung im FLIPR oder Novostar eingesetzt.

Die Ca²⁺-abhängige Fluoreszenz wird dabei vor und nach Zugabe von Substanzen gemessen (λₑₓ = 488 nm, λₑₘ = 540 nm). Die Quantifizierung erfolgt durch die Messung der höchsten Fluoreszenzintensität (FC, Fluorescence Counts) über die Zeit.

### Fluoreszenz-Assay:

Das FLIPR-Protokoll besteht aus 2 Substanzzugaben, die im FLIPR pipettiert werden. Bei Messungen im Novostar wird nur die zweite Substanzzugabe im Gerät pipettiert. Beide Geräte messen die Fluoreszenzintensität über die Zeit. Zunächst werden Testsubstanzen (10 µM) auf die Zellen pipettiert und der intrazelluläre Ca²⁺-Anstieg mit der Kontrolle (hB1 R: Lys-Des-Arg⁹-Bradykinin >= 50 nM; rB1 R: Des-Arg⁹-Bradykinin >= 1 µM) verglichen. Daraus ergibt sich die Angabe in % Aktivierung bezogen auf das Ca²⁺-Signal nach Zugabe von Lys-Des-Arg⁹-Bradykinin (>= 50 nM), bzw. Des-Arg⁹-Bradykinin (>=1 µM).
Nach 6-20 Minuten Inkubation werden Lys-Des-Arg⁹-Bradykinin (hB1 R) bzw. Des-Arg⁹-Bradykinin (rB1 R) in der Konzentration des EC₈₀ appliziert und ebenfalls der Anstieg von Ca²⁺ ermittelt. Antagonisten führen zu einer Unterdrückung des Ca²⁺-Anstiegs in den Zellen. Es werden % Inhibition im Vergleich zu der maximal erreichbaren Inhibition berechnet.

Zur Bestimmung des IC₅₀-Wertes werden die Substanzen in verschiedenen Konzentrationen zugegeben. Es werden Zweifach- oder Dreifach-Bestimmungen (n=2 oder n=3) durchgeführt und diese in mindestens einem weiteren unabhängigen Experiment wiederholt (N>=2). Vorzugsweise weisen die Verbindungen eine B1R antagonistische Wirkung am humanen Rezeptor und/oder am Ratten-Rezeptor auf. Beispielhaft werden in der untenstehenden Tabelle die Daten in % Inhibition angegeben.

Im Folgenden wird die Erfindung anhand von Beispielen erläutert, ohne den allgemeinen Erfindungsgedanken einzuschränken.

### Pharmakologische Daten

Die pharmakologischen Daten wurden wie obenstehend beschrieben bestimmt. Beispielhaft werden in der untenstehenden Tabelle die folgenden Daten angegeben:

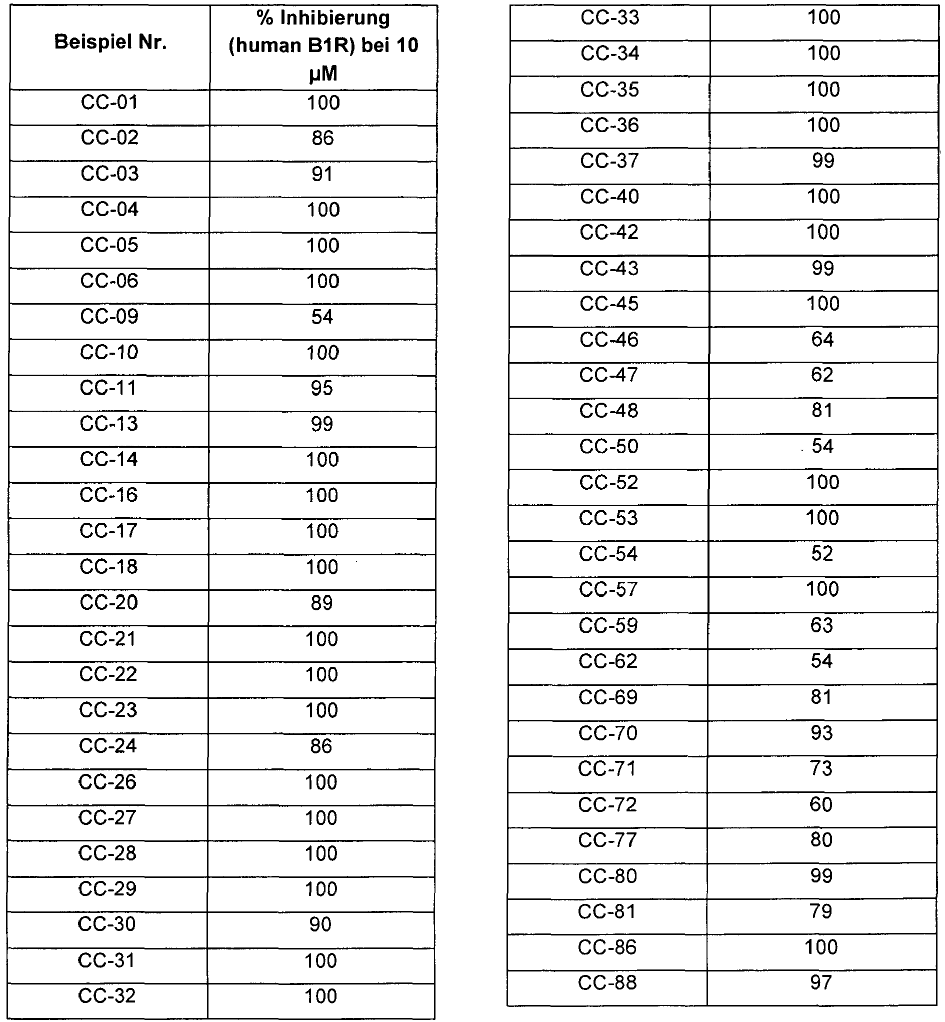

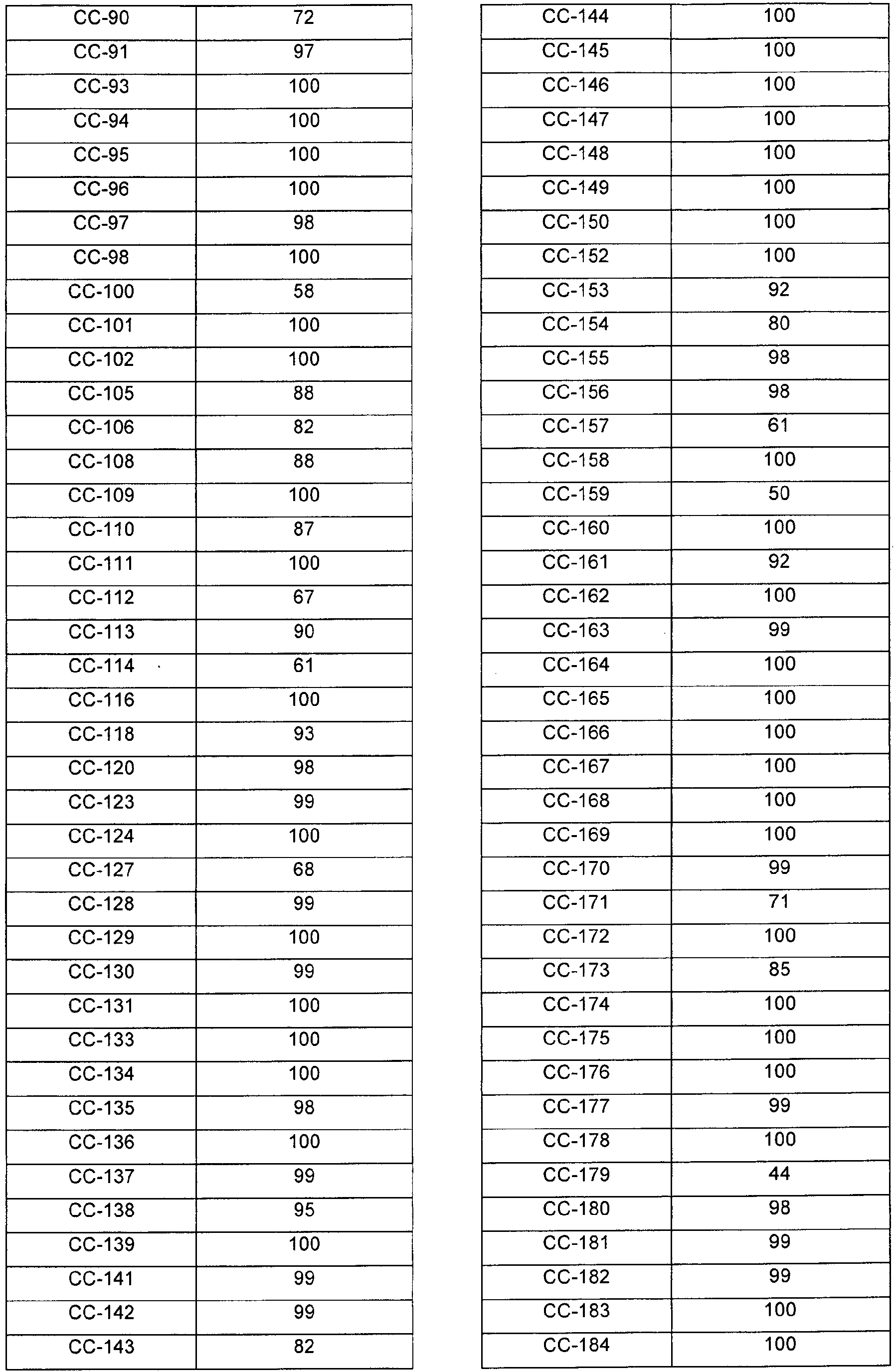

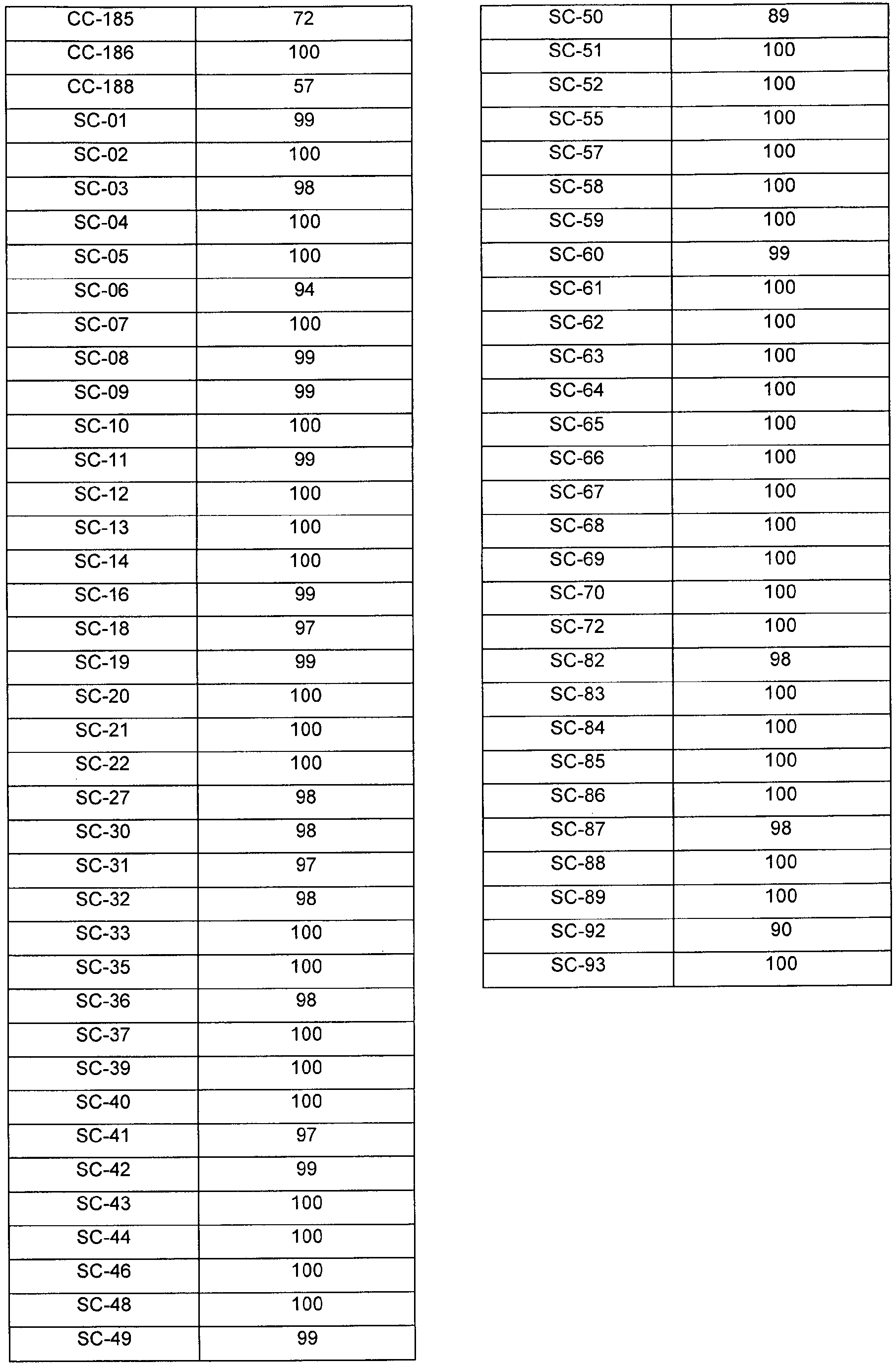

## Patentansprüche

1. Verbindung gemäß allgemeiner Formel (I), 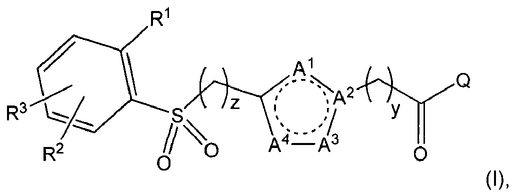 worin
y für 0, 1 oder 2 steht;
z für 1 oder 2 steht;
R¹ für F, Cl, Br, CN, CF₃, CHF₂, CH₂F, C₁₋₄-Alkyl, C₃₋₆-Cycloalkyl, OH, OCH₃, OCF₃, OCHF₂, OCH₂F, O-C₁₋₄-Alkyl, NH₂, N(H)C₁₋₄-Alkyl oder N(C₁₋₄-Alkyl)₂ steht;
R² und R³ jeweils unabhängig voneinander für H, F, Cl, Br, CN, CF₃, CHF₂, CH₂F, C₁₋₄-Alkyl, C₃₋₆-Cycloalkyl, OH, OCH₃, OCF₃, OCHF₂, OCH₂F, O-C₁₋₄-Alkyl, NH₂, N(H)C₁₋₄-Alkyl oder N(C₁₋₄-Alkyl)₂ stehen;
oder zwei benachbarte Substituenten R¹ und R² oder R² und R³ oder R¹ und R³ gemeinsam mit den 2 sie verbindenden C-Atomen bilden einen C₅₋₆-Cycloalkyl oder einen 5-, 6- oder 7-gliedrigen Heterocyclyl, der ein oder zwei O-Atome enthält, wobei besagter Cycloalkyl oder Heterocycloalkyl gesättigt oder ungesättigt, jedoch nicht aromatisch sein kann, und unsubstituiert oder substituiert mit einem oder mehreren Substituenten, jeweils unabhängig ausgewählt aus F, Cl, CN, CF₃; CHF₂; CH₂F; C₁₋₄-Alkyl; O-C₁₋₄-Alkyl; OCF₃; OCHF₂ oder OCH₂F, sein kann;
A¹, A³ und A⁴ jeweils unabhängig voneinander für O, N oder CR⁴ stehen;
worin jedes R⁴ unabhängig für H, F, Cl, Br, CN, CF₃, CHF₂, CH₂F, C₁₋₄-Alkyl, C₃₋₆-Cycloalkyl, OH, OCH₃, OCF₃, OCHF₂, OCH₂F, O-C₁₋₄-Alkyl, NH₂, N(H)C₁₋₄-Alkyl oder N(C₁₋₄-Alkyl)₂ steht;
A² für C oder N steht;
unter der Bedingung, dass A¹, A², A³ und A⁴ und das C-Atom, welches A¹ und A⁴ verbindet, einen 5-gliedrigen Heteroaryl bilden;
und
Q steht für Formel (i), (ii) oder (iii), 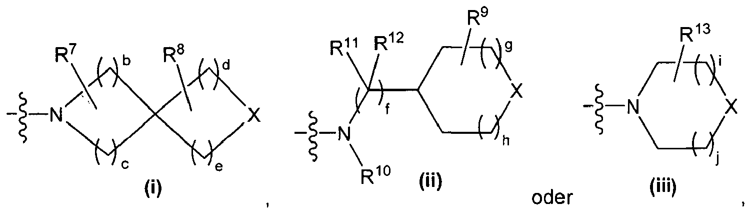 worin
b, c, d und e jeweils unabhängig voneinander für 0, 1, 2, 3 oder 4 stehen,
unter der Bedingung, dass die Summe (b + c) und die Summe (d + e) jeweils wenigstens 2 ist; f für 0, 1, 2, 3 oder 4 steht;
g, h, i und j jeweils unabhängig voneinander für 0, 1 oder 2 stehen;
R⁷, R⁸, R⁹ und R¹³ jeweils unabhängig voneinander für 0, 1, 2, 3, 4 Substituenten stehen, jeweils unabhängig voneinander ausgewählt aus =O, F, Cl, CN, CF₃, CHF₂, CH₂F, C₁₋₄-Alkyl, O-C₁₋₄-Alkyl, OCF₃, OCHF₂ oder OCH₂F;
oder zwei Substituenten R⁹ und R¹³ bilden eine C₁₋₃-Alkylen Gruppe;
R¹⁰ für H, C₁₋₄-Alkyl oder C₃₋₆-Cycloalkyl steht;
jedes R¹¹ und jedes R¹² jeweils unabhängig voneinander für H, F, Cl, CN, C₁₋₄-Alkyl, C₃₋₆-Cycloalkyl, OH, O-CH₃, O-CF₃, O-C₁₋₄-Alkyl, NH₂, N(H)C₁₋₄-Alkyl oder N(C₁₋₄-Alkyl)₂ stehen; oder ein R¹¹ und ein R¹² gemeinsam mit den sie verbindenden C-Atomen einen C₃₋₆-Cycloalkyl oder 4-7-gliedrigen Heterocycloalkyl bilden;
X für
O;
N-(C₁₋₄-Alkylen)ₘ-(Z)ₙ-(C₁₋₄-Alkylen)ₒ-B¹ oder C(R¹⁸)-(C₁₋₄-Alkylen)ₚ-(Z)_{q}-(C₁₋₄-Alkylen)ᵣ-B² steht;
worin
m, n und o jeweils unabhängig voneinander für 0 oder 1 stehen, unter der Bedingung, dass, falls n für 1 steht und Z für O steht, m für 1 steht;
p, q und r jeweils unabhängig voneinander für 0 oder 1 stehen;
R¹⁸ für H, C₁₋₄-Alkyl, C₃₋₆-Cycloalkyl, OH, O-C₁₋₄-Alkyl, NH₂, N(H)C₁₋₄-Alkyl, N(C₁₋₄-Alkyl)₂, Aryl, Heteroaryl oder 4-7-gliedrigen Heterocycloalkyl steht; und
Z bedeutet O oder C=O;
B¹ bedeutet H, C₁₋₄-Alkyl, Cycloalkyl, Heteroaryl, 4-12-gliedrigen Heterocycloalkyl, NR¹⁹R²⁰ oder Aryl,
B² bedeutet H, Heteroaryl, 4-12-gliedrigen Heterocycloalkyl oder NR¹⁹R²⁰;
worin R¹⁹ und R²⁰ jeweils unabhängig voneinander für H, C₁₋₄-Alkyl oder C₃₋₆-Cycloalkyl stehen, worin die vorgenannten Substituenten C₁₋₄-Alkyl, C₁₋₃-Alkylen, C₁₋₄-Alkylen, C₃₋₆-Cycloalkyl,
Heterocycloalkyl, Aryl und Heteroaryl jeweils unsubstituiert oder ein- oder mehrfach substituiert mit gleichen oder verschiedenen Substituenten sind, und die vorgenannten C₁₋₄-Alkyl und C₁₋₄-Alkylen jeweils linear oder verzweigt sein können;
in Form einen individuellen Stereoisomers oder einer Mischung aus Stereoisomeren; in Form eines Tautomers; der freien Verbindung, eines N-Oxids und/oder in Form eines Solvats und/oder eines physiologisch verträglichen Salzes.

2. Verbindung gemäß Anspruch 1, **dadurch gekennzeichnet, dass**
y für 0 oder 1 steht und z für 1 steht,
bevorzugt stehen y für 0 und z für 1.

3. Verbindung gemäß Anspruch 1 oder 2, **dadurch gekennzeichnet, dass**
R¹ für F, Cl, CN, C₁₋₄-Alkyl oder O-C₁₋₄-Alkyl steht; und
R² und R³ unabhängig voneinander für H, F, Cl, CN, C₁₋₄-Alkyl, OH oder O-C₁₋₄-Alkyl stehen.

4. Verbindung gemäß einem oder mehreren der vorigen Ansprüche, **dadurch gekennzeichnet, dass** die Teilstruktur 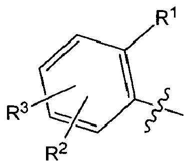 der Formel (I) für 4-Methoxy-2,6-dimethyl-phenyl oder für 2-Chlor-6-methyl-phenyl steht.

5. Verbindung gemäß einem oder mehreren der vorigen Ansprüche, **dadurch gekennzeichnet, dass**
A² für C steht, und eines von A¹, A³ und A⁴ für O steht und die beiden verbleibenden von A¹, A³ und A⁴ für N stehen;
bevorzugt
steht A² für C, A¹ für O und A³ und A⁴ jeweils für N; oder
steht A² für C, A³ für O und A¹ und A⁴ jeweils für N; oder
steht A² für C, A⁴ für O und A¹ und A³ jeweils für N.

6. Verbindung gemäß einem oder mehreren der Ansprüche 1 bis 4, **dadurch gekennzeichnet, dass**
A² für N steht, und A¹, A³ und A⁴ unabhängig voneinander für N oder CR⁴ stehen,
wobei jedes R⁴ jeweils unabhängig voneinander für H, F, Cl, CN, CF₃, CHF₂, CH₂F, C₁₋₄-Alkyl oder C₃₋₆-Cycloalkyl steht;
bevorzugt
stehen A² und A³ für N und stehen A¹ und A⁴ für CR⁴; oder
stehen A¹ und A² für N und stehen A³ und A⁴ für CR⁴; oder
stehen A², A³ und A⁴ für N und steht A¹ für CR⁴.

7. Verbindung gemäß einem oder mehreren der vorigen Ansprüche, **dadurch gekennzeichnet, dass**
Q für Formel (i), (ii) oder (iii) steht,
worin
b, c, d und e jeweils unabhängig voneinander für 0, 1, 2, 3 oder 4 stehen,
unter der Bedingung, dass die Summe (b + c) und die Summe (d + e) jeweils wenigstens 2; f für 0, 1 oder 2 steht;
g, h, i und j jeweils unabhängig voneinander für 0, 1 oder 2 stehen;
R⁷, R⁸, R⁹ und R¹³ jeweils unabhängig voneinander für 0, 1, 2, 3, 4 Substituenten stehen, jeweils unabhängig voneinander ausgewählt aus =O, F, Cl, CH₃ oder OCH₃;
oder zwei Substituenten R¹³ bilden eine C₁₋₃-Alkylen Gruppe;
R¹⁰ für H oder Cyclopropyl oder CH₃ steht;
R¹¹ und R¹² jeweils unabhängig für H, CH₃, CH₂CH₃, OH oder OCH₃ stehen,
oder ein R¹¹ und ein R¹² gemeinsam mit sie verbindenden C-Atomen einen Cyclopropyl, Cyclobutyl, Cyclopentyl oder Cyclohexyl bilden;
X für
O oder
N-(C₁₋₄-Alkylen)ₘ-(Z)ₙ-(C₁₋₄-Alkylen)ₒ-B¹ oder C(R¹⁸)-(C₁₋₄-Alkylen)ₚ-(Z)_{q}-(C₁₋₄-Alkylen)ᵣ-B² steht;
worin
Z für O oder C=O steht;
m, n und o jeweils unabhängig für 0 oder 1 stehen, unter der Bedingung, dass, falls n für 1 steht und Z für O steht, m für 1 steht;
p, q und r jeweils unabhängig für 0 oder 1 stehen;
R¹⁸ für H, C₁₋₄-Alkyl, C₃₋₆-Cycloalkyl, OH, O-C₁₋₄-Alkyl, NH₂, N(H)C₁₋₄-Alkyl, N(C₁₋₄-Alkyl)₂, Aryl, Heteroaryl oder 4-7-gliedrigen Heterocycloalkyl steht;
und
B¹ für H, Alkyl, Cycloalkyl, Heteroaryl, 4-12-gliedrigen Heterocycloalkyl, NR¹⁹R²⁰ oder Aryl steht, B² für H, Heteroaryl, 4-12-gliedrigen Heterocycloalkyl oder NR¹⁹R²⁰ steht;
worin
R¹⁹ und R²⁰ jeweils unabhängig für H, C₁₋₄-Alkyl oder C₃₋₆-Cycloalkyl stehen,
worin
jedes Aryl und jedes Heteroaryl unsubstituiert oder substituiert mit 1, 2, 3 oder 4 Substituenten ist, unabhängig voneinander ausgewählt aus F, Cl, CN, CF₃, CH₃, CH₂CH₃, CH(CH₃)₂, OH, OCH₃, OCF₃, Cyclopropyl, NH₂, N(H)(CH₃), N(CH₃)₂, 4-Morpholinyl, 1-Piperazinyl, 4-Methylpiperazin-1-yl, 1-Piperidinyl und 1-Pyrrolidinyl;
jedes C₁₋₄-Alkyl und jedes C₁₋₄-Alkylen linear oder verzweigt ist und unsubstituiert oder substituiert mit 1, 2 oder 3 Substituenten ist, unabhängig voneinander ausgewählt aus F, Cl, CF₃, CH₃, CH₂CH₃, CH(CH₃)₂, OH oder OCH₃;
und
jedes C₃₋₆-Cycloalkyl und jedes Heterocycloalkyl gesättigt oder ungesättigt, jedoch nicht aromatisch ist, und unsubstituiert oder substituiert mit 1, 2, 3 oder 4 Substituenten ist, unabhängig voneinander ausgewählt aus F, Cl, CN, CF₃, CH₃, CH₂CH₃, CH(CH₃)₂, OH, OCH₃, OCF₃, Cyclopropyl, NH₂, N(H)(CH₃), N(CH₃)₂, 4-Morpholinyl, 1-Piperazinyl, 4-Methyl-piperazin-1-yl, 1-Piperidinyl und 1-Pyrrolidinyl.

8. Verbindung gemäß einem oder mehreren der vorigen Ansprüche, **dadurch gekennzeichnet, dass** R⁷ und R⁸ jeweils unabhängig voneinander oder R⁹ oder R¹³ für 0, 1 oder 2 Substituenten stehen, unabhängig voneinander ausgewählt aus =O, F, Cl, CH₃ oder OCH₃,
bevorzugt stehen R⁷ und R⁸ jeweils für 0 Substituenten oder steht R⁹ für 0 Substituenten oder steht R¹³ für 0 Substituenten oder stehen zwei R¹³ jeweils für CH₃,
X für N-(C₁₋₄-Alkylen)ₘ-B¹ steht,
worin m für 0 oder 1 steht und B¹ für Heteroaryl, 4-12-gliedrigen Heterocycloalkyl oder Aryl steht, oder
X für C(R¹⁸)-(O)_{q}-(C₁₋₄-Alkylen)ᵣ-B² steht;
worin R¹⁸ für H steht, q für 0 oder 1 steht, r für 0 oder 1 steht und B² für 4-12-gliedrigen Heterocycloalkyl oder Heteroaryl oder für N(CH₃)₂, N(CH₃)(CH₂CH₃), N(CH₃)(CH(CH₃)₂), N(CH₃)(C(CH₃)₃), N(CH₃)(cyclopropyl), N(CH₂CH₃)₂ oder N(CH₂CH₃)(cyclopropyl) steht;
worin besagter Heteroaryl jeweils ausgewählt ist aus 2-Pyridyl, 3-Pyridyl, 4-pyridyl, 2-Pyrimidinyl, 4-Pyrimidinyl, 5-Pyrimidinyl, 3-Ppyridazinyl, 4-Pyridazinyl und 2-Pyrazinyl;
worin besagter 4-12-gliedriger Heterocycloalkyl ausgewählt ist aus Tetrahydrofuranyl, Tetrahydropyranyl, Morpholinyl, Piperazinyl, Piperidinyl und Pyrrolidinyl;
worin besagter Heteroaryl, besagter 4-12-gliedriger Heterocycloalkyl oder besagter Aryl unsubstituiert oder substituiert mit 1, 2, 3 oder 4 Substituenten ist, unabhängig voneinander ausgewählt aus F, Cl, CN, CF₃, CH₃, CH₂CH₃, CH(CH₃)₂, OH, OCH₃, OCF₃, Cyclopropyl, NH₂, N(H)(CH₃), N(CH₃)₂, 4-Morpholinyl, 1-Piperazinyl, 4-Methyl-piperazin-1-yl, 1-Piperidinyl und 1-Pyrrolidinyl.

9. Verbindung gemäß einem oder mehreren der vorigen Ansprüche, **dadurch gekennzeichnet, dass** Q ausgewählt ist aus einer der Formeln (i-1) bis (i-46): 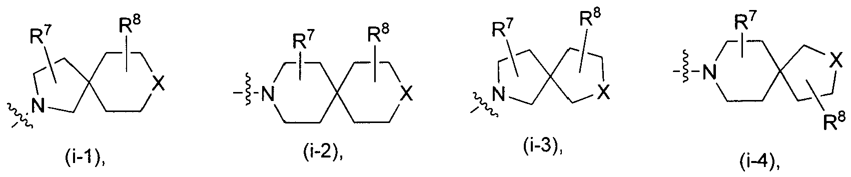 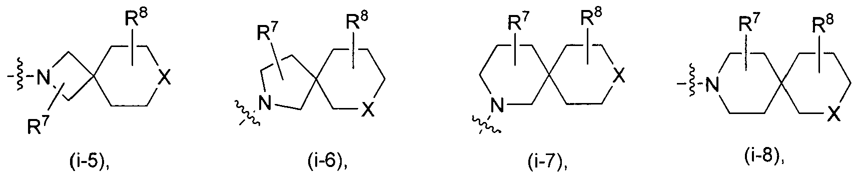 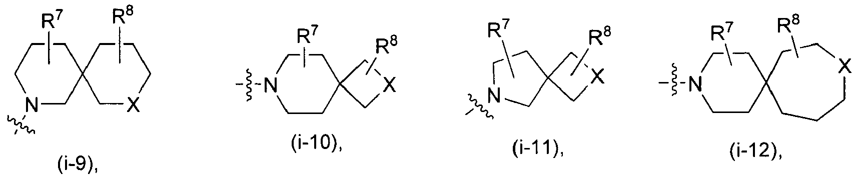 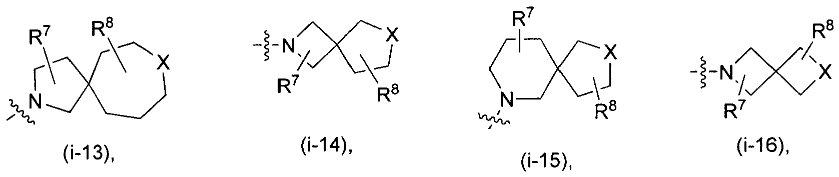 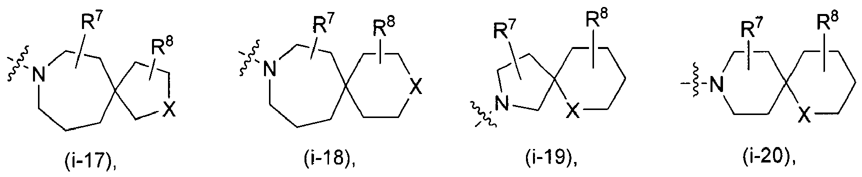 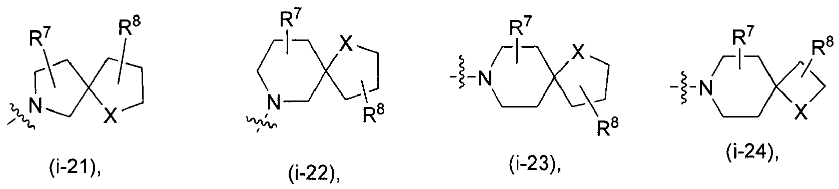 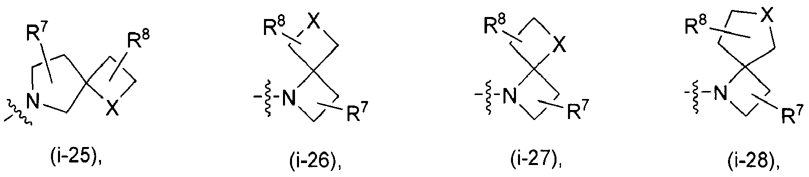 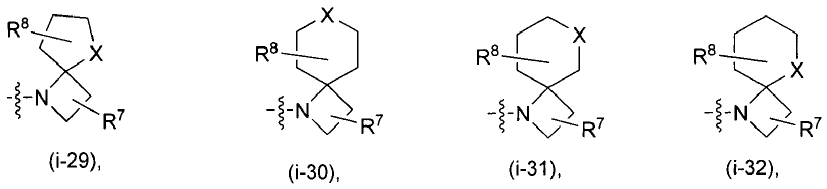 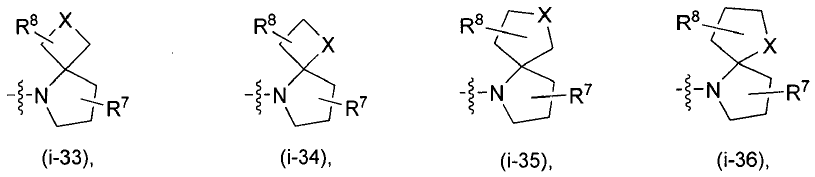 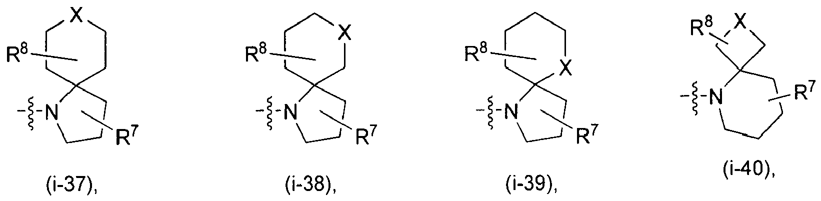 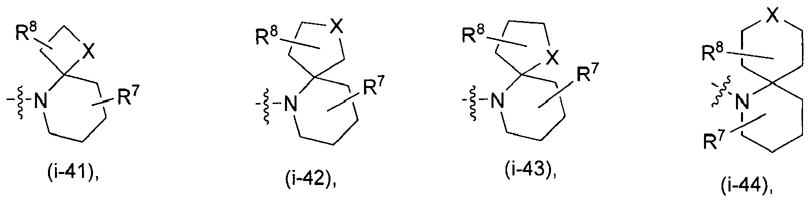 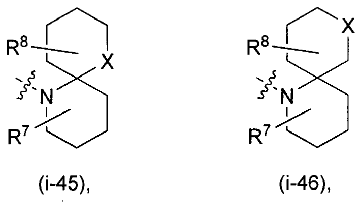 bevorzugt ist Q ausgewählt aus einer der Formeln (i-1) bis (i-5).

10. Verbindung gemäß einem oder mehreren der Ansprüche 1 bis 8, **dadurch gekennzeichnet, dass** Q ausgewählt ist aus einer der Formeln (ii-1) bis (ii-36): 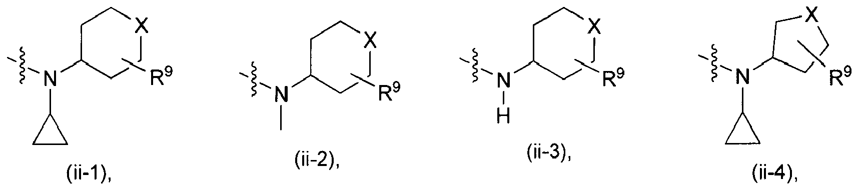 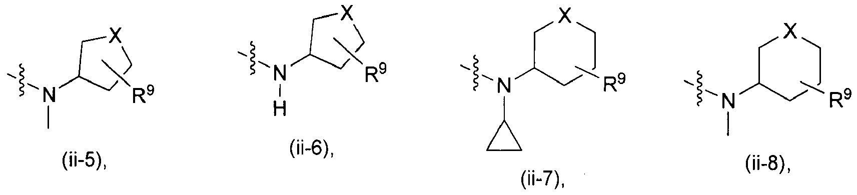 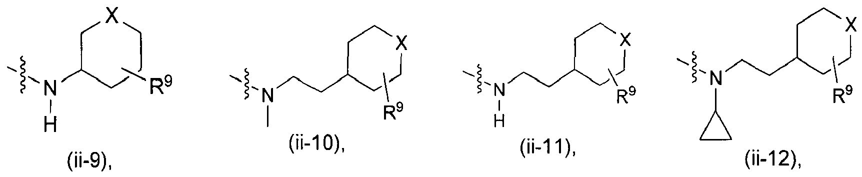 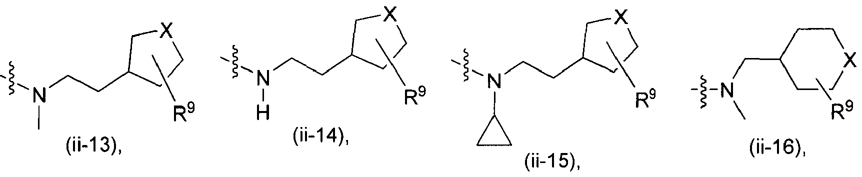 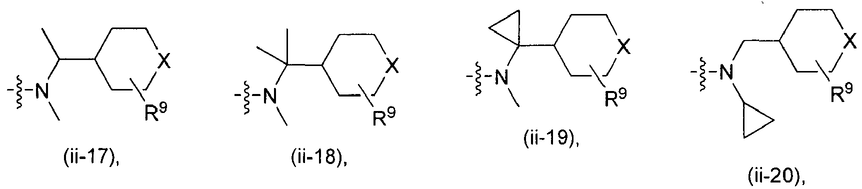 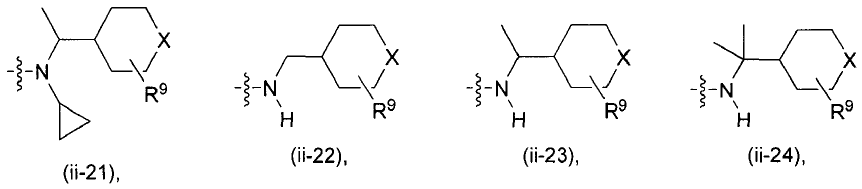 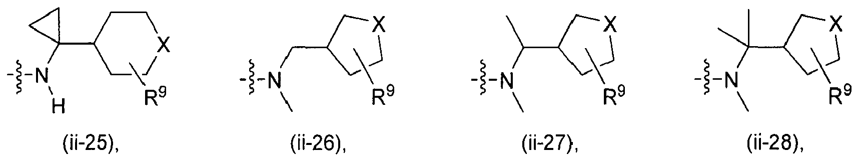 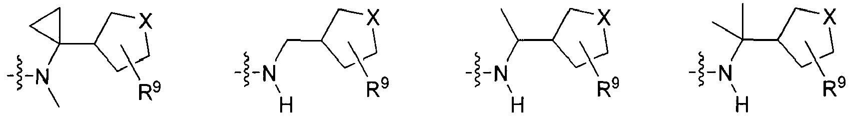 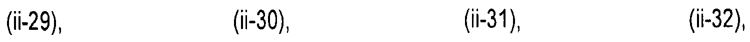 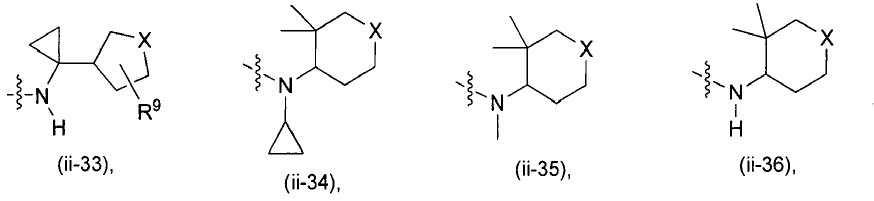 bevorzugt ist Q ausgewählt aus einer der Formeln (ii-1) bis (ii-12), bevorzugt worin R⁹ jeweils für 0 Substituenten steht, und aus einer der Formeln (ii-34) bis (ii-36)..

11. Verbindung gemäß einem oder mehreren der Ansprüche 1 bis 8, **dadurch gekennzeichnet, dass**
Q ausgewählt ist aus einer der Formeln (iii-1) bis (iii-9) 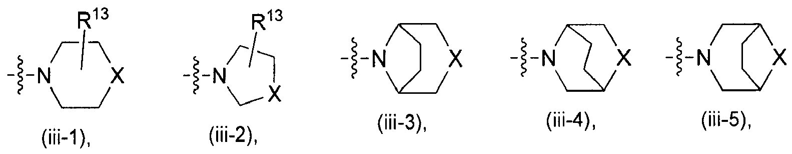 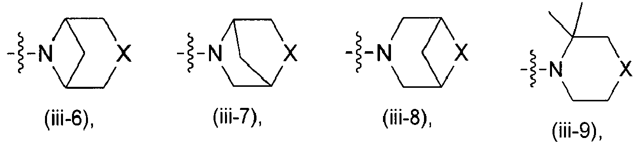 bevorzugt ist Q ausgewählt aus einer der Formeln (iii-1, (iii-2), (iii-3) und (iii-)).

12. Verbindung gemäß einem oder mehreren der vorigen Ansprüche, **dadurch gekennzeichnet, dass**
R¹ für F, Cl, CN, CH₃ oder OCH₃ steht; und
R² und R³ unabhängig voneinander für H, F, Cl, CN, CH₃ oder OCH₃ stehen,
z für 1 steht und y für 0 oder 1, bevorzugt für 0, steht;
A¹, A³ und A⁴ jeweils unabhängig voneinander für O, N oder CR⁴ stehen,
worin jedes R⁴ unabhängig für H oder CH₃ steht;
A² für C oder N steht;
Q ist ausgewählt aus einer der Formeln (i-1), (i-2), (i-3), (i-4), 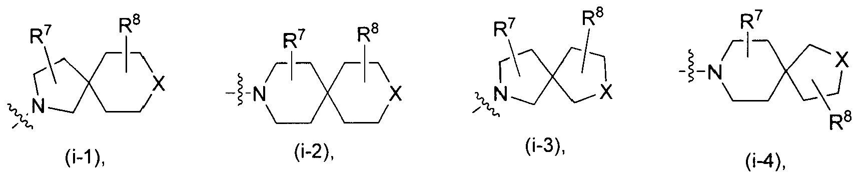 worin
R⁷ und R⁸ unabhängig voneinander für 0, 1 oder 2 Substituenten steht, jeweils unabhängig voneinander ausgewählt aus =O, F, CH₃ oder OCH₃;
oder ist ausgewählt aus einer der Formeln (ii-1) bis (ii-12): 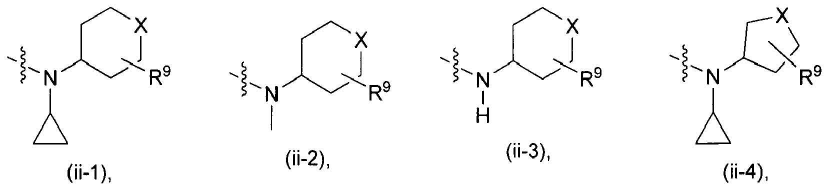 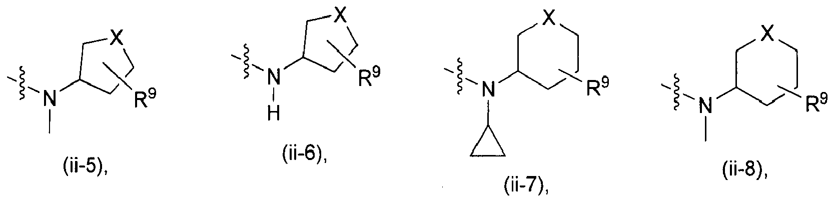 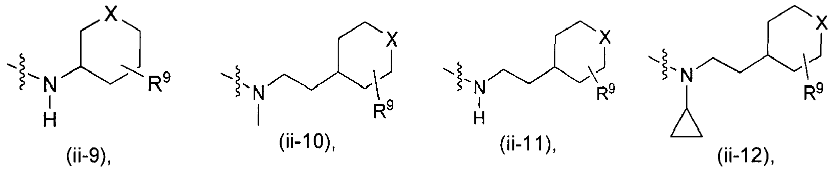 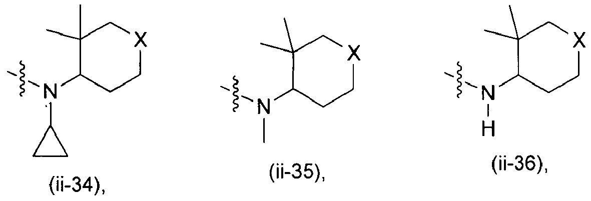 worin
R⁹ für 0, 1 oder 2 Substituenten steht, jeweils unabhängig voneinander ausgewählt aus =O, F, CH₃ oder OCH₃;
oder ist ausgewählt aus einer der Formeln (iii-1) bis (iii-3): 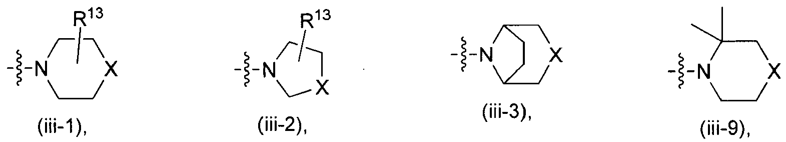 worin R¹³ für 0, 1 oder 2 Substituenten steht, jeweils unabhängig voneinander ausgewählt aus =O, F, CH₃ oder OCH₃, steht;
und worin
X für N-(C₁₋₄-Alkylene)ₘ-B¹ steht,
worin m für 0 oder 1 steht;
und B¹ ausgewählt ist aus 2-Pyridyl, 3-Pyridyl, 4-Pyridyl, 2-Pyrimidinyl oder 4-Pyrimidinyl, jeweils unsubstituiert oder substituiert mit 1, 2 oder 3 Substituenten, jeweils unabhängig voneinander ausgewählt aus F, Cl, CN, CF₃, C₁₋₄-Alkyl, OH, O-C₁₋₄-Alkyl, OCF₃, Cyclopropyl, NH₂, N(H)-C₁₋₄-Alkyl, N(C₁₋₄-Alkyl)₂, 4-Morpholinyl, 1-Piperazinyl, 4-Methyl-piperazin-1-yl, 1-Piperidinyl und 1-Pyrrolidinyl;
oder
X für C(R¹⁸)-(O)_{q}-(C₁₋₄-Alkylene)ᵣ-B² steht,
worin R¹⁸ für H steht, q für 0 oder 1 steht, r für 0 oder 1 steht
und B² ausgewählt ist aus 2-Pyridyl, 3-Pyridyl, 4-Pyridyl, 2-Pyrimidinyl oder 4-Pyrimidinyl, 4-Morpholinyl, 1-Piperazinyl, 1-Piperidinyl und Pyrrolidinyl,
jeweils unsubstituiert oder substituiert mit 1, 2 oder 3 Substituenten, jeweils unabhängig voneinander ausgewählt aus F, Cl, CN, CF₃, CH₃, CH₂CH₃, CH(CH₃)₂, OH, OCH₃, OCF₃, Cyclopropyl, NH₂, N(H)(CH₃), N(CH₃)₂, 4-Morpholinyl, 1-Piperazinyl, 4-Methyl-piperazin-1-yl, 1-Piperidinyl und 1-Pyrrolidinyl,
oder B² für N(CH₃)₂, N(CH₃)(CH₂CH₃), N(CH₃)(CH(CH₃)₂), N(CH₃)(C(CH₃)₃), N(CH₃)(cyclopropyl), N(CH₂CH₃)₂ oder N(CH₂CH₃)(cyclopropyl) steht.

13. Verbindung gemäß einem oder mehreren der vorigen Ansprüche, ausgewählt aus der Gruppe, bestehend aus
CC-01 3-[[(4-Methoxy-2,6-dimethyl-phenyl)sulfonyl]-methyl]-N-[(1S,3R)-3-(4-methyl-piperazin-1-yl)-cyclohexyl]-[1,2,4]oxadiazol-5-carbonsäure amid
CC-02 3-[[(4-Methoxy-2,6-dimethyl-phenyl)sulfonyl]-methyl]-N-[1-[5-(trifluormethyl)-pyridin-2-yl]-piperidin-4-yl]-[1,2,4]oxadiazol-5-carbonsäure amid
CC-03 2-[3-[[(4-Methoxy-2,6-dimethyl-phenyl)sulfonyl]-methyl]-[1,2,4]oxadiazol-5-yl]-N-[1-[5-(trifluormethyl)-pyridin-2-yl]-piperidin-4-yl]-acetamid
CC-04 N-(4-Dimethylamino-cyclohexyl)-3-[[(4-methoxy-2,6-dimethyl-phenyl)sulfonyl]-methyl]-[1,2,4]oxadiazol-5-carbonsäure amid
CC-05 N-(4-Dimethylamino-cyclohexyl)-2-[3-[[(4-methoxy-2,6-dimethyl-phenyl)sulfonyl]-methyl]-[1,2,4]oxadiazol-5-yl]-acetamid
CC-06 2-[3-[[(4-Methoxy-2,6-dimethyl-phenyl)sulfonyl]-methyl]-[1,2,4]oxadiazol-5-yl]-N-methyl-N-[2-(1-pyridin-4-yl-piperidin-4-yl)-ethyl]-acetamid
CC-09 3-[[(2-Chlor-6-methyl-phenyl)sulfonyl]-methyl]-N-[1-(5-(trifluormethyl)-pyridin-2-yl]-piperidin-4-yl]-[1,2,4]oxadiazol-5-carbonsäure amid
CC-10 3-[[(4-Methoxy-2,6-dimethyl-phenyl)sulfonyl]-methyl]-N-methyl-N-[2-(1-pyridin-4-yl-piperidin-4-yl)-ethyl]-[1,2,4]oxadiazol-5-carbonsäure amid
CC-11 3-[[(2-Chlor-6-methyl-phenyl)sulfonyl]-methyl]-N-(4-dimethylamino-cyclohexyl)-[1,2,4]-oxadiazol-5-carbonsäure amid
CC-13 3-[[(4-Chlor-2,5-dimethyl-phenyl)sulfonyl]-methyl]-N-(4-dimethylamino-cyclohexyl)-[1,2,4]oxadiazol-5-carbonsäure amid
CC-14 3-[[(2-Chlor-6-methyl-phenyl)sulfonyl]-methyl]-N-methyl-N-[2-(1-pyridin-4-yl-piperidin-4-yl)-ethyl]-[1,2,4]oxadiazol-5-carbonsäure amid
CC-16 3-[[(4-Chlor-2,5-dimethyl-phenyl)sulfonyl]-methyl]-N-methyl-N-[2-(1-pyridin-4-yl-piperidin-4-yl)-ethyl]-[1,2,4]oxadiazol-5-carbonsäure amid
CC-17 N-(3,3-Dimethyl-piperidin-4-yl)-3-[[(4-methoxy-2,6-dimethyl-phenyl)sulfonyl]-methyl]-[1,2,4]oxadiazol-5-carbonsäure amid
CC-18 N-(3,3-Dimethyl-piperidin-4-yl)-2-[3-[[(4-methoxy-2,6-dimethyl-phenyl)sulfonyl]-methyl]-[1,2,4]oxadiazol-5-yl]-acetamid
CC-20 3-[[(2-Chlor-6-methyl-phenyl)sulfonyl]-methyl]-N-(2,2-dimethyl-4-pyrrolidin-1-yl-cyclo-hexyl)-[1,2,4]oxadiazol-5-carbonsäure amid
CC-21 N-(2,2-Dimethyl-4-pyrrolidin-1-yl-cyclohexyl)-3-[[(4-methoxy-2,6-dimethyl-phenyl)sulfonyl]-methyl]-[1,2,4]oxadiazol-5-carbonsäure amid
CC-22 3-[[(4-Chlor-2,5-dimethyl-phenyl)sulfonyl]-methyl]-N-(2,2-dimethyl-4-pyrrolidin-1-yl-cyclo-hexyl)-[1,2,4]oxadiazol-5-carbonsäure amid
CC-23 N-(2,2-Dimethyl-4-pyrrolidin-1-yl-cyclohexyl)-2-[3-[[(4-methoxy-2,6-dimethyl-phenyl)-sulfonyl]-methyl]-[1,2,4]oxadiazol-5-yl]-acetamid
CC-24 3-[[(4-Chlor-2,5-dimethyl-phenyl)sulfonyl]-methyl]-N-(3,3-dimethyl-piperidin-4-yl)-[1,2,4]-oxadiazol-5-carbonsäure amid
CC-26 N-(2,2-Dimethyl-4-pyrrolidin-1-yl-cyclohexyl)-2-[5-[[(4-methoxy-2,6-dimethyl-phenyl)-sulfonyl]-methyl]-[1,2,4]oxadiazol-3-yl]-acetamid
CC-27 2-[5-[[(4-Methoxy-2,6-dimethyl-phenyl)sulfonyl]-methyl]-[1,2,4]oxadiazol-3-yl]-N-[1-(2-methoxy-ethyl)-3,3-dimethyl-piperidin-4-yl]-acetamid
CC-28 N-(4-Dimethylamino-cyclohexyl)-2-[5-[[(4-methoxy-2,6-dimethyl-phenyl)sulfonyl]-methyl]-[1,2,4]oxadiazol-3-yl]-acetamid
CC-29 N-(2,2-Dimethyl-4-pyrrolidin-1-yl-cyclohexyl)-2-[5-[[(4-methoxy-2,6-dimethyl-phenyl)-sulfonyl]-methyl]-[1,3,4]oxadiazol-2-yl]-acetamid
CC-30 N-(1-Acetyl-3,3-dimethyl-piperidin-4-yl)-2-[5-[[(4-methoxy-2,6-dimethyl-phenyl)sulfonyl]-methyl]-[1,2,4]oxadiazol-3-yl]-acetamid
CC-31 2-[5-[[(4-Methoxy-2,6-dimethyl-phenyl)sulfonyl]-methyl]-[1,3,4]oxadiazol-2-yl]-N-[1-(2-methoxy-ethyl)-3,3-dimethyl-piperidin-4-yl]-acetamid
CC-32 2-[5-[[(4-Methoxy-2,6-dimethyl-phenyl)sulfonyl]-methyl]-[1,2,4]oxadiazol-3-yl]-N-(4-pyrrolidin-1-yl-cyclohexyl)-acetamid
CC-33 N-[2,2-Dimethyl-4-(4-methyl-piperazin-1-yl)-cyclohexyl]-2-[5-[[(4-methoxy-2,6-dimethyl-phenyl)sulfonyl]-methyl]-[1,2,4]oxadiazol-3-yl]-acetamid
CC-34 N-(4-Dimethylamino-cyclohexyl)-2-[5-[[(4-methoxy-2,6-dimethyl-phenyl)sulfonyl]-methyl]-[1,3,4]oxadiazol-2-yl]-acetamid
CC-35 N-[4-(Cyclopropylamino)-2,2-dimethyl-cyclohexyl]-2-[5-[[(4-methoxy-2,6-dimethyl-phenyl)-sulfonyl]-methyl]-[1,2,4]oxadiazol-3-yl]-acetamid
CC-36 N-(2,2-Dimethyl-4-pyridin-4-yl-cyclohexyl)-2-[5-[[(4-methoxy-2,6-dimethyl-phenyl)-sulfonyl]-methyl]-[1,3,4]oxadiazol-2-yl]-acetamid
CC-37 N-(2,2-Dimethyl-4-pyridin-4-yl-cyclohexyl)-2-[5-[[(4-methoxy-2,6-dimethyl-phenyl)-sulfonyl]-methyl]-[1,2,4]oxadiazol-3-yl]-acetamid
CC-40 2-[5-[[(4-Methoxy-2,6-dimethyl-phenyl)sulfonyl]-methyl]-[1,3,4]oxadiazol-2-yl]-N-(4-pyrrolidin-1-yl-cyclohexyl)-acetamid
CC-42 2-[5-[[(4-Methoxy-2,6-dimethyl-phenyl)sulfonyl]-methyl]-[1,2,4]oxadiazol-3-yl]-N-piperidin-4-yl-acetamid
CC-43 2-[3-[[(4-Chlor-2,5-dimethyl-phenyl)sulfonyl]-methyl]-[1,2,4]oxadiazol-5-yl]-N-(2,2-di-methyl-4-pyrrolidin-1-yl-cyclohexyl)-acetamid
CC-45 2-[3-[[(4-Chlor-2,5-dimethyl-phenyl)sulfonyl]-methyl]-[1,2,4]oxadiazol-5-yl]-N-[2,2-di-methyl-4-(4-methyl-piperazin-1-yl)-cyclohexyl]-acetamid
CC-46 N-(1-Acetyl-3,3-dimethyl-piperidin-4-yl)-2-[3-[[(4-chlor-2,5-dimethyl-phenyl)sulfonyl]-methyl]-[1,2,4]oxadiazol-5-yl]-acetamid
CC-47 N-(1-Acetyl-3,3-dimethyl-piperidin-4-yl)-2-[3-[[(2,3-dichlor-phenyl)sulfonyl]-methyl]-[1,2,4]-oxadiazol-5-yl]-acetamid
CC-48 2-[3-[[(2,3-Dichlor-phenyl)sulfonyl]-methyl]-[1,2,4]oxadiazol-5-yl]-N-[2,2-dimethyl-4-(4-methyl-piperazin-1-yl)-cyclohexyl]-acetamid
CC-50 2-[3-[[(3,4-Dichlorphenyl)sulfonyl]-methyl]-[1,2,4]oxadiazol-5-yl]-N-(4-dimethylamino-cyclohexyl)-acetamid
CC-52 N-(3,3-Dimethyl-piperidin-4-yl)-2-[5-[[(4-methoxy-2,6-dimethyl-phenyl)sulfonyl]-methyl]-[1,2,4]oxadiazol-3-yl]-acetamid
CC-53 2-[3-[[(3,4-Dichlorphenyl)sulfonyl]-methyl]-[1,2,4]oxadiazol-5-yl]-N-(2,2-dimethyl-4-pyrrolidin-1-yl-cyclohexyl)-acetamid
CC-54 2-[3-[[(2,3-Dichlor-phenyl)sulfonyl]-methyl]-[1,2,4]oxadiazol-5-yl]-N-piperidin-4-yl-acetamid
CC-57 2-[3-[[(4-Chlor-2,5-dimethyl-phenyl)sulfonyl]-methyl]-[1,2,4]oxadiazol-5-yl]-N-[4-(cyclo-propylamino)-2,2-dimethyl-cyclohexyl]-acetamid
CC-59 2-[3-[[(2,3-Dichlor-phenyl)sulfonyl]-methyl]-[1,2,4]oxadiazol-5-yl]-N-(4-dimethylamino-cyclohexyl)-acetamid
CC-62 2-[3-[[(4-Chlor-2,5-dimethyl-phenyl)sulfonyl]-methyl]-[1,2,4]oxadiazol-5-yl]-N-(3,3-di-methyl-piperidin-4-yl)-acetamid
CC-69 N-(1-Acetyl-3,3-dimethyl-piperidin-4-yl)-2-[5-[[(4-methoxy-2,6-dimethyl-phenyl)sulfonyl]-methyl]-[1,3,4]oxadiazol-2-yl]-acetamid
CC-70 2-[3-[[(4-Chlor-2,5-dimethyl-phenyl)sulfonyl]-methyl]-[1,2,4]oxadiazol-5-yl]-N-(4-pyrrolidin-1-yl-cyclohexyl)-acetamid
CC-71 2-[3-[[(2,3-Dichlor-phenyl)sulfonyl]-methyl]-[1,2,4]oxadiazol-5-yl]-N-(4-pyrrolidin-1-yl-cyclohexyl)-acetamid
CC-72 2-[3-[[(2,3-Dichlor-phenyl)sulfonyl]-methyl]-[1,2,4]oxadiazol-5-yl]-N-[1-(2-methoxy-ethyl)-3,3-dimethyl-piperidin-4-yl]-acetamid
CC-77 2-[3-[[(4-Chlor-2,5-dimethyl-phenyl)sulfonyl]-methyl]-[1,2,4]oxadiazol-5-yl]-N-[1-(2-methoxy-ethyl)-3,3-dimethyl-piperidin-4-yl]-acetamid
CC-80 2-[3-[[(4-Chlor-2,5-dimethyl-phenyl)sulfonyl]-methyl]-[1,2,4]oxadiazol-5-yl]-N-(2,2-di-methyl-4-pyridin-4-yl-cyclohexyl)-acetamid
CC-81 2-[3-[[(4-Chlor-2,5-dimethyl-phenyl)sulfonyl]-methyl]-[1,2,4]oxadiazol-5-yl]-N-(4-dimethyl-amino-cyclohexyl)-acetamid
CC-86 N-[2,2-Dimethyl-4-(4-methyl-piperazin-1-yl)-cyclohexyl]-2-[5-[[(4-methoxy-2,6-dimethyl-phenyl)sulfonyl]-methyl]-[1,3,4]oxadiazol-2-yl]-acetamid
CC-88 N-(3,3-Dimethyl-piperidin-4-yl)-2-[5-[[(4-methoxy-2,6-dimethyl-phenyl)sulfonyl]-methyl]-[1,3,4]oxadiazol-2-yl]-N-methyl-acetamid
CC-90 2-[3-[[(2-Chlor-6-methyl-phenyl)sulfonyl]-methyl]-[1,2,4]oxadiazol-5-yl]-N-piperidin-4-yl-acetamid
CC-91 2-[3-[[(2-Chlor-6-methyl-phenyl)sulfonyl]-methyl]-[1,2,4]oxadiazol-5-yl]-N-(2,2-dimethyl-4-pyrrolidin-1-yl-cyclohexyl)-acetamid
CC-93 2-[3-[[(2-Chlor-6-methyl-phenyl)sulfonyl]-methyl]-[1,2,4]oxadiazol-5-yl]-N-(4-pyrrolidin-1-yl-cyclohexyl)-acetamid
CC-94 2-[3-[[(2-Chlor-6-methyl-phenyl)sulfonyl]-methyl]-[1,2,4]oxadiazol-5-yl]-N-[1-(2-methoxy-ethyl)-3,3-dimethyl-piperidin-4-yl]-acetamid
CC-95 2-[3-[[(2-Chlor-6-methyl-phenyl)sulfonyl]-methyl]-[1,2,4]oxadiazol-5-yl]-N-[4-(cyclopropyl-amino)-2,2-dimethyl-cyclohexyl]-acetamid
CC-96 2-[3-[[(2-Chlor-6-methyl-phenyl)sulfonyl]-methyl]-[1,2,4]oxadiazol-5-yl]-N-(1,3,3-trimethyl-piperidin-4-yl)-acetamid
CC-97 2-[5-[[(4-Methoxy-2,6-dimethyl-phenyl)sulfonyl]-methyl]-[1,3,4]oxadiazol-2-yl]-N-piperidin-4-yl-acetamid
CC-98 2-[3-[[(2-Chlor-6-methyl-phenyl)sulfonyl]-methyl]-[1,2,4]oxadiazol-5-yl]-N-[2,2-dimethyl-4-(4-methyl-piperazin-1-yl)-cyclohexyl]-acetamid
CC-1002-[3-[[(4-Chlor-2,5-dimethyl-phenyl)sulfonyl]-methyl]-[1,2,4]oxadiazol-5-yl]-N-(3,3-di-methyl-piperidin-4-yl)-N-methyl-acetamid
CC-1 01 N-(3,3-Dimethyl-piperidin-4-yl)-2-[5-[[(4-methoxy-2,6-dimethyl-phenyl)sulfonyl]-methyl]-[1,2,4]oxadiazol-3-yl]-N-methyl-acetamid
CC-1022-[5-[[(4-Methoxy-2,6-dimethyl-phenyl)sulfonyl]-methyl]-[1,2,4]oxadiazol-3-yl]-N-(1,3,3-trimethyl-piperidin-4-yl)-acetamid
CC-1052-[3-[[(4-Chlor-2,5-dimethyl-phenyl)sulfonyl]-methyl]-[1,2,4]oxadiazol-5-yl]-N-(1,3,3-tri-methyl-piperidin-4-yl)-acetamid
CC-1062-[3-[[(2,3-Dichlor-phenyl)sulfonyl]-methyl]-[1,2,4]oxadiazol-5-yl]-N-(2,2-dimethyl-4-pyrrolidin-1-yl-cyclohexyl)-acetamid
CC-1082-[3-[[(2-Chlor-6-methyl-phenyl)sulfonyl]-methyl]-[1,2,4]oxadiazol-5-yl]-N-cyclopropyl-N-(4-dimethylamino-cyclohexyl)-acetamid
CC-1092-[3-[[(2,3-Dichlor-phenyl)sulfonyl]-methyl]-[1,2,4]oxadiazol-5-yl]-N-(2,2-dimethyl-4-pyridin-4-yl-cyclohexyl)-acetamid
CC-1102-[3-[[(2-Chlor-6-methyl-phenyl)sulfonyl]-methyl]-[1,2,4]oxadiazol-5-yl]-N-(3,3-dimethyl-piperidin-4-yl)-N-methyl-acetamid
CC-1112-[5-[[(4-Methoxy-2,6-dimethyl-phenyl)sulfonyl]-methyl]-[1,3,4]oxadiazol-2-yl]-N-(1,3,3-trimethyl-piperidin-4-yl)-acetamid
CC-112 N-Cyclopropyl-2-[3-[[(2,3-dichlor-phenyl)sulfonyl]-methyl]-[1,2,4]oxadiazol-5-yl]-N-(4-dimethylamino-cyclohexyl)-acetamid
CC-1132-[3-[[(4-Chlor-2,5-dimethyl-phenyl)sulfonyl]-methyl]-[1,2,4]oxadiazol-5-yl]-N-cyclopropyl-N-(4-dimethylamino-cyclohexyl)-acetamid
CC-1142-[3-[[(2,3-Dichlor-phenyl)sulfonyl]-methyl]-[1,2,4]oxadiazol-5-yl]-N-(1,3,3-trimethyl-piperidin-4-yl)-acetamid
CC-116N-Cyclopropyl-N-(4-dimethylamino-cyclohexyl)-2-[5-[[(4-methoxy-2,6-dimethyl-phenyl)-sulfonyl]-methyl]-[1,2,4]oxadiazol-3-yl]-acetamid
CC-118 N-Cyclopropyl-N-(4-dimethylamino-cyclohexyl)-2-[4-[[(4-methoxy-2,6-dimethyl-phenyl)-sulfonyl]-methyl]-3,5-dimethyl-1H-pyrazol-1-yl]-acetamid
CC-1 202-[3-[[(2-Chlor-6-methyl-phenyl)sulfonyl]-methyl]-[1,2,4]oxadiazol-5-yl]-N-(3,3-dimethyl-piperidin-4-yl)-acetamid
CC-123 N-[4-(Cyclopropylamino)-2,2-dimethyl-cyclohexyl]-2-[3-[[(2,3-dichlor-phenyl)sulfonyl]-methyl]-[1,2,4]oxadiazol-5-yl]-acetamid
CC-124 N-Cyclopropyl-N-(4-dimethylamino-cyclohexyl)-2-[5-[[(4-methoxy-2,6-dimethyl-phenyl)-sulfonyl]-methyl]-[1,3,4]oxadiazol-2-yl]-acetamid
CC-127 N-(2,2-Dimethyl-4-pyridin-4-yl-cyclohexyl)-2-[3-[[(4-methoxy-2,6-dimethyl-phenyl)-sulfonyl]-methyl]-5-methyl-1 H-pyrazol-1-yl]-acetamid
CC-1282-[3-[[(2-Chlor-6-methyl-phenyl)sulfonyl]-methyl]-[1,2,4]oxadiazol-5-yl]-N-(4-dimethyl-amino-cyclohexyl)-acetamid
CC-129[3-[[(4-Methoxy-2,6-dimethyl-phenyl)sulfonyl]-methyl]-[1,2,4]oxadiazol-5-yl]-[4-(4-methyl-piperazin-1-yl)-piperidin-1-yl]-methanon
CC-1302-[3-[[(4-Methoxy-2,6-dimethyl-phenyl)sulfonyl]-methyl]-[1,2,4]oxadiazol-5-yl]-1-(2-methyl-2,8-diazaspiro[4.4]nonan-8-yl)-ethanon
CC-1312-[3-[[(4-Methoxy-2,6-dimethyl-phenyl)sulfonyl]-methyl]-[1,2,4]oxadiazol-5-yl]-1-[4-(4-methyl-piperazin-1-yl)-piperidin-1-yl]-ethanon
CC-1332-[3-[[(4-Methoxy-2,6-dimethyl-phenyl)sulfonyl]-methyl]-[1,2,4]oxadiazol-5-yl]-1-(4-pyridin-4-yl-piperazin-1-yl)-ethanon
CC-1342-[3-[[(4-Methoxy-2,6-dimethyl-phenyl)sulfonyl]-methyl]-[1,2,4]oxadiazol-5-yl]-1-(3-pyridin-4-yl-3,8-diazaspiro[4.5]decan-8-yl)-ethanon
CC-1352-[3-[[(4-Methoxy-2,6-dimethyl-phenyl)sulfonyl]-methyl]-[1,2,4]oxadiazol-5-yl]-1-[(1S,SR)-3-pyridin-4-yloxy-8-azabicyclo[3.2.1]octan-8-yl]-ethanon
CC-136[3-[[(4-Methoxy-2,6-dimethyl-phenyl)sulfonyl]-methyl]-[1,2,4]oxadiazol-5-yl]-(4-pyridin-4-yl-piperazin-1-yl)-methanon
CC-137[3-[[(2-Chlor-6-methyl-phenyl)sulfonyl]-methyl]-[1,2,4]oxadiazol-5-yl]-(4-pyridin-4-yl-piperazin-1-yl)-methanon
CC-138[3-[[(2-Chlor-6-methyl-phenyl)sulfonyl]-methyl]-[1,2,4]oxadiazol-5-yl]-[4-(4-methyl-piperazin-1-yl)-piperidin-1-yl]-methanon
CC-139 [3-[[(4-Methoxy-2,6-dimethyl-phenyl)sulfonyl]-methyl]-[1,2,4]oxadiazol-5-yl]-(3-pyridin-4-yl-3,8-diazaspiro[4.5]decan-8-yl)-methanon
CC-141 [3-[[(4-Chlor-2,5-dimethyl-phenyl)sulfonyl]-methyl]-[1,2,4]oxadiazol-5-yl]-[4-(4-methyl-piperazin-1-yl)-piperidin-1-yl]-methanon
CC-142 [3-[[(4-Chlor-2,5-dimethyl-phenyl)sulfonyl]-methyl]-[1,2,4]oxadiazol-5-yl]-(2-methyl-2,8-diazaspiro[4.4]nonan-8-yl)-methanon
CC-143 [3-[[(2-Chlor-6-methyl-phenyl)sulfonyl]-methyl]-[1,2,4]oxadiazol-5-yl]-(2-methyl-2,8-diaza-spiro[4.4]nonan-8-yl)-methanon
CC-144[3-[[(4-Chlor-2,5-dimethyl-phenyl)sulfonyl]-methyl]-[1,2,4]oxadiazol-5-yl]-(4-pyridin-4-yl-piperazin-1-yl)-methanon
CC-145[3-[[(2-Chlor-6-methyl-phenyl)sulfonyl]-methyl]-[1,2,4]oxadiazol-5-yl]-(3-pyridin-4-yl-3,8-diazaspiro[4.5]decan-8-yl)-methanon
CC-146[3-[[(4-Chlor-2,5-dimethyl-phenyl)sulfonyl]-methyl]-[1,2,4]oxadiazol-5-yl]-(9-pyridin-4-yl-3,9-diazaspiro[5.5]undecan-3-yl)-methanon
CC-147 [3-[[(4-Methoxy-2,6-dimethyl-phenyl)sulfonyl]-methyl]-[1,2,4]oxadiazol-5-yl]-(2-methyl-2,8-diazaspiro[4.4]nonan-8-yl)-methanon
CC-148 [3-[[(2-Chlor-6-methyl-phenyl)sulfonyl]-methyl]-[1,2,4]oxadiazol-5-yl]-(9-pyridin-4-yl-3,9-diazaspiro[5.5]undecan-3-yl)-methanon
CC-149[3-[[(4-Methoxy-2,6-dimethyl-phenyl)sulfonyl]-methyl]-[1,2,4]oxadiazol-5-yl]-[(1S,5R)-3-pyridin-4-yloxy-8-azabicyclo[3.2.1]octan-8-yl]-methanon
CC-150[3-[[(2-Chlor-6-methyl-phenyl)sulfonyl]-methyl]-[1,2,4]oxadiazol-5-yl]-[(1S,5R)-3-pyridin-4-yloxy-8-azabicyclo[3.2.1]octan-8-yl]-methanon
CC-151 [3-[[(4-Chlor-2,5-dimethyl-phenyl)sulfonyl]-methyl]-[1,2,4]oxadiazol-5-yl]-[(1S,5R)-3-pyridin-4-yloxy-8-azabicyclo[3.2.1]octan-8-yl]-methanon
CC-152 [3-[[(4-Chlor-2,5-dimethyl-phenyl)sulfonyl]-methyl]-[1,2,4]oxadiazol-5-yl]-(3-pyridin-4-yl-3,8-diazaspiro[4.5]decan-8-yl)-methanon
CC-1531-[2,2-Dimethyl-4-(pyridin-4-yl-methyl)-piperidin-1-yl]-2-[3-[[(4-methoxy-2,6-dimethyl-phenyl)sulfonyl]-methyl]-[1,2,4]oxadiazol-5-yl]-ethanon
CC-154N-(4-Dimethylamino-cyclohexyl)-2-[3-[[(4-methoxy-2,6-dimethyl-phenyl)sulfonyl]-methyl]-5-methyl-1 H-pyrazol-1-yl]-acetamid
CC-155 N-(2,2-Dimethyl-4-pyrrolidin-1-yl-cyclohexyl)-2-[3-[[(4-methoxy-2,6-dimethyl-phenyl)-sulfonyl]-methyl]-5-methyl-1 H-pyrazol-1-yl]-acetamid
CC-1562-[3-[[(4-Methoxy-2,6-dimethyl-phenyl)sulfonyl]-methyl]-5-methyl-1H-pyrazol-1-yl]-N-(4-pyrrolidin-1-yl-cyclohexyl)-acetamid
CC-1572-[3-[[(4-Methoxy-2,6-dimethyl-phenyl)sulfonyl]-methyl]-5-methyl-1H-pyrazol-1-yl]-N-[1-(2-methoxy-ethyl)-3,3-dimethyl-piperidin-4-yl]-acetamid
CC-158 N-(2,2-Dimethyl-4-pyrrolidin-1-yl-cyclohexyl)-2-[4-[[(4-methoxy-2,6-dimethyl-phenyl)-sulfonyl]-methyl]-1H-[1,2,3]triazol-1-yl]-acetamid
CC-159N-(1-Acetyl-3,3-dimethyl-piperidin-4-yl)-2-[3-[[(4-methoxy-2,6-dimethyl-phenyl)sulfonyl]-methyl]-5-methyl-1 H-pyrazol-1-yl]-acetamid
CC-1602-[4-[[(4-Methoxy-2,6-dimethyl-phenyl)sulfonyl]-methyl]-1H-[1,2,3]triazol-1-yl]-N-[1-(2-methoxy-ethyl)-3,3-dimethyl-piperidin-4-yl]-acetamid
CC-161 N-(1-Acetyl-3,3-dimethyl-piperidin-4-yl)-2-[4-[[(4-methoxy-2,6-dimethyl-phenyl)sulfonyl]-methyl]-1 H-[1,2,3]triazol-1-yl]-acetamid
CC-162 N-[2,2-Dimethyl-4-(4-methyl-piperazin-1-yl)-cyclohexyl]-2-[4-[[(4-methoxy-2,6-dimethyl-phenyl)sulfonyl]-methyl]-1 H-[1,2,3]triazol-1-yl]-acetamid
CC-163 N-[2,2-Dimethyl-4-(4-methyl-piperazin-1-yl)-cyclohexyl]-2-[3-[[(4-methoxy-2,6-dimethylphenyl)sulfonyl]-methyl]-5-methyl-1 H-pyrazol-1-yl]-acetamid
CC-1642-[4-[[(4-Methoxy-2,6-dimethyl-phenyl)sulfonyl]-methyl]-3,5-dimethyl-1H-pyrazol-1-yl]-N-(4-pyrrolidin-1-yl-cyclohexyl)-acetamid
CC-1652-[4-[[(4-Methoxy-2,6-dimethyl-phenyl)sulfonyl]-methyl]-3,5-dimethyl-1H-pyrazol-1-yl]-N-[1-(2-methoxy-ethyl)-3,3-dimethyl-piperidin-4-yl]-acetamid
CC-166 N-[2,2-Dimethyl-4-(4-methyl-piperazin-1-yl)-cyclohexyl]-2-[4-[[(4-methoxy-2,6-dimethyl-phenyl)sulfonyl]-methyl]-3,5-dimethyl-1 H-pyrazol-1-yl]-acetamid
CC-1672-[4-[[(4-Methoxy-2,6-dimethyl-phenyl)sulfonyl]-methyl]-1H-[1,2,3]triazol-1-yl]-N-(4-pyrrolidin-1-yl-cyclohexyl)-acetamid
CC-168 N-(4-Dimethylamino-cyclohexyl)-2-[4-[[(4-methoxy-2,6-dimethyl-phenyl)sulfonyl]-methyl]-3,5-dimethyl-1 H-pyrazol-1-yl]-acetamid
CC-169 N-(4-Dimethylamino-cyclohexyl)-2-[4-[[(4-methoxy-2, 6-dimethyl-phenyl)sulfonyl]-methyl]-1 H-[1,2,3]triazol-1-yl]-acetamid
CC-170 N-(2,2-Dimethyl-4-pyrrolidin-1-yl-cyclohexyl)-2-[4-[[(4-methoxy-2,6-dimethyl-phenyl)-sulfonyl]-methyl]-3,5-dimethyl-1 H-pyrazol-1-yl]-acetamid
CC-171 N-(3,3-Dimethyl-piperidin-4-yl)-2-[3-[[(4-methoxy-2,6-dimethyl-phenyl)sulfonyl]-methyl]-5-methyl-1 H-pyrazol-1-yl]-acetamid
CC-172 N-(2,2-Dimethyl-4-pyridin-4-yl-cyclohexyl)-2-[4-[[(4-methoxy-2,6-dimethyl-phenyl)-sulfonyl]-methyl]-3,5-dimethyl-1 H-pyrazol-1-yl]-acetamid
CC-1732-[4-[[(4-Methoxy-2,6-dimethyl-phenyl)sulfonyl]-methyl]-3,5-dimethyl-1H-pyrazol-1-yl]-N-piperidin-4-yl-acetamid
CC-174 N-[4-(Cyclopropylamino)-2,2-dimethyl-cyclohexyl]-2-[4-[[(4-methoxy-2,6-dimethyl-phenyl)-sulfonyl]-methyl]-3,5-dimethyl-1 H-pyrazol-1-yl]-acetamid
CC-175 N-[4-(Cyclopropylamino)-2,2-dimethyl-cyclohexyl]-2-[4-[[(4-methoxy-2,6-dimethyl-phenyl)-sulfonyl]-methyl]-1 H-[1,2,3]triazol-1-yl]-acetamid
CC-176 N-(3,3-Dimethyl-piperidin-4-yl)-2-[4-[[(4-methoxy-2,6-dimethyl-phenyl)sulfonyl]-methyl]-1 H-[1,2,3]triazol-1-yl]-acetamid
CC-177 N-[4-(Cyclopropylamino)-2,2-dimethyl-cyclohexyl]-2-[3-[[(4-methoxy-2,6-dimethyl-phenyl)-sulfonyl]-methyl]-5-methyl-1H-pyrazol-1-yl]-acetamid
CC-178 N-(2,2-Dimethyl-4-pyridin-4-yl-cyclohexyl)-2-[4-[[(4-methoxy-2,6-dimethyl-phenyl)-sulfonyl]-methyl]-1 H-[1,2,3]triazol-1-yl]-acetamid
CC-179 N-(1-Acetyl-3,3-dimethyl-piperidin-4-yl)-2-[4-[[(4-methoxy-2,6-dimethyl-phenyl)sulfonyl]-methyl]-3,5-dimethyl-1 H-pyrazol-1-yl]-acetamid
CC-180N-(3,3-Dimethyl-piperidin-4-yl)-2-[4-[[(4-methoxy-2,6-dimethyl-phenyl)sulfonyl]-methyl]-3,5-dimethyl-1H-pyrazol-1-yl]-acetamid
CC-181 N-(3,3-Dimethyl-piperidin-4-yl)-2-[4-[[(4-methoxy-2,6-dimethyl-phenyl)sulfonyl]-methyl]-1 H-[1,2,3]triazol-1-yl]-N-methyl-acetamid
CC-1822-[4-[[(4-Methoxy-2,6-dimethyl-phenyl)sulfonyl]-methyl]-1H-[1,2,3]triazol-1-yl]-N-piperidin-4-yl-acetamid
CC-1832-[4-[[(4-Methoxy-2,6-dimethyl-phenyl)sulfonyl]-methyl]-1H-[1,2,3]triazol-1-yl]-N-(1,3,3-trimethyl-piperidin-4-yl)-acetamid
CC-1842-[4-[[(4-Methoxy-2,6-dimethyl-phenyl)sulfonyl]-methyl]-3,5-dimethyl-1H-pyrazol-1-yl]-N-(1,3,3-trimethyl-piperidin-4-yl)-acetamid
CC-185 N-(3,3-Dimethyl-piperidin-4-yl)-2-[4-[[(4-methoxy-2,6-dimethyl-phenyl)sulfonyl]-methyl]-3,5-dimethyl-1 H-pyrazol-1-yl]-N-methyl-acetamid
CC-186 N-Cyclopropyl-N-(4-dimethylamino-cyclohexyl)-2-[4-[[(4-methoxy-2,6-dimethyl-phenyl)-sulfonyl]-methyl]-1 H-[1,2,3]triazol-1-yl]-acetamid
CC-1882-[3-[[(4-Methoxy-2,6-dimethyl-phenyl)sulfonyl]-methyl]-5-methyl-1H-pyrazol-1-yl]-N-(1,3,3-trimethyl-piperidin-4-yl)-acetamid
SC-01 3-[[(4-Methoxy-2,6-dimethyl-phenyl)sulfonyl]-methyl]-N-(1-pyridin-4-yl-piperidin-4-yl)-[1,2,4]oxadiazol-5-carbonsäure amid
SC-02 [3-[[(4-Methoxy-2,6-dimethyl-phenyl)sulfonyl]-methyl]-[1,2,4]oxadiazol-5-yl]-(7-pyridin-4-yl-2,7-diazaspiro[3.5]nonan-2-yl)-methanon
SC-03 [3-[[(4-Methoxy-2,6-dimethyl-phenyl)sulfonyl]-methyl]-[1,2,4]oxadiazol-5-yl]-(3-pyridin-4-yloxy-pyrrolidin-1-yl)-methanon
SC-04 [3-[[(4-Methoxy-2,6-dimethyl-phenyl)sulfonyl]-methyl]-[1,2,4]oxadiazol-5-yl]-(8-pyridin-4-yl-3,8-diazaspiro[4.5]decan-3-yl)-methanon
SC-05 [3-[[(4-Methoxy-2,6-dimethyl-phenyl)sulfonyl]-methyl]-[1,2,4]oxadiazol-5-yl]-(8-pyridin-4-yl-3,8-diazaspiro[4.4]nonan-3-yl)-methanon
SC-06 3-[[(4-Methoxy-2,6-dimethyl-phenyl)sulfonyl]-methyl]-N-[1-[5-(trifluormethyl)-pyridin-2-yl]-pyrrolidin-3-yl]-[1,2,4]oxadiazol-5-carbonsäure amid
SC-07 [3-[[(4-Methoxy-2,6-dimethyl-phenyl)sulfonyl]-methyl]-[1,2,4]oxadiazol-5-yl]-[3-[(4-methyl-piperazin-1-yl)-methyl]-pyrrolidin-1-yl]-methanon
SC-08 [3-[[(4-Methoxy-2,6-dimethyl-phenyl)sulfonyl]-methyl]-[1,2,4]oxadiazol-5-yl]-[4-(pyridin-4-yl-methyl)-piperidin-1-yl]-methanon
SC-09 [3-[[(4-Methoxy-2,6-dimethyl-phenyl)sulfonyl]-methyl]-[1,2,4]oxadiazol-5-yl]-(9-pyridin-4-yloxy-3-azaspiro[5.5]undecan-3-yl)-methanon
SC-10 [3-[[(4-Methoxy-2,6-dimethyl-phenyl)sulfonyl]-methyl]-[1,2,4]oxadiazol-5-yl]-(9-pyridin-4-yl-3,9-diazaspiro[5.5]undecan-3-yl)-methanon
SC-11 2-[3-[[(4-Methoxy-2,6-dimethyl-phenyl)sulfonyl]-methyl]-[1,2,4]oxadiazol-5-yl]-1-(9-pyridin-4-yl-3,9-diazaspiro[5.5]undecan-3-yl)-ethanon
SC-12 N-(3,3-Dimethyl-1-pyridin-4-yl-piperidin-4-yl)-3-[[(4-methoxy-2,6-dimethyl-phenyl)sulfonyl]-methyl]-[1,2,4]oxadiazol-5-carbonsäure amid
SC-13 N-[2,2-Dimethyl-4-(4-methyl-piperazin-1-yl)-cyclohexyl]-2-[3-[[(4-methoxy-2,6-dimethyl-phenyl)sulfonyl]-methyl]-[1,2,4]oxadiazol-5-yl]-acetamid
SC-14 N-(3,3-Dimethyl-1-pyridin-4-yl-piperidin-4-yl)-2-[3-[[(4-methoxy-2,6-dimethyl-phenyl)-sulfonyl]-methyl]-[1,2,4]oxadiazol-5-yl]-acetamid
SC-16 N-[2,2-Dimethyl-4-(4-methyl-piperazin-1-yl)-cyclohexyl]-3-[[(4-methoxy-2,6-dimethyl-phenyl)sulfonyl]-methyl]-[1,2,4]oxadiazol-5-carbonsäure amid
SC-18 3-[[(4-Methoxy-2,6-dimethyl-phenyl)sulfonyl]-methyl]-N-(4-pyridin-4-yl-cyclohexyl)-[1,2,4]-oxadiazol-5-carbonsäure amid
SC-19 3-[[(4-Methoxy-2,6-dimethyl-phenyl)sulfonyl]-methyl]-N-[4-(pyridin-4-yl-methyl)-cyclo-hexyl]-[1,2,4]oxadiazol-5-carbonsäure amid
SC-20 2-[3-[[(4-Methoxy-2,6-dimethyl-phenyl)sulfonyl]-methyl]-[1,2,4]oxadiazol-5-yl]-N-methyl-N-[1-(4-methyl-cyclohexyl)-piperidin-4-yl]-acetamid
SC-21 2-[3-[[(4-Methoxy-2,6-dimethyl-phenyl)sulfonyl]-methyl]-[1,2,4]oxadiazol-5-yl]-N-methyl-N-(1-tetrahydro-pyran-4-yl-piperidin-4-yl)-acetamid
SC-22 N-Cyclopropyl-2-[3-[[(4-methoxy-2,6-dimethyl-phenyl)sulfonyl]-methyl]-[1,2,4]oxadiazol-5-yl]-N-(1-pyridin-4-yl-piperidin-4-yl)-acetamid
SC-27 2-[3-[[(4-Methoxy-2,6-dimethyl-phenyl)sulfonyl]-methyl]-[1,2,4]oxadiazol-5-yl]-N-methyl-N-[1-(pyridin-4-carbonyl)-piperidin-4-yl]-acetamid
SC-30 3-[[(4-Methoxy-2,6-dimethyl-phenyl)sulfonyl]-methyl]-N-[(4-pyridin-4-yl-cyclohexyl)-methyl]-[1,2,4]oxadiazol-5-carbonsäure amid
SC-31 N-(2,2-Dimethyl-4-pyridin-4-yl-cyclohexyl)-3-[[(4-methoxy-2,6-dimethyl-phenyl)sulfonyl]-methyl]-[1,2,4]oxadiazol-5-carbonsäure amid
SC-32 N-(2,2-Dimethyl-4-pyridin-4-yl-cyclohexyl)-2-[3-[[(4-methoxy-2,6-dimethyl-phenyl)-sulfonyl]-methyl]-[1,2,4]oxadiazol-5-yl]-acetamid
SC-33 2-[3-[[(4-Methoxy-2,6-dimethyl-phenyl)sulfonyl]-methyl]-[1,2,4]oxadiazol-5-yl]-N-methyl-N-(4,4,9-trimethyl-9-azaspiro[5.5]undecan-3-yl)-acetamid
SC-35 N-(4-Hydroxy-2,2-dimethyl-cyclohexyl)-2-[3-[[(4-methoxy-2,6-dimethyl-phenyl)sulfonyl]-methyl]-[1,2,4]oxadiazol-5-yl]-acetamid
SC-36 N-(3,3-Dimethyl-tetrahydro-pyran-4-yl)-2-[3-[[(4-methoxy-2,6-dimethyl-phenyl)sulfonyl]-methyl]-[1,2,4]oxadiazol-5-yl]-acetamid
SC-37 2-[3-[[(4-Methoxy-2,6-dimethyl-phenyl)sulfonyl]-methyl]-[1,2,4]oxadiazol-5-yl]-N-(4-pyridin-4-yl-cyclohexyl)-acetamid
SC-39 N-(3,3-Dimethyl-1-tetrahydro-pyran-4-yl-piperidin-4-yl)-2-[3-[[(4-methoxy-2,6-dimethyl-phenyl)sulfonyl]-methyl]-[1,2,4]oxadiazol-5-yl]-acetamid
SC-40 2-[3-[[(4-Methoxy-2,6-dimethyl-phenyl)sulfonyl]-methyl]-[1,2,4]oxadiazol-5-yl]-N-[4-(2-methoxy-pyridin-4-yl)-2,2-dimethyl-cyclohexyl]-acetamid
SC-41 2-[3-[[(4-Methoxy-2,6-dimethyl-phenyl)sulfonyl]-methyl]-[1,2,4]oxadiazol-5-yl]-1-(4-pyridin-4-yl-piperidin-1-yl)-ethanon
SC-42 N-[1-(2,6-Dimethyl-pyrimidin-4-yl)-piperidin-4-yl]-2-[3-[[(4-methoxy-2,6-dimethyl-phenyl)-sulfonyl]-methyl]-[1,2,4]oxadiazol-5-yl]-N-methyl-acetamid
SC-43 3-[[(4-Methoxy-2,6-dimethyl-phenyl)sulfonyl]-methyl]-N-methyl-N-[(1S,3R)-3-(4-methyl-piperazin-1-yl)-cyclohexyl]-[1,2,4]oxadiazol-5-carbonsäure amid
SC-44 2-[3-[[(4-Methoxy-2,6-dimethyl-phenyl)sulfonyl]-methyl]-[1,2,4]oxadiazol-5-yl]-N-methyl-N-[(1 S,3R)-3-(4-methyl-piperazin-1-yl)-cyclohexyl]-acetamid
SC-46 2-[3-[[(4-Methoxy-2,6-dimethyl-phenyl)sulfonyl]-methyl]-[1,2,4]oxadiazol-5-yl]-N-methyl-N-[1-(3-piperidin-1-yl-propanoyl)-piperidin-4-yl]-acetamid
SC-48 N-[1-(4-Fluor-cyclohexyl)-3,3-dimethyl-piperidin-4-yl]-2-[3-[[(4-methoxy-2,6-dimethyl-phenyl)sulfonyl]-methyl]-[1,2,4]oxadiazol-5-yl]-acetamid
SC-49 3-[[(2-Chlor-6-methyl-phenyl)sulfonyl]-methyl]-N-methyl-N-[(1S,3R)-3-(4-methyl-piperazin-1-yl)-cyclohexyl]-[1,2,4]oxadiazol-5-carbonsäure amid
SC-50 2-[3-[[(2-Chlor-6-methyl-phenyl)sulfonyl]-methyl]-[1,2,4]oxadiazol-5-yl]-N-methyl-N-[(1S,3R)-3-(4-methyl-piperazin-1-yl)-cyclohexyl]-acetamid
SC-51 2-[3-[[(4-Methoxy-2,6-dimethyl-phenyl)sulfonyl]-methyl]-[1,2,4]oxadiazol-5-yl]-1-[4-[(6-methoxy-pyridin-3-yl)-methyl]-piperazin-1-yl]-ethanon
SC-52 2-[3-[[(4-Methoxy-2,6-dimethyl-phenyl)sulfonyl]-methyl]-[1,2,4]oxadiazol-5-yl]-1-[4-[(2-pyrrolidin-1-yl-pyridin-4-yl)-methyl]-piperazin-1-yl]-ethanon
SC-55 2-[3-[[(4-Methoxy-2,6-dimethyl-phenyl)sulfonyl]-methyl]-[1,2,4]oxadiazol-5-yl]-N-[1-[(6-methoxy-pyridin-3-yl)-methyl]-piperidin-4-yl]-N-methyl-acetamid
SC-57 2-[3-[[(4-Methoxy-2,6-dimethyl-phenyl)sulfonyl]-methyl]-[1,2,4]oxadiazol-5-yl]-N-methyl-N-[1-[(2-pyrrolidin-1-yl-pyridin-4-yl)-methyl]-piperidin-4-yl]-acetamid
SC-58 2-[3-[[(4-Methoxy-2,6-dimethyl-phenyl)sulfonyl]-methyl]-[1,2,4]oxadiazol-5-yl]-N-methyl-N-[4-(4-methyl-piperazin-1-yl)-cyclohexyl]-acetamid
SC-59 [3-[[(4-Methoxy-2,6-dimethyl-phenyl)sulfonyl]-methyl]-[1,2,4]oxadiazol-5-yl]-[4-[(6-methoxy-pyridin-3-yl)-methyl]-piperazin-1-yl]-methanon
SC-60 [3-[[(4-Methoxy-2,6-dimethyl-phenyl)sulfonyl]-methyl]-[1,2,4]oxadiazol-5-yl]-[4-[(2-pyrrolidin-1-yl-pyridin-4-yl)-methyl]-piperazin-1-yl]-methanon
SC-61 [3-[[(4-Methoxy-2,6-dimethyl-phenyl)sulfonyl]-methyl]-[1,2,4]oxadiazol-5-yl]-[4-[(6-pyrrolidin-1-yl-pyridin-3-yl)-methyl]-piperazin-1-yl]-methanon
SC-62 2-[3-[[(4-Methoxy-2,6-dimethyl-phenyl)sulfonyl]-methyl]-[1,2,4]oxadiazol-5-yl]-1-[4-[(6-pyrrolidin-1-yl-pyridin-3-yl)-methyl]-piperazin-1-yl]-ethanon
SC-63 2-[3-[[(4-Methoxy-2,6-dimethyl-phenyl)sulfonyl]-methyl]-[1,2,4]oxadiazol-5-yl]-N-methyl-N-(1-chinazolin-4-yl-piperidin-4-yl)-acetamid
SC-64 2-[3-[[(4-Methoxy-2,6-dimethyl-phenyl)sulfonyl]-methyl]-[1,2,4]oxadiazol-5-yl]-N-[4-(4-methoxy-piperidin-1-yl)-cyclohexyl]-N-methyl-acetamid
SC-65 2-[3-[[(4-Methoxy-2,6-dimethyl-phenyl)sulfonyl]-methyl]-[1,2,4]oxadiazol-5-yl]-N-methyl-N-[1-[(6-pyrrolidin-1-yl-pyridin-3-yl)-methyl]-piperidin-4-yl]-acetamid
SC-66 N-[1-(2-Dimethylamino-pyrimidin-4-yl)-piperidin-4-yl]-2-[3-[[(4-methoxy-2,6-dimethyl-phenyl)sulfonyl]-methyl]-[1,2,4]oxadiazol-5-yl]-N-methyl-acetamid
SC-67 N-[1-(2-Dimethylamino-pyridin-4-yl)-piperidin-4-yl]-2-[3-[[(4-methoxy-2,6-dimethyl-phenyl)-sulfonyl]-methyl]-[1,2,4]oxadiazol-5-yl]-N-methyl-acetamid
SC-68 N-[1-(2-Dimethylamino-pyrimidin-4-yl)-piperidin-4-yl]-3-[[(4-methoxy-2,6-dimethyl-phenyl)-sulfonyl]-methyl]-N-methyl-[1,2,4]oxadiazol-5-carbonsäure amid
SC-69 N-[1-(2-Dimethylamino-pyridin-4-yl)-piperidin-4-yl]-3-[[(4-methoxy-2,6-dimethyl-phenyl)-sulfonyl]-methyl]-N-methyl-[1,2,4]oxadiazol-5-carbonsäure amid
SC-70 N-[1-(3-Fluor-pyridin-4-yl)-piperidin-4-yl]-2-[3-[[(4-methoxy-2,6-dimethyl-phenyl)sulfonyl]-methyl]-[1,2,4]oxadiazol-5-yl]-N-methyl-acetamid
SC-72 2-[3-[[(4-Methoxy-2,6-dimethyl-phenyl)sulfonyl]-methyl]-[1,2,4]oxadiazol-5-yl]-N-methyl-N-[1-[(3-methyl-isoxazol-5-yl)-methyl]-piperidin-4-yl]-acetamid
SC-73 2-[3-[[(4-Methoxy-2,6-dimethyl-phenyl)sulfonyl]-methyl]-[1,2,4]oxadiazol-5-yl]-N-methyl-N-[1-[2-(4-methyl-piperazin-1-yl)-2-oxo-ethyl]-piperidin-4-yl]-acetamid
SC-77 2-[3-[[(4-Methoxy-2,6-dimethyl-phenyl)sulfonyl]-methyl]-[1,2,4]oxadiazol-5-yl]-N-[1-[(4-methoxy-3,5-dimethyl-pyridin-2-yl)-methyl]-piperidin-4-yl]-N-methyl-acetamid
SC-79 N-[1-(2-Isopropyl-6-methyl-pyrimidin-4-yl)-piperidin-4-yl]-2-[3-[[(4-methoxy-2,6-dimethyl-phenyl)sulfonyl]-methyl]-[1,2,4]oxadiazol-5-yl]-N-methyl-acetamid
SC-80 2-[3-[[(4-Methoxy-2,6-dimethyl-phenyl)sulfonyl]-methyl]-[1,2,4]oxadiazol-5-yl]-N-methyl-N-[1-(2-oxo-2-pyrrolidin-1-yl-ethyl)-piperidin-4-yl]-acetamid
SC-81 2-[3-[[(4-Methoxy-2,6-dimethyl-phenyl)sulfonyl)-methyl]-[1,2,4]oxadiazol-5-yl]-N-methyl-N-[1-(6-methyl-pyrimidin-4-yl)-piperidin-4-yl]-acetamid
SC-82 N-[1-(3-Fluor-pyridin-4-yl)-piperidin-4-yl]-3-[[(4-methoxy-2,6-dimethyl-phenyl)sulfonyl]-methyl]-N-methyl-[1,2,4]oxadiazol-5-carbonsäure amid
SC-83 2-[4-[[(4-Methoxy-2,6-dimethyl-phenyl)sulfonyl]-methyl]-3,5-dimethyl-1H-pyrazol-1-yl]-N-methyl-N-[(1S,3R)-3-(4-methyl-piperazin-1-yl)-cyclohexyl]-acetamid
SC-84 2-[4-[[(4-Methoxy-2,6-dimethyl-phenyl)sulfonyl]-methyl]-1H-[1,2,3]triazol-1-yl]-N-methyl-N-[(1S,3R)-3-(4-methyl-piperazin-1-yl)-cyclohexyl]-acetamid
SC-85 2-[3-[[(4-Methoxy-2,6-dimethyl-phenyl)sulfonyl]-methyl]-[1,2,4]oxadiazol-5-yl]-N-methyl-N-(2-oxo-1-pyridin-4-yl-piperidin-4-yl)-acetamid
SC-86 [5-[[(4-Methoxy-2,6-dimethyl-phenyl)sulfonyl]-methyl]-[1,3,4]oxadiazol-2-yl]-[3-[(4-methyl-piperazin-1-yl)-methyl]-pyrrolidin-1-yl]-methanon
SC-87 N-(3,3-Dimethyl-piperidin-4-yl)-5-[[(4-methoxy-2,6-dimethyl-phenyl)sulfonyl]-methyl]-[1,3,4]oxadiazol-2-carbonsäure amid
SC-88 [5-[[(4-Methoxy-2,6-dimethyl-phenyl)sulfonyl]-methyl]-[1,2,4]oxadiazol-3-yl]-[3-[(4-methyl-piperazin-1-yl)-methyl]-pyrrolidin-1-yl]-methanon
SC-89 5-[[(4-Methoxy-2,6-dimethyl-phenyl)sulfonyl]-methyl]-N-methyl-N-[(1S,3R)-3-(4-methyl-piperazin-1-yl)-cyclohexyl]-[1,2,4]oxadiazol-3-carbonsäure amid
SC-90 N-(3,3-Dimethyl-piperidin-4-yl)-5-[[(4-methoxy-2,6-dimethyl-phenyl)sulfonyl]-methyl]-[1,2,4]oxadiazol-3-carbonsäure amid
SC-91 5-[[(4-Methoxy-2,6-dimethyl-phenyl)sulfonyl]-methyl]-N-methyl-N-[(1S,3R)-3-(4-methyl-piperazin-1-yl)-cyclohexyl]-[1,3,4]oxadiazol-2-carbonsäure amid
SC-92 2-[4-[[(4-Methoxy-2,6-dimethyl-phenyl)sulfonyl]-methyl]-3,5-dimethyl-1H-pyrazol-1-yl]-1-[3-[(4-methyl-piperazin-1-yl)-methyl]-pyrrolidin-1-yl]-ethanon
SC-93 2-[4-[[(4-Methoxy-2,6-dimethyl-phenyl)sulfonyl]-methyl]-1H-[1,2,3]triazol-1-yl]-1-[3-[(4-methyl-piperazin-1-yl)-methyl]-pyrrolidin-1-yl]-ethanon
in Form der freien Verbindung, eines Tautomers oder eines N-Oxids;
in Form eines Racemates, eines individuellen Stereoisomers oder einer Mischung aus Stereoisomeren
und/oder in Form eines Solvats und/oder eines physiologisch verträglichen Salzes.

14. Arzneimittel, enthaltend mindestens eine Verbindung gemäß einem oder mehreren der Ansprüche 1 bis 13.

15. Verbindung gemäß einem oder mehreren der Ansprüche 1 bis 13 zur Behandlung von Schmerz, insbesondere von akutem Schmerz, viszeralem Schmerz, neuropathischen Schmerz, chronischen Schmerz oder Entzündungsschmerz; Migräne; Diabetes; Atemwegserkrankungen; entzündlichen Darmerkrankungen; neurologischen Erkrankungen; neurodegenerativen Erkrankungen, fibrotischen Erkrankungen, Entzündungen der Haut; rheumatischen Erkrankungen; septischen Schock; Reperfusionssyndrom; Fettleibigkeit und/oder Angiogenese.
